# EUROPÄISCHE PATENTSCHRIFT

(11) **EP 1 606 254 B1**
(45) Veröffentlichungstag und Bekanntmachung des Hinweises auf die Patenterteilung: **11.05.2011**
(21) Anmeldenummer: 04716217.7
(22) Anmeldetag: 02.03.2004
(51) Int. Cl.: C07D 207/38, C07D 209/54, C07D 491/10, C07D 495/10, C07D 487/04, C07D 471/04, C07D 491/14, C07D 513/04, C07D 307/60, C07D 307/94, C07D 493/10, C07D 231/32, C07D 498/04, C07D 231/06, C07D 231/14

(54) **2, 4, 6-PHENYLSUBSTITUIERTE CYCLISCHE KETOENOLE**
2,4,6-PHENYL SUBSTITUTED CYCLIC KETOENOLS
CETOENOLS CYCLIQUES SUBSTITUES PAR 2,4,6-PHENYLE

(30) Priorität: 14.03.2003 DE 10311300
(43) Veröffentlichungstag der Anmeldung: 21.12.2005
(73) Patentinhaber: Bayer CropScience AG, 40789 Monheim (DE)
(72) Erfinder: FISCHER, Reiner, 40789 Monheim (DE); KUNZ, Klaus, 40625 Düsseldorf (DE); LEHR, Stefan, 65835 Liederbach (DE); RUTHER, Michael, 40764 Langenfeld (DE); SCHNEIDER, Udo, 51373 Leverkusen (DE); DOLLINGER, Markus, 51381 Leverkusen (DE); DREWES, Mark, Wilhelm, 40764 Langenfeld (DE); FEUCHT, Dieter, 65760 Eschborn (DE); KONZE, Jörg, 51147 Köln (DE); WACHENDORFF-NEUMANN, Ulrike, 56566 Neuwied (DE); BOJACK, Guido, 65207 Wiesbaden (DE); AULER, Thomas, 42799 Leichlingen (DE); HILLS, Martin, Jeffrey, 65510 Idstein (DE); BRETSCHNEIDER, Thomas, 53797 Lohmar (DE); MALSAM, Olga, 53227 Bonn (DE); ERDELEN, Christoph DI, (DE); ANGERMANN, Alfred, 65830 Kriftel (DE); KEHNE, Heinz, 65719 Hofheim (DE); ROSINGER, Christopher Hugh, 65719 Hofheim (DE)
(86) Internationale Anmeldenummer: PCT/EP2004/002053
(87) Internationale Veröffentlichungsnummer: WO 2004/080962

(56) Entgegenhaltungen:
- EP-A- 0 442 077
- EP-A- 0 508 126
- EP-A- 0 521 334
- EP-A- 0 588 137
- WO-A-01/23354
- WO-A-95/01971
- WO-A-96/35664
- WO-A-98/25928
- WO-A-99/16748
- WO-A-99/24437
- US-A- 4 985 063

## Beschreibung

Die vorliegende Erfindung betrifft neue 2,4,6-phenylsubstituierte cyclische Ketoenole, mehrere Verfahren zu ihrer Herstellung und ihre Verwendung als Schädlingsbekämpfungsmittel und/oder Herbizide. Auch Gegenstand der Erfindung sind selektiv herbizide Mittel, die 2,4,6-phenylsubstituierte cyclische Ketoenole einerseits und eine die Kulturpflanzenverträglichkeit verbessernde Verbindung andererseits enthalten.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/16748, WO 99/24437, WO 99/43649, WO 99/48869 und WO 99/55673, WO 01/17972, WO 01/23354, WO 01/74770).

Es ist bekannt, dass bestimmte substituierte Δ³-Dihydrofuran-2-on-Derivate herbizide Eigenschaften besitzen (vgl. DE-A-4014420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4014420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354 und WO 01/74770 bekannt. Auch 3-Aryl-Δ³-dihydrothiphen-on-Derivate sind bekannt (WO 95/26 345, 96/25 395, WO 97/01 535, WO 97/02 243, WO 97/36 868, WO 98/05638, WO 98/25928, WO 99/16748, WO 99/43649, WO 99/48869, WO 99/55673, WO 01/ 17972, WO 01/23354 und WO 01/74770).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941, WO 97/36 868, WO 98/05638, WO 99/43649, WO 99/48869, WO 99/55673, WO 01/17972 und WO 01/74770 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868, WO 99/05638, WO 99/43649, WO 99/48869, WO 99/55673, WO 01/17972 und WO 01/74770 beschrieben.

Es ist bekannt, dass bestimmte substituierte 2-Arylcyclopentandione herbizide, insektizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO 99/48869, WO 99/55673, WO 01/17972 und WO 01/74770). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-A 2 361 084 bekannt, mit Angabe von herbiziden und akariziden Wirkungen.

Es ist bekannt, dass bestimmte substituierte 2-Arylcyclohexandione herbizide, insektizide und akarizide Eigenschaften besitzen (US-4175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-A 2 813 341, sowie Wheeler, T.N., J. Org. Chem. 44, 4906 (1979)), WO 99/43649, WO 99/48869, WO 99/55673, WO 01/17972 und WO 01/74770).

Es ist bekannt, dass bestimmte substituierte 4-Aryl-pyrazolidin-3,5-dione akarizide, insektizide und herbizide Eigenschaften besitzen (vgl. z.B. WO 92/16 510, EP-A-508 126, WO 96/11 574, WO 96/21 652, WO 99/47525, WO 0/17 351, WO 01/17 352, WO 0/17 353, WO 01/17 972, WO 01/17 973, WO 03/028 466 und WO 03/062 244).

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 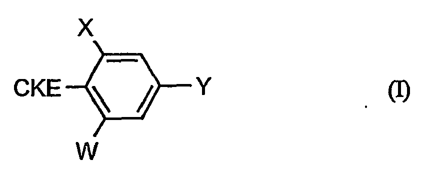 in welcher
- W: für Alkoxy, Halogenalkoxy, Alkoxyalkyloxy, Alkoxy-bis-alkyloxy oder gegebenenfalls substituiertes Cycloalkyl-alkandiyl-oxy, welches gegebenenfalls durch Heteroatome unter- brochen sein kann, steht,
- X: für Alkyl steht,
- Y: für Chlor, Brom oder Iod steht,
- CKE: für eine der Gruppen 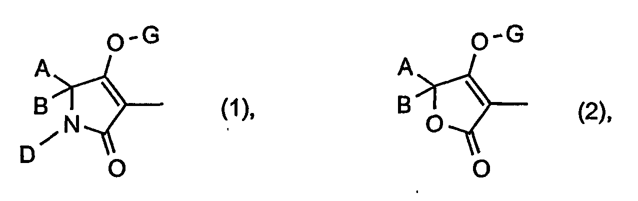 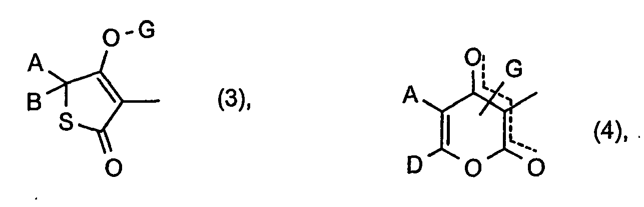 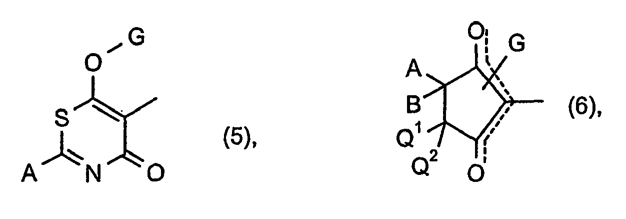 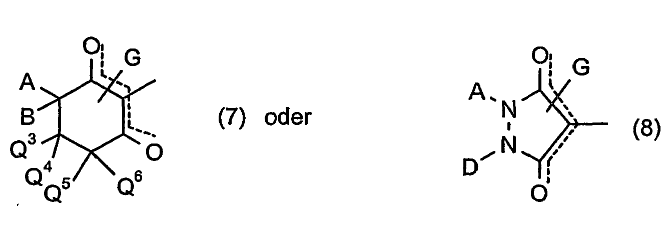 steht
worin
A für Wasserstoff, jeweils gegebenenfalls durch Halogen substituiertes Alkyl, Alkenyl, Alkoxyalkyl, Alkylthioalkyl, gesättigtes oder ungesättigtes, gegebenen- falls substituiertes Cycloalkyl, in welchem gegebenenfalls mindestens ein Ring- atom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind für einen ge- sättigten oder ungesättigten, gegebenenfalls mindestens ein Heteroatom ent- haltenden unsubstituierten oder substituierten Cyclus stehen,
D für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome er- setzt sind, Arylalkyl, Aryl, Hetarylalkyl oder Hetaryl steht oder
A und D gemeinsam mit den Atomen an die sie gebunden sind für einen gesättigten oder ungesättigten und gegebenenfalls mindestens ein (im Falle CKE=8 ein weiteres) Heteroatom enthaltenden, im A,D-Teil unsubstituierten oder substituierten Cyclus stehen, bzw.
A und Q¹ gemeinsam für gegebenenfalls durch Hydroxy, jeweils gegebenenfalls substituiertes Alkyl, Alkoxy, Alkylthio, Cycloalkyl, Benzyloxy oder Aryl substituiertes Alkandiyl oder Alkendiyl stehen oder
Q¹ für Wasserstoff oder Alkyl steht,
Q², Q⁴, Q⁵ und Q⁶ unabhängig voneinander für Wasserstoff oder Alkyl stehen,
Q³ für Wasserstoff, für gegebenenfalls substituiertes Alkyl, Alkoxyalkyl, Alkylthio- alkyl, gegebenenfalls substituiertes Cycloalkyl (worin gegebenenfalls eine Methylengruppe durch Sauerstoff oder Schwefel ersetzt ist) oder gegebenenfalls substituiertes Phenyl steht, oder
Q³ und Q⁴ gemeinsam mit dem Kohlenstoffatom, an das sie gebunden sind, für einen ge- sättigten oder ungesättigten, gegebenenfalls ein Heteroatom enthaltenden un- substituierten oder substituierten Cyclus stehen,
G für Wasserstoff (a) oder für eine der Gruppen 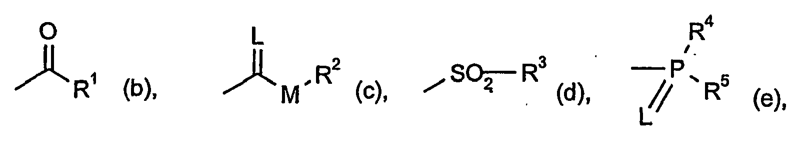 E (f) oder 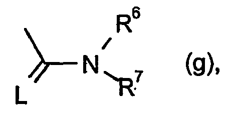 steht,
worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxy- alkyl steht,
R² für jeweils gegebenenfalls durch Halogen substituiertes Alkyl, Alkenyl, Alkoxyalkyl, Polyalkoxyalkyl oder für jeweils gegebenenfalls sub- stituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alke- nylthio, Cycloalkylthio oder für jeweils gegebenenfalls substituiertes Phe- nyl, Benzyl, Phenoxy oder Phenylthio stehen,
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbro- chenen Cyclus stehen.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (8) der Gruppe CKE ergeben sich folgende hauptsächliche Strukturen (I-1) bis (I-8): 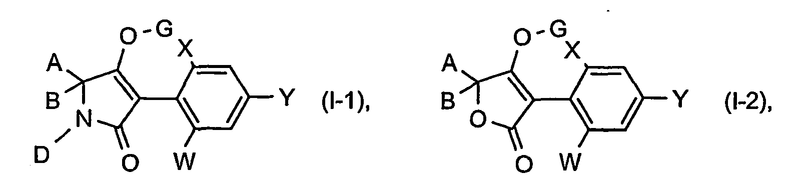 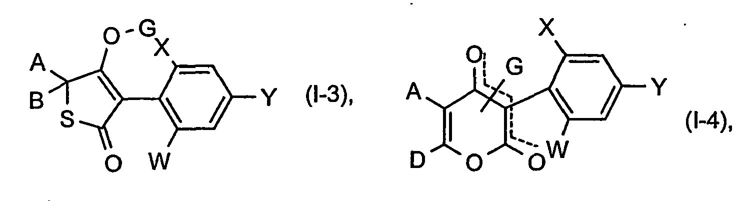 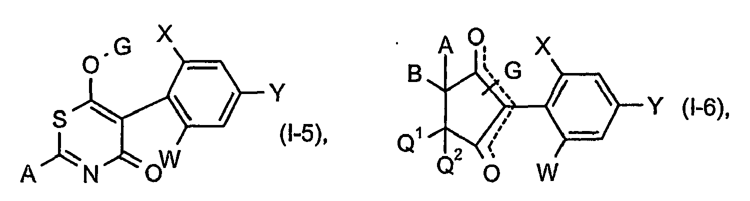 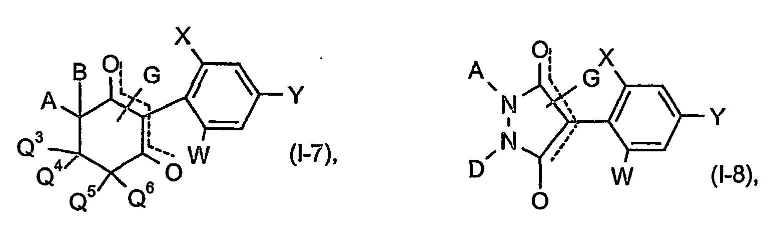 worin
A, B, D, G, Q¹, Q², Q³, Q⁴, Q⁵, Q⁶, W, X und Y die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn CKE für die Gruppe (1) steht, 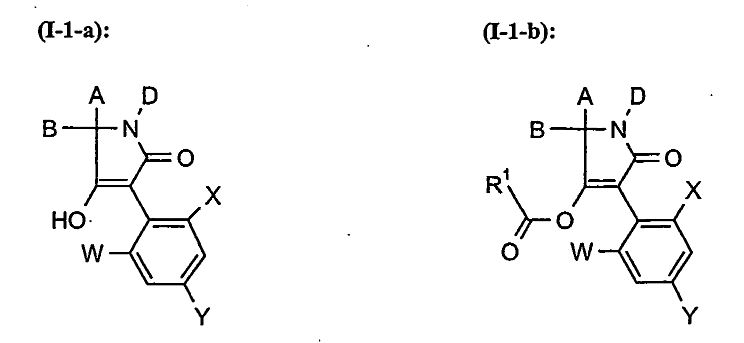 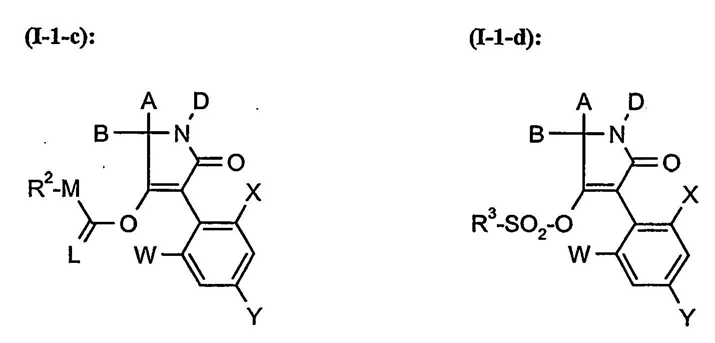 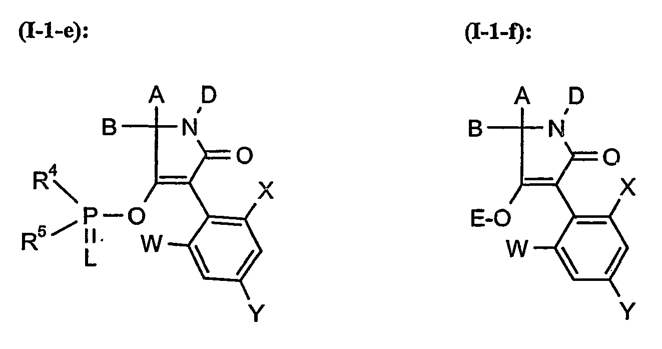 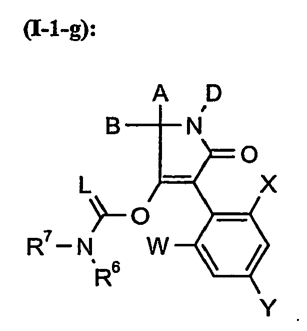 worin
A, B, D, E, L, M, W, X, Y, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn CKE für die Gruppe (2) steht, 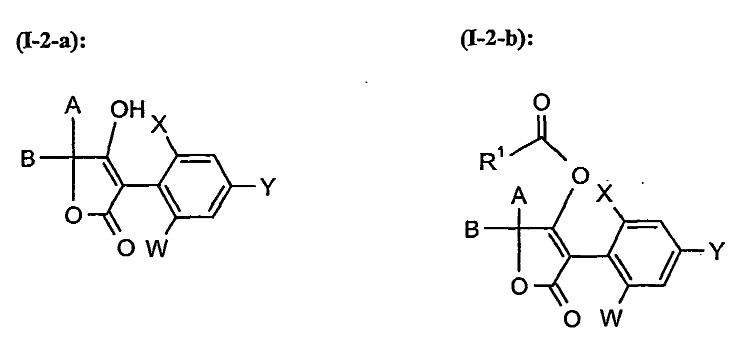 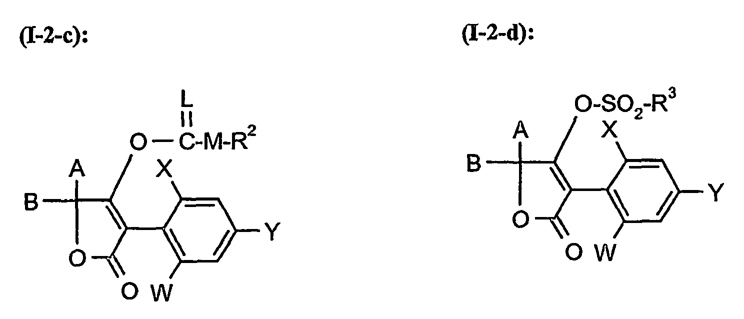 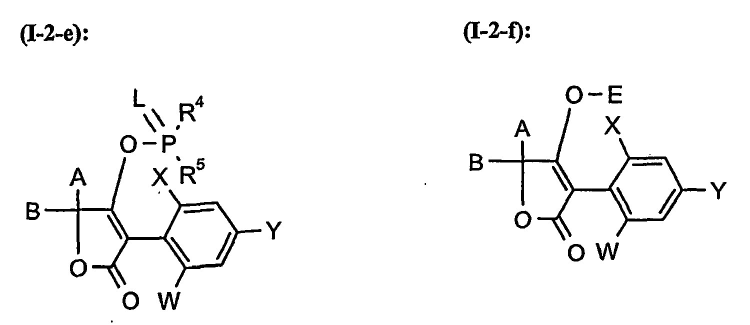 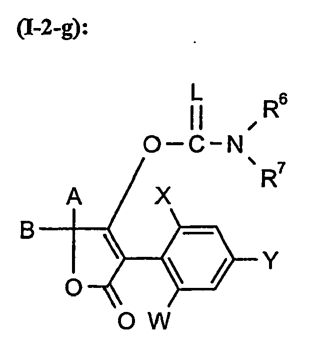 worin
A, B, E, L, M, W, X, Y, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn CKE für die Gruppe (3) steht, 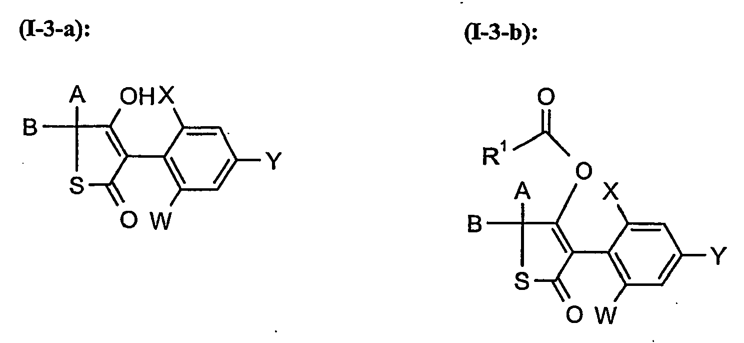 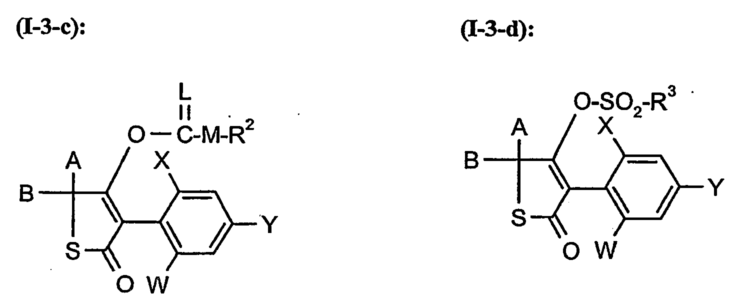 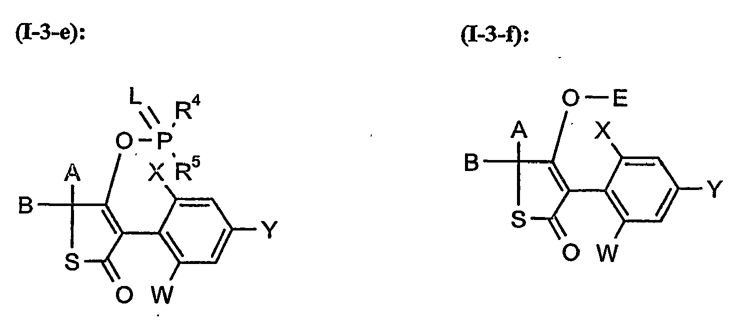 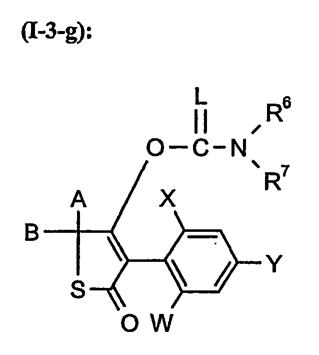 worin
A, B, E, L, M, W, X, Y, R¹, R², R³, R⁴, R⁵, R⁶ und R⁷ die oben angegebenen Bedeutung besitzen.

Die Verbindungen der Formel (I-4) können in Abhängigkeit von der Stellung des Substituenten G in den zwei isomeren Formen der Formeln (I-4-A) und (I-4-B) vorliegen, 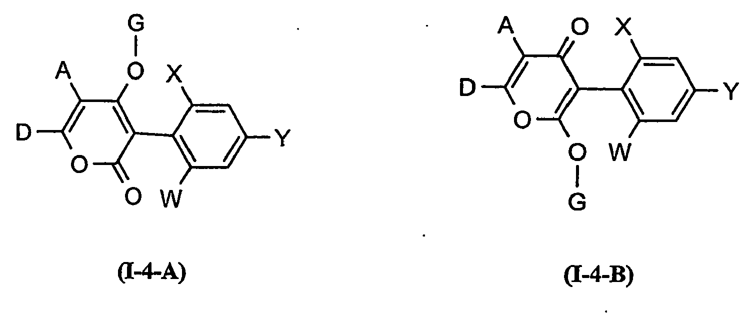 was durch die gestrichelte Linie in der Formel (I-4) zum Ausdruck gebracht werden soll.

Die Verbindungen der Formeln (I-4-A) und (I-4-B) können sowohl als Gemische als auch in Form ihrer reinen Isomeren vorliegen. Gemische der Verbindungen der Formeln (I-4-A) und (I-4-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-4-a) bis (I-4-g), wenn CKE für die Gruppe (4) steht, 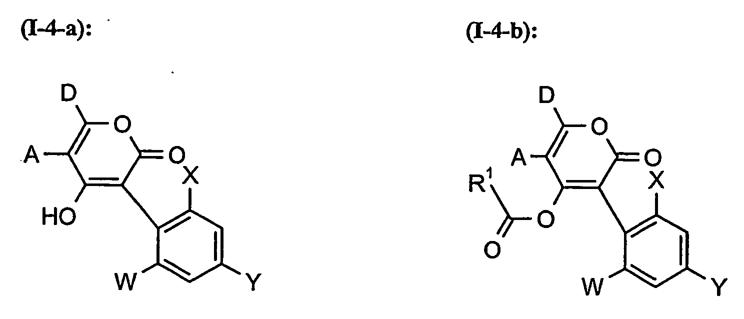 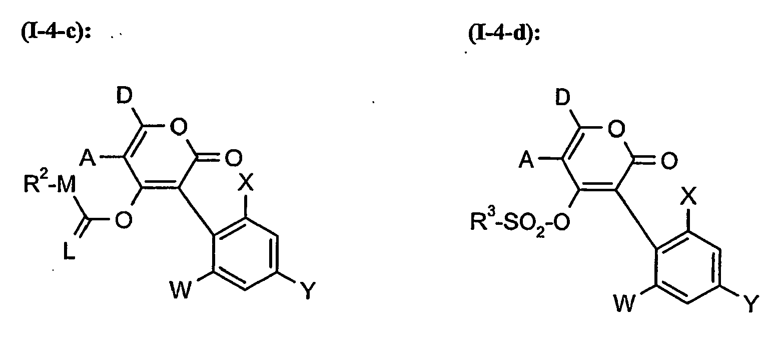 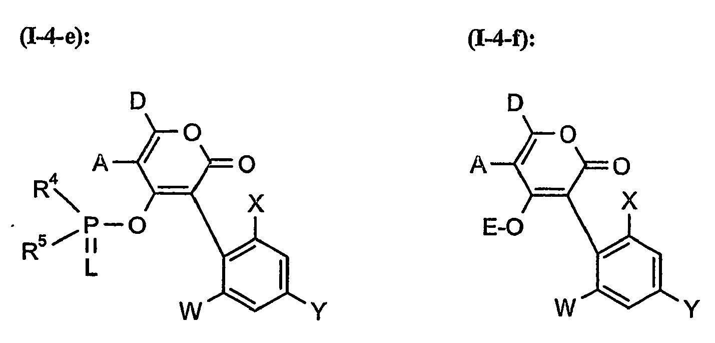 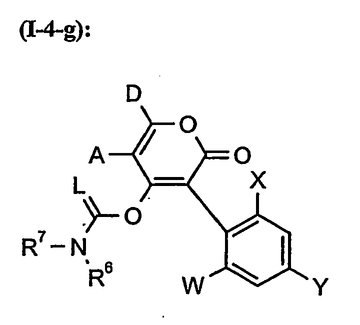 worin
A, D, E, L, M, W, X, Y, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der.Gruppe G ergeben sich folgende hauptsächliche Strukturen (I-5-a) bis (I-5-g), wenn CKE für die Gruppe (5) steht, 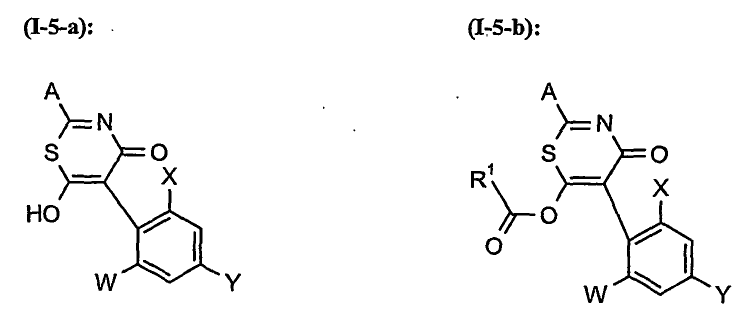 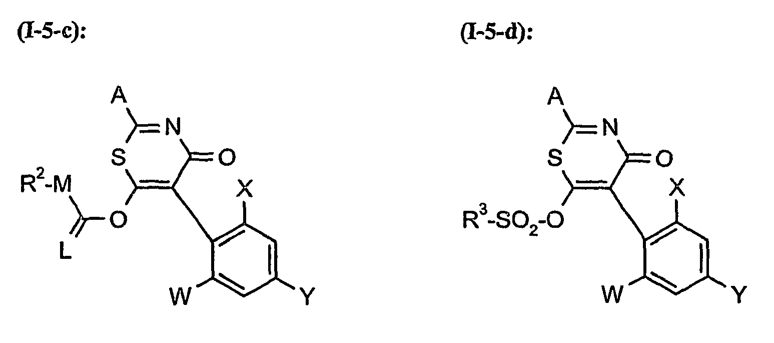 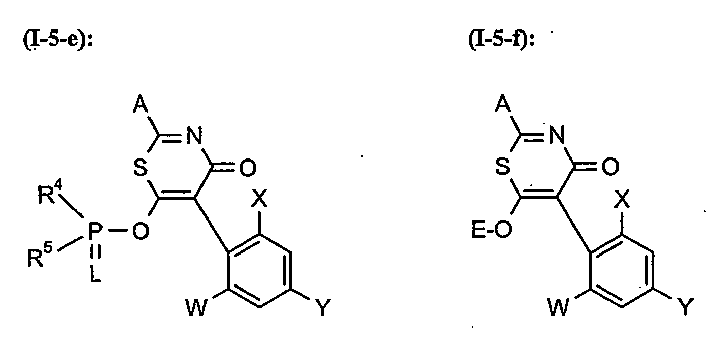 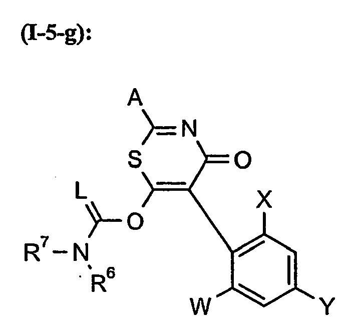 worin
A, E, L, M, W, X, Y, R¹, R², R³, R⁴, R⁵, R⁶ und R⁷ die oben angegebenen Bedeutungen besitzen.

Die Verbindungen der Formel (I-6) können in Abhängigkeit von der Stellung des Substituenten G in den zwei isomeren Formen der Formeln (I-6-A) und (I-6-B) vorliegen, 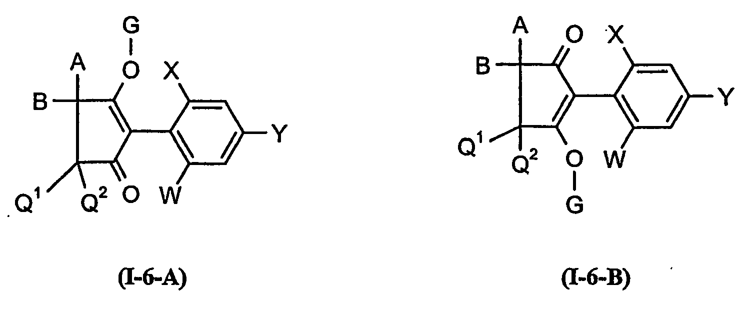 was durch die gestrichelte Linie in der Formel (I) zum Ausdruck gebracht werden soll.

Die Verbindungen der Formeln (I-6-A) und (I-6-B) können sowohl als Gemische als auch in Form ihrer reinen Isomeren vorliegen. Gemische der Verbindungen der Formeln (I-6-A) und (I-6-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgerührt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächlichen Strukturen (I-6-a) bis (I-6-g): 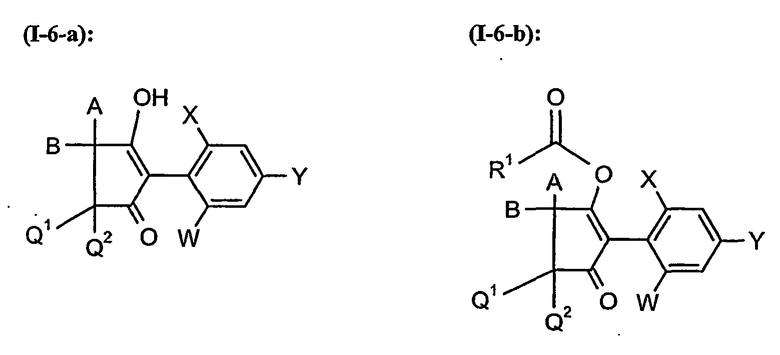 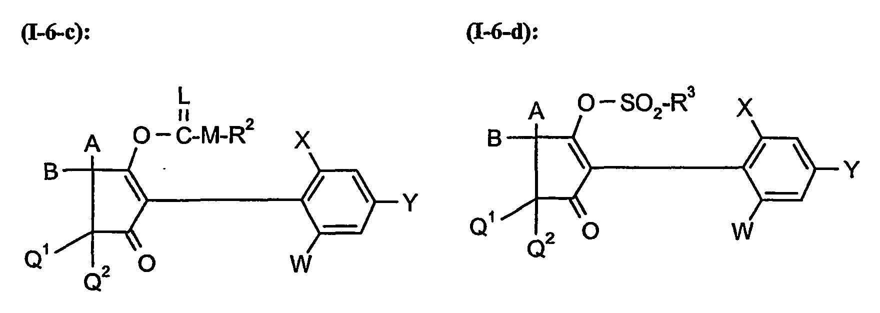 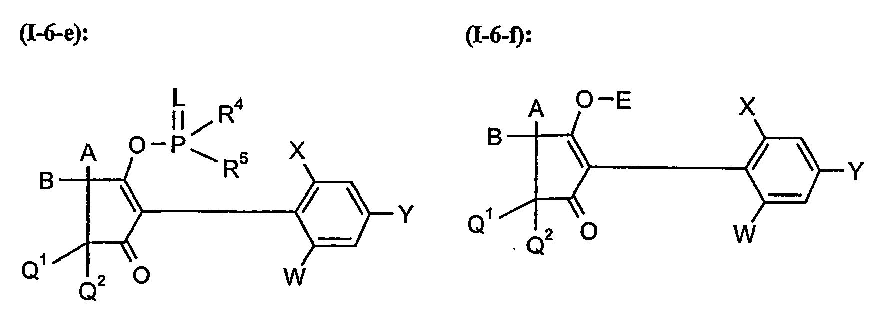 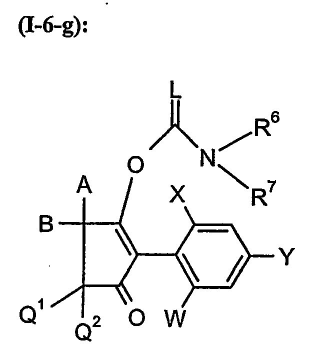 worin
A, B, Q¹ Q², E, L, M, W, X, Y, R¹, R², R³, R⁴, R⁵, R⁶ und R⁷ die oben angegebenen Bedeutungen besitzen.

Die Verbindungen der Formel (I-7) können in Abhängigkeit von der Stellung des Substituenten G in den zwei isomeren Formen der Formeln (I-7-A) bzw. (I-7-B) vorliegen, was durch die gestrichelte Linie in der Formel (I-7) zum Ausdruck gebracht werden soll:

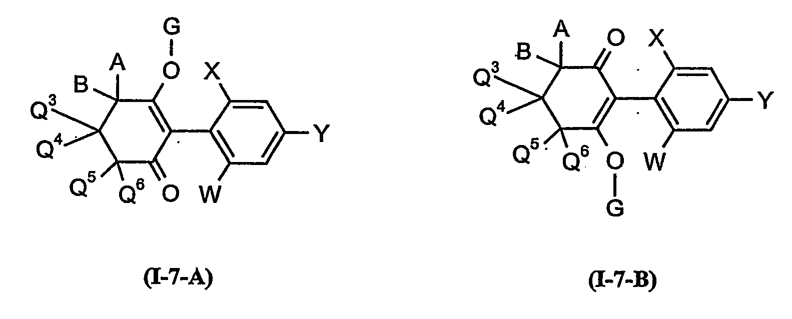

Die Verbindungen der Formeln (I-7-A) bzw. (I-7-B) können sowohl als Gemische als auch in Form ihrer reinen Isomeren vorliegen. Gemische der Verbindungen der Formeln (I-7-A) und (I-7-B) lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt ein, dass die betreffende Verbindung gegebenenfalls als Isomerengemisch oder in der jeweils anderen isomeren Form vorliegen kann.

Unter Einbeziehung der verschiedenen Bedeutungen (a), (b), (c), (d), (e), (f) und (g) der Gruppe G ergeben sich folgende hauptsächlichen Strukturen (I-7-a) bis (I-7-g): 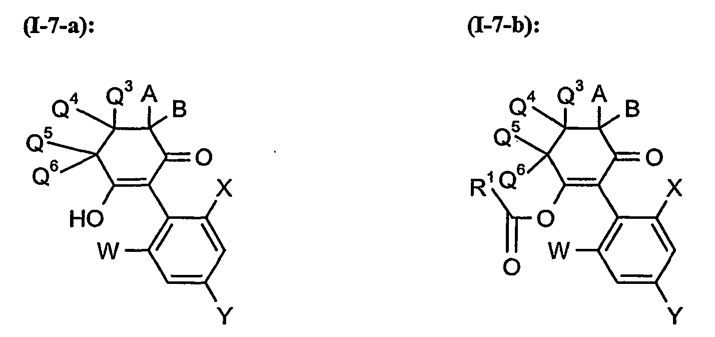 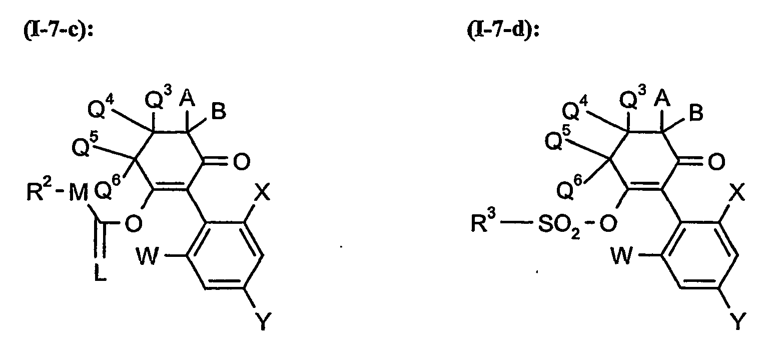 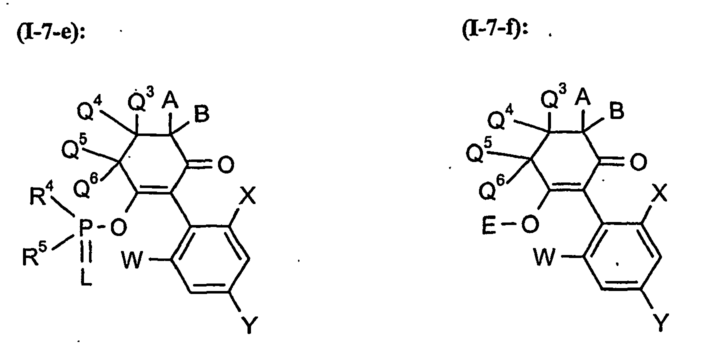 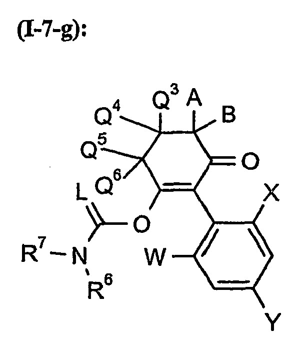 worin
A, B, E, L, M, Q³, Q⁴, Q⁵, Q⁶, W, X, Y, R¹, R², R³, R⁴, R⁵, R⁶ und R⁷ die oben angegebenen Bedeutungen besitzen.

Die Verbindungen der Formel (I-8) können in Abhängigkeit von der Stellung des Substituenten G in den zwei isomeren Formeln (I-8-A) und (I-8-B) vorliegen, 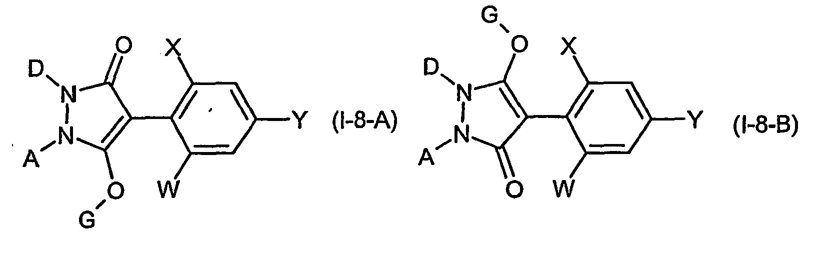 was durch die gestrichelte Linie in der Formel (I-8) zum Ausdruck gebracht werden soll.

Die Verbindungen der Formeln (I-8-A) und (I-8-B) können sowohl als Gemische als auch in Form ihrer reinen Isomeren vorliegen. Gemische der Verbindungen der Formel (I-8-A) und (I-8-B) lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-8-a) (bis (I-8-g), wenn Het für die Gruppe (8) steht, 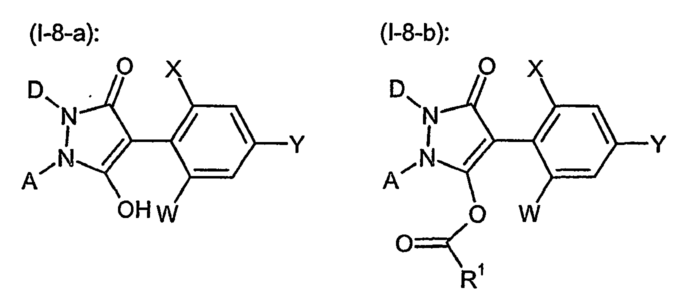 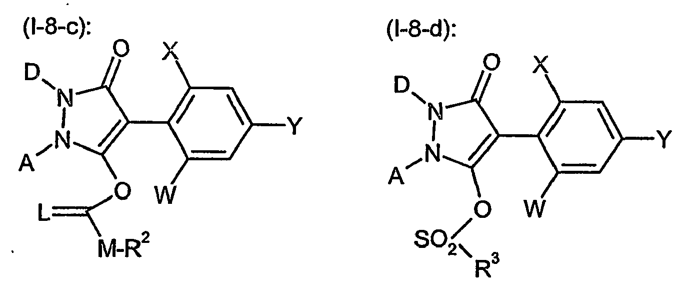 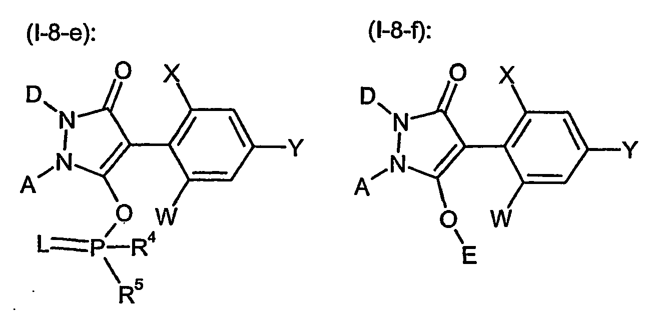 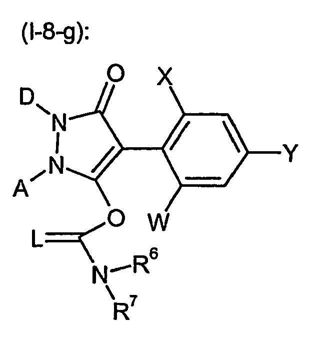 worin
A, D, E, L, M, W, X, Y, R¹, R², R³, R⁴, R⁵, R⁶ und R⁷ die oben angegebenen Bedeutungen besitzen.

Weiterhin wurde gefunden, dass man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 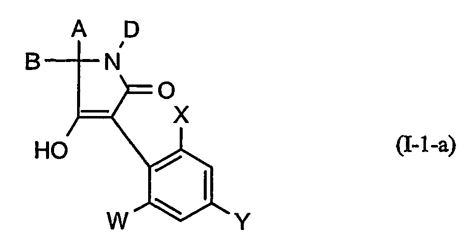 in welcher
   A, B, D, W, X und Y die oben angegebenen Bedeutungen haben,
   wenn man
   N-Acylaminosäureester der Formel (II) 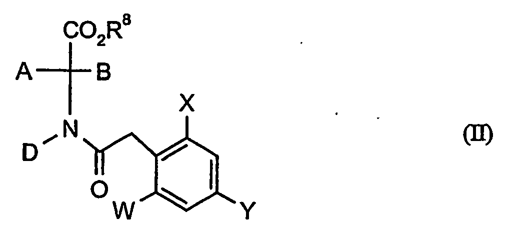 in welcher
   - A, B, D, W, X und Y: die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man substituierte 3-Phenyl-4-hydroxy-Δ³dihydrofuran-on-Derivate der Formel (I-2-a) 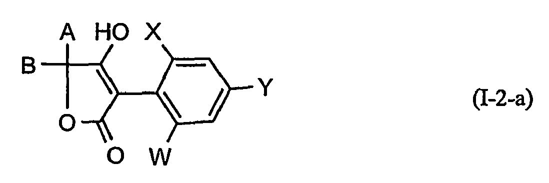 in welcher
   A, B, W, X und Y die oben angegebenen Bedeutungen haben,
   erhält, wenn man
   Carbonsäureester der Formel (III) 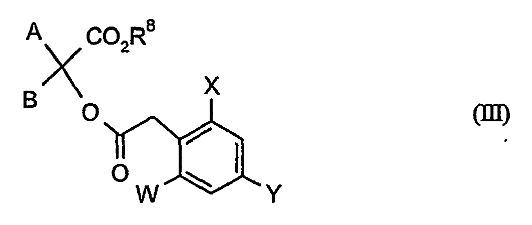 in welcher
   A, B, W, X , Y und R⁸ die oben angegebenen Bedeutungen haben,
   in Gegenwart eines Verdünnungsmittels und in Gegenwart einer Base intramolekular kondensiert.
(C) Weiterhin wurde gefunden, dass man substituierte 3-Phenyl-4-hydroxy-Δ³-dihydrothiophenon-Derivate der Formel (I-3-a) 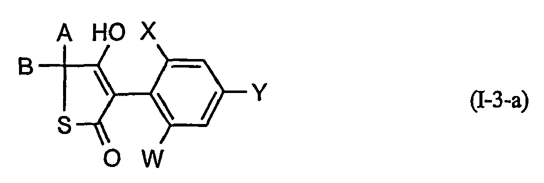 in welcher
   - A, B, W, X und Y: die oben angegebenen Bedeutungen haben,
   erhält, wenn man
   B-Ketocarbonsäureester der Formel (IV) 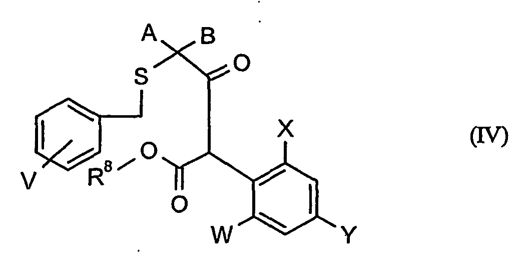 in welcher
   - A, B, W, X, Y und R⁸: die oben angegebenen Bedeutungen haben und
   - V: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin wurde gefunden, dass man die neuen substituierten 3-Phenylpyron-Derivate der Formel (I-4-a) 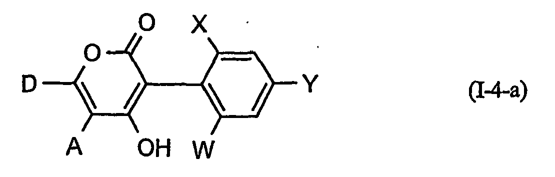 in welcher
   - A, D, W, X und Y: die oben angegebenen Bedeutungen haben,
   erhält, wenn man
   Carbonylverbindungen der Formel (V) 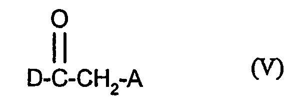 in welcher
   - A und D: die oben angegebenen Bedeutungen haben,
   oder deren Silylenolether der Formel (Va) 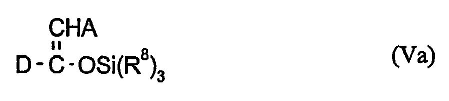 in welcher
   A, D und R⁸ die oben angegebene Bedeutung haben,
   mit Ketensäurehalogeniden der Formel (VI) 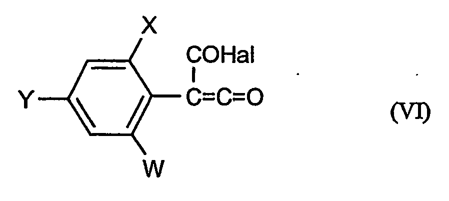 in welcher
   - W, X und Y: die oben angegebenen Bedeutungen haben und
   - Hal: für Halogen (vorzugsweise für Chlor oder Brom) steht,
   gegebenenfalls in Gegenwart eines Verdünnungsmittels und gegebenenfalls in Gegenwart eines Säureakzeptors umsetzt.
   Weiterhin wurde gefunden,
(E) dass man die neuen substituierten Phenyl-1,3-thiazin-Derivate der Formel (I-5-a) 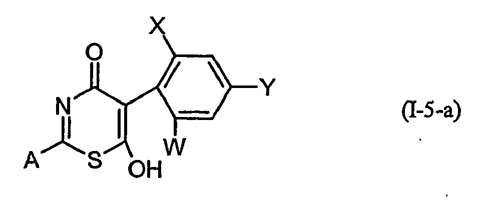 in welcher
   A, W, X und Y die oben angegebene Bedeutung haben,
   erhält, wenn man Thioamide der Formel (VII) 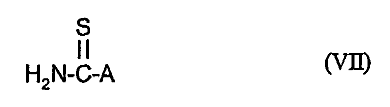 in welcher
   - A: die oben angegebene Bedeutung hat,
   mit Ketensäurehalogeniden der Formel (VI) 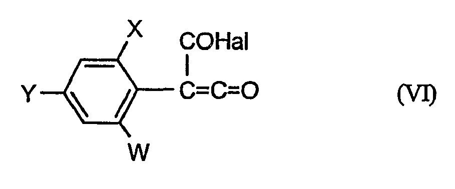 in welcher
   - Hal, W, X und Y: die oben angegebenen Bedeutungen haben,
   gegebenenfalls in Gegenwart eines Verdünnungsmittels und gegebenenfalls in Gegenwart eines Säureakzeptors umsetzt.
   Weiterhin wurde gefunden,
(F) dass man Verbindungen der Formel (I-6-a) 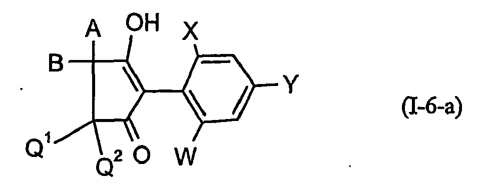 in welcher
   - A, B,Q¹, Q², W, X und Y: die oben angegebene Bedeutung haben,
   erhält, wenn man
   Ketocarbonsäureester der Formel (VIII) 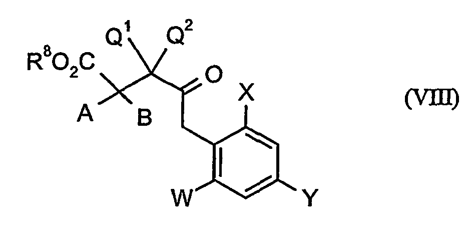 in welcher
   - A, B, Q¹, Q², W, X und Y: die oben angegebene Bedeutung haben, und
   - R⁸: für Alkyl (insbesondere C₁-C₈-Alkyl) steht,
   gegebenenfalls in Gegenwart eines Verdünnungsmittels und in Gegenwart einer Base intramolekular cyclisiert.
   Außerdem wurde gefunden,
(G) dass man Verbindungen der Formel (I-7-a) 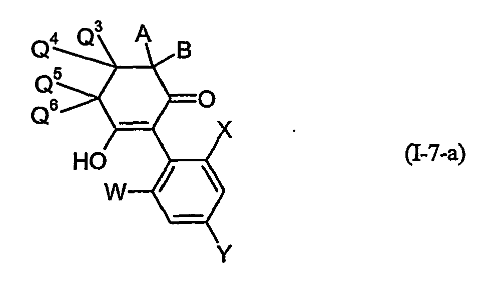 in welcher
   - A, B, Q³, Q⁴, Q⁵, Q⁶, W, X und Y: die oben angegebene Bedeutung haben,
   erhält, wenn man
   6-Aryl-5-keto-hexansäureester der Formel (IX) 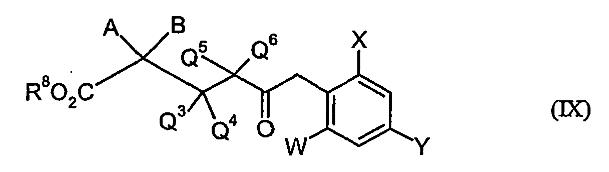 in welcher
   - A, B, Q³, Q⁴, Q⁵, Q⁶, W, X und Y: die oben angegebene Bedeutung haben
   und
   - R⁸ 8: für Alkyl (bevorzugt C₁-C₆-Alkyl) steht,
   in Gegenwart eines Verdünnungsmittels und in Gegenwart einer Base intramolekular kondensiert.
(H) Weiterhin wurde gefunden, dass man die Verbindungen der Formel (I-8-a) 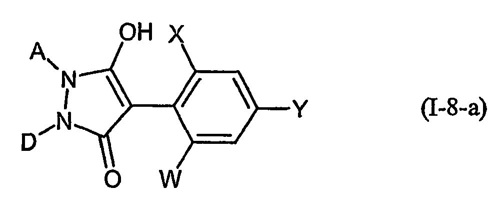 in welcher
   - A, D, W, X und Y: die oben angegebenen Bedeutungen haben,
   erhält, wenn man
   Verbindungen der Formel (X) 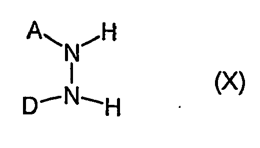 in welcher
   - A und D: die oben angegebene Bedeutung haben,

   α) mit Verbindungen der Formel (VI) 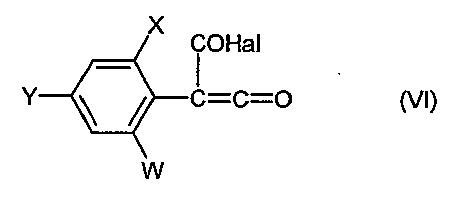 in welcher
      - Hal, X, Y und W: die oben angegebenen Bedeutungen haben,
      gegebenenfalls in Gegenwart eines Verdünnungsmittels und gegebenenfalls in Gegenwart eines Säureakzeptors umsetzt, oder
   ß) mit Verbindungen der Formel (XI) 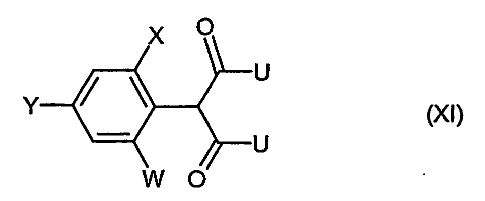 in welcher
      - W, X und Y: die oben angegebene Bedeutung haben,
      und U für NH₂ oder O-R⁸ steht, wobei R⁸ die oben genannte Bedeutung hat,
      gegebenenfalls in Gegenwart eines Verdünnungsmittels und gegebenenfalls in Gegenwart einer Base umsetzt, oder
   γ) Verbindungen der Formel (XII) 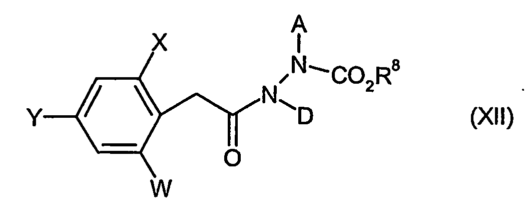 in welcher
      A, D, W, X, Y und R⁸ die oben angegebene Bedeutung haben,
      gegebenenfalls in Gegenwart eines Verdünnungsmittels und gegebenenfalls in Gegenwart einer Base umsetzt.
   Außerdem wurde gefunden
(I) dass man die Verbindungen der oben gezeigten Formeln (I-1-b) bis (I-8-b), in welchen A, B, D, Q¹, Q², Q³, Q⁴, Q⁵, Q⁶, R¹, W, X, und Y die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
   (α) mit Säurehalogeniden der Formel (XIII) 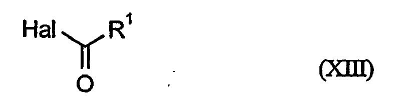 in welcher
      - R¹: die oben angegebene Bedeutung hat und
      - Hal: für Halogen (insbesondere Chlor oder Brom) steht
      oder
   (ß) mit Carbonsäureanhydriden der Formel (XIV)

      R¹-CO-O-CO-R¹ (XIV)

      in welcher
      - R¹: die oben angegebene Bedeutung hat,
   gegebenenfalls in Gegenwart eines Verdünnungsmittels und gegebenenfalls in Gegenwart eines Säurebindemittels umsetzt;
(J) dass man die Verbindungen der oben gezeigten Formeln (I-1-c) bis (I-8-c), in welchen A, B, D, Q¹, Q², Q³, Q⁴, Q⁵, Q⁶, R², M, W, X und Y die oben angegebenen Bedeutungen haben und L für Sauerstoff steht, erhält, wenn man Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
   mit Chlorameisensäureestern oder Chlorameisensäurethioestern der Formel (XV)

   R²-M-CO-Cl (XV)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(K) dass man Verbindungen der oben gezeigten Formeln (I-1-c) bis (I-8-c), in welchen A, B, D, Q¹, Q², Q³, Q⁴, Q⁵ , Q⁶, R², M, W, X u nd Y die oben angegebenen Bedeutungen haben und L für Schwefel steht, erhält, wenn man Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
   mit Chlormonothioameisensäureestern oder Chlordithioameisensäureestern der Formel (XVI) 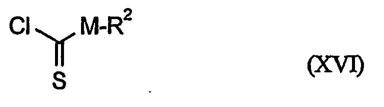 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt
   und
(L) dass man Verbindungen der oben gezeigten Formeln (I-1-d) bis (I-8-d), in welchen A, B, D, Q¹ Q², Q³, Q⁴, Q⁵, Q⁶, R³, W, X und Y die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
   mit Sulfonsäurechloriden der Formel (XVTI)

   R³-SO₂-Cl (XVII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(M) dass man Verbindungen der oben gezeigten Formeln (I-1-e) bis (I-8-e), in welchen A, B, D, L, Q¹, Q², Q³, Q⁴, Q⁵, Q⁶, R⁴, R⁵, W, X und Y die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
   mit Phosphorverbindungen der Formel (XVIII) 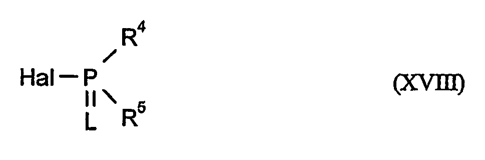 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(N) dass man Verbindungen der oben gezeigten Formeln (I-1-f) bis (I-8-f), in welchen A, B, D, E, Q¹, Q², Q³, Q⁴, Q⁵, Q⁶, W, X und Y die oben angegebenen Bedeutungen haben, erhält, wenn man Verbindungen der Formeln (I-1-a) bis (I-8-a), in welchen A, B, D, Q¹, Q², Q³, Q⁴, Q⁵, Q⁶, W, X und Y die oben angegebenen Bedeutungen haben, jeweils
   mit Metallverbindungen oder Aminen der Formeln (XIX) oder (XX)
   Me(OR¹⁰)ₜ (XIX) 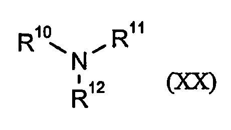 in welchen
   - Me: für ein ein- oder zweiwertiges Metall (bevorzugt ein Alkali- oder Erdalkalimetall wie Lithium, Natrium, Kalium, Magnesium oder Calcium), oder für ein Ammoniumion 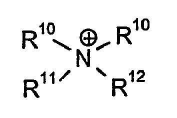
   - t: für die Zahl 1 oder 2 und
   - R¹⁰, R¹¹, R¹²: unabhängig voneinander für Wasserstoff oder Alkyl (bevorzugt C₁-C₈- Alkyl) stehen,
   gegebenenfalls in Gegenwart eines Verdünnungsmittels umsetzt,
(O) dass man Verbindungen der oben gezeigten Formeln (I-1-g) bis (I-8-g), in welchen A, B, D, L, Q¹, Q², Q³, Q⁴, Q⁵, Q⁶, R⁶, R⁷, W, X und Y die oben angegebenen Bedeutungen haben, erhält, wenn man Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
   (α) mit Isocyanaten oder Isothiocyanaten der Formel (XXI)

      R6-N=C=L (XXI)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   (ß) mit Carbamidsäurechloriden oder Thiocarbamidsäurechloriden der Formel (XXII) 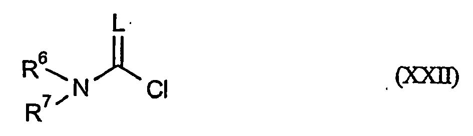 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt,
(P) dass man Verbindungen der oben gezeigten Formeln (I-1-a) bis I-8-a), in welchen A, B, D, Q¹ Q², Q³, Q⁴, Q⁵, Q⁶ W, X und Y die oben angegebene Bedeutung haben, erhält, wenn man Verbindungen der Formeln (I-1-a') bis (I-8-a'), in welchen A, B, D, Q¹, Q², Q³, Q⁴, Q⁵, Q⁶, X und Y die oben genannte Bedeutung haben und W' bevorzugt für Brom steht 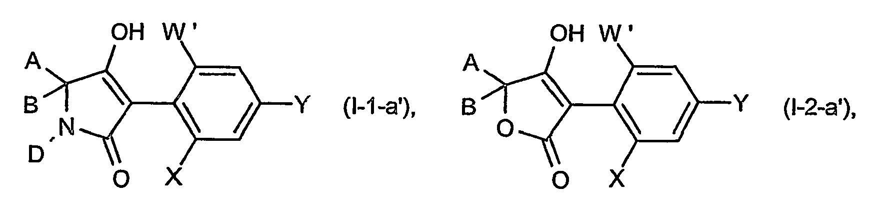 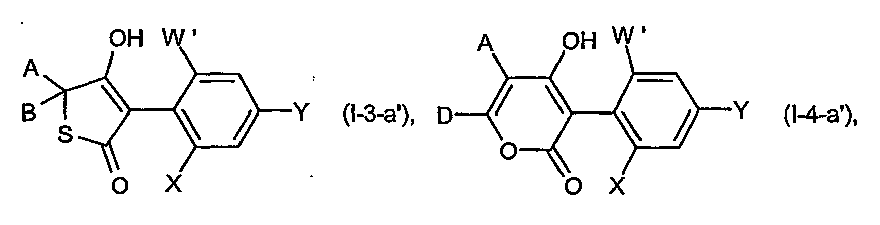 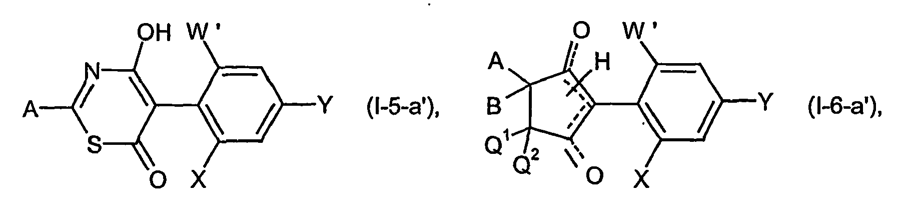 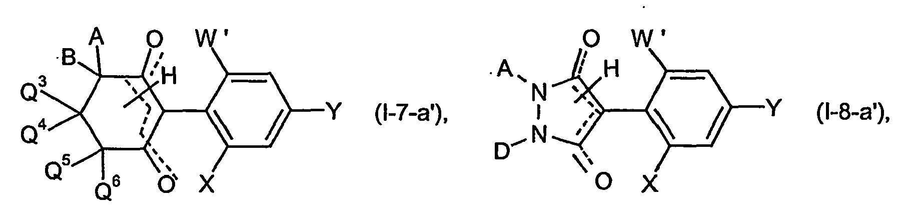 mit Alkoholen der Formel

   W-OH

   in welcher
   - W: die oben angegebene Bedeutung hat, gegebenenfalls in Gegenwart eines Verdünnungsmittels, eines Cu-(1)-Salzes (z.B. CuBr, CuJ) und einer starken Base (z.B. Natriumhydrid, Kalium-tert-butylat) umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstofflcombination umfassend als Komponenten
(a') mindestens ein substituiertes, cyclisches Ketoenol der Formel (I), in welcher CKE, W, X und Y die oben angegebene Bedeutung haben
   und
(b') zumindest eine die Kulturpflanzen-Verträglichkeit verbessernde Verbindung aus der folgenden Gruppe von Verbindungen:
   4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlonnethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl -vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2 -(4-Chlor-o-tolyloxy)-propionsäure (Mecoprop), Diethyl-1-(2,4-dichlor-phenyl)-4,5-dihydro-5-methyl-1H-pyrazol-3,5-di-carboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-di-allylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlorphenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxybenzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 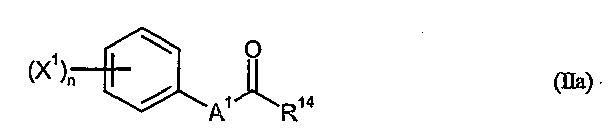 oder der allgemeinen Formel (IIb) 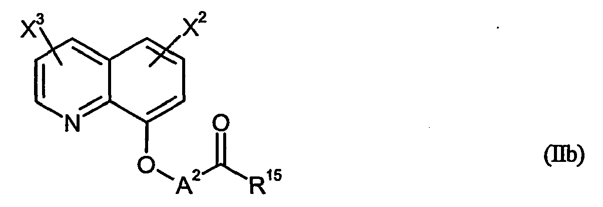 oder der Formel (IIc) 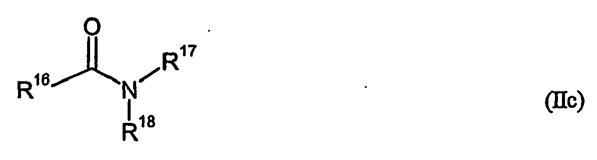 wobei
- n: für eine Zahl zwischen 0 und 5 steht,
- A¹: für eine der nachstehend skizzierten divalenten heterocyclischen Gruppierungen steht, 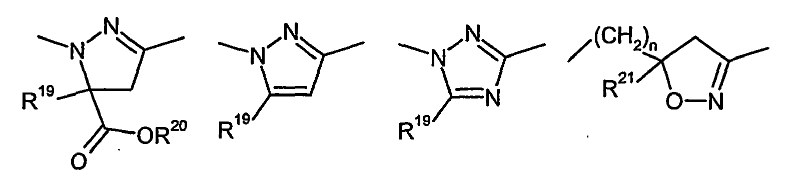
- n: für eine Zahl zwischen 0 und 5 steht,
- A²: für gegebenenfalls durch C₁-C₄-Alkyl und/oder C₁-C₄-Alkoxy-carbonyl substituiertes Alkan- diyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alkylamino oder Di- (C₁-C₄-alkyl)-amino steht,
- R¹⁵: für Hydroxy, Mercapto, Amino, C₁-C₆Alkoxy, C₁-C₆-Alkylthio, C₁-C₆-Alkylamino oder Di- (C₁-C₄-alkyl)-amino steht,
- R¹⁶: für jeweils gegebenenfalls durch Fluor, Chlor und/oder Brom substituiertes C₁-C₄-Alkyl steht,
- R¹⁷: für Wasserstoff, jeweils gegebenenfalls durch Fluor, Chlor und/oder Brom substituiertes C₁- C₆-Alkyl, C₂-C₆-Alkenyl oder C₂-C₆-Akhyl, C₁-C₄-Alkoxy-C₁-C₄-alkyl, Dioxolanyl-C₁-C₄- alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁- C₆-Alkyl, C₂-C₆-Alkenyl oder C₂-C₆-Alkinyl, C₁-C₄-Alkoxy-C₁-C₄-alkyl, Dioxolanyl-C₁-C₄- alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, oder zusammen mit R¹⁷ für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅- Oxaalkandiyl steht,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- R²⁰: für Wasserstoff, gegebenenfalls durch Hydroxy, Cyano, Halogen oder C₁-C₄-Alkoxy sub- stituiertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl, C₁-C₄-Alkoxy oder C₁-C₄- Halogenalkoxy steht,
- X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 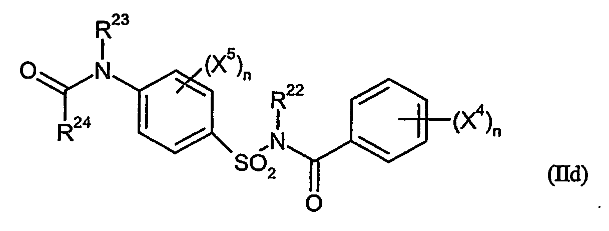 oder der allgemeinen Formel (IIe) 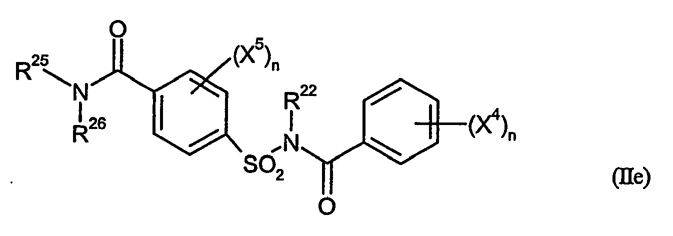 wobei
- n: für eine Zahl zwischen 0 und 5 steht,
- R²²: für Wasserstoff oder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substitu- iertes C₁-C₆-Alkyl, C₁-C₆Alkoxy, C₁-C₆-Alkylthio, C₁-C₆-Alkylamino oder Di-(C₁-C₄-alkyl)- amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substitu- iertes C₁-C₆-Alkyl, jeweils gegebenenfalls durch Cyano oder Halogen substituiertes C₃-C₆- Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substitu- iertes C₁-C₆-Alkyl, jeweils gegebenenfalls durch Cyano oder Halogen substituiertes C₃-C₆- Alkenyl oder C₃-C₆-Alkinyl, gegebenenfalls durch Cyano, Halogen oder C₁-C₄-Alkyl sub- stituiertes C₃-C₆-Cycloalkyl, oder gegebenenfalls durch Nitro, Cyano, Halogen, C₁-C₄-Alkyl, C₁-C₄-Halogenalkyl, C₁-C₄-Mkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆- Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁- C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁- C₄-Alkyl, C₁-C₄-Halogenalkyl, C₁-C₄ Alkoxy oder C₁-C₄-Halogenalkoxy steht.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- W: steht bevorzugt für C₁-C₆-Alkoxy, C₁-C₆-Halogenalkoxy, C₁-C₄-Alkoxy-C₂-C₄-alkyloxy, C₁-C₄-Alkoxy-bis-C₂-C₄-alkyloxy oder gegebenenfalls einfach bis dreifach durch Fluor, Chlor, C₁-C₃-Alkyl oder C₁-C₃-Alkoxy substituiertes C₃-C₆-Cycloalkyl-C₁-C₃-alkandiyl- oxy, worin gegebenenfalls eine Methylengruppe des Rings durch Sauerstoff oder Schwefel unterbrochen sein kann,
- X: steht bevorzugt für C₁-C₆-Alkyl,
- Y: steht bevorzugt für Chlor, Brom oder Iod,
- CKE: steht bevorzugt für eine der Gruppen 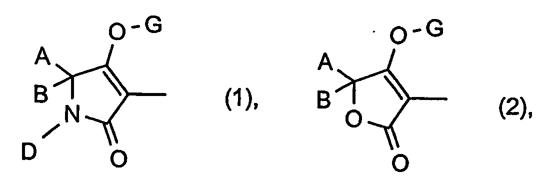 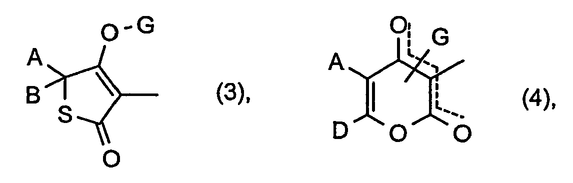 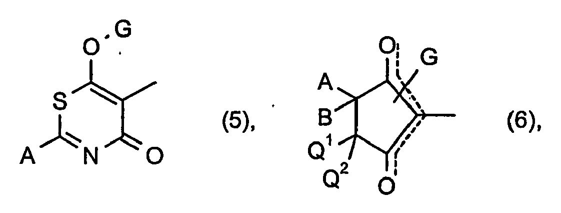 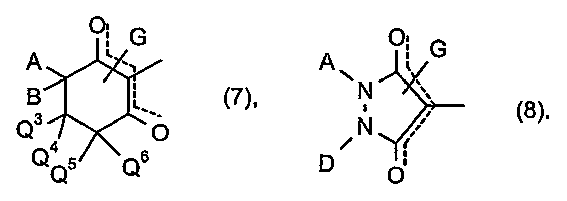
- A: steht bevorzugt für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₁-C₆- alkyl, gegebenenfalls durch, Halogen, C₁-C₆-Alkyl oder C₁-C₆-Alkoxy substituiertes C₃- C₈-Cycloalkyl, in welchem gegebenenfalls ein oder zwei nicht direkt benachbarte Ring- glieder durch Sauerstoff und/oder Schwefel ersetzt sind oder für jeweils gegebenenfalls durch Halogen, C₁-C₆-Alkyl, C₁-C₆ Halogenalkyl, C₁-C₆-Alkoxy, C₁-C₆-Halogen- alkoxy, Cyano oder Nitro substituiertes Phenyl, Naphthyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl), Phenyl-C₁-C₆-alkyl oder Naphthyl-C₁-C₆alkyl,
- B: steht bevorzugt für Wasserstoff, C₁-C₁₂-Alkyl oder C₁-C₈-Alkoxy-C₁-C₆-alkyl oder
- A, B: und das Kohlenstoffatom an das sie gebunden sind, stehen bevorzugt für gesättigtes C₃- C₁₀-Cycloalkyl oder ungesättigtes C₅-C₁₀-Cycloalkyl, worin gegebenenfalls ein Ring- glied durch Sauerstoff oder Schwefel ersetzt ist und welche gegebenenfalls einfach oder
- A, B: zweifach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder und das Kohlenstoffatom, an das sie gebunden sind, stehen bevorzugt für C₃-C₆-Cyclo- alkyl, welches durch eine gegebenenfalls ein oder zwei nicht direkt benachbarte Sauerstoff- und/oder Schwefelatome enthaltende gegebenenfalls durch C₁-C₄-Alkyl sub- stituierte Alkylendiyl-, oder durch eine Alkylendioxyl- oder durch eine Alkylendithioyl- Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, stehen bevorzugt für C₃-C₈-Cyclo- alkyl oder C₅-C₈-Cycloalkenyl, in welchen zwei Substituenten gemeinsam mit den Kohlenstoffatomen, an die sie gebunden sind, für jeweils gegebenenfalls durch C₁-C₆- Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- D: steht bevorzugt für Wasserstoff, jeweils gegebenenfalls durch Halogen substituiertes C₁- C₁₂-Alkyl, C₃-C₈-Alkenyl, C₃-C₈-Alkinyl, C₁-C₁₀-Alkoxy-C₂-C₈-alkyl, gegebenenfalls durch Halogen, C₁-C₄-Alkyl, C₁-C₄-Alkoxy oder C₁-C₄ Halogenalkyl substituiertes C₃- C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen (beispielsweise Furanyl, Imidazolyl, Pyridyl, Thiazolyl, Pyrazo- lyl, Pyrimidyl, Pyrrolyl, Thienyl oder Triazolyl), Phenyl-C₁-C₆-alkyl oder Hetaryl-C₁-C₆- alkyl mit 5 oder 6 Ringatomen (beispielsweise Furanyl, Imidazolyl, Pyridyl, Thiazolyl, Pyrazolyl, Pyrimidyl, Pyrrolyl, Thienyl oder Triazolyl) oder
- A und D: stehen gemeinsam bevorzugt für jeweils gegebenenfalls substituiertes C₃-C₆-Alkandiyl oder C₃-C₆-Alkendiyl, worin gegebenenfalls eine Methylengruppe durch eine Carbonyl- gruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist
oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemein- sam mit den Atomen, an die sie gebunden sind beispielsweise für die weiter unten ge- nannten Gruppen AD-1 bis AD-10), der Sauerstoff oder Schwefel enthalten kann, oder worin gegebenenfalls eine der folgenden Gruppen 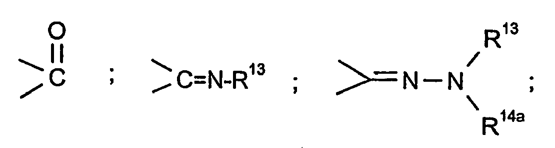 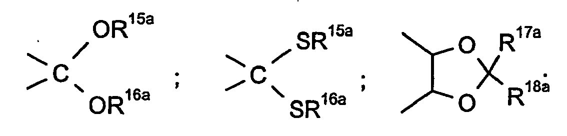 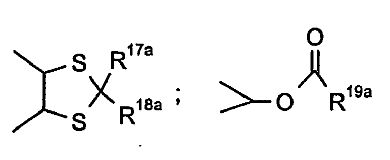 oder 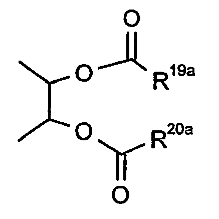 enthalten ist, oder
- A und Q¹: stehen gemeinsam bevorzugt für jeweils gegebenenfalls einfach oder zweifach, gleich oder verschieden durch Halogen, Hydroxy, durch jeweils gegebenenfalls einfach bis drei- fach, gleich oder verschieden durch Halogen substituiertes C₁-C₁₀-Alkyl, C₁-C₆-Alkoxy, C₁-C₆-Alkylthio, C₃-C₇-Cycloalkyl oder durch jeweils gegebenenfalls einfach bis dreifach, gleich oder verschieden durch Halogen, C₁-C₆-Alkyl oder C₁-C₆-Alkoxy substi- tuiertes Benzyloxy oder Phenyl substituiertes C₃-C₆-Alkandiyl oder C₄-C₆-Alkendiyl, welches außerdem gegebenenfalls eine der nachstehenden Gruppen 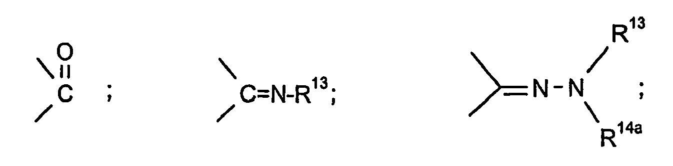 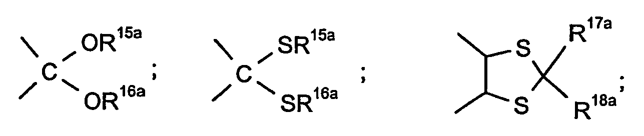 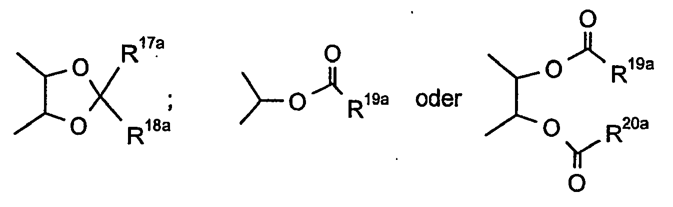 enthält oder durch eine C₁-C₂-Alkandiylgruppe oder durch ein Sauerstoffatom überbrückt ist oder
- Q¹: steht bevorzugt für Wasserstoff oder C₁-C₄-Alkyl,
- Q², Q⁴, Q⁵ und Q⁶: stehen unabhängig voneinander bevorzugt für Wasserstoff oder C₁-C₄-Alkyl,
- Q³: steht bevorzugt für Wasserstoff, C₁-C₆-Alkyl, C₁-C₆-Alkoxy-C₁-C₂-alkyl, C₁-C₆-Alkyl- thio-C₁-C₂-alkyl, gegebenenfalls durch C₁-C₄-Alkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl oder
- Q³ und Q⁴: stehen bevorzugt gemeinsam mit dem Kohlenstoffatom, an das sie gebunden sind, für einen gegebenenfalls durch C₁-C₄-Alkyl, C₁-C₄-Alkoxy oder C₁-C₂-Halogenalkyl sub- stituierten C₃-C₇-Ring, in welchem gegebenenfalls ein Ringglied durch Sauerstoff oder Schwefel ersetzt ist,
- G: steht bevorzugt für Wasserstoff (a) oder für eine der Gruppen 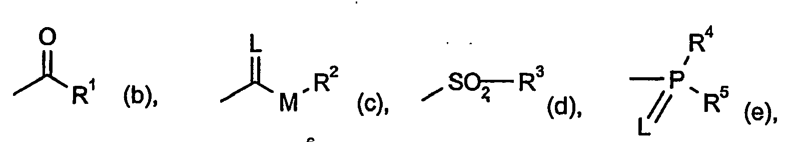 E (f) oder 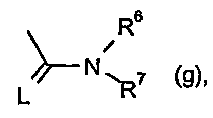 insbesondere für (a), (b), (c) oder (g)
in welchen
E für ein Metallionäquivalent oder ein Ammoniumion steht,
L für Sauerstoff oder Schwefel steht und
M für Sauerstoff oder Schwefel steht.
- R¹: steht bevorzugt für jeweils gegebenenfalls durch Halogen substituiertes C₁-C₂₀-Alkyl, C₂- C₂₀-Alkenyl, C₁-C₈-Alkoxy-C₁-C₈-alkyl, C₁-C₈-Alkylthio-C₁-C₈-alkyl, Poly-C₁-C₈- alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere (bevorzugt nicht mehr als zwei) nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆- Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆- Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl (beispielsweise Pyrazolyl, Thiazolyl, Pyridyl, Pyrimidyl, Furanyl oder Thienyl),
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6- gliedriges Hetaryloxy-C₁-C₆-alkyl (beispielsweise Pyridyloxy-C₁-C₆-alkyl, Pyrimidyloxy- C₁-C₆-alkyl oder Thiazolyloxy-C₁-C₆-alkyl),
- R²: steht bevorzugt für jeweils gegebenenfalls durch Halogen substituiertes C₁-C₂₀-Alkyl, C₂- C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈- Cycloalkyl oder
für jeweils gegebenenfalls durch Halogen, Cyano, Nitro, C₁-C₆-Alkyl, C₁-C₆-Alkoxy, C₁- C₆-Halogenalkyl oder C₁-C₆-Halogenalkoxy substituiertes Phenyl oder Benzyl,
- R³: steht bevorzugt für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl,
- R⁴ und R⁵: stehen bevorzugt unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenen- falls durch Halogen, Nitro, Cyano, C₁-C₄-Alkoxy, C₁-C₄-Halogenalkoxy, C₁-C₄-Alkyl- thio, C₁-C₄-Halogenalkylthio, C₁-C₄-Alkyl oder C₁-C₄-Halogenalkyl substituiertes Phenyl, Phenoxy oder Phenylthio,
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈- Alkenyl, C₁-C₈-Alkoxy-C₁-C₈-alkyl, für gegebenenfalls durch Halogen, C₁-C₈- Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆- Alkylenrest, in welchem gegebenenfalls ein Kohlenstoffatom durch Sauerstoff oder Schwefel ersetzt ist,
- R¹³: steht bevorzugt für Wasserstoff, für jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄-Halogenalkyl, C₁-C₄- Halogenalkoxy, Nitro oder Cyano substituiertes Phenyl, Phenyl-C₁-C₄-alkyl oder Phenyl- C₁-C₄-alkoxy,
- R^{14a}: steht bevorzugt für Wasserstoff oder C₁-C₈-Alkyl oder
- R¹³und R^{14a}: stehen gemeinsam bevorzugt für C₄-C₆-Alkandiyl,
- R^{15a} und R^{16a}: sind gleich oder verschieden und stehen bevorzugt für C₁-C₆-Alkyl oder
- R^{15a} und R^{16a}: stehen gemeinsam bevorzugt für einen C₂-C₄-Alkandiylrest, der gegebenenfalls durch C₁-C₆-Alkyl, C₁-C₆-Halogenalkyl oder durch gegebenenfalls durch Halogen, C₁- C₆-Alkyl, C₁-C₄-Halogenalkyl, C₁-C₆-Alkoxy, C₁-C₄-Halogenalkoxy, Nitro oder Cyano substituiertes Phenyl substituiert ist,
- R^{17a} und R^{18a}: stehen unabhängig voneinander bevorzugt für Wasserstoff, für gegebenenfalls durch Halogen substituiertes C₁-C₈-Alkyl oder für gegebenenfalls durch Halogen, C₁-C₆- Alkyl, C₁-C₆-Alkoxy, C₁-C₄-Halogenalkyl, C₁-C₄-Halogenalkoxy, Nitro oder Cyano substituiertes Phenyl oder
- R^{17a} und R^{18a}: stehen gemeinsam mit dem Kohlenstoffatom, an das sie gebunden sind, bevorzugt für eine Carbonylgruppe oder für gegebenenfalls durch Halogen, C₁-C₄-Alkyl oder C₁- C₄-Alkoxy substituiertes C₅-C₇-Cycloalkyl, in dem gegebenenfalls eine Methylengruppe durch Sauerstoff oder Schwefel ersetzt ist,
- R^{19a} und R^{20a}: stehen unabhängig voneinander bevorzugt für C₁-C₁₀-Alkyl, C₂-C₁₀-Alkenyl, C₁-C₁₀-Alkoxy, C₁-C₁₀-Alkylamino, C₃-C₁₀-Alkenylanimo, Di-(C₁-C10-alkyl)amino oder Di-(C₃-C₁₀-alkenyl)amino.

In den als bevorzugt genannten Restedefinitionen steht Halogen für Fluor, Chlor, Brom und Jod, insbesondere für Fluor, Chlor und Brom.
- W: steht besonders bevorzugt für C₁-C₄-Alkoxy, C₁-C₄-Halogenalkoxy, C₁-C₃-Alkoxy-C₂-C₃- alkyloxy, C₁-C₂-Alkoxy-bis-C₂-C₃-alkyloxy oder C₃-C₆-Cycloalkyl-C₁-C₂-alkandiyloxy, worin gegebenenfalls eine Methylengruppe des Rings durch Sauerstoff unterbrochen sein kann,
- X: steht besonders bevorzugt für C₁-C₃-Alkyl,
- Y: steht besonders bevorzugt für Chlor oder Brom,
- CKE: steht besonders bevorzugt für eine der Gruppen 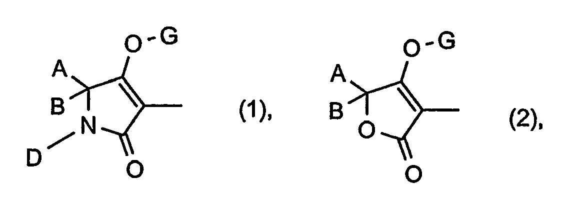 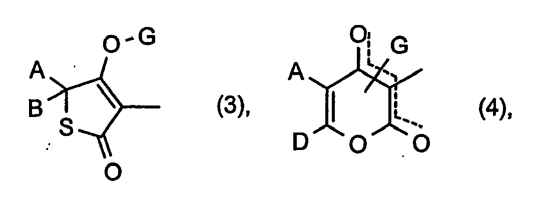 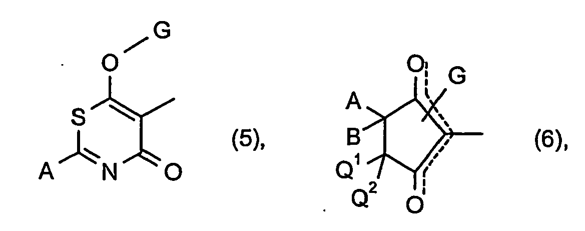 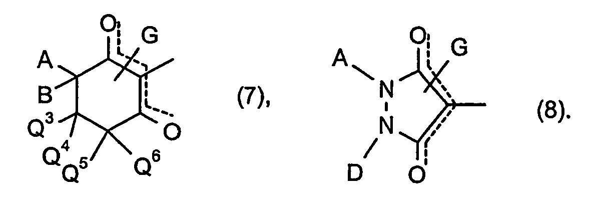
- A: steht besonders bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebe- nenfalls einfach bis zweifach durch C₁-C₂-Alkyl oder C₁-C₂-Alkoxy substituiertes C₃- C₆-Cycloalkyl oder (jedoch nicht im Fall der Verbindungen der Formeln (I-3), (I-4), (I-6) und (I-7)) jeweils einfach bis zweifach gegebenenfalls durch Fluor, Chlor, Brom, C₁-C₄- Alkyl, C₁-C₂-Halogenalkyl, C₁-C₄-Alkoxy, C₁-C₂-Halogenalkoxy, Cyano oder Nitro sub- stituiertes Phenyl oder Benzyl,
- B: steht besonders bevorzugt für Wasserstoff, C₁-C₄-Alkyl oder C₁-C₂-Alkoxyl-C₁-C₂-alkyl oder
- A, B: und das Kohlenstoffatom an das sie gebunden sind, stehen besonders bevorzugt für ge- sättigtes oder ungesättigtes C₅-C₇-Cycloalkyl, worin gegebenenfalls ein Ringglied durch Sauerstoff oder Schwefel ersetzt ist und welches gegebenenfalls einfach bis zweifach durch C₁-C₆-Alkyl, Trifluormethyl oder C₁-C₆-Alkoxy, substituiert ist mit der Maßgabe, dass dann Q³ besonders bevorzugt für Wasserstoff oder Methyl steht oder
- A,B: und das Kohlenstoffatom, an das sie gebunden sind, stehen besonders bevorzugt für C₅- C₆-Cycloalkyl, welches durch eine gegebenenfalls ein oder zwei nicht direkt benachbarte Sauerstoff- oder Schwefelatome enthaltende gegebenenfalls durch Methyl oder Ethyl sub- stituierte Alkylendiyl- oder durch eine Alkylendioxyl- oder durch eine Alkylendithiol- Gruppe substituiert ist, die mit dem Kohlenstoffatom, an das sie gebunden ist, einen weiteren fünf- oder sechsgliedrigen Ring bildet mit der Maßgabe, dass dann Q³ besonders bevorzugt für Wasserstoff oder Methyl steht,
- A, B: und das Kohlenstoffatom, an das sie gebunden sind, stehen besonders bevorzugt für C₃- C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂- Alkyl oder C₁-C₂-Alkoxy substituiertes C₂-C₄-Alkandiyl, C₂-C₄-Alkendiyl oder Butadiendiyl stehen, mit der Maßgabe, dass dann Q³ besonders bevorzugt für Wasserstoff oder Methyl steht,
- D: steht besonders bevorzugt für Wasserstoff, für jeweils gegebenenfalls einfach bis dreifach durch Fluor substituiertes C₁-C₆-Alkyl, C₃-C₆-Alkenyl, C₁-C₄-Alkoxy-C₂-C₃-alkyl, für gegebenenfalls einfach bis zweifach durch C₁-C₄-Alkyl, C₁-C₄-Alkoxy oder C₁-C₂- Halogenalkyl substituiertes C₃-C₆-Cycloalkyl, in welchem gegebenenfalls eine Methylen- gruppe durch Sauerstoff ersetzt ist oder (jedoch nicht im Fall der Verbindungen der Formeln (I-1)) für jeweils gegebenenfalls einfach bis zweifach durch Fluor, Chlor, Brom, C₁-C₄-Alkyl, C₁-C₄-Halogenalkyl, C₁-C₄-Alkoxy oder C₁-C₄-Halogenalkoxy substi- tuiertes Phenyl oder Pyridyl, oder
- A und D: stehen gemeinsam besonders bevorzugt für gegebenenfalls einfach bis zweifach sub- stituiertes C₃-C₅-Alkandiyl, in welchem eine Methylengruppe durch eine Carbonylgruppe (nicht jedoch im Fall der Verbindungen der Formel (I-1)), Sauerstoff oder Schwefel ersetzt sein kann, wobei als Substituenten C₁-C₂-Alkyl oder C₁-C₂-Alkoxy in Frage kommen oder
- A und D: stehen (im Fall der Verbindungen der Formel (I-1)) gemeinsam mit den Atomen, an die sie gebunden sind, für eine der Gruppen AD-1 bis AD-10: 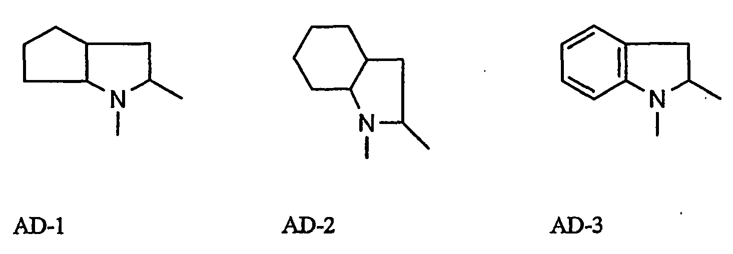 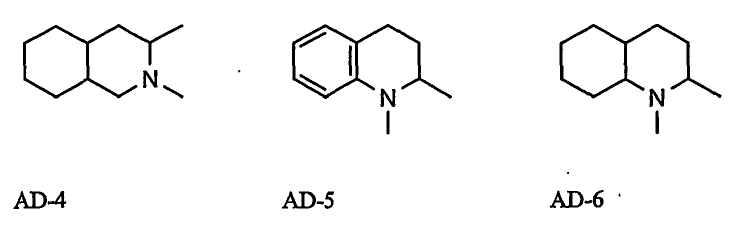 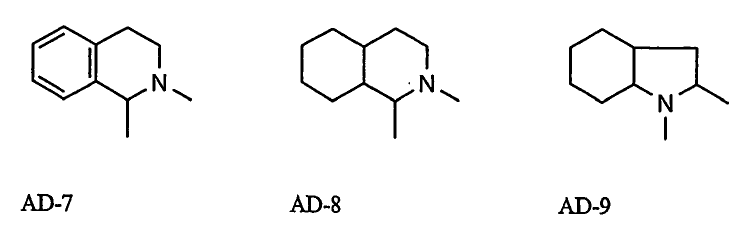 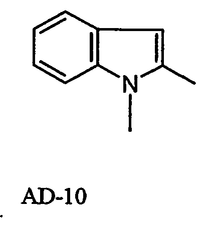 oder
- A und Q¹: stehen gemeinsam besonders bevorzugt für jeweils gegebenenfalls einfach oder zweifach, gleich oder verschieden durch C₁-C₂-Alkyl oder C₁-C₂-Alkoxy substituiertes C₃-C₄-Alkandiyl oder
- Q¹: steht besonders bevorzugt für Wasserstoff,
- Q²: steht besonders bevorzugt für Wasserstoff,
- Q⁴, Q⁵ und Q⁶: stehen besonders bevorzugt unabhängig voneinander für Wasserstoff oder C₁-C₃- Alkyl,
- Q³: steht besonders bevorzugt für Wasserstoff, C₁-C₄-Alkyl, oder gegebenenfalls einfach bis zweifach durch Methyl oder Methoxy substituiertes C₃-C₆-Cycloalkyl, oder
- Q³ und Q⁴: stehen besonders bevorzugt gemeinsam mit dem Kohlenstoff, an das sie gebunden sind, für einen gegebenenfalls durch C₁-C₂-Alkyl oder C₁-C₂-Alkoxy substituierten gesättigten C₅-C₆-Ring, in welchem gegebenenfalls ein Ringglied durch Sauerstoff oder Schwefel ersetzt ist, mit der Maßgabe, dass dann A besonders bevorzugt für Wasserstoff oder Methyl steht, oder
- G: steht besonders bevorzugt für Wasserstoff (a) oder für eine der Gruppen 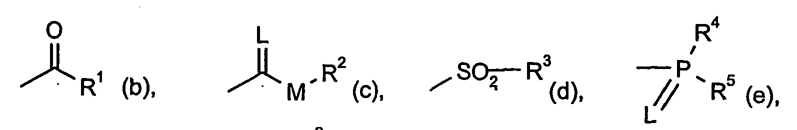 E (f) oder 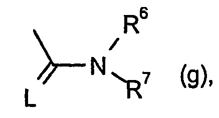 insbesondere für (a), (b) oder (c),
in welchen
E für ein Metallionäquivalent oder ein Ammoniumion steht,
L für Sauerstoff oder Schwefel steht und
M für Sauerstoff oder Schwefel steht,
- R¹: steht besonders bevorzugt für jeweils gegebenenfalls einfach bis dreifach durch Fluor oder Chlor substituiertes C₁-C₈-Alkyl, C₂-C₁₈-Alkenyl, C₁-C₄-Alkoxy-C₁-C₂-alkyl, C₁-C₄- Alkylthio-C₁-C₂-alkyl oder gegebenenfalls einfach bis zweifach durch Fluor, Chlor, C₁-C₂-Alkyl oder C₁-C₂-Alkoxy substituiertes C₃-C₆-Cycloalkyl, in welchem gegebenen- falls ein oder zwei nicht direkt benachbarte Ringglieder durch Sauerstoff ersetzt sind,
für gegebenenfalls einfach bis zweifach durch Fluor, Chlor, Brom, Cyano, Nitro, C₁-C₄- Alkyl, C₁-C₄-Alkoxy, C₁-C₂-Halogenalkyl oder C₁-C₂-Halogenalkoxy substituiertes Phenyl,
- R²: steht besonders bevorzugt für jeweils gegebenenfalls einfach bis dreifach durch Fluor sub- stituiertes C₁-C₈-Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆- Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substituiertes Phenyl oder Benzyl,
- R³: steht besonders bevorzugt für gegebenenfalls einfach bis dreifach durch Fluor substi- tuiertes C₁-C₆-Alkyl oder für jeweils gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl,
- R⁴: steht besonders bevorzugt für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder Tri- fluormethyl substituiertes Phenyl, Phenoxy oder Phenylthio,
- R⁵: steht besonders bevorzugt für C₁-C₆-Alkoxy oder C₁-C₆-Alkylthio,
- R⁶: steht besonders bevorzugt für Wasserstoff, C₁-C₆-Alkyl, C₃-C₆-Cycloalkyl, C₁-C₆- Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phe- nyl, für gegebenenfalls einfach durch Fluor, Chlor, Brom, C₁-C₄-Alkyl, Trifluormethyl oder C₁-C₄-Alkoxy substituiertes Benzyl,
- R⁷: steht besonders bevorzugt für C₁-C₆-Alkyl, C₃-C₆-Alkenyl oder C₁-C₆-Alkoxy-C₁-C₄-alkyl,
- R⁶ und R⁷: stehen besonders bevorzugt zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist.

In den als besonders bevorzugt genannten Restedefinitionen steht Halogen für Fluor, Chlor und Brom, insbesondere für Fluor und Chlor.
- W: steht ganz besonders bevorzugt für Methoxy, Ethoxy, n-Propoxy, iso-Propoxy, n-Butoxy, iso-Butoxy, sec-Butoxy, Methoxy-ethyloxy, Ethoxy-ethyloxy, Cyclopropyl-methoxy, Cyclopentyl-methoxy oder Cyclohexyl-methoxy,
- X: steht ganz besonders bevorzugt für Methyl oder Ethyl,
- Y: steht ganz besonders bevorzugt für Chlor oder Brom,
- CKE: steht ganz besonders bevorzugt für eine der Gruppen 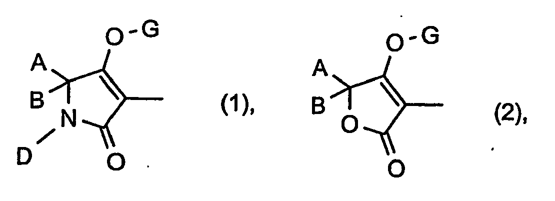 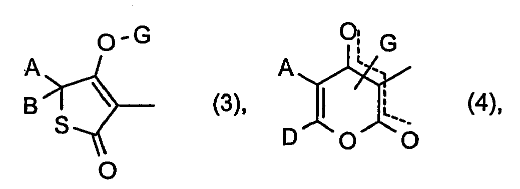 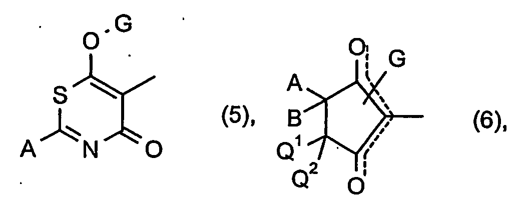 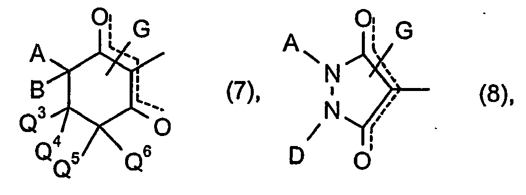
- A: steht ganz besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl und nur im Fall der Verbindungen der Formel (I-5) für jeweils gegebenenfalls durch Fluor, Chlor, Brom, Methyl, Ethyl, n-Propyl, iso- Propyl, Methoxy, Ethoxy, Trifluormethyl, Trifluormethoxy, Cyano oder Nitro sub- stituiertes Phenyl,
- B: steht ganz besonders bevorzugt für Wasserstoff, Methyl oder Ethyl oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Trifluormethyl, Methoxy, Ethoxy, Propoxy oder Butoxy substituiert ist, mit der Maßgabe, dass dann Q³ ganz besonders bevorzugt für Wasserstoff steht oder
- A, B: und das Kohlenstoffatom, an das sie gebunden sind, stehen ganz besonders bevorzugt für C₆-Cycloalkyl, welches gegebenenfalls durch mit zwei nicht direkt benachbarten Sauer- stoffatomen enthaltende Alkylendioxyl-Gruppe substituiert ist, mit der Maßgabe, dass dann Q³ ganz besonders bevorzugt für Wasserstoff steht oder
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄- Alkendiyl oder Butadiendiyl stehen, mit der Maßgabe, dass dann Q³ ganz besonders bevorzugt für Wasserstoff steht,
- D: steht ganz besonders bevorzugt für Wasserstoff, für jeweils gegebenenfalls einfach bis dreifach durch Fluor substituiertes C₁-C₄₋Alkyl, C₃-C₄-Alkenyl, C₁-C₄-Alkoxy-C₂-C₃- alkyl, für Cyclopropyl, Cyclopentyl oder Cyclohexyl oder (jedoch nicht im Fall der Verbindungen der Formeln (I-1)) für gegebenenfalls einfach durch Fluor, Chlor, Methyl, Ethyl, n-Propyl, iso-Propyl, Methoxy, Ethoxy oder Trifluormethyl substituiertes Phenyl oder Pyridyl,
oder
- A und D: stehen gemeinsam ganz besonders bevorzugt für gegebenenfalls einfach durch Methyl oder Methoxy substituiertes C₃-C₅-Alkandiyl, worin gegebenenfalls ein Kohlenstoffatom durch Sauerstoff oder Schwefel ersetzt ist oder für die Gruppe AD-1
- A und Q¹: stehen gemeinsam ganz besonders bevorzugt für gegebenenfalls einfach oder zweifach durch Methyl oder Methoxy substituiertes C₃-C₄-Alkandiyl oder
- Q¹: steht ganz besonders bevorzugt für Wasserstoff,
- Q²: steht ganz besonders bevorzugt für Wasserstoff,
- Q⁴, Q⁵ und Q⁶: stehen ganz besonders bevorzugt unabhängig voneinander für Wasserstoff oder Methyl,
- Q³: steht ganz besonders bevorzugt für Wasserstoff, Methyl, Ethyl oder Propyl, oder
- Q³ und Q⁴: stehen ganz besonders bevorzugt gemeinsam mit dem Kohlenstoff, an den sie ge- bunden sind, für einen gegebenenfalls einfach durch Methyl oder Methoxy substituierten gesättigten C₅-C₆-Ring, mit der Maßgabe, dass dann A ganz besonders bevorzugt für Wasserstoff steht,
- G: steht ganz besonders bevorzugt für Wasserstoff (a) oder für eine der Gruppen

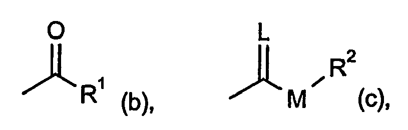

-SO₂-R³

(d) oder E (f),
in welchen
L für Sauerstoff oder Schwefel steht,
M für Sauerstoff oder Schwefel steht und
E für ein Ammoniumion steht,
- R¹: steht ganz besonders bevorzugt für C₁-C₆-Alkyl, C₂-C₁₇-Alkenyl, C₁-C₂-Alkoxy-C₁- alkyl, C₁-C₂-Alkylthio-C₁-alkyl oder jeweils gegebenenfalls einfach durch Fluor, Chlor, Methyl oder Methoxy substituiertes Cyclopropyl oder Cyclohexyl, für gegebenenfalls einfach durch Fluor, Chlor, Brom, Cyano, Nitro, Methyl, Methoxy, Tri- fluormethyl oder Trifluormethoxy substituiertes Phenyl,
- R²: steht ganz besonders bevorzugt für jeweils gegebenenfalls einfach durch Fluor sub- stituiertes C₁-C₈-Alkyl, C₂-C₆-Alkenyl oder C₁-C₄-Alkoxy-C₂-C₃-alkyl, Phenyl oder Benzyl,
- R³: steht ganz besonders bevorzugt für C₁-C₈-Alkyl.
- W: steht hervorgehoben für Methoxy, Ethoxy, n-Propoxy, iso-Propoxy, n-Butoxy, iso-Butoxy, sec-Butoxy, Methoxy-ethyloxy, Ethoxy-ethyloxy oder Cyclopropylinethoxy,
- X: steht hervorgehoben für Methyl oder Ethyl,
- Y: steht hervorgehoben für Chlor,
- CKE: steht hervorgehoben für eine der Gruppen 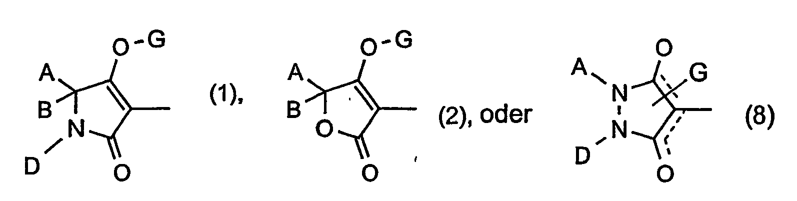
- A: steht hervorgehoben für Wasserstoff, Methyl, Ethyl, Cyclopropyl, Isopropyl, n-Propyl, Isobutyl, n-Butyl, t-Butyl oder s-Butyl (insbesondere für Wasserstoff, Methyl oder Ethyl),
- B: steht hervorgehoben für Wasserstoff, Methyl oder Ethyl,
- A, B: und das Kohlenstoffatom an das sie gebunden sind, stehen hervorgehoben für gesättigtes C₅-C₆-Cycloalkyl, in welchem gegebenenfalls ein Ringglied durch Sauerstoff ersetzt ist und welches gegebenenfalls einfach durch Methyl, Methoxy, Ethoxy, n-Propoxy, n-Butoxy oder Trifluormethyl (insbesondere durch Methyl oder Methoxy), substituiert ist,
- D: steht hervorgehoben für Wasserstoff, Methyl, Ethyl, Isopropyl, Cyclopropyl oder Cyclohexyl,
oder
- A und D: stehen gemeinsam hervorgehoben für C₃-C₅-Alkandiyl oder für die Gruppe AD-1,
- G: steht hervorgehoben für Wasserstoff (a) oder für eine der Gruppen 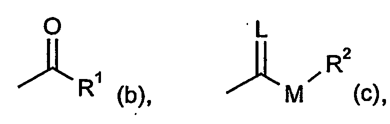 -SO₂-R³ (d) oder E (f)
in welchen
L für Sauerstoff steht,
M für Sauerstoff steht und
E für ein Ammoniumion (N⁺ (C₆H₁₃)₄) steht,
- R¹: steht hervorgehoben für C₁-C₈-Alkyl, C₁-C₂-Alkoxy-C₁-alkyl, oder C₂-C₁₇-Alkenyl,
- R²: steht hervorgehoben für C₁-C₈-Alkyl oder C₂-C₆-Alkenyl,
- R³: steht hervorgehoben für C₁-C₄-Alkyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert s ein, wobei b ei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

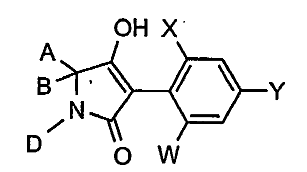

**Tabelle 1: W = OCH₃, X = CH₃, Y = Cl.**

| A | B | D |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 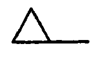 | CH₃ | H |
| 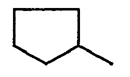 | CH₃ | H |
| 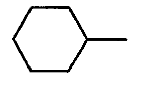 | CH₃ | H |
| -(CH₂)₂- | | H |
| -(CH₂)₄- | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHO₂H₅-(CH₂)₂- | | H |
| -(CH₂)-CHOC₃H₇-(CH₂)₂- | | H |
| -(CH₂)₂-CHO-i-C₃H₇-(CH₂)₂- | | H |
| -(CH₂)₂-C(CH₃)₂-(CH₂)₂- | | H |
| -CH₂-(CHCH₃)₂-(CH₂)₂- | | H |
| 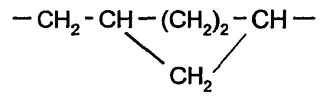 | | H |
| 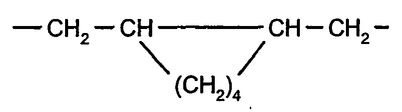 | | H |
| 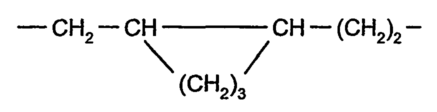 | | H |
| 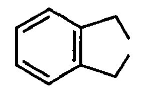 | | H |
| 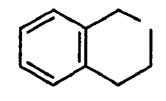 | | H |
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-CH(OCH₃)-CH₂- | | H |
| -CH₂-CH=CH-CH₂- | | H |
| 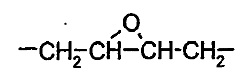 | | H |
| -CH₂-S-CH₂- | | H |
| -CH₂-S-(CH₂)₂- | | H |
| -(CH₂)₂-S-CH₂- | | H |
| 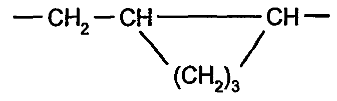 | | H |
| H | CH₃ | H |
| H | C₂H₅ | H |
| H | C₃H₇ | H |
| H | i-C₃H₇ | H |
| H | 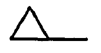 | H |
| H | 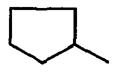 | H |
| H | 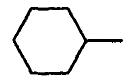 | H |
| CH₃ | CH₃ | H |
| CH₃ | C₂H₅ | H |
| CH₃ | C₃H₇ | H |
| CH₃ | i-C₃H₇ | H |
| CH₃ | 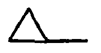 | H |
| CH₃ | 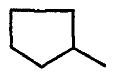 | H |
| CH₃ | 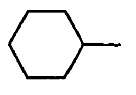 | H |
| 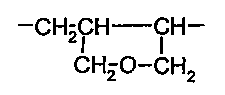 | | H |
| C₂H₅ | CH₃ | H |
| C₂H₅ | C₂H₅ | H |

**Tabelle 2:** A, B und D wie in Tabelle 1 angegeben
W=OCH₃; X = CH₃; Y = Br

**Tabelle 3:** A, B und D wie in Tabelle 1 angegeben
W = OCH₃; X = C₂H₅; Y = Cl.

**Tabelle 4:** A, B und D wie in Tabelle 1 angegeben
W = OCH₃; X=C₂H₅; Y=Br.

**Tabelle 5:** A, B und D wie in Tabelle 1 angegeben
W = OC₂H₅; X = CH₃; Y = Cl.

**Tabelle 6:** A, B und D wie in Tabelle 1 angegeben
W = OC₂H₅; X = C₂H₅; Y = Cl.

Im einzelnen seien außer den bei den Herstellungsbeispielen genannten Verbindungen die folgenden Verbindungen der Formel (I-2-a) genannt:

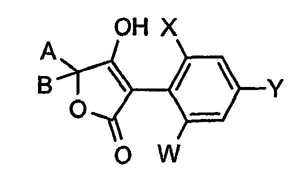

**Tabelle 7: W = OCH₃, X = CH₃, Y = Cl.**

| A | B |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 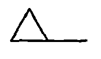 | CH₃ |
| 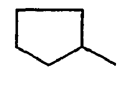 | CH₃ |
| 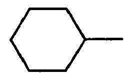 | CH₃ |
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -(CH₂)-CHCH₃-(CH₂)₂- | |
| (CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHO-i-C₃H₇-(CH₂)₂- | |
| -(CH₂)₂-C(CH₃)₂-(CH₂)₂- | |
| -CH₂-(CHCH₃)₂-(CH₂)₂- | |
| 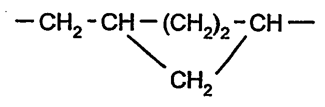 | |
| 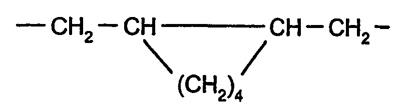 | |
| 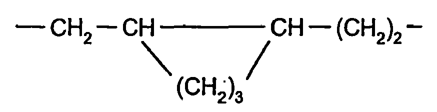 | |
| 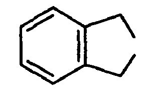 | |
| 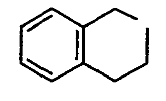 | |

**Tabelle 8**: A und B wie in Tabelle 7 angegeben
W = OCH₃; X = CH₃; Y = Br.

**Tabelle 9**: A und B wie in Tabelle 7 angegeben
W = OCH; X = C₂H₅; Y = Cl.

**Tabelle 10**: A und B wie in Tabelle 7 angegeben
W = OCH₃; X = C₂H₅; Y = Br.

**Tabelle 11**: A und B wie in Tabelle 7 angegeben
W = OC₂H₅; X = CH₃; Y = Cl.

**Tabelle 12**: A und B wie in Tabelle 7 angegeben
W = OC₂H₅; X = C₂H₅; Y = Cl.

Im einzelnen seien außer bei den Herstellungsbeispielen genannten Verbindungen die folgenden Verbindungen deer Formel (I-8-a) genannt:

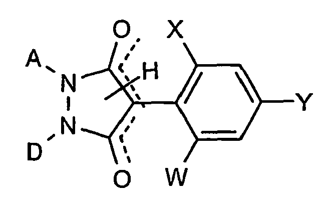

**Tabelle 13: W = OCH₃, X = CH₃, Y = Cl.**

| **A** | **D** |
|---|---|
| CH₃ | CH₃ |
| CH₃ | -(CH₂)₂OH- |
| CH₃ | -(CH₂)₂OCH₃- |
| CH₃ | -(CH₂)₂-O-(CH₂₎₂-OCH₃- |
| -(CH₂)₂-O-CH₃- | -(CH₂)₂-O-CH₃- |
| -(CH₂)₂-O-(CH₂)₂-OCH₃- | -(CH₂)₂-O-(CH₂)₂-OCH₃- |
| -(CH₂)₃- | |
| -(CH₂)₄- | |
| -(CH₂)₂-O-(CH₂)₂- | |
| 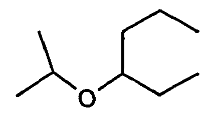 | |
| 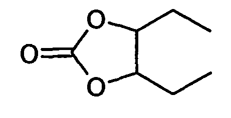 | |
| 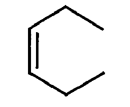 | |
| 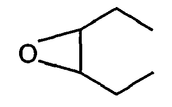 | |
| 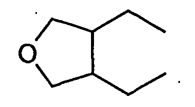 | |
| 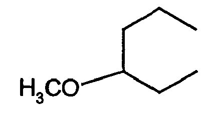 | |
| 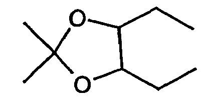 | |
| 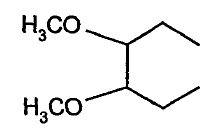 | |
| 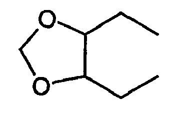 | |
| 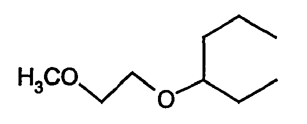 | |

**Tabelle 14:** A und D wie in Tabelle 13 angegeben
W = OCH₃; X = CH₃; Y = Br.

**Tabelle 15:** A und D wie in Tabelle 13 angegeben
W = OCH₃; X = C₂H₅; Y = Cl.

**Tabelle 16:** A und D wie in Tabelle 13 angegeben
W = OCH₃; X = C₂H₅; Y = Br.

**Tabelle 17**: A und D wie in Tabelle 13 angegeben
W = OC₂H₅; X = CH₃; Y = Cl.

**Tabelle 18:** A und D wie in Tabelle 13 angegeben
W = OC₂H₅; X = C₂H₅; Y = Cl.

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- n: steht bevorzugt für die Zahlen 0, 1, 2, 3 oder 4.
- A¹: steht bevorzugt für eine der nachstehend skizzierten divalenten heterocyclischen Gruppierungen 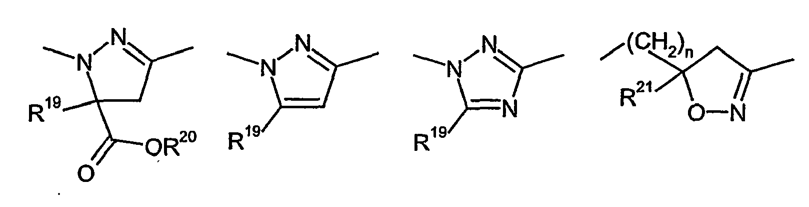
- A²: steht bevorzugt für jeweils gegebenenfalls durch Methyl, Ethyl, Methoxycarbonyl oder Ethoxy-carbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethyl- amino oder Diethylamino.
- R¹⁵: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethyl- amino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substitu- iertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substitu- iertes Methyl, Ethyl, n- oder i-Propyl, n-, i-, s- oder t-Butyl, Propenyl, Butenyl, Propinyl oder Butenyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclo- propyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s- oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s- oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluor- methyl, Fluordichlormethyl, Methoxy, Ethoxy, n- oder i-Propoxy, Difluormethoxy oder Tri- fluormethoxy.
- X²: steht bevorzugt für Wasserstoff, Nitro, Cyano, Fluor, Chlor, Brom, Methyl, Ethyl, n- oder i- Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluor- methoxy oder Trifluormethoxy.
- X³: steht bevorzugt für Wasserstoff, Nitro, Cyano, Fluor, Chlor, Brom, Methyl, Ethyl, n- oder i- Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluor- methoxy oder Trifluormethoxy.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i- Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclo- propylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch C yano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Di- fluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan- 1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle Beispiele für die Verbindungen der Formel (IIa)**

| | | | |
|---|---|---|---|
| 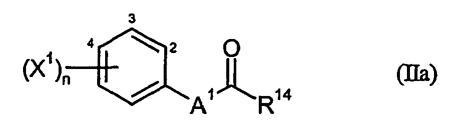 | | | |

| **Beispiel-Nr.** | **(Positionen) (X¹)ₙ** | **A¹** | **R¹⁴** |
|---|---|---|---|
| IIa-1 | (2) Cl, (4) Cl | 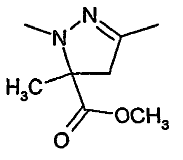 | OCH₃ |
| IIa-2 | (2) Cl, (4) Cl | 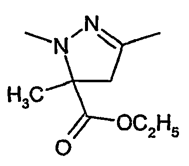 | OCH₃ |
| Ila-3 | (2) Cl, (4) Cl | 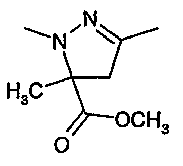 | OC₂H₅ |
| IIa-4 | (2) Cl, (4) Cl | 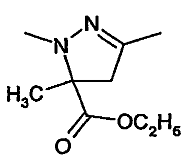 | OC₂H₅ |
| IIa-5 | (2) Cl | 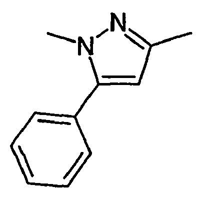 | OCH₃ |
| IIa-6 | (2) Cl, (4) Cl | 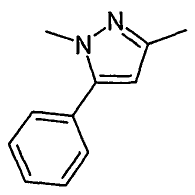 | OCH₃ |
| IIa-7 | (2) F | 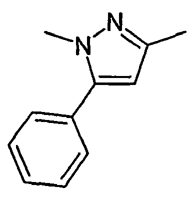 | OCH₃ |
| IIa-8 | (2) F | 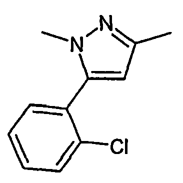 | OCH₃ |
| IIa-9 | (2) Cl, (4) Cl | 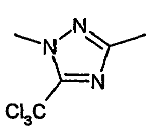 | OC₂H₅ |
| IIa-10 | (2) Cl, (4) CF₃ | 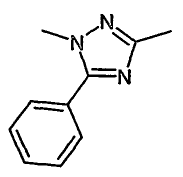 | OCH₃ |
| IIa-11 | (2) Cl | 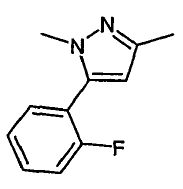 | OCH₃ |
| IIa-12 | - | 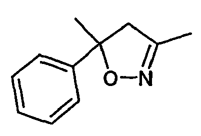 | OC₂H₅ |
| IIa-13 | (2) Cl, (4) Cl | 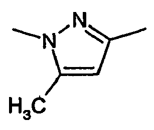 | OC₂H₅ |
| IIa-14 | (2) Cl, (4) Cl | 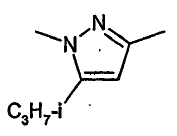 | OC₂H₅ |
| IIa-15 | (2) Cl, (4) Cl | 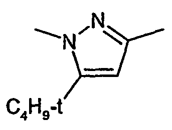 | OC₂H₅ |
| IIa-16 | (2) Cl, (4) Cl | 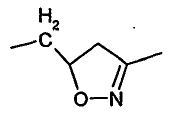 | OC₂H₅ |
| IIa-17 | (2) Cl, (4) Cl | 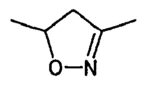 | OC₂H₅ |
| IIa-18 | - | 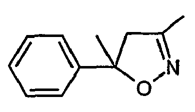 | OH |

Beispiele für die als erfindungsgemäße Herbizid-Safener ganz besonders bevorzugten Verbindungen der Formel (IIb) sind in der nachstehenden Tabelle aufgeführt.

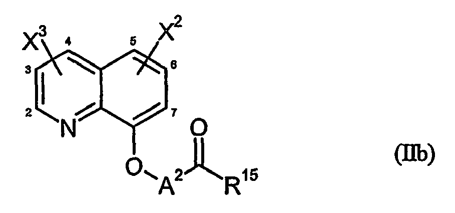

**Tabelle Beispiele für die Verbindungen der Formel (IIb)**

| **Beispiel-Nr.** | **(Position) X²** | **(Position) X³** | **A²** | **R¹⁵** |
|---|---|---|---|---|
| IIb-1 | (5) | - | CH₂ | OH |
| | Cl | | | |
| IIb-2 | (5) | - | CH₂ | OCH₃ |
| | Cl | | | |
| IIb-3 | (5) | - | CH₂ | OC₂H₅ |
| | Cl | | | |
| IIb-4 | (5) | - | CH₂ | OC₃H₇₋ₙ |
| | Cl | | | |
| IIb-5 | (5) | - | CH₂ | OC₃H₇-i |
| | Cl | | | |
| IIb-6 | (5) | - | CH₂ | OC₄H₉-n |
| | Cl | | | |
| IIb-7 | (5) | - | CH₂ | OCH(CH₃)C₅H₁₁-n |
| | Cl | | | |
| IIb-8 | (5) | (2) | CH₂ | OH |
| | Cl | F | | |
| IIb-9 | (5) | (2) | CH₂ | OH |
| | Cl | Cl | | |
| IIb-10 | (5) | - | CH₂ | OCH₂CH=CH₂ |
| | Cl | | | |
| IIb-11 | (5) | - | CH₂ | OC₄H₉-i |
| | Cl | | | |
| IIb-12 | (5) | - | CH₂ | 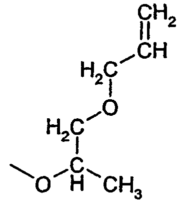 |
| | Cl | | | |
| IIb-13 | (5) | - | 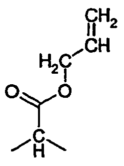 | OCH₂CH=CH₂ |
| | Cl | | | |
| IIb-14 | (5) | - | 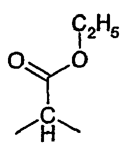 | OC₂H₅ |
| | Cl | | | |
| IIb-15 | (5) | - | 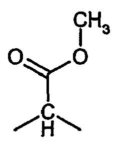 | OCH3 |
| | Cl | | | |

Beispiele für die als erfindungsgemäße Herbizid-Safener ganz besonders bevorzugten Verbindungen der Formel (IIc) sind in der nachstehenden Tabelle aufgeführt.

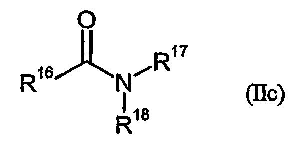

**Tabelle Beispiele für die Verbindungen der Formel (IIc)**

| **Beispiel-Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 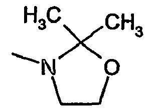 |
| IIc-3 | CHCl₂ | 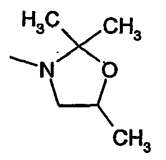 |
| IIc-4 | CHCl₂ | 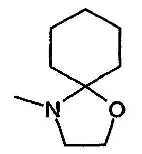 |
| IIc-5 | CHCl₂ | 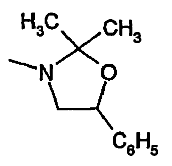 |
| IIc-6 | CHCl₂ | 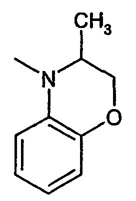 |
| IIc-7 | CHCl₂ | 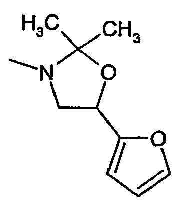 |

Beispiele für die als erfindungsgemäße Herbizid-Safener ganz besonders bevorzugten Verbindungen der Formel (IId) sind in der nachstehenden Tabelle aufgeführt.

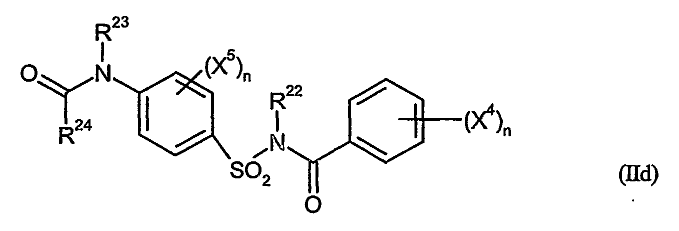

**Tabelle Beispiele für die Verbindungen der Formel (IId)**

| **Beispiel-Nr.** | **R²²** | **R²³** | **R²⁴** | **(Positionen) (X⁴)ₙ** | **(Positionen) (X⁵)ₙ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 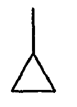 | (2) OCH₃ | - |
| IId-6 | H | H | CH₃ | (2) OCH₃ (5) CH₃ | - |
| IId-7 | H | H | C₂H₅ | (2) OCH₃ | - |
| | | | | (5) CH₃ | |
| IId-8 | H | H | C₃H₇-n | (2) OCH₃ | - |
| | | | | (5) CH₃ | |
| IId-9 | H | H | C₃H₇-i | (2) OCH₃ | - |
| | | | | (5) CH₃ | |
| IId-10 | H | H | 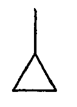 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 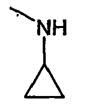 | (2) OCH₃ | - |
| | | | | (5) CH₃ | |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

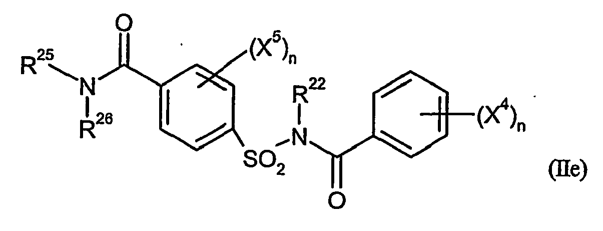

**Tabelle Beispiele für die Verbindungen der Formel (IIe)**

| **Beispiel-Nr.** | **R²²** | **R²⁵** | **R²⁶** | **(Positionen) (X⁴)ₙ** | **(Positionen) (X⁵)ₙ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 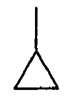 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| IIe-8 | H | H | C₂H₅ | (2) OCH₃ | - |
| | | | | (5) CH₃ | |
| IIe-9 | H | H | C₃H₇-n | (2) OCH₃ | - |
| | | | | (5) CH₃ | |
| IIe-10 | H | H | C₃H₇-i | (2) OCH₃ | - |
| | | | | (5) CH₃ | |
| IIe-11 | H | H | 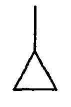 | (2)OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfmdungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle Beispiele für die erfindungsgemäßen Kombinationen**

| **Wirkstoffe der Formel (I)** | **Safener** |
|---|---|
| I-1 | Cloquintocet-mexyl |
| I-1 | Fenchlorazole-ethyl |
| I-1 | Isoxadifen-ethyl |
| I-1 | Mefenpyr-diethyl |
| I-1 | Furilazole |
| I-1 | Fenclorim |
| I-1 | Cumyluron |
| I-1 | Daimuron /Dymron |
| I-1 | Dimepiperate |
| I-1 | IIe-11 |
| I-1 | IIe-5 |
| I-2 | Cloquintocet-mexyl |
| I-2 | Fenchlorazole-ethyl |
| I-2 | Isoxadifen-ethyl |
| I-2 | Mefenpyr-diethyl |
| I-2 | Furilazole |
| I-2 | Fenclorim |
| I-2 | Cumyluron |
| I-2 | Daimuron /Dymron |
| I-2 | Dimepiperate |
| I-2 | IIe-11 |
| I-2 | IIe-5 |
| I-3 | Cloquintocet-mexyl |
| I-3 | Fenchlorazole-ethyl |
| I-3 | Isoxadifen-ethyl |
| I-3 | Mefenpyr-diethyl |
| I-3 | Furilazole |
| I-3 | Fenclorim |
| I-3 | Cumyluron |
| I-3 | Daimuron /Dymron |
| I-3 | Dimepiperate |
| I-3 | IIe-5 |
| I-3 | IIe-11 |
| I-4 | Cloquintocet-mexyl |
| I-4 | Fenchlorazole-ethyl |
| I-4 | Isoxadifen-ethyl |
| I-4 | Mefenpyr-diethyl |
| I-4 | Furilazole |
| I-4 | Fenclorim |
| I-4 | Cumyluron |
| I-4 | Daimuron /Dymron |
| I-4 | Dimepiperate |
| I-4 | IIe-11 |
| I-4 | IIe-5 |
| I-5 | Cloquintocet-mexyl |
| I-5 | Fenchlorazole-ethyl |
| I-5 | Isoxadifen-ethyl |
| 1-5 | Mefenpyr-diethyl |
| I-5 | Furilazole |
| I-5 | Fenclorim |
| I-5 | Cumyluron |
| I-5 | Daimuron/Dymron |
| I-5 | Dimepiperate |
| I-5 | IIe-5 |
| I-5 | IIe-11 |
| I-6 | Cloquintocet-mexyl |
| I-6 | Fenchlorazole-ethyl |
| I-6 | Isoxadifen-ethyl |
| I-6 | Mefenpyr-diethyl |
| I-6 | Furilazole |
| I-6 | Fenclorim |
| I-6 | Cumyluron |
| I-6 | Daimuron /Dymron |
| I-6 | Dimepiperate |
| I-6 | IIe-5 |
| I-6 | IIe-11 |
| 1-7 | Cloquintocet-mexyl |
| I-7 | Fenchlorazole-ethyl |
| I-7 | Isoxadifen-ethyl |
| I-7 | Mefenpyr-diethyl |
| I-7 | Furilazole |
| I-7 | Fenclorim |
| I-7 | Cumyluron |
| I-7 | Daimuron /Dymron |
| I-7 | Dimepiperate |
| I-7 | IIe-5 |
| I-7 | IIe-11 |
| I-8 | Cloquintocet-mexyl |
| I-8 | Fenchlorazole-ethyl |
| I-8 | Isoxadifen-ethyl |
| I-8 | Mefenpyr-diethyl |
| I-8 | Furilazole |
| I-8 | Fenclorim |
| I-8 | Cumyluron |
| I-8 | Daimuron /Dymron |
| I-8 | Dimepiperate |
| I-8 | IIe-5 |
| I-8 | IIe-11 |

Es wurde nun überraschend gefunden, dass die oben definierten Wirkstoffkombinationen aus substituierten cyclischen Ketoenole der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Verwendet man beispielsweise gemäß Verfahren (A) N-(2-Methyl-4-chlor-6-methoxy-phenylacetyl)-1-amino-cyclohexan-carbonsäureethylester als Ausgangsstoff, so kann der Verlauf des erfmdungsgemäßen Verfahrens durch folgendes Reaktionsschema wiedergegeben werden:

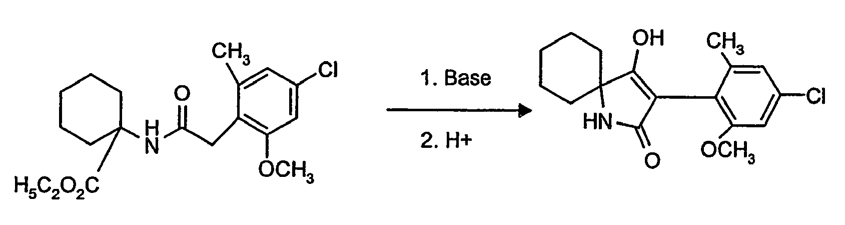

Verwendet man beispielsweise gemäß Verfahren (B) O-(2-Ethyl-4-chlor-6-methoxy-phenylacetyl)-2-hydroxyisobuttersäureethylester, so kann der Verlauf des erfindungsgemäßen Verfahrens durch folgendes Reaktionsschema wiedergegeben werden:

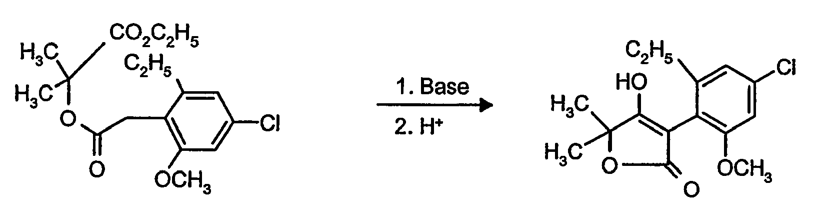

Verwendet man beispielsweise gemäß Verfahren (C) 2-(2-Methyl-4-chlor-6-methoxy-phenyl)-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

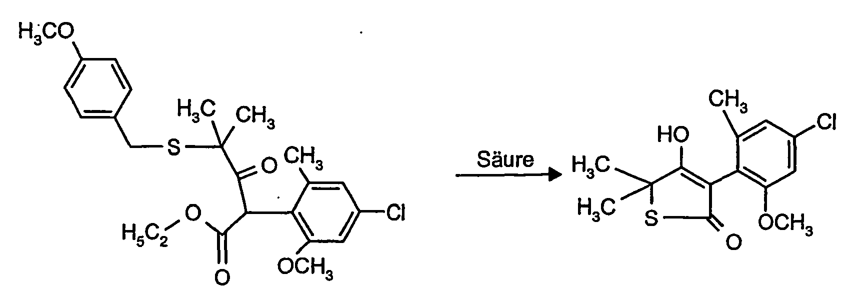

Verwendet man beispielsweise gemäß Verfahren (D) (Chlorcarbonyl)-2-[(2-ethyl-4-chlor-6-methoxy)-phenyl)]-keten und Aceton als Ausgangsverbindungen, so kann der Verlauf des erfindungsgemäßen Verfahrens durch das folgende Reaktionsschema wiedergegeben werden:

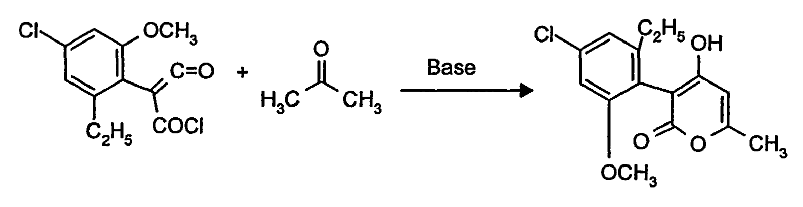

Verwendet man beispielsweise gemäß Verfahren (E) (Chlorcarbonyl)-2-(2-ethyl-4-chlor-6-methoxy-phenyl)-keten und Thiobenzamid als Ausgangsverbindungen, so kann der Verlauf des erfindungsgemäßen Verfahrens durch das folgende Reaktionsschema wiedergegeben werden:

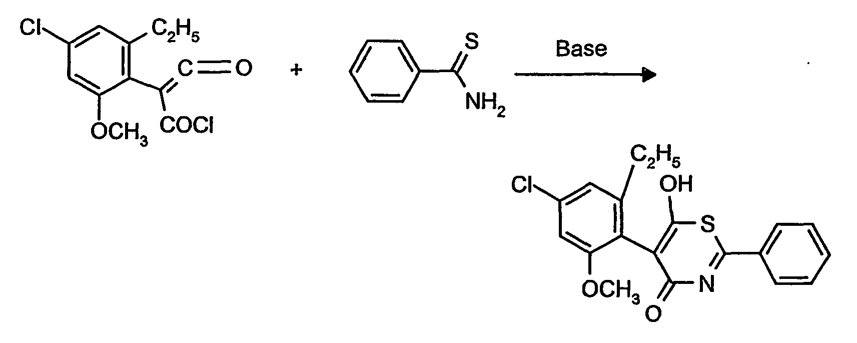

Verwendet man beispielsweise gemäß Verfahren (F) 5-(2-Ethyl-4-chlor-6-methoxy-phenyl)-2,3-trimethylen-4-oxo-valeriansäureethylester, so kann der Verlauf des erfindungsgemäßen Verfahrens durch folgendes Reaktionsschema wiedergegeben werden:

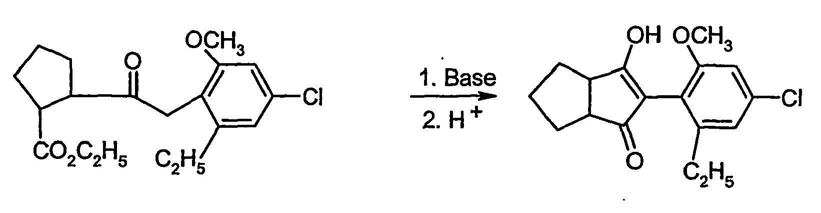

Verwendet man beispielsweise gemäß Verfahren (G) 5-[(2-Ethyl-4-chlor-6-methoxy)-phenyl]-2-methyl-5-oxo-hexansäure-ethylester, so kann der Verlauf des erfindungsgemäßen Verfahrens durch folgendes Reaktionsschema wiedergegeben werden:

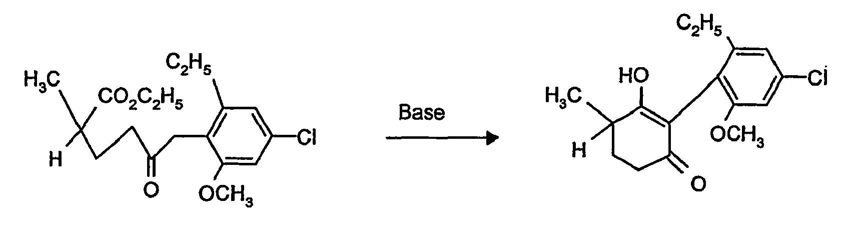

Verwendet man beispielsweise gemäß Verfahren (Hα) Hexahydropyridazin und (Chlorcarbonyl)-2-[(2-ethyl-4-chlor-6-methoxy)-phenyl]-keten als Ausgangsverbindungen, so kann der Reaktionsverlauf des erfindungsgemäßen Verfahrens durch folgendes Reaktionsschema wiedergegeben werden:

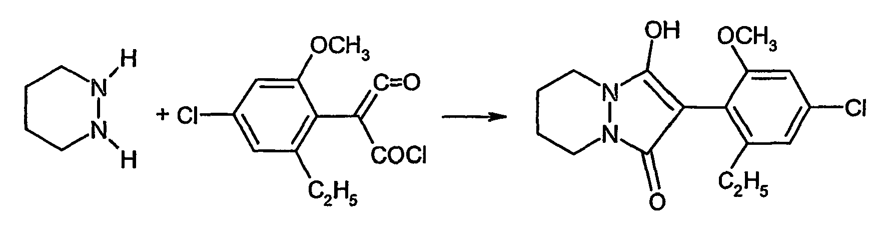

Verwendet man beispielsweise gemäß Verfahren (Hβ) Hexahydropyridazin und (2-Ethyl-4-chlor-6-methoxy)-phenylmalonsäuredimethylester als Ausgangsprodukte, so kann der Verlauf des erfindungsgemäßen Verfahrens durch folgendes Reaktionsschema wiedergegeben werden:

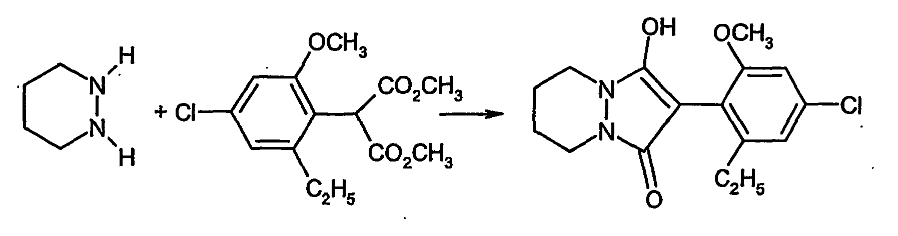

Verwendet man beispielsweise gemäß V erfahren (Hγ) 1-Ethoxycarbonyl-2-[(2-methyl-4-brom-6-methoxy)-phenylacetyl]-hexahydropyridazin als Ausgangsprodukt, so kann der Reaktionsverlauf durch folgendes Schema wiedergegeben werden:

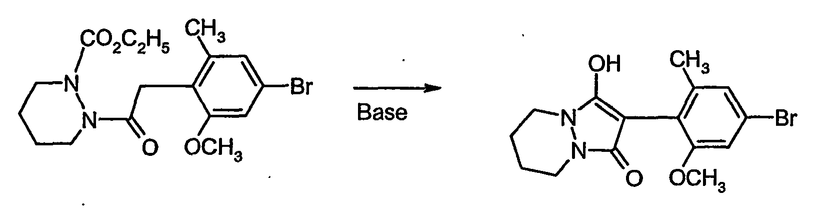

Verwendet man beispielsweise gemäß Verfahren (Iα) 3-(2-Methyl-4-chlor-6-methoxy-phenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

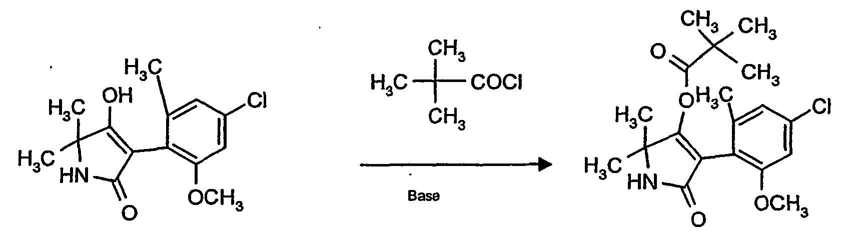

Verwendet man beispielsweise gemäß Verfahren (Iβ) 3-(2-Ethyl-4-chlor-6-methoxy-phenyl)-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

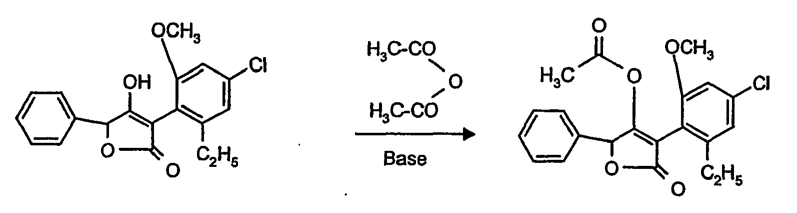

Verwendet man beispielsweise gemäß Verfahren (J) 8-[(2-Ethyl-4-chlor-6-methoxy)-phenyl]-1-aza-bicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

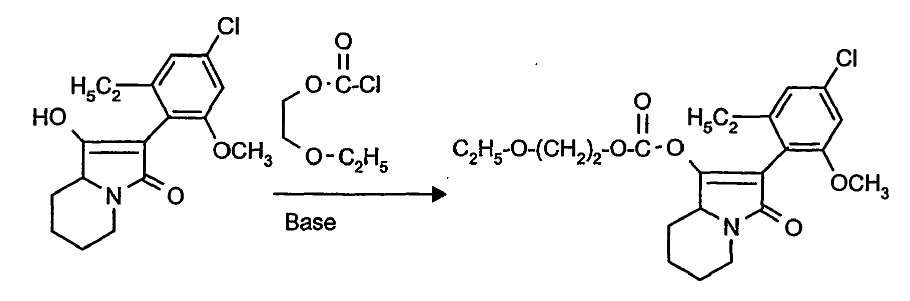

Verwendet man beispielsweise gemäß Verfahren (K), 3-(2-Ethyl-4-chlor-6-methoxy-phenyl)-4-hydroxy-5-methyl-6-(3-pyridyl)-pyron und Chlormonothioameisensäuremethylester als Ausgangsprodukte, so kann der Reaktionsverlauf folgendermaßen wiedergegeben werden:

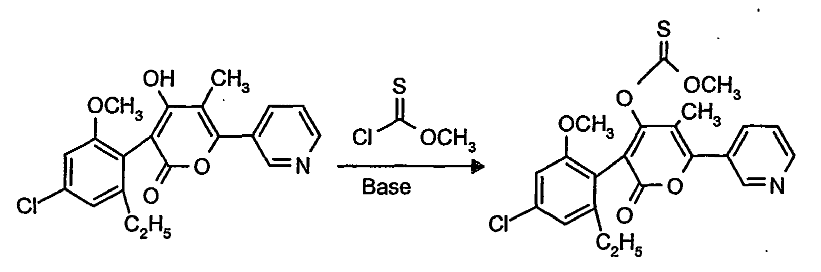

Verwendet man beispielsweise gemäß Verfahren (L) 3-(2-Methyl-4-chlor-6-methoxy-phenyl)-5,5-pentamethylen-pyrrolidin-2,4-dion und Methansulfonsäurechlorid als Ausgangsprodukt, so kann der Reaktionsverlauf durch folgendes Reaktionsschema wiedergegeben werden:

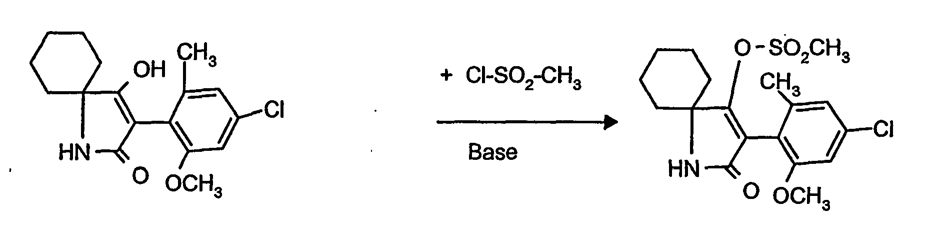

Verwendet man beispielsweise gemäß Verfahren (M) 3-(2-Ethyl-4-chlor-6-methoxy-phenyl)-4-hydroxy-5,5-dimethyl-Δ³-dihydrofuran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

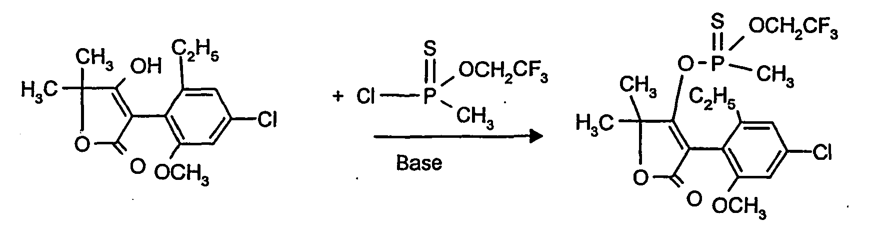

Verwendet man beispielsweise gemäß Verfahren (N) 3-(2-Ehyl-4-chlor-6-methoxy-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

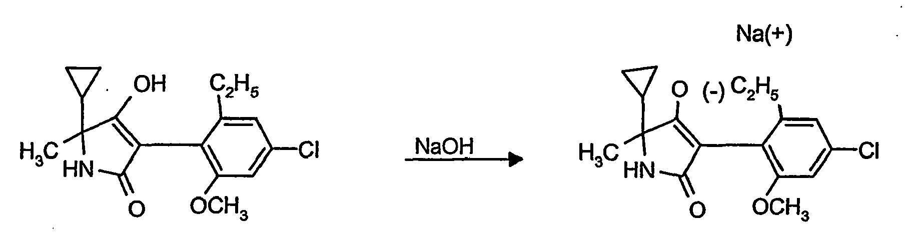

Verwendet man beispielsweise gemäß Verfahren (O) Variante α 3-(2-Ethyl-4-chlor-6-methoxyphenyl)-4-hydroxy-5-tetramethylen-Δ³-dihydro-furan-2-on und Ethylisocyanat als Ausgangsprodukte, so kann der Reaktionsverlauf durch folgendes Reaktionsschema wiedergegeben werden:

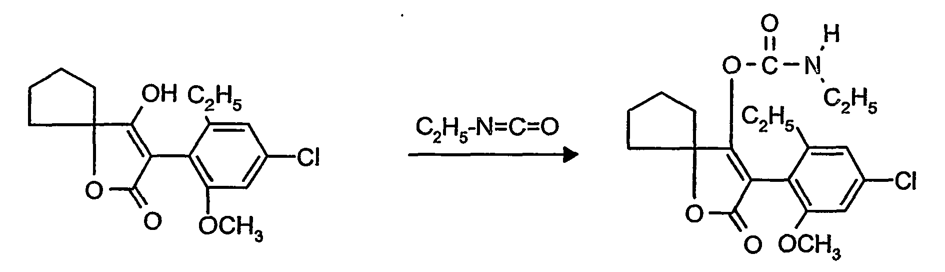

Verwendet man beispielsweise gemäß Verfahren (O) Variante β 3-(2-Methyl-4-chlor-6-methoxyphenyl)-5-methyl-pyrrolidin-2,4-dion und Dimethylcarbamidsäurechlorid als Ausgangsprodukte, so kann der Reaktionsverlauf durch folgendes Schema wiedergegeben werden:

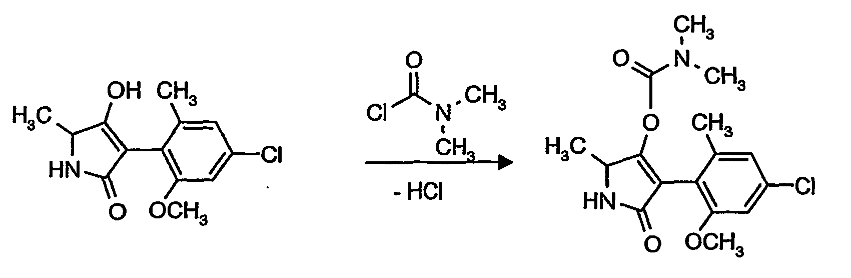

Verwendet man beispielsweise gemäß Verfahren (P) 3-(2-Brom-4-chlor-6-ethyl-phenyl)-5,5-dimethyl-pyrrolidin-2,4-dion und Natriummethylat als Ausgangsstoffe, so kann der Reaktionsverlauf durch folgendes Schema wiedergegeben werden:

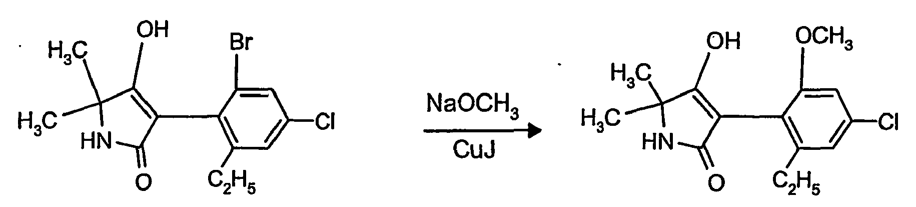

Die beim erfindungsgemäßen Verfahren (a) als Ausgangsstoffe benötigten Verbindungen der Formel (II) 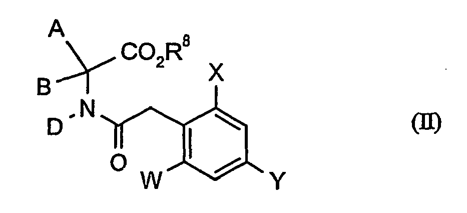 in welcher
- A, B, D, W, X, Y und R⁸: die oben angegebenen Bedeutungen haben,
sind neu.

Man erhält die Acylaminosäureester der Formel (II) beispielsweise, wenn man Aminosäurederivate der Formel (XXIII) 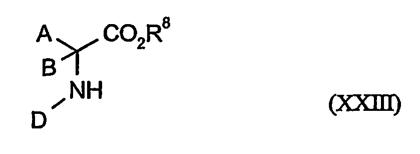 in welcher
A, B, R⁸ und D die oben angegebenen Bedeutungen haben,
mit substituierten Phenylessigsäurederivaten Formel (XXIV) 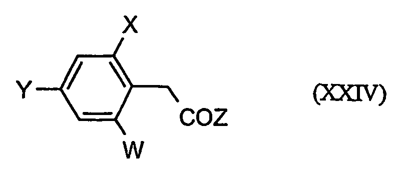 in welcher
- W, X und Y: die oben angegebenen Bedeutungen haben und
- Z: für eine durch Carbonsäureaktivierungsreagenzien wie Carbonyldiimidazol, Carbonyl- diimide (wie z.B. Dicyclohexylcarbondiimid), Phosphorylierungsreagenzen (wie z.B. POCl₃, BOP-Cl), Halogenierungsmittel z.B. Thionylchlorid, Oxalylchlorid, Phosgen oder Chlorameisensäsureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XXV) 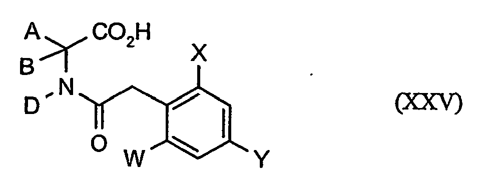 in welcher
- A, B, D, W, X und Y: die oben angegebenen Bedeutungen haben,
verestert (Chem. Ind. (London) 1568 (1968)).

Die Verbindungen der Formel (XXV) 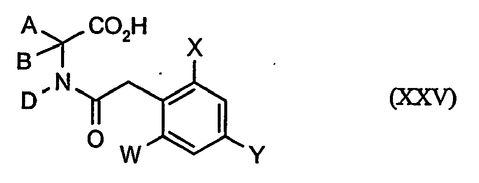 in welcher
- A, B, D, W, X und Y: die oben angegebenen Bedeutungen haben,
sind neu.

Man erhält die Verbindungen der Formel (XXV), wenn man Aminosäuren der Formel (XXVI) 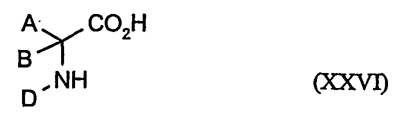 in welcher
- A, B und D: die oben angegebenen Bedeutungen haben,
mit substituierten Phenylessigsäurederivaten der Formel (XXIV) 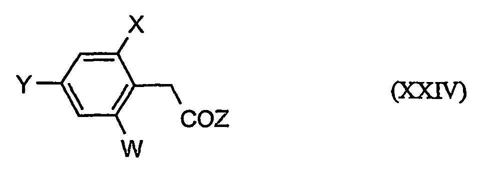 in welcher
- W, X und Y: die oben angegebenen Bedeutungen haben und
- Z: die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XXIV) sind neu. Sie lassen sich nach im Prinzip bekannten Verfahren und wie aus den Beispielen ersichtlich darstellen (s. z.B. H. Henecka, Houben-Weyl, Methoden der Organischen Chemie, Bd. 8, S. 467-469 (1952)).

Man erhält die Verbindungen der Formel (XXIV) beispielsweise, indem man substituierte Phenylessigsäuren der Formel (XXVII) 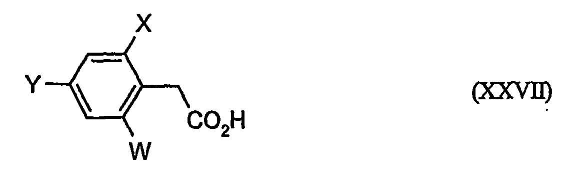 in welcher
- W, X und Y: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphonylierungsreagenzien wie (z.B. POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von -10°C bis 100°C, umsetzt.

Die Verbindungen der Formel (XXIII) und (XXVI) sind teilweise bekannt und/oder lassen sich nach bekannten Verfahren darstellen (siehe z.B. Compagnon, Miocque Ann. Chim. (Paris) [14] 5, S. 11-22,23-27 (1970)).

Die substituierten cyclischen Aminocarbonsäuren der Formel (XXVI), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 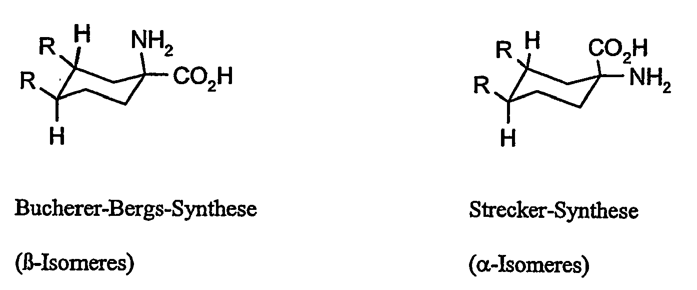 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 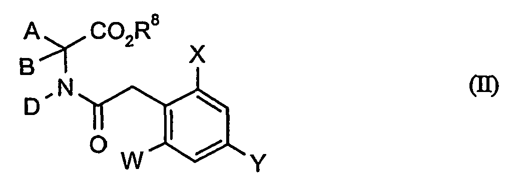 in welcher
- A, B, D, W, X, Y und R⁸: die oben angegebenen Bedeutungen haben,
herstellen, wenn man Aminonitrile der Formel (XXVIII) 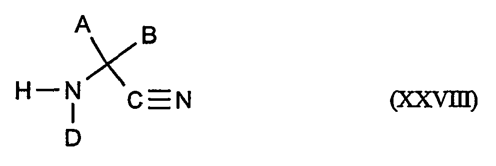 in welcher
- A, B und D: die oben angegebenen Bedeutungen haben,
mit substituierten Phenylessigsäurederivaten der Formel (XXIV) 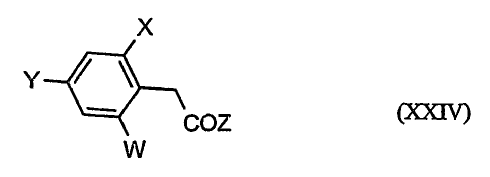 in welcher
- W, X, Y und Z: die oben angegebenen Bedeutungen haben,
zu Verbindungen der Formel (XXIX) 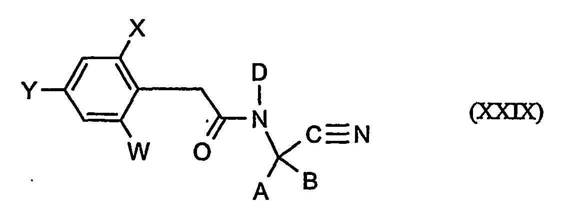 in welcher
- A, B, D, W, X und Y: die oben angegebenen Bedeutungen haben,
umsetzt,
und diese anschließend einer sauren Alkoholyse unterwirft.

Die Verbindungen der Formel (XXIX) sind ebenfalls neu.

Die b ei dem erfindungsgemäßen V erfahren (B) als Ausgangstoffe benötigten Verbindungen der Formel (III) 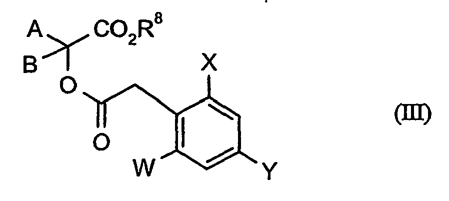 in welcher
- A, B, W, X, Y und R⁸: die oben angegebenen Bedeutungen haben,
sind neu.

Sie lassen sich nach im Prinzip bekannten Methoden herstellen.

So erhält man die Verbindungen der Formel (III) beispielsweise, wenn man
2-Hydroxycarbonsäureester der Formel (XXX-A) 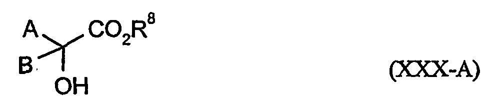 in welcher
- A, B und R⁸: die oben angegebenen Bedeutungen haben,
mit substituierten Phenylessigsäurederivaten der Formel (XXIV) 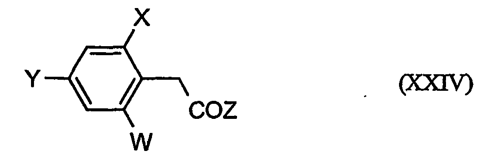 in welcher
- W, X und Y: die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXVII) 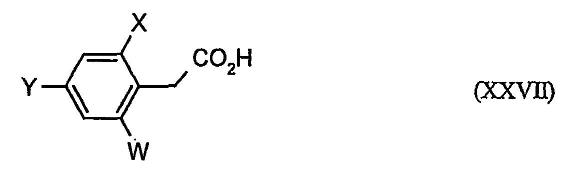 in welcher
- W, X und Y: die oben angegebenen Bedeutungen haben,
mit α-Halogencarbonsäureestern der Formel (XXX-B) 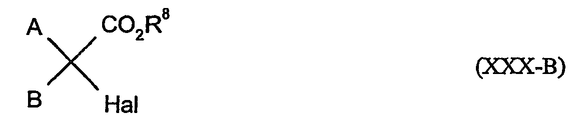 in welcher
- A, B und R⁸: die oben angegebenen Bedeutungen haben und
- Hal: für Chlor oder Brom steht,
alkyliert.

Die Verbindungen der Formel (XXVII) sind neu.

Die Verbindungen der Formel (XXX-B) sind käuflich.

Beispielsweise erhält man die Verbindungen der Formel (XXVII), 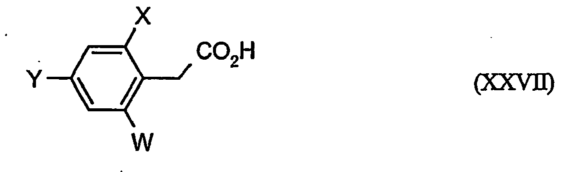 in welcher
- W, X und Y: die oben angegebenen Bedeutungen haben,
wenn man Phenylessigsäureester der Formel (XXXI) 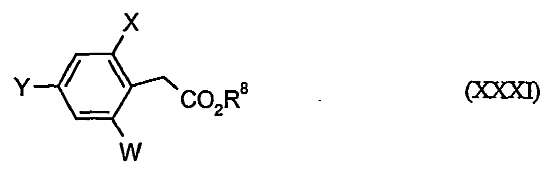 in welcher
- W, X, Y und R⁸: die oben angegebene Bedeutung haben,
in Gegenwart von Säuren oder Basen, in Gegenwart eines Lösungsmittels unter allgemein bekannten Standardbedingungen verseift. Weiterhin erhält man Phenylessigsäuren der Formel (XXVII) nach Verfahren (Q).

Die Verbindungen der Formel (XXXI) sind neu.

Die Verbindungen der Formel (XXXI) 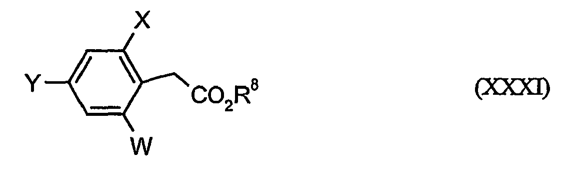 in welcher
- W, X Y und R⁸: die oben angegebene Bedeutung haben,
erhält man beispielsweise nach dem in den Beispielen beschriebenen Verfahren (R),
wenn man Phenylessigsäureester der Formel (XXXI-a) 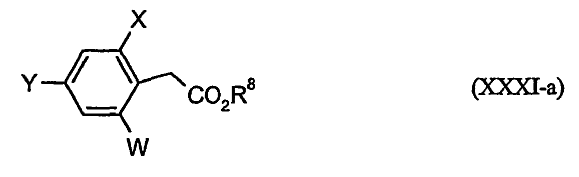 in welcher
- R⁸, X und Y: die oben angegebene Bedeutung haben, und
- W: für Halogen (insbesondere für Brom) steht,
in Gegenwart eines Alkohols, in Gegenwart einer Base und gegebenenfalls in Gegenwart eines Katalysators (bevorzugt Kupfersalze wie z.B. Kupfer(I)bromid) umsetzt.

Die Phenylessigsäureester der Formel (XXXI-a) sind prinzipiell aus der Anmeldung WO 96/35 664 und DE-A-10 301 804 bekannt und lassen sich nach den dort beschriebenen Verfahren herstellen.

Weiterhin erhält man Phenylessigsäureester der Formel (XXXI) nach dem weiter hinten beschriebenen Verfahren (Q), in dem man die dort erhaltenen Phenylessigsäuren der Formel (XXVII) nach Standardmethoden verestert.

Die bei dem obigen Verfahren (C) als Ausgangsstoffe benötigten Verbindungen der Formel (IV) 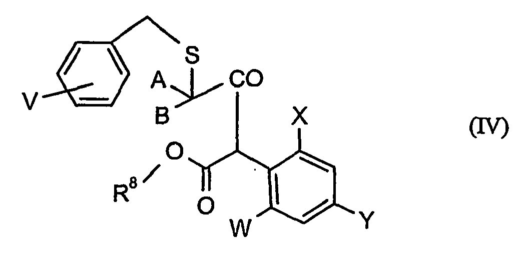 in welcher
- A, B, V, W, X, Yund R⁸: die oben angegebenen Bedeutungen haben,
sind neu.

Sie lassen sich nach im Prinzip bekannten Methoden herstellen.

Man erhält die Verbindungen der Formel (IV) beispielsweise, wenn man
substituierte Phenylessigsäureester der Formel (XXXI) 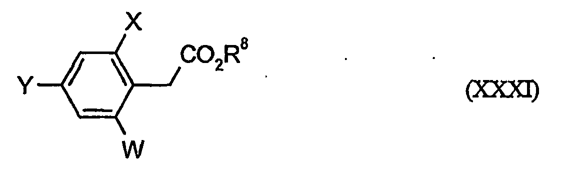 in welcher
- W, X, Y und R⁸: die oben angegebenen Bedeutungen haben,
mit 2-Benzylthio-carbonsäurehalogeniden der Formel (XXXII) 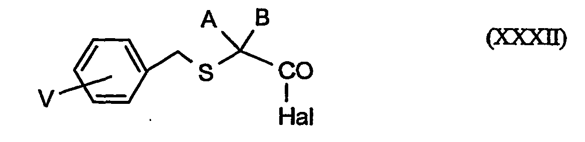 in welcher
- A, B und V: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsäurehalogenide der Formel (XXXII) sind teilweise bekannt und/oder lassen sich nach bekannten Verfahren herstellen (J. Antibiotics (1983), 26, 1589).

Die bei den obigen Verfahren (D), (E) und (H-α) als Ausgangsstoffe benötigten Halogencarbonylketene der Formel (VI) sind neu. Sie lassen sich nach im Prinzip bekannten Methoden herstellen (vgl. beispielsweise Org. Prep. Proced. Int., 7, (4), 155-158, 1975 und DE 1 945 703). So erhält man z.B. die Verbindungen der Formel (VI) 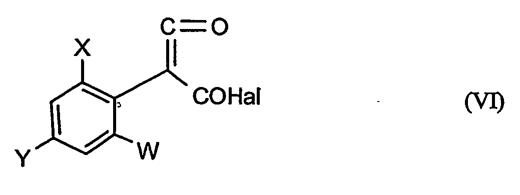 in welcher
- W, X und Y: die oben angegebenen Bedeutungen haben und
- Hal: für Chlor oder Brom steht,
wenn man
substituierte Phenylmalonsäuren der Formel (XXXIII) 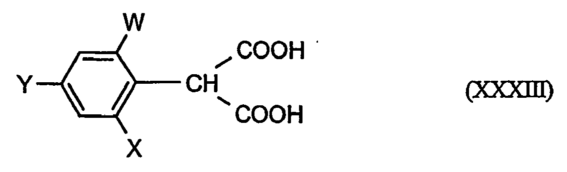 in welcher
- W, X und Y: die oben angegebenen Bedeutungen haben,
mit Säurehalogeniden, wie beispielsweise Thionylchlorid, Phosphor(V)chlorid, Phosphor(III)-chlorid, Oxalylchlorid, Phosgen oder Thionylbromid gegebenenfalls in Gegenwart von Katalysatoren, wie beispielsweise Dimethylformamid, Methyl-Sterylformamid oder Triphenylphosphin und gegebenenfalls in Gegenwart von Basen wie z.B. Pyridin oder Triethylamin, umsetzt.

Die substituierten Phenylmalonsäuren der Formel (XXXIII) sind neu. Sie lassen sich in einfacher Weise nach bekannten Verfahren herstellen (vgl. z.B. Organikum, VEB Deutscher Verlag der Wissenschaften, Berlin 1977, S. 517 ff, EP-A-528 156, WO 96/35 664, WO 97/02 243, WO 97/01535, WO 97/36868 und WO 98/05638).

So erhält man Phenylmalonsäuren der Formel (XXXIII) 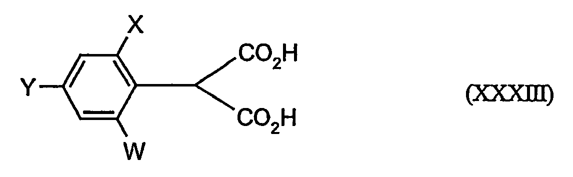 in welcher
- W, X und Y: die oben angegebenen Bedeutungen haben,
wenn man Phenylmalonsäureester der Formel (XI) 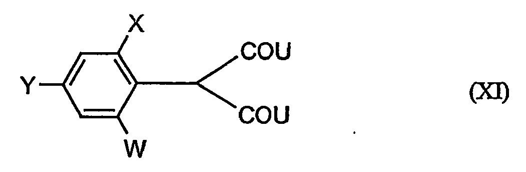 in welcher
- W, X und Y: die oben angegebene Bedeutung haben,
- und U: für OR⁸ oder NH₂ steht,
wobei R⁸ die oben angegebene Bedeutung hat,
zunächst in Gegenwart einer Base und einem Lösungsmittel verseift und anschließend vorsichtig ansäuert (EP-A-528 156, WO 96/35 664, WO 97/02 243).

Die Malonsäureester der Formel (XI) 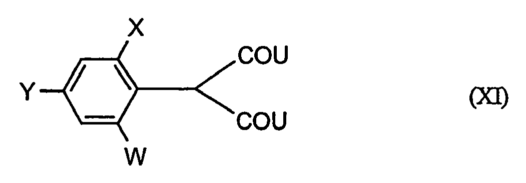 in welcher
- W, X und Y: die oben angegebene Bedeutung haben,
- und U: für OR⁸ oder NH₂ steht,
wobei R⁸ die oben angegebene Bedeutung hat
sind neu.

Sie lassen sich nach allgemein bekannten Methoden der Organischen Chemie darstellen (vgl. z.B. Tetrahedron Lett. 27, 2763 (1986), Organikum VEB Deutscher Verlag der Wissenschaften, Berlin 1977, S. 587 ff., WO 96/35664, WO 97/02243, WO 97/01535, WO 97/36868, WO 98/05638 und WO 99/47525).

Die für das erfindungsgemäße Verfahren (D) als Ausgangsstoffe benötigten Carbonylverbindungen der Formel (V) 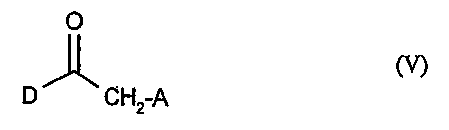 in welcher
- A und D: die oben angegebenen Bedeutungen haben,
oder deren Silylenolether der Formel (Va) 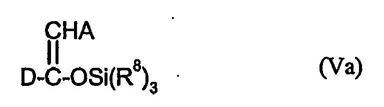 in welcher
- A, D und R⁸: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (E) als Ausgangsstoffe benötigten Ketensäurechloride der Formel (VI) wurden bereits oben beschrieben. Die zur Durchführung des erfindungsgemäßen Verfahrens (E) benötigten Thioamide der Formel (VII) 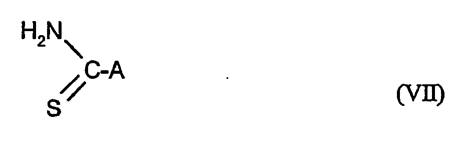 in welcher
- A: die oben angegebene Bedeutung hat,
sind allgemein in der Organischen Chemie bekannte Verbindungen.

Die bei dem obigen Verfahren (F) als Ausgangsstoffe benötigten Verbindungen der Formel (VIII) 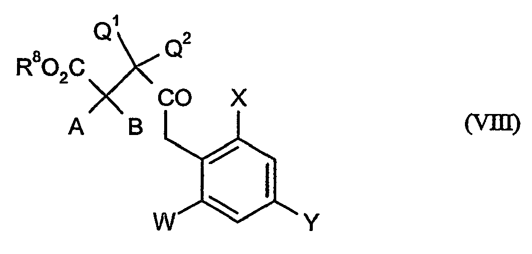 in welcher
A, B, Q¹, Q², W, X, Y und R⁸ die oben angegebene Bedeutung haben,
sind neu.

Sie lassen sich nach im Prinzip bekannten Methoden herstellen.

Man erhält die 5-Aryl-4-ketocarbonsäureester der Formel (VIII) beispielsweise, wenn man 5-Aryl-4-ketocarbonsäuren der Formel (XXXIV) 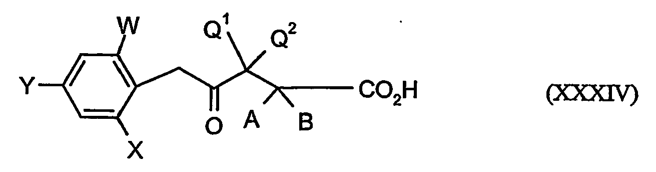 in welcher
- W, X, Y, A, B, Q¹ und Q²: die oben angegebene Bedeutung haben,
verestert (vgl. z.B. Organikum, 15. Auflage, Berlin, 1977, Seite 499) oder alkyliert (siehe Herstellungsbeispiel).

Die 5-Aryl-4-ketocarbonsäuren der Formel (XXXIV) 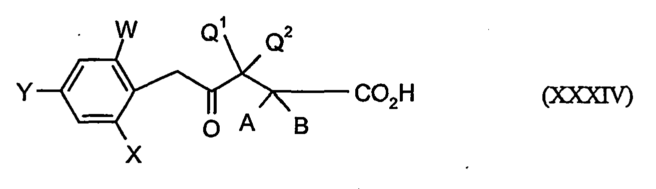 in welcher
A, B, Q¹, Q², W, X und Y die oben angegebene Bedeutung haben,
sind neu, lassen sich aber nach im Prinzip bekannten Methoden herstellen (WO 96/01 798, WO 97/14667, WO 98/39281).

Man erhält die 5-Aryl-4-ketocarbonsäuren der Formel (XXXIV) beispielsweise, wenn man 2-Phenyl-3-oxo-adipinsäureester der Formel (XXXV) 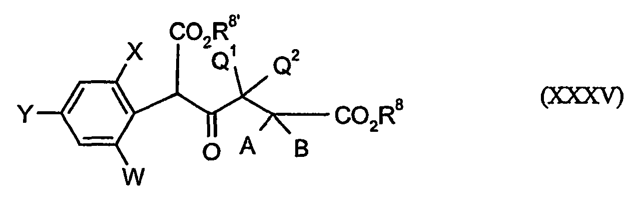 in welcher
- A, B, Q¹, Q², W, X und Y: die oben angegebene Bedeutung haben und
- R⁸ und R^{8'}: fur Alkyl (insbesondere C₁-C₈-Alkyl) stehen und
bei Einsatz der Verbindung der Formel (XXXVII-a) R⁸ für Wasserstoff steht,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XXXV) 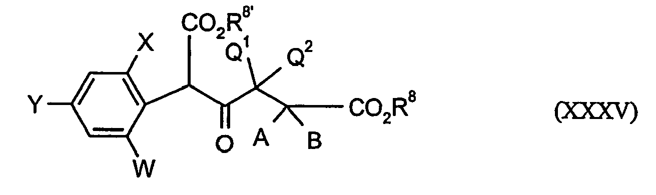 in welcher
A, B, Q¹, Q², W, X, Y, R⁸, R^{8'} die oben angegebene Bedeutung haben und
bei Einsatz der Verbindung der Formel (XXXVII-a) R⁸ für Wasserstoff steht
sind neu.

Man erhält die Verbindungen der Formel (XXXV) beispielsweise,
wenn man Dicarbonsäurehalbesterchloride der Formel (XXXVI), 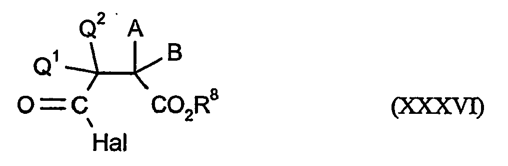 in welcher
- A, B, Q¹, Q² und R⁸: die oben angegebene Bedeutung haben und
- Hal: für Chlor oder Brom steht,
oder Carbonsäureanhydride der Formel (XXXVII-a) 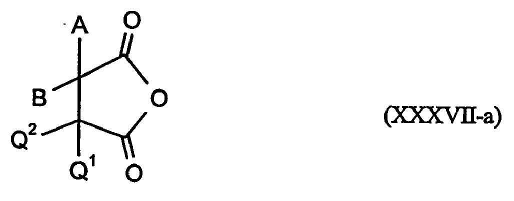 in welcher
A, B, Q¹ und Q² die oben angegebene Bedeutung haben,
mit einem Phenylessigsäureester der Formel (XXXI) 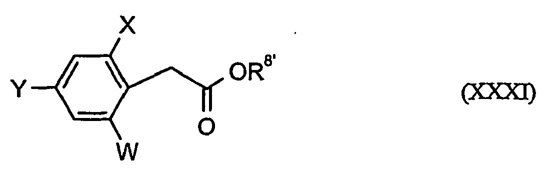 in welcher
- W, X, Y und R^{8'}: die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XXXVI) und (XXXVII-a) sind teilweise bekannte Verbindungen der Organischen Chemie und/oder lassen sich nach im Prinzip bekannten Methoden in einfacher Weise herstellen.

Die bei dem obigen Verfahren (G) als Ausgangsstoffe benötigten Verbindungen der Formel (IX) 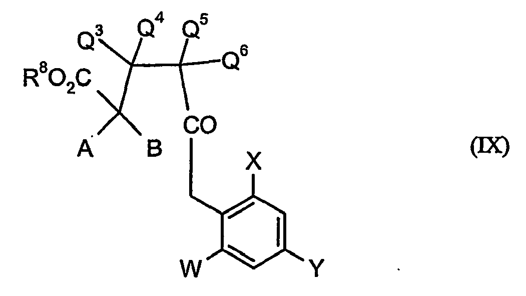 in welcher
A, B, Q³, Q⁴, Q⁵, Q⁶, W, X, Y und R⁸ die oben angegebene Bedeutung haben,
sind neu.

Sie lassen sich nach im Prinzip bekannten Methoden herstellen.

Man erhält die 6-Aryl-5-ketocarbonsäureester der Formel (IX) beispielsweise, wenn man 6-Aryl-5-ketocarbonsäuren der Formel (XXXVIII) 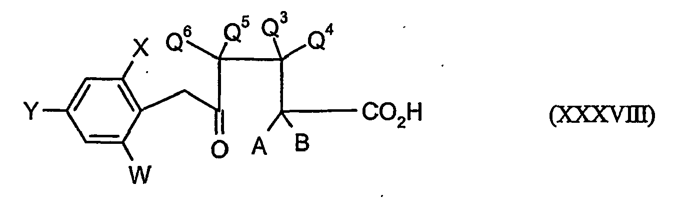 in welcher
- A, B, Q³, Q⁴, Q⁵, Q⁶, W, X und Y: die oben angegebene Bedeutung haben,
verestert, (vgl. z.B. Organikum, 15. Auflage, Berlin, 1977, Seite 499).

Die 6-Aryl-5-ketocarbonsäuren der Formel (XXXVIII) 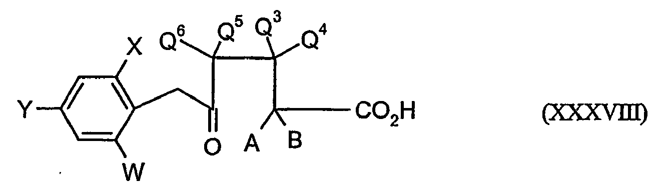 in welcher
- A, B, Q³, Q⁴, Q⁵, Q⁶, W, X und Y: die oben angegebene Bedeutung haben,
sind neu. Sie lassen sich nach im Prinzip bekannten Methoden herstellen (WO 99/43649, WO 99/48869), beispielsweise wenn man
substituierte 2-Phenyl-3-oxo-heptandisäureester der Formel (XXXIX) 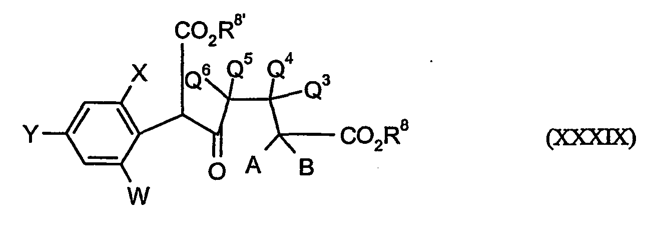 in welcher
- A, B, Q³, Q⁴, Q⁵, Q⁶, W, X und Y: die oben angegebene Bedeutung haben und
- R⁸ und R^{8'}: für Alkyl (bevorzugt C₁-C₆-Alkyl), stehen, und
bei Einsatz der Verbindung der Formel (XXXVII-b) R⁸ für Wasserstoff steht,
gegebenenfalls in Gegenwart eines Verdünnungsmittels und gegebenenfalls in Gegenwart einer Base oder Säure verseift und decarboxyliert (vgl. z.B. Organikum, 15. Auflage, Berlin, 1977, Seite 519 bis 521).

Die Verbindungen der Formel (XXXIX) 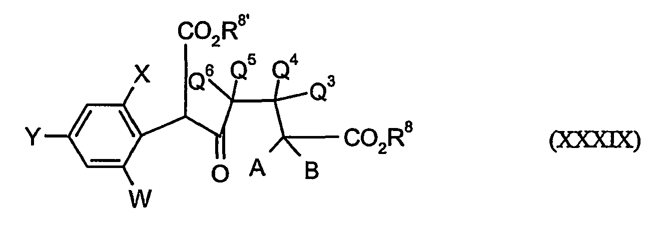 in welcher
A, B, Q³, Q⁴, Q⁵, Q⁶, W, X, Y, R⁸ und R^{8'} die oben angegebene Bedeutung haben,
sind neu und erhältlich,
wenn man Dicarbonsäureester der Formel (XL), 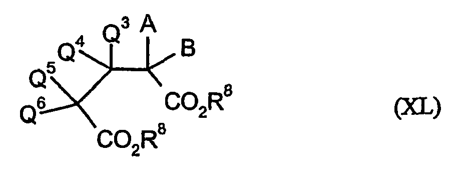 in welcher
- A, B, Q³, Q⁴, Q⁵, Q⁶ und R⁸: die oben angegebene Bedeutung haben,
oder Carbonsäureanhydride der Formel (XXXVII-b) 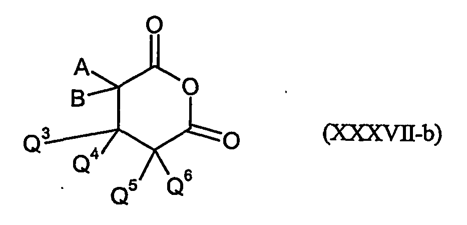
- in welcher A, B, Q³, Q⁴, Q⁵, Q⁶: die oben angegebene Bedeutung haben
mit einem substituierten Phenylessigsäureester der Formel (XXXI) 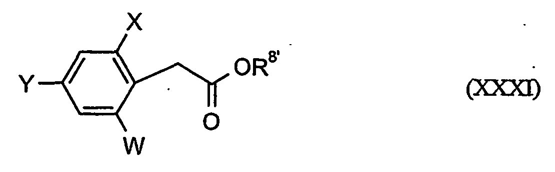 in welcher
W, X, Y und R^{8'} die oben angegebene Bedeutung haben,
in Gegenwart eines Verdünnungsmittels und in Gegenwart einer Base kondensiert.

Die Verbindungen der Formel (XL) sind teilweise bekannt und/oder lassen sich nach bekannten Verfahren darstellen.

Die für das erfindungsgemäße Verfahren (H-α) und (H-ß) als Ausgangsstoffe benötigten Hydrazine der Formel (X)

A-NH-NH-D (X)

in welcher
- A und D: die oben angegebenen Bedeutungen haben,
sind teilweise bekannt und/oder nach literaturbekannten Methoden herstellbar (vgl. beispielsweise Liebigs Ann. Chem. 585, 6 (1954); Reaktionen der organischen Synthese, C. Ferri, Seite 212, 513; Georg Thieme Verlag Stuttgart, 1978; Liebigs Ann. Chem. 443, 242 (1925); Chem. Ber. 98, 2551 (1965), EP-A-508 126, WO 92/16510, WO 99/47 525, WO 01/17 972).

Die für das erfindungsgemäße Verfahren (H-γ) benötigten Verbindungen der Formel (XII) 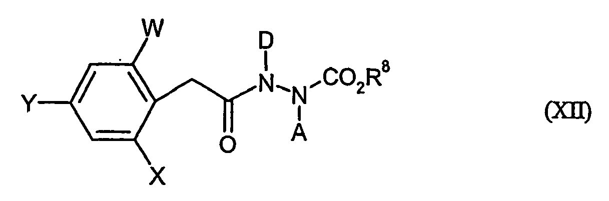 in welcher
A, D, W, X, Y und R⁸ die oben angegebene Bedeutung haben,
sind neu.

Man erhält die Acylcarbazate der Formel (XII) beispielsweise, wenn man Carbazate der Formel (XLI) 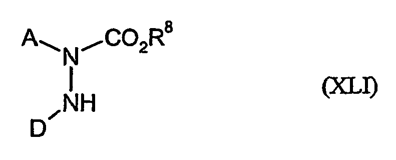 in welcher
- A, R⁸ und D: die oben angegebenen Bedeutungen haben,
mit substituierten Phenylessigsäurederivaten der Formel (XXIV) 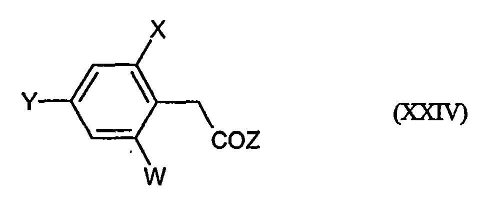 in welcher
W, X, Y und Z die oben angegebenen Bedeutungen haben
acyliert (Chem. Reviews 52, 237-416 (1953); Bhattacharya, Indian J. Chem. 6, 341-5, 1968).

Die Carbazate der Formel (XLI) sind teilweise käufliche und teilweise bekannte Verbindungen oder lassen sich nach im Prinzip bekannten Verfahren der organischen Chemie herstellen.

Die Verbindungen der Formel (XXIV) wurden bereits bei den Vorstufen für das Verfahren (A) und (B) beschrieben.
(Q) Man erhält weiterhin Phenylessigsäuren der Formel (XXVII), 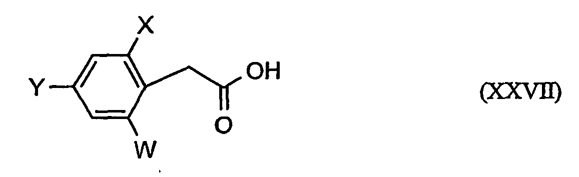 in welcher
   - W, X und Y: die oben angegebene Bedeutung haben,
wenn man Phenylacetaldehyde der Formel (XLII) 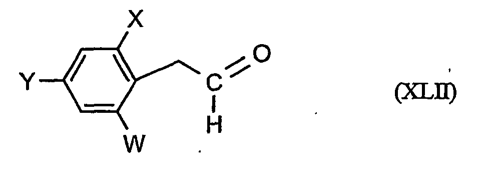 in welcher
- W, X und Y: die oben angegebene Bedeutung haben,
gegebenenfalls in Gegenwart eines Lösungsmittels mit geeigneten Oxidationsmitteln (wie z.B. NaOCl) oxidiert.

Die Verbindungen der Formel (XLII) sind neu.

Man erhält Verbindungen der Formel (XLII) 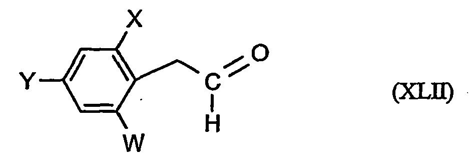 in welcher
- W, X und Y: die oben angegebene Bedeutung haben,
wenn man 3-Phenylpropene der Formel (XLIII) 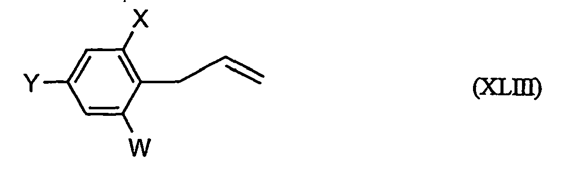 in welcher
- W, X und Y: die oben angegebene Bedeutung haben
in Gegenwart eines Lösungsmittels ozonolysiert und das erhaltene Ozonid beispielsweise mit Dimethylsulfid reduktiv aufarbeitet.

Die zur Herstellung der Verbindungen der Formel (XLII) benötigten 2-Alkoxy-substituierte 3-Phenyl-propene der Formel (XLIII) sind im Prinzip bekannte Verbindungen in der organischen Chemie und lassen sich nach Standardverfahren durch Alkylierung von Phenolen mit Allylhalogeniden, gefolgt von einer Claisen-Umlagerung und anschließender Alkylierung herstellen (WO 96/25 395).

Die zur Durchführung der erfindungsgemäßen Verfahren (I), (J), (K), (L), (M), (N) und (O) außerdem als Ausgangsstoffe benötigten Säurehalogenide der Formel (XIII), Carbonsäureanhydride der Formel (XIV), C hlorameisensäureester oder C hlorameisensäurethioester d er Formel (XV), Chlormonothioameisensäureester oder Chlordithioameisensäureester der Formel (XVI), Sulfonsäurechloride der Formel (XVII), Phosphorverbindungen der Formel (XVIII) und Metallhydroxide, Metallalkoxide oder Amine der Formel (XIX) und (XX) und Isocyanate der Formel (XXI) und Carbamidsäurechloride der Formel (XXII) sind allgemein bekannte Verbindungen der Organischen bzw. Anorganischen Chemie:

Die Verbindungen der Formeln (V), (VII), (XIII) bis (XXII), (XXIII), (XXVI), (XXVIII), (XXX-A), (XXX-B), (XXXII), (XXXVI), (XXXVII-a), (XXXVII-b), (XL) und (XLI) sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, D, W, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass Verbindungen der Formel (III), in welcher A, B, W, X, Y und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formel (III) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (C) ist dadurch gekennzeichnet, dass man Verbindungen der Formel (IV) in welcher A, B, V, W, X, Y und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (C) wird im Allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure als Lösungsmittel oder als Katalysator zu verwenden.

Das erfindungsgemäße Verfahren (D) ist dadurch gekennzeichnet, dass man Carbonylverbindungen der Formel (V) oder deren Enolether der Formel (V-a) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (D) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Glykoldimethylether Diglykoldimethylether und Diphenylether, außerdem polare Lösungsmittel, wie Dimethylsulfoxid, Sulfolan, Dimethylformamid oder N-Methyl-pyrrolidon.

Als Säureakzeptoren können bei der Durchführung der erfindungsgemäßen Verfahrensvariante (D) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (D) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (D) wird zweckmäßigerweise unter Normaldruck durchgeführt.

Bei der Durchführung des erfindungsgemäßen Verfahrens (D) setzt man die Reaktionskomponenten der Formeln (V) und (VI), in welchen A, D, W, X und Y die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das erfindungsgemäße Verfahren (E) ist dadurch gekennzeichnet, dass man Thioamide der Formel (VIII) mit Ketensäurehalogeniden der Formel (VI) in Gegenwart eines Verdünnungsmittels und gegebenenfalls in Gegenwart eines Säureakzeptors umsetzt.

Als Verdünnungsmittel können bei der erfindungsgemäßen Verfahrensvariante (E) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VII) und (VI), in welchen A, W, X und Y die oben angegebenen Bedeutungen haben und Hal für Halogen steht und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (F) ist dadurch gekennzeichnet, dass man Verbindungen der Formel (VIII), in welcher A, B, Q¹, Q², W, X, Y und R⁸ die oben angegebene Bedeutung haben, in Gegenwart einer Base einer intramolekularen Kondensation unterwirft.

Als Verdünnungsmittel können bei dem erfindungsgemäßen Verfahren (F) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (F) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (F) innerhalb eines größeren Bereiches variiert werden. Im Allgemeinen arbeitet man bei Temperaturen zwischen -75°C und 250°C, vorzugsweise zwischen -50°C und 150°C.

Das erfindungsgemäße Verfahren (F) wird im Allgemeinen unter Normaldruck durchgeführt.

Bei der Durchführung des erfindungsgemäßen Verfahrens (F) setzt man die Reaktionskomponenten der Formel (VIII) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (G) ist dadurch gekennzeichnet; dass man Verbindungen der Formel (IX), in welcher A, B, Q³, Q⁴, Q⁵, Q⁶, W, X, Y und R⁸ die oben angegebene Bedeutung haben, in Gegenwart von Basen einer intramolekularen Kondensation unterwirft.

Als Verdünnungsmittel können bei dem erfindungsgemäßen Verfahren (G) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (G) alle üblichen Protonenakzeptoren eingesetzt werden.

Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (G) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (G) wird im Allgemeinen unter Normaldruck durchgeführt.

Bei der Durchführung des erfindungsgemäßen Verfahrens (G) setzt man die Reaktionskomponenten der Formel (IX) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das erfmdungsgemäße Verfahren (H-α) ist dadurch gekennzeichnet, dass man Hydrazine der Formel (X) oder Salze dieser Verbindungen mit Ketensäurehalogeniden der Formel (VI) in Gegenwart eines Verdünnungsmittels und gegebenenfalls in Gegenwart eines Säureakzeptors umsetzt.

Als Verdünnungsmittel können bei dem erfindungsgemäßen Verfahren (H-α) alle inerten organischen Solventien eingesetzt werden. Vorzugsweise verwendbar sind gegebenenfalls chlorierte Kohlenwasserstoffe, wie beispielsweise Mesitylen, Chlorbenzol und Dichlorbenzol, Toluol, Xylol, ferner Ether, wie Dibutylether, Glykoldimethylether, Diglykoldimethylether und Diphenylethan, außerdem polare Lösungsmittel, wie Dimethylsulfoxid, Sulfolan, Dimethylformamid oder N-Methyl-pyrrolidon.

Als Säureakzeptoren können bei der Durchführung der erfmdungsgemäßen Verfahrensvariante (H-α) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante (H-α) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (H-α) wird zweckmäßigerweise unter Normaldruck durchgeführt.

Bei der Durchführung des erfindungsgemäßen Verfahrens (H-α) setzt man die Reaktionskomponenten der Formeln (VI) und (X), in welchen A, D, W, X und Y die oben angegebenen Bedeutungen haben und Hal für Halogen steht, und gegebenenfalls die Säureakzeptoren im Allgemeinen in etwa äquimolaren Mengen ein. Es ist jedoch auch möglich, die eine oder andere Komponente in einem größeren Überschuss (bis zu 5 Mol) zu verwenden.

Das Verfahren (H-ß) ist dadurch gekennzeichnet, dass man Hydrazine der Formel (X) oder Salze dieser Verbindung, in welcher A und D die oben angegebenen Bedeutungen haben, mit Malonestern oder Malonsäureamiden der Formel (XI), in welcher U, W, X, Y und R⁸ die oben angegebene Bedeutung haben, in Gegenwart einer Base einer Kondensation unterwirft.

Als Verdünnungsmittel können bei dem erfindungsgemäßen Verfahren (H-ß) alle inerten organischen Solventien eingesetzt werden. Vorzugsweise verwendbar sind gegebenenfalls halogenierte Kohlenwasserstoffe, wie Toluol, Xylol, Mesitylen, Chlorbenzol und Dichlorbenzol, ferner Ether, wie Dibutylether, Tetrahydrofuran, Dioxan, Diphenylether, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-ß) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Verwendbar sind auch tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung des erfindugnsgemäßen Verfahrens (H-ß) innerhalb eines größeren Bereiches variiert werden. Im Allgemeinen arbeitet man bei Temperaturen zwischen 0°C und 280°C, vorzugsweise zwischen 50°C und 180°C.

das erfindungsgemäße Verfahren (H-ß) wird im Allgemeinen unter Normaldruck durchgeführt.

Bei der Durchführung des erfindungsgemäßen Verfahrens (H-ß) setzt man die Reaktionskomponenten der Formeln (XI) und (X) im Allgemeinen in etwa äquimolaren Mengen ein. Es ist jedoch auch möglich, die eine oder andere Komponente in einem größeren Überschuss (bis zu 3 mol) zu verwenden.

Das Verfahren (H-γ) ist dadurch gekennzeichnet, dass man Verbindungen der Formel (XII), in welcher A, D, W, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (H-γ) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie M ethanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (H-γ) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und - hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (H-γ) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (H-γ) wird im Allgemeinen unter Normaldruck durchgeführt.

Bei der Durchführung des erfindungsgemäßen Verfahrens (H-γ) setzt man die Reaktionskomponenten der Formel (XII) und die deprotonierenden Basen im Allgemeinen in etwa doppeltäquimolaren Mengen ein. Es ist jedoch auch möglich, die eine oder andere Komponente in einem größeren Überschuss (bis zu 3 Mol) zu verwenden.

Das Verfahren (I-α) ist dadurch gekennzeichnet, dass man Verbindungen der Formeln (I-1-a) bis (I-8-a) jeweils mit Carbonsäurehalogeniden der Formel (XIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare S olventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (I-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (I-α) innerhalb eines größeren Bereiches variiert w erden. Im Allgemeinen arbeitet man bei Temperaturen zwischen - 20°C und +150°C, vorzugsweise zwischen 0°C und 100°C.

Bei der Durchführung des erfindungsgemäßen Verfahrens (I-α) werden die Ausgangsstoffe der Formeln (I-1-a) bis (I-8-a) und das Carbonsäurehalogenid der Formel (XIII) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (I-ß) ist dadurch gekennzeichnet, dass man Verbindungen der Formeln (I-1-a) bis (I-8-a) mit Carbonsäureanhydriden der Formel (XIV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-ß) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (I-ß) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (I-ß) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (I-ß) werden die Ausgangsstoffe der Formeln (I-1-a) bis (I-8-a) und das Carbonsäureanhydrid der Formel (XIV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (J) ist dadurch gekennzeichnet, dass man Verbindungen d er Formeln (I-1-a) bis (I-a) jeweils mit Chlorameisensäureestern oder Chlorameisensäurethiolestern der Formel (XV) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (J) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (J) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (J) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (J) wird im Allgemeinen unter Normaldruck durchgeführt.

Bei der Durchführung des erfindungsgemäßen Verfahrens (J) werden die Ausgangsstoffe der Formeln (I-1-a) bis (I-8-a) und der entsprechende Chlorameisensäureester bzw. Chlorameisensäurethiolester der Formel (XIII) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (K) ist dadurch gekennzeichnet, dass man Verbindungen der Formeln (I-1-a) bis (I-8-a) jeweils mit Verbindungen der Formel (XVI) in Gegenwart eines Verdünnungsmittels und gegebenenfalls in Gegenwart eines Säurebindemittels umsetzt.

Beim Herstellungsverfahren (K) setzt man pro Mol Ausgangsverbindung der Formeln (I-1-a) bis (I-8-a) ca. 1 Mol Chlormonothioameisensäureester bzw. Chlordithioameisensäureester der Formel (XVI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (L) ist dadurch gekennzeichnet, dass man Verbindungen der Formeln (I-1-a) bis (I-8-a) jeweils mit Sulfonsäurechloriden der Formel (XVII) gegebenenfalls in Gegenwart eines Verdünnungsmittels und gegebenenfalls in Gegenwart eines Säurebindemittels umsetzt.

Beim Herstellungsverfahren (L) setzt man pro Mol Ausgangsverbindung der Formel (I-1-a bis I-8-a) ca. 1 Mol Sulfonsäurechlorid der Formel (XVII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann b ei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (M) ist dadurch gekennzeichnet, dass man Verbindungen der Formeln (I-1-a) bis (I-8-a) jeweils mit Phosphorverbindungen der Formel (XVIII) gegebenenfalls in Gegenwart eines Verdünnungsmittels und gegebenenfalls in Gegenwart eines Säurebindemittels umsetzt.

Beim Herstellungsverfahren (M) setzt man zum Erhalt von Verbindungen der Formeln (I-1-e) bis (I-8-e) auf 1 Mol der Verbindungen (I-1-a) bis (I-8-a), 1 bis 2, vorzugsweise 1 bis 1,3 Mol der Phosphorverbindung der Formel (XVIII) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (N) ist dadurch gekennzeichnet, dass man Verbindungen der Formeln (I-1-a) bis (I-8-a) mit Metallhydroxiden bzw. Metallalkoxiden der Formel (XIX) oder Aminen d er Formel (XX), gegebenenfalls in Gegenwart eines Verdünnungsmittels, umsetzt.

Als Verdünnungsmittel können bei dem erfindungsgemäßen Verfahren (N) vorzugsweise Ether wie Tetrahydrofuran, Dioxan, Diethylether oder aber Alkohole wie Methanol, Ethanol, Iso-propanol, aber auch Wasser eingesetzt werden.

Das erfindungsgemäße Verfahren (N) wird im Allgemeinen unter Normaldruck durchgeführt.

Die Reaktionstemperaturen liegen im Allgemeinen zwischen -20°C und 100°C, vorzugsweise zwischen 0°C und 50°C.

Das erfmdungsgemäße Verfahren (O) ist dadurch gekennzeichnet, dass man Verbindungen der Formeln (I-1-a) bis (I-8-a) jeweils mit (O-α) Verbindungen der Formel (XXI) gegebenenfalls in Gegenwart eines Verdünnungsmittels und gegebenenfalls in Gegenwart eines Katalysators oder (O-ß) mit Verbindungen der Formel (XXII) gegebenenfalls in Gegenwart eines Verdünnungsmittels und gegebenenfalls in Gegenwart eines Säurebindemittels umsetzt.

Bei Herstellungsverfahren (O-α) setzt man pro Mol Ausgangsverbindung der Formeln (I-1-a) bis (I-8-a) ca. 1 Mol Isocyanat der Formel (XXI) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (O-ß) setzt man pro Mol Ausgangsverbindung der Formeln (I-1-a) bis (I-8-a) ca. 1 Mol Carbamidsäurechlorid der Formel (XXII) bei -20 bis 150°C, vorzugsweise bei 0 bis 70°C um.

Als gegebenenfalls zugesetzte Verdünnungsmittel kommen alle inerten polaren organischen Lösungsmittel in Frage wie Ether, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-8-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann b ei Normaldruck oder unter erhöhtem Druck durchgeführt werden, vorzugsweise wird bei Normaldruck gearbeitet. Die Aufarbeitung geschieht nach üblichen Methoden.

Das Verfahren (P) ist dadurch gekennzeichnet, dass man Verbindungen der Formeln (I-1-a') bis (I-8-a'), in welchen A, B, D, Q¹, Q², Q³, Q⁴, Q⁵, Q⁶, X und Y die oben angegebenen Bedeutungen haben und W' bevorzugt für Brom steht, mit Alkoholen der Formel WOH, in welcher W die oben angegebene Bedeutung hat, in Gegenwart einer Base und eines Cu-(I)-Salzes (z.B. CuBr oder CuJ) umsetzt.

Als Verdünnungsmittel können bei dem erfindungsgemäßen Verfahren (P) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, Ester wie Methylacetat, Ethylacetat, Propylacetat sowie Alkohole der Formel WOH wie z.B. Methanol, Ethanol, Propanol, Iso-Propanol, Butanol und Iso-Butanol.

Als Base (Deprotonierungsmittel) können bei der Durchführung des erfindungsgemäßen Verfahrens (P) alle üblichen Protonenakzeptoren eingesetzt werden. Vorzugsweise verwendbar sind Alkalimetalle wie Natrium oder Kalium. Ferner sind Alkalimetall- und Erdalkalimetallamide und -hydride, wie Natriumamid, Natriumhydrid und Calciumhydrid, und bevorzugt auch Alkalimetallalkoholate, wie Natriummethylat, Natriumethylat, Natriumisopropylat, Natrium-tert.-butylat und Kalium-tert.-butylat einsetzbar.

Die Reaktionstemperatur kann bei der Durchführung des erfindungsgemäßen Verfahrens (P) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (P) wird im Allgemeinen unter Normaldruck durchgeführt.

Bei der Durchführung des erfindungsgemäßen Verfahrens (P) setzt man die Reaktionskomponente der Formel (I-1-a') bis (I-8-a') im Allgemeinen mit Überschüssen der Alkohole WOH und der Basen bis zu 20 Mol, bevorzugt 3 bis 5 Mol um. Die Kupfer-I-Salze werden in der Regel katalytisch eingesetzt; 0,001 bis 0,5 Mol, bevorzugt 0,01 bis 0,2 Mol. Es ist jedoch auch möglich diese äquimolar einzusetzen.

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella occidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, P tinus s pp., Niptus hololeucus, Gibbium psylloides, Tribolium spp., T enebrio molitor, A griotes s pp., C onoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus d ipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfmdungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injezieren und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothalisopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]-ethyl]-3-methyl-2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxamide; 3,4,5-tri-chloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
      sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxinecopper.
Bakterizide:
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
Insektizide / Akarizide / Nematizide:
   Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Alpha-Gypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
   Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
   Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, C lothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
   DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimefluthrin, Dimethoate, Dimethylvinphos, Dinobuton, Dinocap, Dinotefuran, Diofenolan, Disulfoton, Docusat-sodium, Dofenapyn, DOWCO-439,
   Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
   Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
   Gamma-Cyhalothrin, Gamma-HCH, Gossyplure, Grandlure, Granuloseviren,
   Halfenprox, Halofenozide, HCH, HCN-801, Heptenophos, Hexaflumuron, Hexythiazox, Hydramethylnone, Hydroprene,
   IKA-2002, Imidacloprid, Imiprothrin, Indoxacarb, Iodofenphos, Iprobenfos, Isazofos, Isofenphos, Isoprocarb, Isoxathion, Ivermectin,
   Japonilure,
   Kadethrin, Kernpolyederviren, Kinoprene,
   Lambda-Cyhalothrin, Lindane, Lufenuron,
   Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, M ethoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800,
   Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
   OK-5101, OK-5201, OK-9601, OK-9602, OK-9701, OK-9802, Omethoate, Oxamyl, Oxydemetonmethyl,
   Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, P yridaben, Pyridalyl, Pyridaphenthion, Pyridathion, Pyrimidifen, Pyriproxyfen,
   Quinalphos,
   Resmethrin, RH-5849, Ribavirin, RU-12457, RU-15525,
   S-421, S-1833, Salithion, Sebufos, SI-0009, Silafluofen, Spinosad, Spirodiclofen, Spiromesifen, Sulfluramid, Sulfotep, Sulprofos, SZI-121,
   Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IR-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb,
   Vamidothion, Vaniliprole, Verbutin, Verticillium lecanii,
   WL-108477, WL-40027,
   YI-5201, YI-5301, YI-5302,
   XMC, Xylylcarb,
   ZA-3274, Zeta-Cypermethrin, Zolaprofos, ZXI-8901,
   die Verbindung 3-Methyl-phenyl-propylcarbamat (Tsumacide Z),
   die Verbindung 3-(5-Chlor-3 pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
   sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren Safenern bzw. Semichemicals ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. " Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, B aumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnsttoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari, (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren- seien die folgenden Insekten genannt:
Käfer wie
Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
Sirex juvencus, Urocerus gigas, Urocerus gigas taignus, Urocerus augur.
Termiten wie
Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteil, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganzallgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.%, insbesondere 0,001 bis 60 Gew.-%.

Die M enge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Gumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurden un überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
2-tert.-Butylamino-4-cyaopropylamino-6-methylthio-1,3,5-triazin, Dichlorophen, Diuron, Endothal, Fentinacetat, Isoproturon, Methabenzthiazoron, Oxyfluorfen, Quinoclamine und Terbutryn;
Fungizide wie
Benzo(b)thiophencarbonsäurecyclohexylamid-S,S-dioxid, Dichlofluanid, Fluorfolpet, 3-Iod-2-propinyl-butylcarbamat, Tolylfluanid und Azole wie
Azaconazole, Cyproconazole, Epoxyconazole, Hexaconazole, Metconazole, Propiconazole und Tebuconazole;
Molluskizide wie
Fentinacetat, Metaldehyd, Methiocarb, Niclosamid, Thiodicarb und Trimethacarb, Fe-chelate;
oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/- Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit, anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoelea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische, Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen(-sodium), Aclonifen, Alachlor, Alloxydim(-sodium), Ametryne, Amicarbazone, Amidochlor, Amidosulfuron, Anilofos, Asulam, Atrazine, Azafenidin, Azimsulfuron, Beflubutamid, Benazolin(-ethyl), Benfuresate, Bensulfuron(-methyl), Bentazon, Benzfendizone, Benzobicyclon, Benzofenap, Benzoylprop (-ethyl), Bialaphos, Bifenox, Bispynbac(-sodium), Bromobutide, Bromofenoxim, Bromoxynil, Butachlor, Butafenacil(-allyl), Butroxydim, Butylate, Cafenstrole, Caloxydim, Carbetamide, Carfentrazone(-ethyl), Chlomethoxyfen, Chloramben, Chloridazon, Chlorimuron (-ethyl), Chlornitrofen, Chlorsulfuron, Chlortoluron, Cinidon(-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise:
AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäβen Wirkstoffe geht aus den nachfolgenden Beispielen hervor.

### Herstellungsbeispiele

### Verfahren A

### Beispiel I-1-a-1

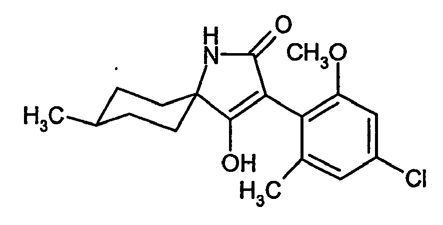

Zu 5 ,43 g (0,047 Mol) Kalium-tert.-butylat in 18 ml wasserfreiem Tetrahydrofuran tropft man bei Rückflusstemperatur 6,7 g der Verbindung gemäß Herstellungsbeispiel II-1 in 40 ml wasserfreiem Toluol.

Das Reaktionsgemisch wird 1,5 h unter Rückfluss gerührt. Man gibt anschließend 60 ml Wasser zu, trennt die wässrige Phase ab und extrahiert die organische Phase mit Wasser. Die wässrigen Phasen werden nochmals mit Toluol gewaschen und bei 0-20°C mit konzentrierter Salzsäure auf pH 1 eingestellt. Der Niederschlag wird abgesaugt, gewaschen und getrocknet. Es erfolgt säulenchromatographische Reinigung an Kieselgel (Dichlormethan: Essigsäureethylester, 5:1).

Ausbeute: 3,2 g / 52 % der Theorie). Fp.: > 220°C.

### Verfahren P

### Beispiel-Nr. I-1-a-18

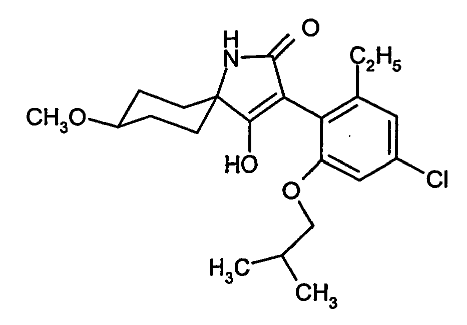

12,12 g iso-Butanol vorlegen und 3,54 g Kalium-tert-butylat (95 %ig) bei 0°C bis 5°C zugeben, kurz erwärmen. Dann das Kupfer-(I)-iodid 0,571 g (3 mmol) und 1,34 g der Verbindung gemäß Beispiel I-a-10 (DE-A-10301804) zugeben, dann auf 110°C erwärmen. Man rührt 24 Stunden bei 100°C bis 110°C. Es wird über Celite abgesaugt, der Feststoff verworfen, das Filtrat angesäuert und einrotiert.

Es erfolgt eine Flashsäulentrennung an Kieselgel mit Essigsäureethylester. Die Fraktionen, die nach LC/MS das Produkt enthielten, wurden einer RP-Säulenchromatographie mit einem Gradientenprogramm unterworfen. Die Säule wurde mit einem 50:50-Gemisch aus Wasser/Methanol vorkonditioniert.

Ausbeute: 190 mg weißes Pulver = 15 % d. Th.

Fp.=117°C

In Analogie zu Beispiel (I-1-a-1) und gemäß den allgemeinen Angaben zur Herstellung erhält man folgende Verbindungen der Formel (I-1-a)

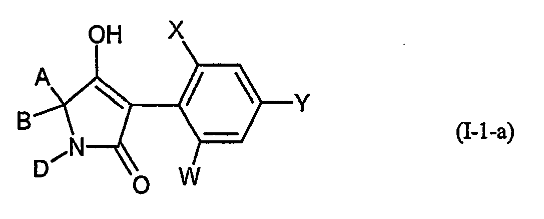

| **Bsp.-Nr** | **W** | **X** | **Y** | **D** | **A** | **B** | Fp°C | Isomer |
|---|---|---|---|---|---|---|---|---|
| I-1-a-2 | OCH₃ | CH₃ | Cl | H | -(CH₂)₂O-(CH₂)₂- | | >220 | - |
| I-1-a-3 | OCH₃ | C₂H₅ | Cl | H | -(CH₂-)₂-CHOCH₃-(CH₂)₂- | | 223 | β |
| I-1-a-4 | OCH₃ | C₂H₅ | Cl | H | -(CH₂-)₂-CHCH₃-(CH₂)₂- | | 232 | β |
| I-1-a-5 | OCH₃ | C₂H₅ | Cl | H | CH₃ | CH₃ | 141 | - |
| I-1-a-6 | OCH₃ | C₂H₅ | Cl | H | -(CH₂)₅₋ | | 236 | - |
| I-1-a-7 | OCH₃ | C₂H₅ | Cl | 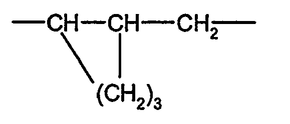 | | H | 169 | Isomeren-gemisch |
| I-1-a-8 | OCH₃ | CH₃ | Cl | H | -(CH₂-)₂-CHOCH₃-(CH₂)₂- | | 318 | β |
| I1-a-9 | OC₂H₅ | C₂H₅ | Cl | H | -(CH₂-)₂-CHOCH₃-(CH₂)₂- | | 96 | β |
| I-1-a-10 | OCH₃ | CH₃ . | Cl | H | CH₃ | CH₃ | 266 | - |
| I-1-a-11 | OC₂H₅ | C₂H₅ | Cl | H | CH₃ | CH₃ | 207 | - |
| I-1-a-12 | OCH₃ | C₂H₅ | Cl | C₂H₅ | CH₃ | H | 176-178 | - |
| I-1-a-13 | OCH₃ | C₂H₅ | Cl | _{C}-C₆H₁₁ | CH₃ | H | 184 | - |
| I-1-a-14 | OCH₃ | C₂H₅ | Cl | 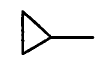 | H | H | 154-157 | - |
| I-l-a-15 | OCH₃ | C₂H₅ | Cl | CH₃ | C₂H₅ | H | *1) | - |
| I-1-a-16 | OCH₃ | C₂H₅ | Cl | -(CH₂)₃- | | H | Zers. | - |
| I-1-a-17 | OC₂H₅ | CH₃ | Cl | H | -(CH₂-)₂-CHOCH₃-(CH₂)₂- | | 202 | β |
| I-1-a-18¹⁾ | O-i-C₄H₉ | C₂H₅ | Cl | H | -(CH₂-)₂-CHOCH₃-(CH₂)₂- | | 117 | β |
| I-1-a-19¹⁾ | 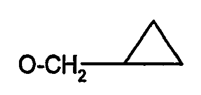 | C₂H₅ | Cl | H | -(CH₂-)₂-CHOCH₃-(CH₂)₂- | | Wachs *2) | β |
| I-1-a-20¹⁾ | O-CH₂-CH₂-OCH₃ | C₂H₅ | Cl | H | -(CH₂-)₂-CHOCH₃-(CH₂)₂- | | 61 | β |
| I-1-a-21¹⁾ | OC₃H₇ | C₂H₅ | Cl | H | -(CH₂-)₂-CHOCH₃-(CH₂)₂- | | Wachs *3) | β |

| | | | | | | | | |
|---|---|---|---|---|---|---|---|---|
| *1) ¹H-NMR (400 MHz, d₆-DMSO): δ = 2.43 (m, 2-H, Ar-CH₂-CH₃), 2,77 (s, 3H, N-CH₃) ppm *2) ¹H-NMR (400 MHz, d₆-DMSO): δ = 2.42 (m, 2-H, Ar-CH₂-CH₃), 3,82 (m, 2H, O-CH₂CH₂ ) ppm *3) ¹H-NMR (400 MHz, d₆-DMSO): δ = 0.27-0.3,0.42-0.45 (2m, 4H, cyclopropyl-CH₂), 1.02 (t, 3H, Ar CH₂-CH₃) ppm 1) Verfahren P | | | | | | | | |

### Beispiel-Nr. I-1-b-1

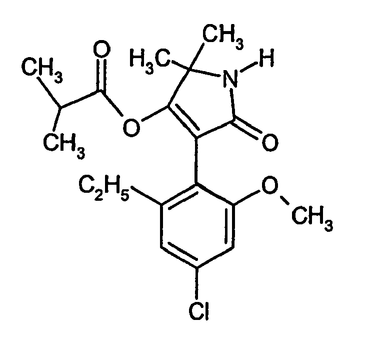

200 mg (0,57 mol) der Verbindung gemäß Beispiel I-1-a-S werden in 5ml wasserfreiem Essigsäureethylester vorgelegt und mit 0,94 ml (0,57 mmol) Triethylamin versetzt. Bei Rückfluss werden 0,71 ml (0,57 mmol) Isobuttersäurechlorid in 1 ml Essigsäureethylester zugegeben und 2,5 h unter Rückfluss erhitzt.

Die Reaktionslösung wird abgekühlt und eingeengt und der Rückstand an Kieselgel mit einem Gradienten Heptan/Essigsäureethylester 100/0 bis 0/100 chromatographiert.

Ausbeute: 90 mg (43 % d. Th.) Fp. 131°C.

In Analogie zu Beispiel (I-1-b-1) und gemäß den allgemeinen Angaben zur Herstellung, erhält man folgende Verbindungen der Formel (I-1-b)

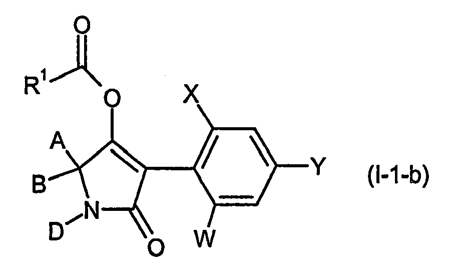

| **Bsp.-Nr.** | **W** | **X** | **Y** | **D** | **A** | **B** | **R¹** | **Fp. °C** | **Isomer** |
|---|---|---|---|---|---|---|---|---|---|
| I-1-b-2 | OCH₃ | C₂H₅ | Cl | H | -(CH₂)₅- | | i-C₃H₇ | 221 | - |
| I-1-b-3 | OCH₃ | C₂H₅ | Cl | H | -(CH₂)₂-CHCH₃-(CH₂)₂- | | i-C₃H₇ | 161 | β |
| I-1-b-4 | OCH₃ | C₂H₅ | Cl | H | -(CH₂)₂-CHCH₃-(CH₂)₂- | | t-C₄H₉ | 169 | β |
| I-1-b-5 | OCH₃ | C₂H₅ | Cl | H | CH₃ | CH₃ | t-C₄H₉ | 148 | - |
| I-1-b-6 | OCH₃ | C₂H₅ | Cl | H | -(CH₂)₂-CHOCH₃-(CH₂)₂- | | i-C₃H₇ | 201 | β |
| I-1-b-7 | OC₂H₅ | C₂H₅ | Cl | H | CH₃ | CH₃ | H₃C-O-CH₂ | 130 | - |
| I-1b-8 | OCH₃ | CH₃ | Cl | H | CH₃ | CH₃ | H₃C-O-CH₂ | 149-153 | - |
| I-1-b-9 | OCH₃ | C₂H₅ | Cl | 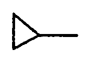 | H | H | t-C₄H₉- | *1.07(s,9H,t-Bu)- 2.40(m,2H,CH₂-Ar) | - |
| I-1-b-10 | OCH₃ | C₂H₅ | Cl | CH₃ | C₂H₅ | H | t-C₄H₉- | Öl | - |
| I-1-b-11 | OC₂H₅ | C₂H₅ | Cl | H | -(CH₂)₂-CHOCH₃-(CH₂)₂- | | i-C₃H₇- | 226-229 | β |
| I-1-b-12 | OCH₃ | C₂H₅ | Cl | H | -(CH₂)₂-CHOCH₃-(CH₂)₂- | | 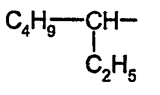 | 138 | β |
| I-1-b-13 | OC₂H₅ | C₂H₅ | Cl | H | -(CH₂)₂-CHOCH₃-(CH₂)₂- | | 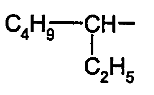 | 171 | β |
| I-1 b-14 | OCH₃ | C₂H₅ | Cl | H | -(CH₂)₂-CHOCH₃-(CH₂)₂- | | C₂H₅-C(CH₃)₂- | 216-218 | β |
| I-1-b-15 | OC₂H₅ | C₂H₅ | Cl | H | -(CH₂)₂-CHOCH₃-(CH₂)₂- | | C₂H₅-C(CH₃)₂- | 237-239 | β |
| I-1-b-16 | OCH₃ | C₂H₅ | Cl | H | -(CH₂)₂-CHOCH₃-(CH₂)₂- | | H₅C₂-O-CH₂ | 154-159 | β |
| I-1-b-17 | OCH₃ | C₂H₅ | Cl | H | -(CH₂)₂-CHOCH₃-(CH₂)₂- | | (C₈H₁₇-CH=CH-C₇H₁₄- | *3.21(m,1H,CHOCH₃) 5,35(m,2H,CH=CH) | β |

| | | | | | | | | | |
|---|---|---|---|---|---|---|---|---|---|
| * ¹H-NMR (400 MHz, CDCl₃): Verschiebungen δ in ppm | | | | | | | | | |

### Beispiel-Nr. I-1-c-1

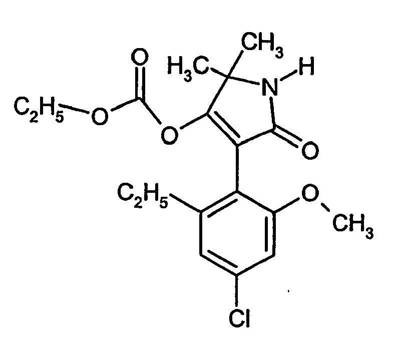

200 mg (0,57 mmol) der Verbindung gemäß Beispiel I-1a-5 werden in 5 ml wasserfreiem CH₂Cl₂ vorgelegt und mit 0,94 ml (0,57 mmol) Triethylamin versetzt. Bei 10 bis 20°C werden 0,64 ml (0,57 mmol) Chlorameisensäureethylester in 1 ml CH₂Cl₂ zugegeben und 1,5 h bei Raumtemperatur gerührt. Die Reaktionslösung wurde eingeengt und der Rückstand an Kieselgel mit einem Gradientin Heptan/Essigester 100/0 bis 0/100 chromatographiert.

Ausbeute: 0,19 g (95 % d. Th.) Fp. 222°C.

In Analogie zu Beispiel (I-1-c-1) und gemäß den allgemeinen Angaben zur Herstellung erhält man folgende Verbindungen der Formel (I-1-c):

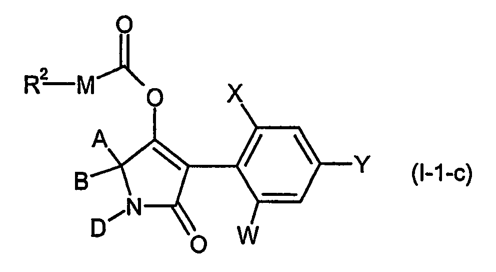

| **Bsp.-Nr.** | **W** | **X** | **Y** | **D** | **A** | **B** | **M** | **R²** | **Fp. °C** | **Isomer** |
|---|---|---|---|---|---|---|---|---|---|---|
| I-1-c-2 | OCH₃ | C₂H₅ | Cl | H | -(CH₂)₅- | | O | C₂H₅ | 208 | - |
| I-1-c-3 | OCH₃ | C₂H₅ | Cl | H | -(CH₂)₂-CHCH₃-(CH₂)₂- | | O | C₂H₅ | 187 | ß |
| I-1-c-4 | OCH₃ | C₂H₅ | Cl | H | -(CH₂)₂-CHOCH₃-(CH₂)₂- | | O | C₂H₅ | 161 | ß |
| I-1-c-5 | OCH₃ | C₂H₅ | Cl | CH₃ | C₂H₅ | H | O | C₂H₅ | *2.98(s,3H,NCH₃)- 3.71 (s,3H,OCH₃) | - |
| I-1-c-6 | OCH₃ | C₂H₅ | Cl | c-C₆H₁₁ | CH₃ | H | O | C₂H₅ | *4.2 (m,2H,OCH₂) | - |
| I-1-c-7 | OCH₃ | C₂H₅ | Cl | C₂H₅ | CH₃ | H | O | C₂H₅ | *4.2(m,2H,OCH₂) | - |
| I-1-c-8 | OCH₃ | C₂H₅ | Cl | 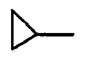 | H | H | O | C₂H₅ | *2.8(m,1H,H 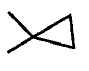 | - |
| I-1-c-9 | OCH₃ | CH₃ | Cl | H | CH₃ | CH₃ | O | C₂H₅ | 141-145 | - |
| I-1-c-10 | OC₂H₅ | C₂H₅ | Cl | H | CH₃ | CH₃ | O | C₂H₅ | *1,46 (d,6H,C(CH₃)₂) | - |
| I-1-c-11 | OC₂H₅ | C₂H₅ | Cl | H | -(CH₂)₂-CHOCH₃-(CH₂)₂- | | O | C₂H₅ | 188-191 | ß |
| I-1-c-12 | OC₂H₅ | C₂H₅ | Cl | H | -(CH₂)₂-CHOCH₃-(CH₂)₂- | | O | CH₂=CH-CH₂- | 159-161 | ß |
| I-1-c-13 | OCH₃ | C₂H₅ | Cl | H | -(CH₂)₂-CHOCH₃-(CH₂)₂- | | O | CH₂=CH-CH₂- | 138-140 | ß |
| I-1-c-14 | OCH₃ | CH₃ | Cl | H | -(CH₂)₂-CHOCH₃-(CH₂)₂- | | O | C₂H₅ | 170 | ß |
| I-1-c-15 | OCH₃ | C₂H₅ | Cl | H | -(CH₂)₂-CHOCH₃-(CH₂)₂- | | O | 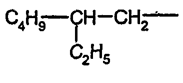 | 98-101 | ß |
| I-1-c-16 | OC₂H₅ | C₂H₅ | Cl | H | -(CH₂)₂-CHOCH₃-(CH₂)₂- | | O | 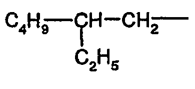 | 114 | ß |
| I-1-c-17 | OCH₃ | C₂H₅ | Cl | -(CH₂)₃- | | H | O | C₂H₅ | *4.2 (m,2H,OCH₂)- | - |

| | | | | | | | | | | |
|---|---|---|---|---|---|---|---|---|---|---|
| * ¹H-NMR (400 MHz, CDCl₃) : Verschiebungen δ in ppm | | | | | | | | | | |

### Beispiel I-d-1

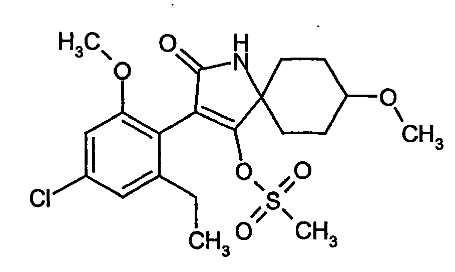

Zu einer Lösung von 0,2 g (0,547 mmol) gemäß Beispiel I-1-a-3 in wasserfreiem CH₂Cl₂ werden zunächst 0,09 ml Triethylamin und anschließend 0,069 g (0,601 mmol) Methansulfonsäurechlorid gegeben. Nach Rühren über Nacht bei Raumtemperatur wird mit 10 ml Natriumhydrogencarbonatlösung versetzt, die Phasen getrennt und anschließend die organische Phase nach Trocknen mit Natriumsulfat unter Vakuum vom Lösungsmittel befreit. Der so erhaltene Rückstand wird mit einem Gradienten n-Heptan/Essigsäureethylester 100/0 bis 0/100 an Kieselgel chromatographiert.

Ausbeute: 0,14 g (58 % d. Th.) Fp.: 210-214°C.

### Beispiel I-1-f-1

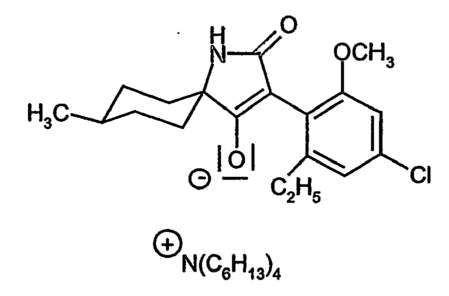

Zu einer Lösung von 0,75 g gemäß Beispiel I-1-a-3 (1,95 mmol) in 20 ml wasserfreiem Methanol werden 9 ml einer 10 %igen Lösung von Tetrahexylammoniumhydroxid in Methanol gegeben und 30 Minuten bei Raumtemperatur nachgerührt. Man befreit im Vakuum vom Lösungsmittel, versetzt noch insgesamt dreimal mit je 50 ml Methanol und entfernt das Lösungsmittel und erhält so 1,44 g (97 % Ausbeute) in Form eines wachsartigen Feststoffs.

¹H-NMR (400 MHz, d₆-DMSO): δ = 0,88 (t, 12, 4 -CH₃), 3,56 (s, 3H, OCH₃) ppm

In Analogie zu Beispiel I-1-f-1 erhält man in Beispiel I-1-f-2

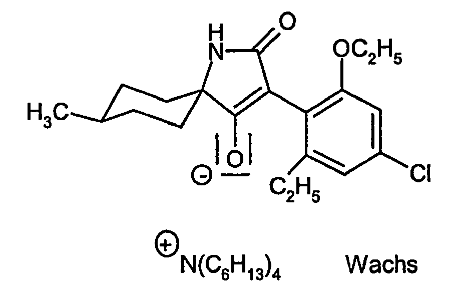

¹H-NMR (400 MHz, d₆-DMSO): δ = 0,88 (t, 12,4 CH₃), 3,83 (s, 2H, OCH₂) ppm

### Beispiel-Nr. II-1

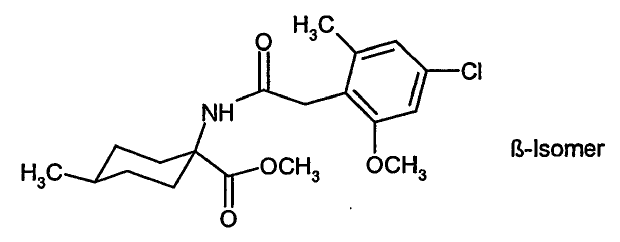

5,2 g 4-Chlor-2-methoxy-6-methyl-phenylessigsäure und 5,4 ml (0,073 Mol) Thionylchlorid werden bei 50°C gerührt bis die Gasentwicklung beendet ist.

Bei 50°C wird überschüssiges Thionylchlorid abrotiert, den Rückstand, nimmt man in 50 ml wasserfreies Toluol auf und rotiert erneut überschüssiges Thionylchlorid ab. Der Rückstand wird in 30 ml wasserfreiem Tetrahydrofuran aufgenommen (Lösung 1). 5,1 g 1-Amino-4-methyl-cyclohexancarbonsäuremethylester-hydrochlorid werden in 50 ml wasserfreiem Tetrahydrofuran vorgelegt und 7,5 ml (0,053 Mol) Triethylamin zugeben. Bei 0-10°C tropft man anschließend Lösung 1 zu.

Man rührt 1 h bei Raumtemperatur nach.

Das Lösungsmittel wird abrotiert und der Rückstand in 0,5 N Salzsäure-Dichlormethanlösung aufgenommen, extrahiert, getrocknet und das Lösungsmittel abdestilliert. Der Rückstand wird aus MTB-Ether/n-Hexan umkristallisiert.

Ausbeute: 6,7 g (68 % der Theorie), Fp.: 166°C.

### Beispiel-Nr. II-2

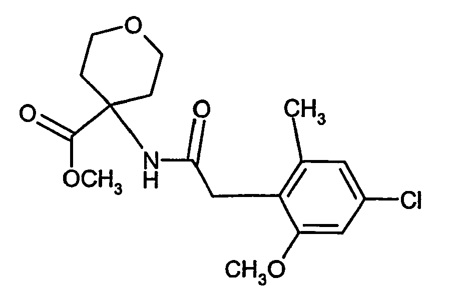

Zu 9,8 g (0,1 Mol) konz. Schwefelsäure gibt man 6,4 g der Verbindung gemäß Herstellungsbeispiel Nr. XXIX-1 in 60 ml Methylenchlorid bei einer Innentemperatur von 30-40°C. Man rührt 2 h bei 30-40°C. Anschließend tropft man 13,5 ml wasserfreies Methanol zu, so dass sich eine Innentemperatur von 40°C einstellt. Man rührt 6 h bei 40-70°C weiter.

Die Reaktionslösung wird auf 0,1 kg Eis gegossen, mit Dichlormethan extrahiert und mit NaHCO₃-Lösung gewaschen. Danach wird getrocknet, das Lösungsmittel abdestilliert und aus MTB-Ether/n-Hexan umkristallisiert.

Ausbeute: 5,9 g (83 % d. Th.), Fp.: 156°C.

In Analogie zu den Beispielen II-1 und II-2 und gemäß den allgemeinen Angaben zur Herstellung erhält man folgende Verbindungen der Formel II

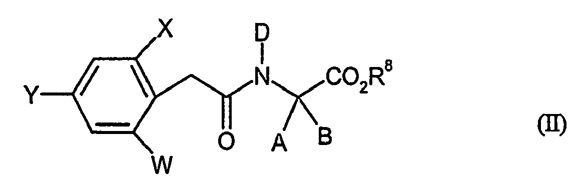

| **Bsp.-Nr.** | **W** | **X** | **Y** | **D** | **A** | **B** | **R⁸** | **Isomer** | **Fp°C** |
|---|---|---|---|---|---|---|---|---|---|
| II-3 | OCH₃ | C₂H₅ | Cl | H | -(CH₂)₂-CHOCH₃-(CH₂)₂- | | CH₃ | ß | 105 |
| II-4 | OCH₃ | C₂H₅ | Cl | H | -(CH₂)₂-CH CH₃-(CH₂)₂- | | CH₃ | ß | 131 |
| II-5 | OCH₃ | C₂H₅ | Cl | H | CH₃ | CH₃ | CH₃ | - | 163 |
| II-6 | OCH₃ | C₂H₅ | Cl | H | -(CH₂)₅- | | CH₃ | - | 124 |
| II-7 | OCH₃ | C₂H₅ | Cl | 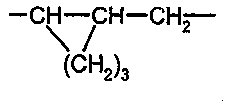 | | H | C₂H₅ | Isomeren-gemisch | Öl |
| II-8 | OCH₃ | CH₃ | Cl | H | -(CH₂)₂-CHOCH₃-(CH₂)₂ | | CH₃ | ß | 127 |
| II-9 | OC₂H₅ | C₂H₅ | Cl | H | -(CH₂)₂-CHOCH₃-(CH₂)₂ | | CH₃ | ß | 100 |
| II-10 | OCH₃ | CH₃ | Cl | H | CH₃ | CH₃ | CH₃ | - | 157 |
| II-11 | OC₂H₅ | C₂H₅ | Cl | H | CH₃ | CH₃ | CH₃ | - | 118 |
| II-12 | OCH₃ | C₂H₅ | Cl | C₂H₅ | CH₃ | H | C₂H₅ | - | *1,47 (d, 3H, CH(CH₃)) |
| II-13 | OCH₃ | C₂H₅ | Cl | c-C₆H₁₁ | CH₃ | H | C₂H₅ | - | *1,44 (d, 3H, CH(CH₃)) |
| II-14 | OCH₃ | C₂H₅ | Cl | 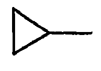 | H | H | C₂H₅ | - | *3,03 (m, 1H, 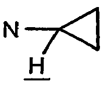 ) |
| II-15 | OCH₃ | C₂H₅ | Cl | CH₃ | C₂H₅ | H | C₂H₅ | - | *2,59 (q, 2H, Ar-CH₂) |
| II-16 | OCH₃ | C₂H₅ | Cl | -(CH₂)₃- | | H | CH₃ | - | ** 1.13 (t,3H,ArCH₂-CH₃), 3.76 (s, 3H, Ar OCH₃) |
| II-17 | OC₂H₅ | CH₃ | Cl | H | -(CH₂)₂-CHOCH₃-(CH₂)₂- | | CH₃ | ß | ***1.31 (t,3H,Ar-O-CH₂-CH₃), 4.00 (q,2H,Ar-O-CH₂-CH₃) |

| | | | | | | | | | |
|---|---|---|---|---|---|---|---|---|---|
| * ¹H-NMR (400 MHz, CDCl₃): Verschiebungen δ in ppm ** ¹H-NMR (400 MHz, d₆-DMSO): Verschiebungen δ in ppm *** ¹H-NMR (400 MHz, CD₃CN): Verschiebungen δ in ppm | | | | | | | | | |

### Beispiel Nr. XXIX-1

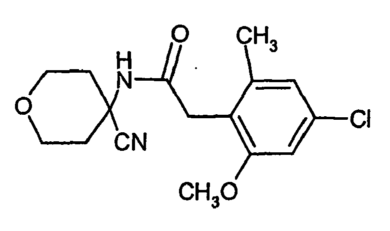

5,2 g 4-Chlor-2-methoxy-6-methyl-phenylessigsäure und 5,4 ml (0,073 Mol) Thionylchlorid werden bei 50°C gerührt, bis die Gasentwicklung beendet ist.

Bei 50°C wird überschüssiges Thionylchlorid abrotiert, den Rückstand nimmt man in 50 ml wasserfreies Toluol auf und rotiert erneut überschüssiges Thionylchlorid ab. Der Rückstand wird in 30 ml wasserfreiem Tetrahydrofuran aufgenommen (Lösung 1). 6,11 g 4-Amino-tetrahydropyran-4-carbonsäurenitril werden in 50 ml wasserfreiem Tetrahydrofuran vorgelegt, mit 3,4 ml Triethylamin versetzt und bei 0-10°C Lösung 1 zugetropft.

Man rührt 1 h bei Raumtemperatur nach.

Das Lösungsmittel wird abrotiert und der Rückstand in 0,5 N Salzsäure-Dichlormethanlösung aufgenommen, extrahiert, getrocknet und das Lösungsmittel abdestilliert. Der Rückstand wird aus MTB-Ether/n-Hexan umkristallisiert.

Ausbeute: 6,4 g (82 % d.Th.), Fp.: 149°C

### Beispiel I-2-a-1

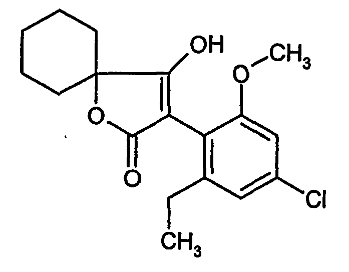

1,84 g KOtBu in 5 mol DMF werden bei 0°C vorgelegt und 4,2 g der Verbindung gemäß Beispiel III-1 in DMF (5 ml) gelöst und bei 0 bis 10°C zugetropft. Man lässt über Nacht bei Raumtemperatur rühren und destilliert das DMF im Vakuum ab. Der Rückstand wird mit Wasser verrührt, mit HCl angesäuert und der Niederschlag abgesaugt, getrocknet.

Ausbeute: 2,8 g (59 % der Theorie), 90°C.

In Analogie zu Beispiel (I-2-a-1) und gemäß den allgemeinen Angaben zur Herstellung erhält man folgende Verbindungen der Formel (I-2-a)

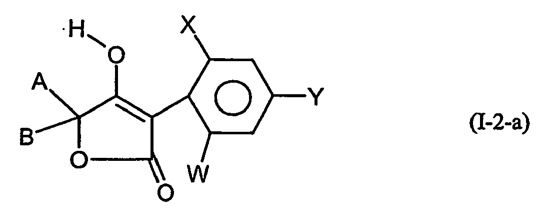

| **Bsp.- Nr.** | **W** | **X** | **Y** | **A** | **B** | **Fp. °C** |
|---|---|---|---|---|---|---|
| I-2-a-2 | OCH₃ | C₂H₅ | Cl | -(CH₂)₂-CHOCH₃-(CH₂)₂- | | 90-95 |

### Beispiel I-2-b-1

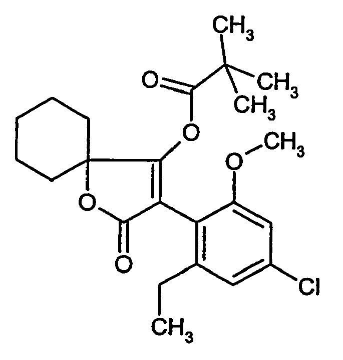

0,34 g der Verbindung gemäß Beispiel I-2-a-1 werden in Dichlormethan (10 ml) und Triethylamin (0,15 ml) vorgelegt und unter Eiskühlung m it 0,13 g P ivalinsäurechlorid versetzt. E s wird über Nacht bei Raumtemperatur gerührt, die Lösung mit 10 % Citronensäure und 10 % NaOH gewaschen, getrennt, getrocknet und eingeengt.

Ausbeute: 0,3 g (57 % der Theorie).

¹H-NMR (400 MHz, CD₃CN): δ = 1.11 (s, 9H, C(CH₃)₃), 3,72 (s, 3H, ArOCH₃) ppm.

In Analogie zu Beispiel (I-2-b-1) und gemäß den allgemeinen Angaben zur Herstellung erhält man folgende Verbindungen der Formel (I-2-b)

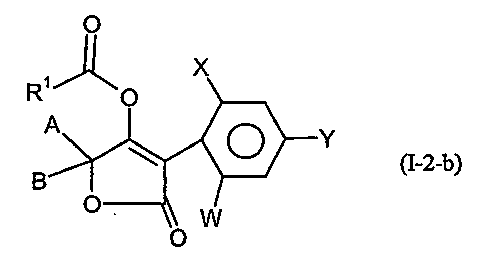

| **Bsp.-Nr.** | **W** | **X** | **Y** | **A** | **B** | **R¹** | **Fp. °C** |
|---|---|---|---|---|---|---|---|
| I-2-b-2 | OCH₃ | C₂H₅ | Cl | -(CH₂)₅- | | H₅C₂-C(CH₃)₂ | * 1,07, 1,19, (2s, 6H, 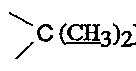 ), 3,72, (s, 3H, OCH₃) |
| I-2-b-3 | OCH₃ | C₂H₅ | Cl | -(CH₂)₅- | | i-C₃H₇ | *2,66 (m, 1H, -CH(CH₃)₂, 3,72 (s, 3H, OCH₃ |
| I-2-b-4 | OCH₃ | C₂H₅ | Cl | -(CH₂)₂-CHOCH₃-(CH₂)₂- | | H₅C₂-C(CH₃)₂- | *3,28, 3,33 (2s, 3H, CH-OCH₃) |
| I-2-b-5 | OCH₃ | C₂H₅ | Cl | -(CH₂)₂-CHOCH₃-(CH₂)₂- | | i-C₃H₇ | *3,30, 3,32 (2s, 3H, CH-OCH₃) |
| I-2-b-6 | OCH₃ | C₂H₅ | Cl | -(CH₂)₂-CHOCH₃-(CH2)2- | | t-C₄H₉ | *3,29, 3,33 (2s, 3H, CHOCH₃), 1,11, 1,12 (2s, 9H, C(CH₃)₃) |

| | | | | | | | |
|---|---|---|---|---|---|---|---|
| * ¹H-NMR (400 MHz, CD₃CN): Verschiebungen δ in ppm | | | | | | | |

### Beispiel I-2-c-1

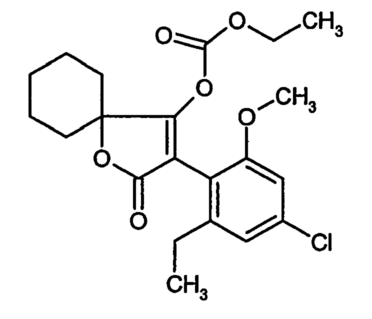

0,67 g der Verbindung gemäß Beispiel I-2-a-1 werden in Dichlormethan (10 ml) und Triethylamin (0,31 ml) vorgelegt und unter Eiskühlung mit 0,239 g Chlorameisensäureethylester versetzt. Es wird über Nacht bei Raumtemperatur gerührt, die Lösung mit 10 % Citronensäure und 10 % NaOH gewaschen, getrennt, getrocknet und eingeengt.

Ausbeute: 0,76 g (73 % der Theorie).

¹H-NMR (400 MHz, CD₃CN): δ = 3,73 (s, 3H, OCH₃), 4,03 (q, 2H, OCH₂CH₃), 6,87 (d, 1H, Ar-H), 6,95 (d, 1H, Ar-H) ppm.

In Analogie zu Beispiel (I-2-c-1) und gemäß den allgemeinen Angaben zur Herstellung erhält man folgende Verbindungen der Formel (I-2-c)

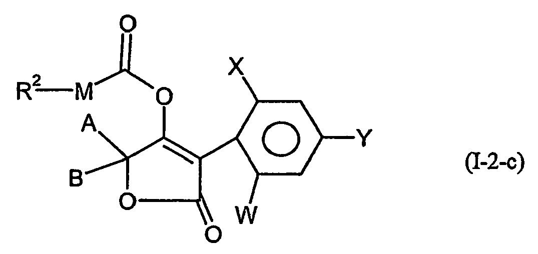

| **Bsp.-Nr.** | **W** | **X** | **Y** | **A** | **B** | **M** | **R²** | **Fp.°C** |
|---|---|---|---|---|---|---|---|---|
| I-2-c-2 | OCH₃ | C₂H₅ | Cl | -(CH₂)-CHOCH₃-(CH₂)₂- | | O | C₂H₅ | *3,31, 3,33 (2s, 3H, CHOCH₃), 3,73 (s, 3H, OCH₃ |

| | | | | | | | | |
|---|---|---|---|---|---|---|---|---|
| * ¹H-NMR (400 MHz, CDCN): Verschiebungen δ in ppm | | | | | | | | |

### Beispiel III-1

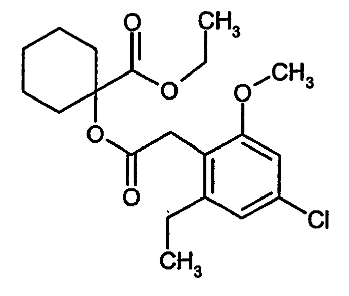

2,09 g 1-Hydroxycyclohexancarbonsäure-ethylester und 3 g der Verbindung gemäß Beispiel XXIV-1 werden im Ölbad auf 120°C erhitzt und bis zum Ende der Gasentwicklung verrührt und dann noch kurz auf 140°C erhitzt. Ausbeute: 42 g (59 % der Theorie).

¹H-NMR (400 MHz, CD₃CN): δ = 2,58 (q, 2H, CH₂-Ar), 3,77 (s, 3H, OCH₃), 4,05 (m, 2H, O-CH₂-CH₃) ppm.

In Analogie zu Beispiel III-1 und gemäß den allgemeinen Angaben zur Herstellung erhält m an folgende Verbindungen der Formel (III)

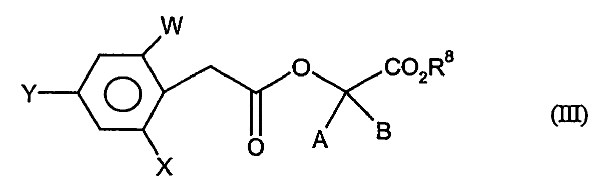

| **Bsp.-Nr.** | **W** | **X** | **Y** | **A** | **B** | **R⁸** | **Fp. °C** |
|---|---|---|---|---|---|---|---|
| III-2 | OCH₃ | C₂H₅ | Cl | -(CH₂)₂CHOCH3-(CH₂)₂- | | C₂H₅ | * 2,58 (q, 2H, CH₂-Ar), 4,06 (m, 2H, O-CH₂-CH₃), 6,88 (d, 1H, Ar-H), 6,91 (d, 1H, Ar-H) |

### Beispiel I-8-a-1

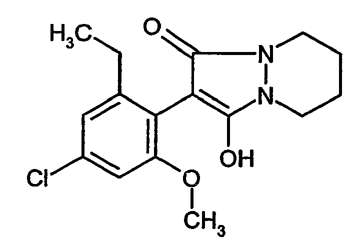

2,5 g (0,007 mol) der Verbindung gemäß Beispiel XII-1 und 1,673 g (0,015 mol) Kalium-tert.-butylat werden in 100 ml N,N-Dimethylacetamid auf 60 bis 120°C erhitzt. Man lässt abkühlen, säuert an, verdünnt mit Wasser und extrahiert 3 mal mit Toluol. Die organische Phase wird getrocknet, das Lösungsmittel im Vakuum abdestilliert und der Rückstand über Kieselgel filtriert. Man erhielt 0.9 g Produkt.

Die wässrige Phase wurde erneut mit Essigester extrahiert und der Extrakt analog aufgereinigt. Dabei wurden weitere 0.48 g Produkt erhalten.

Gesamtausbeute: 1,38 g (60 % der Theorie), Fp. 163°C.

### Beispiel I-8-b-1

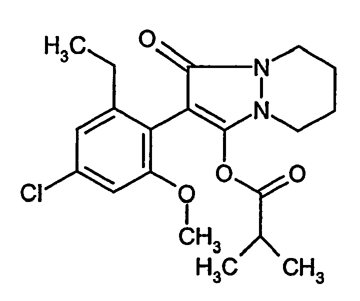

0,14 g der Verbindung gemäß Beispiel I-8-a-1 und 0.08 ml Triethylamin werden in 25 ml Dichlormethan vorgelegt. Nach Zutropfen von 0.05 ml Isobuttersäurechlorid wird 1 S tunde b ei Raumtemperatur gerührt, mit Wasser verdünnt, die organische Phase abgetrennt und das Lösungsmittel abdestilliert. Das Reaktionsgemisch wird mit n-Hexan und wenig Toluol verrührt und der Niederschlag abgesaugt.

Ausbeute: 120 mg (70 % der Theorie), 127,5°C.

### Beispiel I-8-c-1

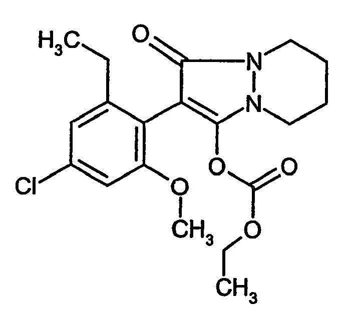

0,15 g der Verbindung gemäß Beispiel I-8-a-1 und 0.08 ml Triethylamin werden in 35 ml Dichlormethan vorgelegt, 0.04 ml Chlorameisensäureethylester zugetropft und 1 Stunde bei Raumtemperatur gerührt. Die Reaktionslösung wird mit Wasser verdünnt, die organische Phase abgetrennt, getrocknet und das Lösungsmittel abdestilliert. Der Rückstand wird mit n-Hexan und wenig Toluol verrührt und der Niederschlag abgesaugt.

Ausbeute: 110 mg (60 % der Theorie), 110°C.

### Beispiel XII-1

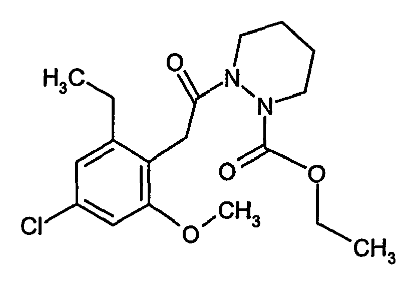

1,9 g der Verbindung gemäß Beispiel XXVII-2 in 50 ml Dichlormethan werden mit 2,10 g Oxalsäuredichlorid versetzt, 30 Minuten unter Rückfluss erhitzt, 1 ml Dimethylformamid hinzugeben und weitere 30 Minuten unter Rückfluss gerührt, unter N₂-Atmosphäre abgekühlt und das Lösungsmittel abdestilliert und der Rückstand in Acetonitril gelöst, (Lösung A).

1.31 g der Verbindung gemäß Beispiel XLI-2 und 1.38 g Kaliumcarbonat werden in 100 ml Acetonitril vorgelegt, Lösung A bei Raumtemperatur zugetropft und 4 Stunden bei Raumtemperatur nachgerührt. Der Feststoff wird abfiltriert, das Lösungsmittel eingedampft und der Rückstand an Kieselgel filtriert.

Ausbeute: 2,5 g (83 % der Theorie).

¹H-NMR (400 MHz, CDCl₃): δ = 6,85 (d, 1H); 6,70 (d, 1H); 4,50 (ddbr, 1H); 4,30-4,10 (m, 3H); 3,90 (d, 1H); 3,75 (s, 3H); 3,50 (d, 1H); 2,70 (mbr, 2H); 2,60 (q, 2H); 1,65 (mbr, 4H); 1,30 (tr, 3H); 1,20 (tr, 3H) ppm.

### Beispiel XLI-1

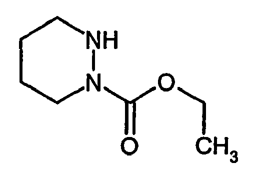

5 g (0,031 mol) Hexahydropyridazin werden in 35 ml Dichlormethan vorgelegt und mit 32 ml Wasser versetzt und 0,1 g Tetra-n-butylammoniumbromid hinzugegeben. Unter Eisbadkühlung werden 4,1 g (0,104 mmol) Natriumhydroxid in 32 ml Wasser dazudosiert. Bei 0°C lässt man 3.0 ml Chlorameisensäureethylester in 30 ml Dichlormethan dazutropfen und 2 Stunden bei 0°C rühren. Nach 8 Stunden Rühren bei Raumtemperatur wird die organische Phase abgetrennt, die wässrige Phase mit Dichlormethan extrahiert und die organische Phase einrotiert. Das Reaktionsgemisch wird mit Wasser verrührt, leicht angesäuert, 2 mal mit Diethylether gewaschen, die wässrige Phase basisch gestellt und mit Dichlormethan extrahiert, getrocknet und einrotiert.

Ausbeute: 3,1 g (63 % der Theorie)

¹H-NMR (400 MHz, CDCl₃): δ = 4,20 (q, 2H); 3,55 (tr, br, 2H); 2,90 (tr, br, 2H); 1,65 (m, 4H); 1,30 (tr, 3H) ppm.

### Beispiel XXIV-1

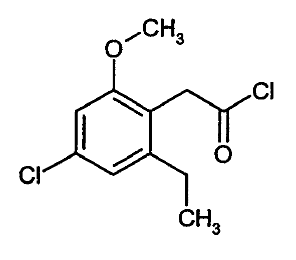

5,78 g der Verbindung gemäß Beispiel XXVII-2 werden in 50 ml Toluol und einem Tropfen DMF vorgelegt. Bei Raumtemperatur wird 3,6 g Thionylchlorid zugetropft, über Nacht unter Rückfluss verrührt, abgekühlt, eingeengt und entgast.

Ausbeute: 6,17 g (98 % der Theorie).

Das Produkt wurde ohne weitere Aufreinigung, z.B. für die Herstellung der Beispiele III-1 und III-2 verwendet.

### Verfahren Q

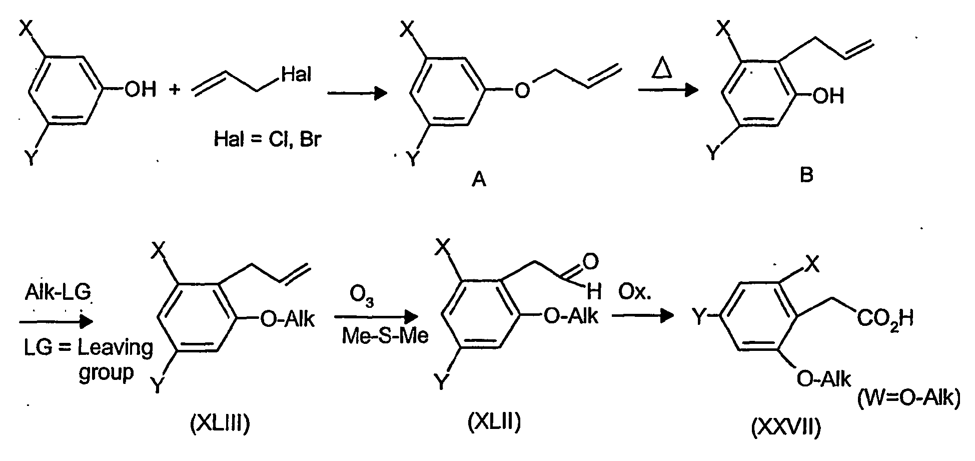

### Bsp.-Nr. XXVII-1

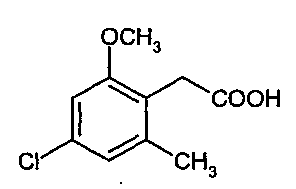

34 g (0,11 Mol) der Verbindung gemäß Herstellungsbeispiel XLII-1 werden in 350 ml tert.-Butanol und 115 g 2-Methyl-2-buten bei Raumtemperatur vorgelegt. Dann wird eine Lösung aus 456 ml Wasser, 155,9 g NaH₂PO₄ und 53,9 g Natriumchlorit als 20 %ige Lösung bei Raumtemperatur zugetropft. 4 h bei Raumtemperatur nachrühren.

Die Reaktionslösung wird in Essigsäureethylester eingerührt, die organische Phase abgetrennt und zweimal mit Essigsäureethylester extrahiert. Anschließend wird getrocknet und das Lösungsmittel abdestilliert. Der Rückstand wird in Wasser aufgenommen, alkalisch gestellt und extrahiert. Die wässrige Phase wird sauergestellt, der Niederschlag abgesaugt und getrocknet.

Ausbeute: 11,2 g (47,5 % d. Th.), Fp.: 130-135°C

### Beispiel-Nr. XLII-1

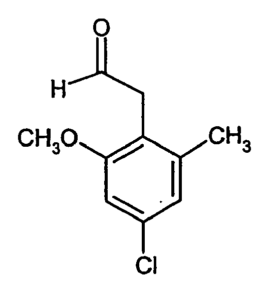

22 g (0,11 Mol) der Verbindung gemäß Herstellungsbeispiel XLIII-1 wird in 60 ml CH₂Cl₂ bei-70°C vorgelegt. Dann wird 2 Stunden Ozon eingeleitet bis keine Aufnahme mehr sichtbar ist. (KJ-Lösung färbt sich gelb-braun). Es wird mit Sauerstoff nachgespült. Wenn die Reaktion beendet ist, wird bei -70°C 19,4 g Dimethylsulfid mit einer Pipette zugetropft und 30 Minuten nachgerührt. Man lässt langsam auf Raumtemperatur kommen und rührt 30 Minuten bei Raumtemperatur nach. Das Lösungsmittel wird im Abzug im Vakuum abrotiert.

Es erfolgt chromatographische Reinigung des Rückstandes an Kieselgel (Petrolether: Essigsäureethylester, 15:1).

Ausbeute: 34 g (40 % d.Th.).

### Beispiel-Nr. XLIII-1

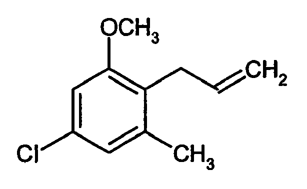

28 g (0,15 Mol) 3 -Chlor-6-allyl-5-mefhyl-phenol gemäß Beispiel B werden mit 7,4 g (0,18 Mol) NaOH in 70 ml H₂O vorgelegt. Bei 20-30°C werden 20,4 g (0,165 Mol) Dimethylsulfat zugetropft und 7 h bei 100°C nachgerührt. Die wässrige Phase wird 3 x mit Diethylether extrahiert und die organische Phase 2 x mit 1N NaOH-Lösung und Wasser gewaschen. Man trocknet und rotiert das Lösungsmittel ab und destilliert den Rückstand im Vakuum.

Ausbeute: 22 g (Kp.: 65°C; 0,2 mbar, 76 % d.Th.).

### Beispiel A

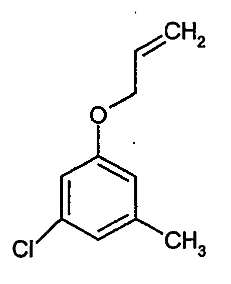

50 g (0,35 mol) 3-Chlor-5-methylphenol, 46,7 g (0,38 Mol) Brompropen und 50 g Kaliumcarbonat werden in 80 ml wasserfreiem Aceton bei Raumtemperatur vorgelegt. Über Nacht wird unter Rückfluss erhitzt.

Das Reaktionsgemisch wird abgekühlt, mit 150 ml Wasser versetzt und 2 x mit Methyl-tert.-butylether extrahiert. Die organische Phase wird anschließend mit 10 % NaOH-Lösung gewaschen und über Kaliumcarbonat getrocknet. Das Lösungsmittel wird einrotiert und der Rückstand in Vakuum destilliert.

Ausbeute: 54 g (Kp.: 105 bei 0,1 mbar; 85 % d.Th.)

### Beispiel B

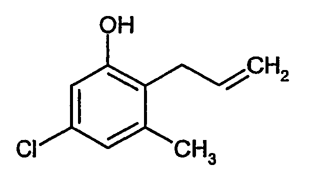

53 g (0,29 Mol) der Verbindung gemäß Herstellungsbeispiel A bei Raumtemperatur vorlegen. Dann 150 ml Mesilyen zugeben und 1-2 Tage bei Rückfluss kochen. Das Lösungsmittel wird nach Reaktionsende (DC-Kontrolle) im Vakuum abrotiert. Nach Feindestillation von 110 g Rohprodukt im Vakuum erhält man zwei Isomere, die ohne weitere Aufreinigung in die Folgereaktion zur Herstellung von Bsp. XLIII-1 eingegeben werden.

Ausbeute: 28 g (Kp.: 84°C; 0,12 mbar, 53 % d.Th.) (Rohprodukt).

### Verfahren R

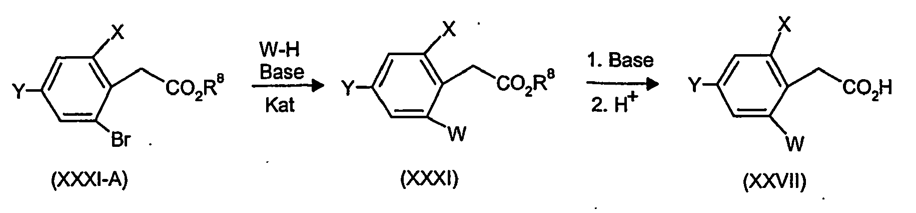

### Beispiel-Nr. XXXI-1

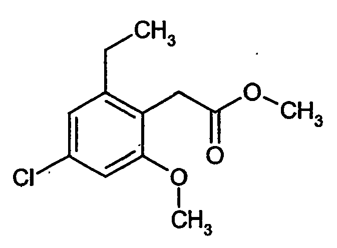

30,1 g (103 mmol) 2-Brom-4-chlor-6-ethyl-phenylessigsäuremethylester, 3 g (21 mmol) Kupfer(I)-bromid, 30 ml Essigsäureethylester und 210 ml (1105 mmol) 30 % Natriummethylatlösung werden über Nacht unter Rückfluss erhitzt. Anschließend wird das Lösungsmittel einrotiert, der Rückstand mit Wasser/Dichlormethan aufgenommen, extrahiert, getrocknet und das Lösungsmittel einrotiert.

Ausbeute: 9,4 g (38% d.Th.)

¹H-NMR {400 MHz, DMSO-d₆}: 1.09 (t, ³J_{HH} = 7 Hz, 3H, CH₃); 2.55 (q, ³J_{HH} = 7 Hz, 2H, CH₂); 3.58 (s, 3H, OCH₃); 3.61 (s, 2H, CH₂) 3.78 (s, 3H, OCH₂); 6.89 (s, 1H, Ph-H); 6.94 (s, 1H, Ph-H).

MS/CI: 243 (M+1)

In Analogie zu Beispiel XXX-1 erhält man 2-Ethyl-6-ethoxy-4-chlorphenylessigsäureethylester (XXX-2)

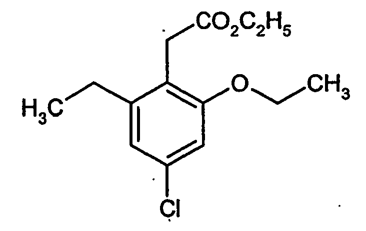

¹H-NMR {400 MHz, DMSO-d₆}: 1.10 (t, ³J_{HH} = 7 Hz, 3H, CH₃); 1.18 (t, ³J_{HH} = 7 Hz, 3H, CH₃); 1.27 (t, ³J_{HH} = 7 Hz, 3H, CH₃); 2.54 (q, ³J_{HH} = 7 Hz, 2H, CH₂); 3.58 (s, 2H, CH₂); 4.01 (q, ³J_{HH} = 7 Hz, 2H, OCH₂) 4.09 (q, ³J_{HH} = 7 Hz, 2H, OCH₂); 6.86 (s, 1H, Ph-H); 6.88 (s,1H, Ph-H) ppm.

MS/CI271 (M+1).

### Beispiel Nr. XXVII-2

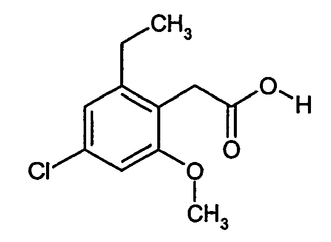

6,5 g (116 mmol) KOH, 30 ml Wasser und 40 ml Methanol werden vorgelegt, 9,4 g (38 mmol) der Verbindung gemäß Beispiel XXXI-1 zugegeben und über Nacht unter Rückfluss erhitzt. Dann wird das Lösungsmittel im Vakuum entfernt, der Rückstand in Wasser aufgenommen und mit konz. HCl ausgefällt. Der Niederschlag wird mit abfiltriert, mit wenig Wasser gewaschen und im Vakuum getrocknet.

Ausbeute: 8,6 g (97 % d. Th.)

¹H-NMR {400 MHz, DMSO-d₆} : 1.08 (t, ³J_{HH} = 7 Hz, 3H, CH₃); 2.53 (q, ³J_{HH} = 7 Hz, 2H, CH₂; 3.51 (s, 3H, CH₂); 3.76 (s, 3H, OCH₃); 6.86 (s, 1H, Ph-H); 6.89 (s, 1H, Ph-H); 12.2 (s, 1H, CO₂H).

MS/CI: 229 (M+1)

In Analogie zu Beispiel XXVII-2 erhält man 2-Ethyl-6-ethoxy-4-chlorphenylessigsäure XXXVII-3

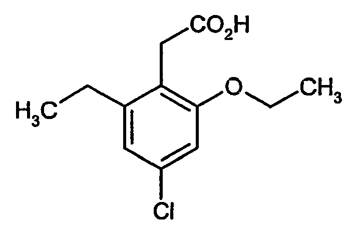

¹H-NMR {400 MHz, DMSO-d₆}: 1.09 (t, ³J_{HH} = 7 Hz, 3H, CH₃); 1.28 (t, ³J_{HH} = 7 Hz, 3H, CH₃); 2.54 (q, ³J_{HH} = 7 Hz, 2H, CH₂); 3.51 (s, 2H, CH₂); 4.01 (q, ³J_{HH} = 7 Hz, 2H,OCH₂); 6.85 (s, 1H, Ph-H); 6.87 (s, 1H, Ph-H); 12.2 (s 1H, CO₂H) ppm.

MS/CI:243 (M+I).

### Anwendungsbeispiele:

### Beispiel A

### Post-emergence-Test

| | |
|---|---|
| Lösungsmittel: | 5 Gewichtsteile Aceton |
| Emulgator: | 1 Gewichtsteil Alkyarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | = keine Wirkung (wie unbehandelte Kontrolle) |
| 100 % | = totale Vernichtung |

### Beispiel B

### Pre-emergence-Test

| | |
|---|---|
| Lösungsmittel: | 5 Gewichtsteile Aceton |
| Emulgator: | 1 Gewichtsteil Alkyarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % | = keine Wirkung (wie unbehandelte Kontrolle) |
| 100 % | = totale Vernichtung |

| post-emergence | Gewächshaus | g a.i./ha | Zuckerrüben | Alopecurus | Avena fatua | Echinochloa | Setaria | Abutilon | Sinapis |
|---|---|---|---|---|---|---|---|---|---|
| Bsp. I-1-a-3 | | 250 | 0 | 100 | 100 | 100 | 100 | 70 | 80 |

| post-emergence | Gewächshaus | g a.i./ha | Zuckerrüben | Alopecurus | Avena fatua | Echinochloa | Setaria |
|---|---|---|---|---|---|---|---|
| Bsp.I-1-a-4 | | 250 | 0 | 100 | 100 | 100 | 100 |

| post-emergence | Gewächshaus | g a.i./ha | Alopecurus | Avena fatua | Echinochloa | Setaria | Sinapis |
|---|---|---|---|---|---|---|---|
| Bsp. I-1-a-5 | | 250 | 100 | 100 | 100 | 100 | 80 |
| Bsp. I-1-a-6 | | 250 | 90 | 100 | 100 | - | 80 |

| post-emergence | Gewächshaus | g a.i./ha | Zuckerrüben | Alopecurus | Avena fatua | Setaria | Amaranthus |
|---|---|---|---|---|---|---|---|
| Bsp. I-1-a-1 | | 250 | 0 | 100 | 100 | 100 | - |
| Bsp. I-1-a-2 | | 250 | 0 | - | 100 | 100 | 90 |

| pre-emergence | Gewächshaus | g a.i./ha | Alopecurus | Avena fatua | Echinochloa | Setaria | Abutilon | Sinapis |
|---|---|---|---|---|---|---|---|---|
| Bsp. I-1-a-3 | | 250 | 80 | 100 | 100 | 100 | 80 | 80 |
| Bsp. I-1-a-4 | | 250 | 90 | 100 | 100 | 100 | - | 90 |

| pre-emergence | Gewächshaus | g a.i./ha | Alopecurus | Avena fatua | Echinochloa | Setaria |
|---|---|---|---|---|---|---|
| Bsp. I-1-a-5 | | 250 | 90 | 100 | 100 | 100 |

| pre-emergence | Gewächshaus | g a.i./ha | Zuckerrüben | Alopecurus | Lolium | Setaria | Cassia | Matricaria | Viola |
|---|---|---|---|---|---|---|---|---|---|
| Bsp. I-1-a-1 | | 125 | 0 | 100 | 100 | 100 | 100 | 90 | 90 |

| pre-emergence | Gewächshaus | g a.i./ha | Zuckerrüben | Alopecurus | Avena fatua | Setaria |
|---|---|---|---|---|---|---|
| Bsp. I-1-a-2 | | 250 | 0 | 100 | 80 | 80 |

### Beispiel C

### Kulturpflanzenverträglichkeit im Nachauflauf

Samen von mono- bzw. dikotylen Unkraut- bzw. Kulturpflanzen werden in Holzfasertöpfen oder in Plastiktöpfen in sandigem Lehmboden ausgelegt, mit Erde abgedeckt und im Gewächshaus, während der Vegetationsperiode auch im Freien außerhalb des Gewächshauses, unter guten Wachstumsbedingungen angezogen. 2 bis 3 Wochen nach der Aussaat werden die Versuchspflanzen im Ein- bis Drei-Blattstadium behandelt. Die als Spritzpulver (WP) oder Flüssigkeit (EC) formulierten Testverbindungen werden in verschiedenen Dosierungen mit einer Wasseraufwandmenge von umgerechnet 3001/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der Präparate visuell im Vergleich zu unbehandelten Kontrollen bonitiert (herbizide Wirkung in Prozent (%): 100 % Wirkung = Pflanzen sind abgestorben, 0 % Wirkung = wie Kontrollpflanzen).

### Verwendung von Safenern

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen).
- Der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz im Vergleich zu unbehandelten Kontrollpflanzen beurteilt werden.

### Ergebnisse aus Gewächshausversuchen mit Safener/Vorbehandlung (Safener Mefenpyr, (100 g a.i./ha), einen Tag vor der Nachauflaufbehandlung des Herbizids)

**Tabelle 1'**

| | Aufwandmenge g a.i./ha | Sommergerste beobachtet (%) |
|---|---|---|
| Beispiel I-1-c-7 | 50 | 85 |
| | 25 | 30 |
| | 13 | 15 |
| Beispiel I-1-c-7 | 500 + 100 | 50 |
| + Mefenpyr | 25 + 100 | 10 |
| | 13 + 100 | 5 |

**Tabelle 2'**

| | Aufwandmenge g a.i./ha | Sommergerste beobachtet (%) | Sommerweizen beobachtet (%) |
|---|---|---|---|
| Beispiel I-1-c-8 | 100 | | 55 |
| | 50 | 97 | 50 |
| | 25 | 35 | |
| Beispiel I-1-c-8 | 100 + 100 | | 20 |
| + Mefenpyr | 50 + 100 | 50 | 15 |
| | 25 + 100 | 15 | |

**Tabelle 3'**

| | Aufwandmenge g a.i./ha | Sommergerste beobachtet (%) | Sommerweizen beobachtet (%) |
|---|---|---|---|
| Beispiel I-2-a-1 | 100 | 20 | 30 |
| | 50 | 15 | 20 |
| | 25 | 10 | 20 |
| Beispiel I-2-a-1 | 100 + 100 | 10 | 15 |
| + Mefenpyr | 50 + 100 | 0 | 10 |
| | 25 + 100 | 0 | 5 |

**Tabelle 4'**

| | Aufwandmenge g a.i./ha | Sommergerste beobachtet (%) |
|---|---|---|
| Beispiel I-2-a-2 | 100 | 97 |
| | 50 | 60 |
| | 25 | 60 |
| Beispiel I-2-a-2 | 100 + 100 | 40 |
| + Mefenpyr | 50 + 100 | 30 |
| | 25 + 100 | 20 |

**Tabelle 5'**

| | Aufwandmenge g a.i./ha | Sommerweizen beobachtet (%) |
|---|---|---|
| Beispiel I-2-c-1 | 100 | 30 |
| | 50 | 20 |
| Beispiel I-2-c-1 | 100 + 100 | 10 |
| + Mefenpyr | 50 + 100 | 5 |

**Tabelle 6'**

| | Aufwandmenge g a.i./ha | Sommergerste beobachtet (%) | Sommerweizen beobachtet (%) |
|---|---|---|---|
| Beispiel I-2-b-1 | 100 | 20 | 60 |
| | 50 | 10 | 40 |
| | 25 | | 20 |
| Beispiel I-2-b-1 | 100 + 100 | 10 | 15 |
| + Mefenpyr | 50 +100 | 0 | 10 |
| | 25 + 100 | | 5 |

**Tabelle 7'**

| | Aufwandmenge g a.i./ha | Sommergerste beobachtet (%) |
|---|---|---|
| Beispiel I-2-b-6 | 100 | 98 |
| | 50 | 97 |
| | 25 | 50 |
| Beispiel I-2-b-6 | 100 + 100 | 20 |
| + Mefenpyr | 50 + 100 | 15 |
| | 25 + 100 | 10 |

**Tabelle 8'**

| | Aufwandmenge g a.i./ha | Sommerweizen beobachtet (%) |
|---|---|---|
| Beispiel I-1-c-1 | 100 | 70 |
| | 50 | 20 |
| | 25 | 20 |
| Beispiel + Mefenpyr | 100 + 100 | 55 |
| | 50 + 100 | 5 |
| | 25 + 100 | 5 |

**Tabelle 9'**

| | Aufwandmenge g a.i./ha | Sommergerste beobachtet (%) | Sommerweizen beobachtet (%) |
|---|---|---|---|
| Beispiel I-2-b-4 | 100 | 80 | |
| | 25 | | 100 |
| Beispiel I-2-b-4 | 100 + 100 | 20 | |
| + Mefenpyr | 25 + 100 | | 20 |

**Tabelle 10'**

| | Aufwandmenge g a.i./ha | Sommergerste beobachtet (%) | Sommerweizen beobachtet (%) |
|---|---|---|---|
| Beispiel I-2-b-5 | 100 | 100 | |
| | 50 | 97 | |
| | 25 | 50 | 99 |
| Beispiel I-2-b-5 | 100 + 100 | 30 | |
| + Mefenpyr | 50 + 100 | 20 | |
| | 25 + 100 | 10 | 20 |

### Getreideversuch im Gewächshaus mit 100 g a.i./ha Mefenpyr im Nachauflauf; Auswertung 21 Tage nach Applikation

**Tabelle 11'**

| | Aufwandmenge g a.i./ha | Wintergerste (%) | Winterweizen (%) |
|---|---|---|---|
| Beispiel I-1-a-6 | 50 | 20 | 10 |
| Beispiel I-1-a-6 + Mefenpyr | 50 + 100 | 0 | 3 |

### Gefäßversuche mit Getreide außerhalb des Gewächshauses

### Herbizid: Mefenpyr 1:2 Tankmischung

**Tabelle F'-1**

| | Aufwandmenge g a.i./ha | Sommergerste beobachtet (%) | Sommerweizen beobachtet (%) |
|---|---|---|---|
| Beispiel I-2-a-2 | 50 | 20 | 95 |
| Beispiel I-2-a-2 + Mefenpyr | 50 + 100 | 5 | 15 |

**Tabelle F'-2**

| | Aufwandmenge g a.i./ha | Sommergerste beobachtet (%) | Sommerweizen beobachtet (%) |
|---|---|---|---|
| Beispiel I-2-c-1 | 100 | 15 | 40 |
| Beispiel I-2-c-1 + Mefenpyr | 100 + 200 | 0 | 5 |

**Tabelle F'- 3**

| | Aufwandmenge g a.i./ha | Sommergerste beobachtet (%) | Sommerweizen beobachtet (%) |
|---|---|---|---|
| Beispiel I-2-c-2 | 100 | 100 | 100 |
| Beispiel I-2-c-2 + Mefenpyr | 100 + 200 | 15 | 70 |

**Tabelle F'-4**

| | Aufwandmenge g a.i./ha | Sommergerste beobachtet (%) | Sommerweizen beobachtet (%) |
|---|---|---|---|
| Beispiel I-2-b-1 | 100 | 20 | 30 |
| Beispiel I-2-b-1 + Mefenpyr | 100 + 200 | 0 | 0 |

**Tabelle F'-5**

| | Aufwandmenge g a.i./ha | Sommergerste beobachtet (%) | Sommerweizen beobachtet (%) |
|---|---|---|---|
| Beispiel I-2-b-6 | 50 | 40 | 100 |
| Beispiel I-2-b-6 + Mefenpyr | 50 + 100 | 10 | 15 |

**Tabelle F'-6**

| | Aufwandmenge g a.i./ha | Sommergerste beobachtet (%) | Sommerweizen beobachtet (%) |
|---|---|---|---|
| Beispiel I-1-c-1 | 100 | 98 | 98 |
| Beispiel I-1-c-1 + Mefenpyr | 100+200 | 20 | 65 |

**Tabelle F'-7**

| | Aufwandmenge g a.i./ha | Sommergerste beobachtet (%) |
|---|---|---|
| Beispiel I-1-b-4 | 50 | 60 |
| Beispiel I-1-b-4 + Mefenpyr | 50 + 100 | 15 |

### Gefäßversuche mit Getreide außerhalb des Gewächshauses

### Herbizid: Mefenpyr g a.i./ha : 50 g a.i./ha

**Tabelle F'-8**

| | Aufwandmenge g a.i./ha | Sommergerste beobachtet (%) |
|---|---|---|
| Beispiel I-2-c-2 | 100 | 98 |
| Beispiel I-2-c-2 + Mefenpyr | 100 + 50 | 25 |

**Tabelle F'-9**

| | Aufwandmenge g a.i./ha | Sommergerste beobachtet (%) | Sommerweizen beobachtet (%) |
|---|---|---|---|
| Beispiel I-2-c-1 | 100 | 50 | 65 |
| Beispiel I-2-c-1 + Mefenpyr | 100+50 | 5 | 5 |

### Beispiel D

### Aphis-Test kontakt

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Triebe von jungen Dicken Bohnen-Pflanzen (Vicia faba),die stark von der Schwarzen Bohnenblattlaus (Aphis fabae) befallen sind, werden durch Tauchen in die Wirkstoff zubereitung der gewünschten Konzentration behandelt.

Nach der gewünschten Zeit wird die Abtötung in % bestimmt. Dabei bedeutet 100 %, dass alle alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

**Tabelle D**

| **pflanzenschädigende Insekten** | | |
|---|---|---|
| **Aphis fabae kontakt-Test** | | |
| Wirkstoffe | Wirkstoffkonzentration in ppm | Abtötungsgrad in % nach 6^{d} |
| Bsp.I-1-a-1 | 1000 | 100 |

### Beispiel E

### Meloidogyne-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Bei diesem Test zeigen z. B. die folgenden Verbindungen der Herstellungsbeispiele gute Wirksamkeit:

**Tabelle E**

| **pflanzenschädigende Nematoden** | | |
|---|---|---|
| **Meloidogyne-Test** | | |
| Wirkstoffe | Wirkstoffkonzentration in ppm | Abtötungsgrad in % nach 14^{d} |
| Bsp. I-1-a-3 | 20 | 98 |
| Bsp. I-1-a-4 | 20 | 98 |

### Beispiel F

### Myzus-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Grünen Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele gute Wirksamkeit:

**Tabelle F**

| pflanzenschädigende Insekten | | |
|---|---|---|
| **Myzus-Test** | | |
| Wirkstoffe | Wirkstoffkonzentration in ppm | Abtötungsgrad in % nach 6^{d} |
| Bsp. I-1-a-1 | 1000 | 95 |
| Bsp. 1-1-a-2 | 1000 | 100 |
| Bsp. I-1-a-3 | 1000 | 90 |

### Beispiel G

### Nephotettix-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Blätter noch feucht sind.

Nach der gewünschten Zeit wird die Abtötung in % bestimmt. Dabei bedeutet 100 %, dass alle Zikaden abgetötet wurden; 0 % bedeutet, dass keine Zikaden abgetötet wurden.

Bei diesem Test zeigen z. B. die folgenden Verbindungen der Herstellungsbeispiele gute Wirksamkeit:

**Tabelle G**

| **pflanzenschädigende Insekten** | | |
|---|---|---|
| **Nephotettix-Test** | | |
| Wirkstoffe | Wirkstoffkonzentration in ppm | Abtötungsgrad in % nach 6^{d} |
| Bsp.I-1-a-1 | 1000 | 100 |
| Bsp. I-1-a-2 | 1000 | 100 |

### Beispiel H

### Phaedon-Larven-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit:

**Tabelle H**

| pflanzenschädigende Insekten | | |
|---|---|---|
| **Phaedon-Larven-Test** | | |
| Wirkstoffe | Wirkstoffkonzentration in ppm | Abtötungsgrad in % nach 7^{d} |
| Bsp. I-1-a-1 | 1000 | 100 |
| Bsp.I-1-a-2 | 1000 | 100 |
| Bsp.I-1-a-3 | 1000 | 80 |
| Bsp. I-1-a-4 | 1000 | 100 |
| Bsp.I-1-a-6 | 1000 | 90 |

### Beispiel I

### Plutella-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit:

**Tabelle I**

| pflanzenschädigende Insekten | | |
|---|---|---|
| **Plutella-Test** | | |
| Wirkstoffe | Wirkstoffkonzentration in ppm | Abtötungsgrad in % nach 7^{d} |
| Bsp. I-1-a-1 | 1000 | 100 |
| Bsp. I-1-a-2 | 1000 | 100 |

### Beispiel J

### Spodoptera frugiperda-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen d es Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit:

**Tabelle J**

| pflanzenschädigende Insekten | | |
|---|---|---|
| **Spodoptera frugiperda -Test** | | |
| Wirkstoffe | Wirkstoffkonzentration in ppm | Abtötungsgrad in % nach 7^{d} |
| Bsp. I-1-a-1 | 1000 | 100 |

### Beispiel K

### Tetranychus-Test (OP-resistent/Tauchbehandlung)

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der Gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit:

**Tabelle K**

| pflanzenschädigende Milben | | |
|---|---|---|
| **Tetranychus-Test** (OP-resistent/Tauchbehandlung) | | |
| Wirkstoffe | Wirkstoffkonzentration in ppm | Abtötungsgrad in % nach 7^{d} |
| Bsp. I-1-a-1 | 1000 | 100 |
| Bsp. I-1-a-2 | 1000 | 95 |
| Bsp. I-1-a-3 | 100 | 80 |

### Beispiel L

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel M

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I), 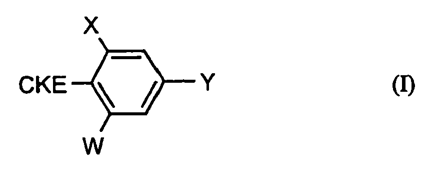 in welcher
W für Alkoxy, Halogenalkoxy, Alkoxyalkyloxy, Alkoxy-bis-alkyloxy oder gege- benenfalls substituiertes Cycloalkyl-alkandiyl-oxy, welches gegebenenfalls durch Heteroatome unterbrochen sein kann, steht,
X für Alkyl steht,
Y für Chlor, Brom oder Iod steht,
CKE für eine der Gruppen 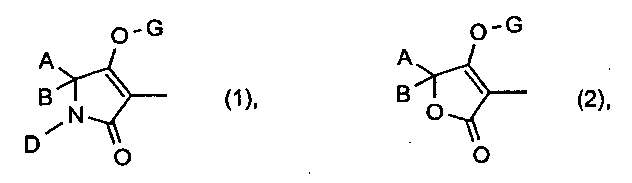 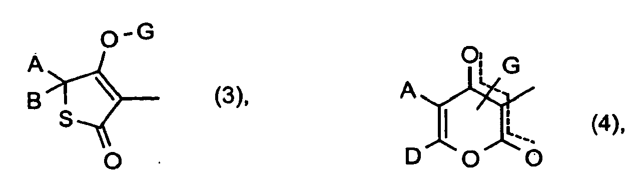 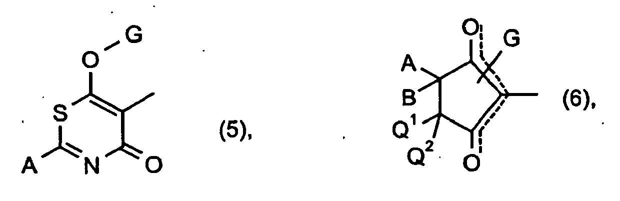 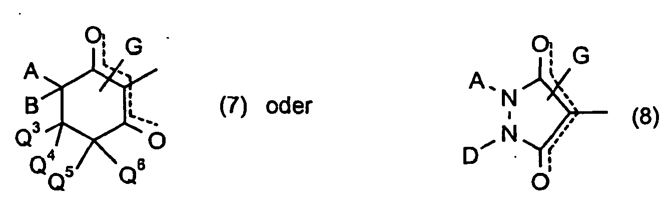 steht
worin
A für Wasserstoff, jeweils gegebenenfalls durch Halogen substituiertes Alkyl, Alkenyl, Alkoxyalkyl, Alkylthioalkyl, gesättigtes oder ungesättigtes, gegebenen- falls substituiertes Cycloalkyl, in welchem gegebenenfalls mindestens ein Ring- atom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, oder
A und B gemeinsam mit dem Kohlenstoffatom an das sie gebunden sind für einen ge- sättigten oder ungesättigten, gegebenenfalls mindestens ein Heteroatom ent- haltenden unsubstituierten oder substituierten Cyclus stehen,
D für Wasserstoff oder einen gegebenenfalls substituierten Rest aus der Reihe Alkyl, Alkenyl, Alkinyl, Alkoxyalkyl, gesättigtes oder ungesättigtes Cycloalkyl, in welchem gegebenenfalls eines oder mehrere Ringglieder durch Heteroatome er- setzt sind, Arylalkyl, Aryl, Hetarylalkyl oder Hetaryl steht oder
A und D gemeinsam mit den Atomen an die sie gebunden sind für einen gesättigten oder ungesättigten und gegebenenfalls mindestens ein (im Falle CKE=8 ein weiteres) Heteroatom enthaltenden, im A,D-Teil unsubstituierten oder substituierten Cyclus stehen, bzw.
A und Q¹ gemeinsam für gegebenenfalls durch Hydroxy, jeweils gegebenenfalls substi- tuiertes Alkyl, Alkoxy, Alkylthio, Cycloalkyl, Benzyloxy oder Aryl substituiertes Alkandiyl oder Alkendiyl stehen oder
Q¹ für Wasserstoff oder Alkyl steht,
Q², Q⁴, Q⁵ und Q⁶ unabhängig voneinander für Wasserstoff oder Alkyl stehen,
Q³ für W asserstoff, für g egebenenfalls substituiertes Alkyl, Alkoxyalkyl, Alkylthio- alkyl, gegebenenfalls substituiertes Cycloalkyl (worin gegebenenfalls eine Methylengruppe durch Sauerstoff oder Schwefel ersetzt ist) oder gegebenenfalls substituiertes Phenyl steht, oder
Q³ und Q⁴ gemeinsam mit dem Kohlenstoffatom, an das sie gebunden sind, für einen ge- sättigten oder ungesättigten, gegebenenfalls ein Heteroatom enthaltenden un- substituierten oder substituierten Cyclus stehen,
G für Wasserstoff (a) oder für eine der Gruppen 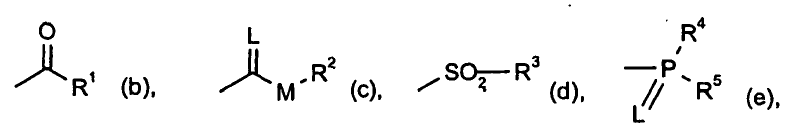 E (f) oder 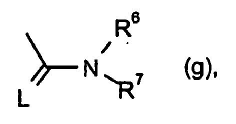 steht,
worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl, Polyalkoxyalkyl oder gegebenenfalls durch Halogen, Alkyl oder Alkoxy substituiertes Cycloalkyl, das durch mindestens ein Heteroatom unterbrochen sein kann, jeweils gegebenenfalls substituiertes Phenyl, Phenylalkyl, Hetaryl, Phenoxyalkyl oder Hetaryloxy- alkyl steht,
R² für jeweils gegebenenfalls durch Halogen substituiertes Alkyl, Alkenyl, Alkoxyalkyl, Polyalkoxyalkyl oder für jeweils gegebenenfalls sub- stituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alke- nylthio, Cycloalkylthio oder für jeweils gegebenenfalls substituiertes Phe- nyl, Benzyl, Phenoxy oder Phenylthio stehen,
R⁶ und R⁷ unabhängig voneinander für Wasserstoff, jeweils gegebenenfalls durch Halogen substituiertes Alkyl, Cycloalkyl, Alkenyl, Alkoxy, Alkoxyalkyl, für gegebenenfalls substituiertes Phenyl, für gegebenenfalls substituiertes Benzyl stehen, oder gemeinsam mit dem N-Atom, an das sie gebunden sind, für einen gegebenenfalls durch Sauerstoff oder Schwefel unterbro- chenen Cyclus stehen.

2. Verbindungen der Formel (I) gemäß Anspruch 1, in welcher
W für C₁-C₆-Alkoxy, C₁-C₆-Halogenalkoxy, C₁-C₄-Alkoxy-C₂-C₄-alkyloxy, C₁-C₄- Alkoxy-bis-C₂-C₄-alkyloxy oder gegebenenfalls einfach bis dreifach durch Flur, Chlor, C₁-C₃-Alkyl oder C₁-C₃-Alkoxy substituiertes C₃-C₆-Cycloalkyl-C₁-C₃- alkandiyl-oxy steht, worin gegebenenfalls eine Methylengruppe des Rings durch Sauerstoff oder Schwefel unterbrochen sein kann,
X für C₁-C₆-Alkyl steht,
Y für Chlor, Brom oder Iod steht,
CKE für eine der Gruppen 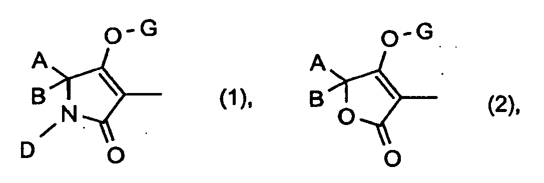 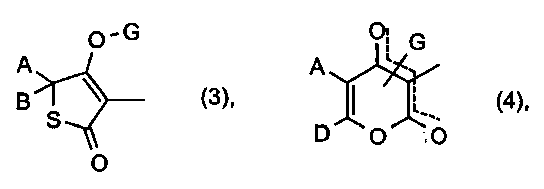 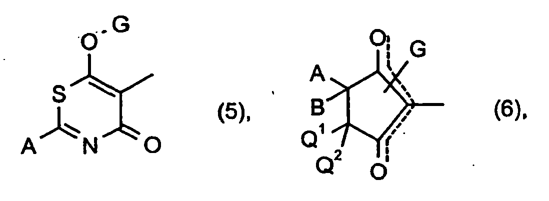 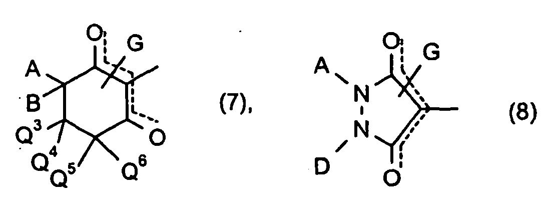 steht.
A für Wasserstoff oder jeweils gegebenenfalls durch Halogen substituiertes C₁-C₁₂- Alkyl, C₃-C₈-Alkenyl, C₁-C₁₀-Alkoxy-C₁-C₈-alkyl, C₁-C₁₀-Alkylthio-C₁-C₆- alkyl, gegebenenfalls durch Halogen, C₁-C₆-Alkyl oder C₁-C₆-Alkoxy sub- stituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆- Alkoxy, C₁-C₆-Halogenalkoxy, Cyano oder Nitro substituiertes Phenyl, Naphthyl, Hetaryl mit 5 bis 6 Ringatomen, Phenyl-C₁-C₆-alkyl oder Naphthyl-C₁-C₆-alkyl, steht
B für Wasserstoff, C₁-C₁₂-Alkyl oder C₁-C₈-Alkoxy-C₁-C₆-alkyl steht, oder
A, B und das Kohlenstoffatom an das sie gebunden sind, für gesättigtes C₃-C₁₀-Cyclo- alkyl oder ungesättigtes C₅-C₁₀-Cycloallcyl stehen, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogen- alkyl, C₁-C₈-Alkoxy, C₁-C₈-Alkylthio, Halogen oder Phenyl substituiert sind oder
A, B und das Kohlenstoffatom, an das sie gebunden sind, für C₃-C₆-Cycloalkyl stehen, welches durch eine gegebenenfalls ein oder zwei nicht direkt benachbarte Sauer- stoff- und/oder Schwefelatome enthaltende gegebenenfalls durch C₁-C₄-Alkyl substituierte Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁- C₆-Alkyl, C₁-C₆-Alkoxy oder Halogen substituiertes C₂-C₆-Alkandiyl, C₂-C₆- Alkendiyl oder C₄-C₆-Alkandiendiyl stehen, worin gegebenenfalls eine Methylen- gruppe durch Sauerstoff oder Schwefel ersetzt ist,
D für Wasserstoff, jeweils gegebenenfalls durch Halogen substituiertes C₁-C₁₂- Alkyl, C₃-C₈-Alkenyl, C₃-C₈-Alkinyl, C₁-C₁₀-Alkoxy-C₂-C₈-alkyl, gegebenen- falls durch Halogen, C₁-C₄-Alkyl, C₁-C₄-Alkoxy oder C₁-C₄-Halogenalkyl sub- stituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Hetaryl mit 5 oder 6 Ringatomen, Phenyl-C₁-C₆- alkyl oder Hetaryl-C₁-C₆-alkyl mit 5 oder 6 Ringatomen steht oder
A und D gemeinsam für jeweils gegebenenfalls substituiertes C₃-C₆-Alkandiyl oder C₃- C₆-Alkendiyl stehen, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen sub- stituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆- Alkendiylgruppierung oder eine Butadienylgruppierung, die gegebenenfalls durch C₁-C₆-Alkyl substituiert ist oder in der gegebenenfalls zwei benachbarte Sub- stituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD- 1 bis AD-10), der Sauerstoff oder Schwefel enthalten kann, oder worin gege- benenfalls eine der folgenden Gruppen 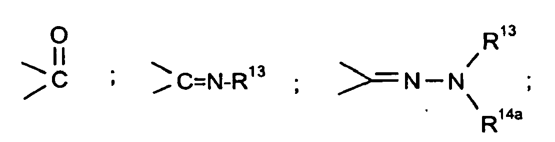 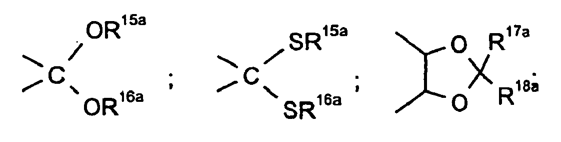 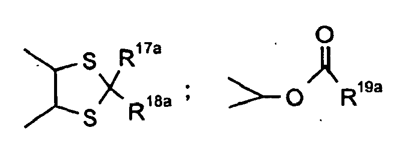 oder 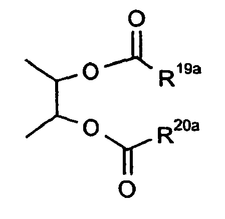 enthalten ist, oder
A und Q¹ gemeinsam für jeweils gegebenenfalls einfach oder zweifach, gleich oder ver- schieden durch Halogen, Hydroxy, durch jeweils gegebenenfalls einfach bis drei- fach, gleich oder verschieden durch Halogen substituiertes C₁-C₁₀-Alkyl. C₁-C₆- Alkoxy, C₁-C₆-Alkylthio, C₃-C₇-Cyloalkyl oder durch jeweils gegebenenfalls einfach bis dreifach, gleich oder verschieden durch Halogen, C₁-C₆-Alkyl oder C₁-C₆-Alkoxy substituiertes Benzyloxy oder Phenyl substituiertes C₃-C₆- Alkandiyl oder C₄-C₆-Alkendiyl stehen, welches außerdem gegebenenfalls eine der nachstehenden Gruppen 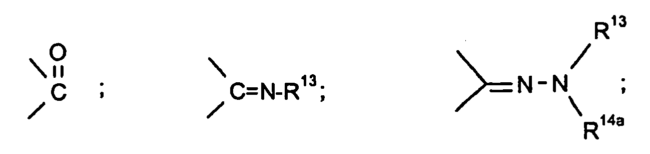 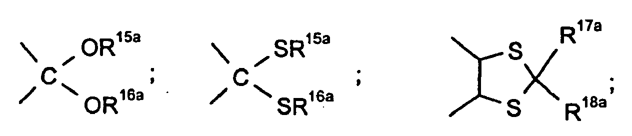 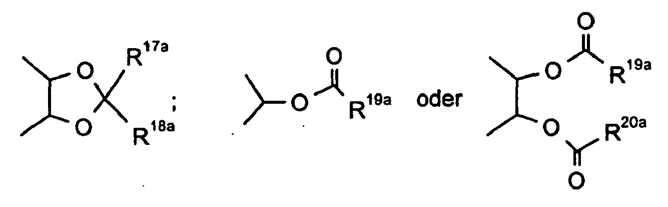 enthält oder durch eine C₁-C₂-Alkandiylgruppe oder durch ein Sauerstoffatom überbrückt ist oder
Q¹ für Wasserstoff oder C₁-C₄-Alkyl steht,
Q², Q⁴, Q⁵ und Q⁶ unabhängig voneinander für Wasserstoff oder C₁-C₄-Alkyl stehen,
Q³ für Wasserstoff, C₁-C₆-Alkyl, C₁-C₆-Alkoxy-C₁-C₂-alkyl, C₁-C₆-Alkylthio-C₁ C₂-alkyl, gegebenenfalls durch C₁-C₄-Alkyl oder C₁-C₄-Alkoxy substituiertes C₃-C₈-Cycloalkyl, worin gegebenenfalls eine Methylengruppe durch Sauerstoff oder Schwefel ersetzt ist oder gegebenenfalls durch Halogen, C₁-C₄-Alkyl, C₁-C₄-Alkoxy, C₁-C₂-Halogenalkyl, C₁-C₂-Halogenalkoxy, Cyano oder Nitro substituiertes Phenyl steht oder
Q³ und Q⁴ gemeinsam mit dem Kohlenstoffatom, an das sie gebunden sind, für einen ge- gebenenfalls durch C₁-C₄-Alkyl, C₁-C₄-Alkoxy oder C₁-C₂-Halogenalkyl sub- stituierten C₃-C₇-Ring stehen, in welchem gegebenenfalls ein Ringglied durch Sauerstoff oder Schwefel ersetzt ist,
G für Wasserstoff (a) oder für eine der Gruppen 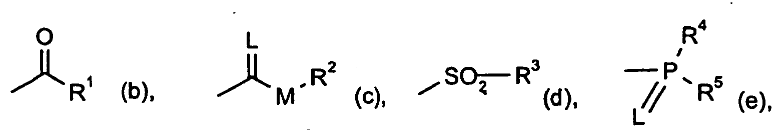 E (f) oder 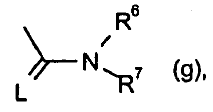 steht
in welchen
E für ein Metallionäquivalent oder ein Ammoniumion steht,
L für Sauerstoff oder Schwefel steht und
M für Sauerstoff oder Schwefel steht.
R¹ für jeweils gegebenenfalls durch Halogen substituiertes C₁-C₂₀-Alkyl, C₂-C₂₀- Alkenyl, C₁-C₈-Alkoxy-C₁-C₈-alkyl, C₁-C₈-Alkylthio-C₁-C₈-alkyl, Poly-C₁-C₈- alkoxy-C₁-C₈-alkyl oder gegebenenfalls durch Halogen, C₁-C₆-Alkyl oder C₁-C₆- Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkyl- sulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆- alkyl,
für gegebenenfalls durch Halogen oder C₁-C₆-Alkyl substituiertes 5- oder 6- gliedriges Hetaryl,
für gegebenenfalls durch Halogen oder C₁-C₆-Alkyl substituiertes Phenoxy-C₁- C₆-alkyl oder
für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀- Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆- Alkoxy, C₁-C₆-Halogenalkyl oder C₁-C₆-Halogenalkoxy substituiertes Phenyl oder Benzyl, steht
R³ für gegebenenfalls durch Halogen substituiertes C₁-C₈-Alkyl oder für jeweils ge- gebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl, steht
R⁴ und R⁵ unabhängig voneinander für jeweils gegebenenfalls durch Halogen substituier- tes C₁-C₈-Alkyl, C₁-C₈-Alkoxy, C₁-C₈-Alkylamino, Di-(C₁-C₈-alkyl)amino, C₁- C₈-Alkylthio, C₂-C₈-Alkenylthio, C₃-C₇-Cycloalkylthio oder für jeweils ge- gebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogen- alkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈- Alkenyl, C₁-C₈-Alkoxy-C₁-C₈-alkyl, für gegebenenfalls durch Halogen, C₁-C₈- Halogenalkyl, C₁-C₈-Alkyl oder C₁-C₈-Alkoxy substituiertes Phenyl, gegebe- nenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlen- stoffatom durch Sauerstoff oder Schwefel ersetzt ist,
R¹³ für Wasserstoff, für jeweils gegebenenfalls durch Halogen substituiertes C₁ -C₈- Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gege- benenfalls durch Halogen, C₁-C₆-Alkyl, C₁-C₆-Alkoxy, C₁-C₄-Halogenalkyl, C₁- C₄-Halogenalkoxy, Nitro oder Cyano substituiertes Phenyl, Phenyl-C₁-C₄-alkyl oder Phenyl-C₁-C₄-alkoxy steht,
R^{14a} für Wasserstoff oder C₁-C₈-Alkyl steht, oder
R¹³ und R^{14a} gemeinsam für C₄-C₆-Alkandiyl stehen,
R^{15a} und R^{16a} sind gleich oder verschieden und für C₁-C₆-Alkyl stehen, oder
R^{15a} und R^{16a} gemeinsam für einen C₂-C₄-Alkandiylrest stehen, der gegebenenfalls durch C₁-C₆-Alkyl, C₁-C₆-Halogenalkyl oder durch gegebenenfalls durch Halo- gen, C₁-C₆-Alkyl, C₁-C₄-Halogenalkyl, C₁-C₆-Alkoxy, C₁-C₄-Halogenalkoxy, Nitro oder Cyano substituiertes Phenyl substituiert ist,
R^{17a} und R^{18a} unabhängig voneinander für Wasserstoff, für gegebenenfalls durch Halogen substituiertes C₁-C₈-Alkyl oder für gegebenenfalls durch Halogen, C₁- C₆-Alkyl, C₁-C₆-Alkoxy, C₁-C₄-Halogenalkyl, C₁-C₄-Halogenalkoxy, Nitro oder Cyano substituiertes Phenyl stehen, oder
R^{17a} und R^{18a} gemeinsam mit dem Kohlenstoffatom, an das sie gebunden sind, für eine Carbonylgruppe oder für gegebenenfalls durch Halogen, C₁-C₄-Alkyl oder C₁-C₄- Alkoxy substituiertes C₅-C₇-Cyloalkyl stehen, in dem gegebenenfalls eine Methylengruppe durch Sauerstoff oder Schwefel ersetzt ist,
R^{19a} und R^{20a} unabhängig voneinander für C₁-C₁₀-Alkyl, C₂-C₁₀-Alkenyl, C₁-C₁₀- Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen.

3. Verbindungen der Formel (I) gemäß Anspruch 1, in welcher
W für C₁-C₄-Alkoxy, C₁-C₄-Halogenalkoxy, C₁-C₃-Alkoxy-C₂-C₃-alkyloxy, C₁-C₂- Alkoxy-bis-C₂-C₃-alkyloxy oder C₃-C₆-Cycloalkyl-C₁-C₂-alkandiyloxy steht, worin gegebenenfalls eine Methylengruppe des Rings durch Sauerstoff unterbrochen sein kann,
X für C₁-C₃-Alkyl steht,
Y für Chlor oder Brom steht,
CKE für eine der Gruppen 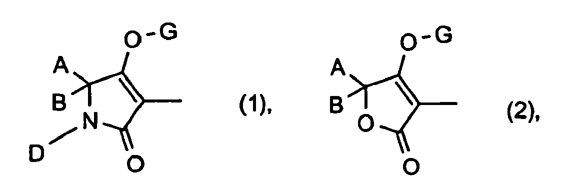 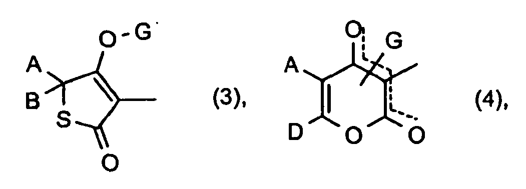 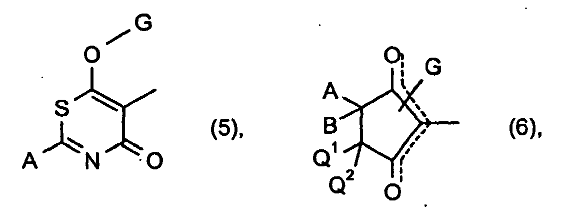 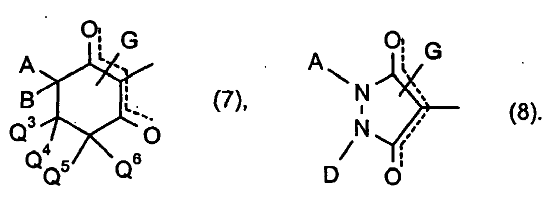 steht.
A für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls ein- fach bis zweifach durch C₁-C₂-Alkyl oder C₁-C₂-Alkoxy substituiertes C₃-C₆- Cycloalkyl oder (jedoch nicht im Fall der Verbindungen der Formeln (I-3), (I-4), (I-6) und (I-7)) jeweils einfach bis zweifach gegebenenfalls durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl oder Benzyl steht,
B für Wasserstoff, C₁-C₄-Alkyl oder C₁-C₂-Alkoxyl-C₁-C₂-alkyl steht, oder
A, B und das Kohlenstoffatom an das sie gebunden sind, für gesättigtes oder unge- sättigtes C₅-C₇-Cyloalkyl stehen, worin gegebenenfalls ein Ringglied durch Sauerstoff oder Schwefel ersetzt ist und welches gegebenenfalls einfach bis zwei- fach durch C₁-C₆-Alkyl, Trifluormethyl oder C₁-C₆-Alkoxy, substituiert ist mit der Maßgabe, dass dann Q³ für Wasserstoff oder Methyl steht oder
A, B und das Kohlenstoffatom, an das sie gebunden sind, für C₅-C₆-Cycloalkyl stehen, welches durch eine gegebenenfalls ein oder zwei nicht direkt benachbarte Sauer- stoff- oder Schwefelatome enthaltende gegebenenfalls durch Methyl oder Ethyl substituierte Alkylendiyl- oder durch eine Alkylendioxyl- oder durch eine Alkylen- dithiol-Gruppe substituiert ist, die mit dem Kohlenstoffatom, an das sie gebunden ist, einen weiteren fünf- oder sechsgliedrigen Ring bildet mit der Maßgabe, dass dann Q³ für Wasserstoff oder Methyl steht,
A, B und das Kohlenstoffatom, an das sie gebunden sind, für C ₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alken- diyl oder Butadiendiyl stehen, mit der Maßgabe, dass dann Q³ besonders bevor- zugt für Wasserstoff oder Methyl steht,
D für Wasserstoff, für jeweils gegebenenfalls einfach bis dreifach durch Fluor sub- stituiertes C₁-C₆-Alkyl, C₃-C₆-Alkenyl, C₁-C₄-Alkoxy-C₂-C₃-alkyl, für gege- benenfalls einfach bis zweifach durch C₁-C₄-Alkyl, C₁-C₄-Alkoxy oder C₁-C₂- Halogenalkyl substituiertes C₃-C₆-Cycloalkyl, in welchem gegebenenfalls eine Methylengruppe durch Sauerstoff ersetzt ist oder (jedoch nicht im Fall der Ver- bindungen der Formeln (I-1)) für jeweils gegebenenfalls einfach bis zweifach durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl oder Pyridyl steht, oder
A und D gemeinsam für gegebenenfalls einfach bis zweifach substituiertes C₃-C₅- Alkandiyl stehen, in welchem eine Methylengruppe durch eine Carbonylgruppe (nicht jedoch im Fall der Verbindungen der Formel (I-1)), Sauerstoff oder Schwefel ersetzt sein kann, wobei als Substituenten C₁-C₂-Alkyl oder C₁-C₂- Alkoxy in Frage kommen oder
A und D (im Fall der Verbindungen der Formel (I-1)) gemeinsam mit den Atomen, an die sie gebunden sind, für eine der Gruppen AD-1 bis AD-10 stehen: 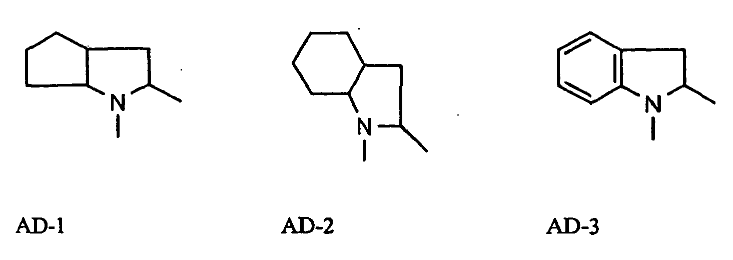 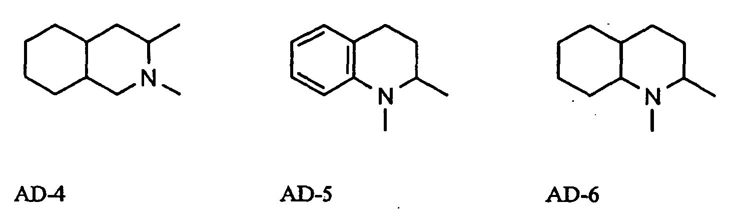 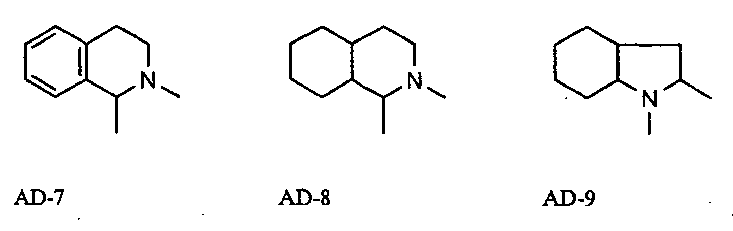 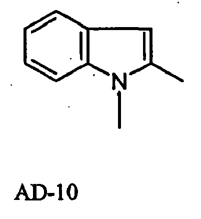 oder
A und Q¹ gemeinsam für jeweils gegebenenfalls einfach oder zweifach, gleich oder ver- schieden durch C₁-C₂-Alkyl oder C₁-C₂-Alkoxy substituiertes C₃-C₄-Alkandiyl stehen, oder
Q¹ für Wasserstoff steht,
Q² für Wasserstoff steht,
Q⁴, Q⁵ und Q⁶ unabhängig voneinander für Wasserstoff oder C₁-C₃-Alkyl stehen,
Q³ für Wasserstoff, C₁-C₄-Alkyl, oder gegebenenfalls einfach bis zweifach durch Methyl oder Methoxy substituiertes C₃-C₆-Cycloalkyl steht, oder
Q³ und Q⁴ gemeinsam mit dem Kohlenstoff, an das sie gebunden sind, für einen gege- benenfalls durch C₁-C₂-Alkyl oder C₁-C₂-Alkoxy substituierten gesättigten C₅-C₆-Ring stehen, in welchem gegebenenfalls ein Ringglied durch Sauerstoff oder Schwefel ersetzt ist, mit der Maßgabe, dass dann A für Wasserstoff oder Methyl steht, oder
G für Wasserstoff (a) oder für eine der Gruppen 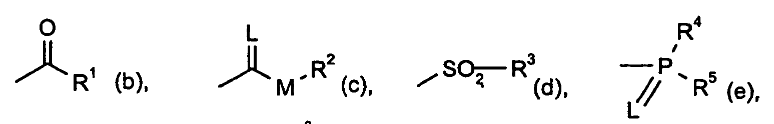 E (f) oder 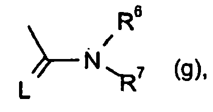 steht
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls einfach bis dreifach durch Fluor oder Chlor substituier- tes C₁-C₈-Alkyl, C₂-C₁₈-Alkenyl, C₁-C₄-Alkoxy-C₁-C₂-alkyl, C₁-C₄-Alkylthio- C₁-C₂₋alkyl oder gegebenenfalls einfach bis zweifach durch Fluor, Chlor, C₁-C₂- Alkyl oder C₁-C₂-Alkoxy substituiertes C₃-C₆-Cycloalkyl, in welchem gege- benenfalls ein oder zwei nicht direkt benachbarte Ringglieder durch Sauerstoff er- setzt sind, für gegebenenfalls einfach bis zweifach durch Fluor, Chlor, Brom, Cyano, Nitro, C₁-C₄-Alkyl, C₁-C₄-Alkoxy, C₁-C₂-Halogenalkyl oder C₁-C₂-Halogenalkoxy substituiertes Phenyl steht,
R² für jeweils gegebenenfalls einfach bis dreifach durch Fluor substituiertes C₁-C₈- Alkyl, C₂-C₈-Alkenyl oder C₁-C₄-Alkoxy-C₂-C₄-alkyl,
für gegebenenfalls einfach durch C₁-C₂-Alkyl oder C₁-C₂-Alkoxy substituiertes C₃-C₆-Cycloalkyl oder
für jeweils gegebenenfalls einfach bis zweifach durch Fluor, Chlor, Brom, Cyano, Nitro, C₁-C₄-Alkyl, C₁-C₃-Alkoxy, Trifluormethyl oder Trifluormethoxy substitu- iertes Phenyl oder Benzyl steht,
R³ für gegebenenfalls einfach bis dreifach durch Fluor substituiertes C₁-C₆-Alkyl oder für jeweils gegebenenfalls einfach durch Fluor, Chlor, Brom, C₁-C₄-Alkyl, C₁-C₄-Alkoxy, Trifluormethyl, Trifluormethoxy, Cyano oder Nitro substituiertes Phenyl steht,
R⁴ für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthiou oder für jeweils ge- gebenenfalls einfach durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder Trifluormethyl substituiertes Phenyl, Phenoxy oder Phenylthio, steht
R⁵ für C₁-C₆-Alkoxy oder C₁-C₆-Alkylthio steht,
R⁶ für Wasserstoff, C₁-C₆-Alkyl, C₃-C₆-Cycloalkyl, C₁-C₆-Alkoxy, C₃-C₆-Alkenyl, C₁-C₆-Alkoxy-C₁-C₄-alkyl, für gegebenenfalls einfach durch Fluor, Chlor, Brom, Trifluormethyl, C₁-C₄-Alkyl oder C₁-C₄-Alkoxy substituiertes Phenyl, für gege- benenfalls einfach durch Fluor, Chlor, Brom, C₁-C₄-Alkyl, Trifluormethyl oder C₁-C₄-Alkoxy substituiertes Benzyl steht,
R⁷ für C₁C₆-Alkyl, C₃-C₆-Alkenyl oder C₁-C₆-Alkoxy-C₁-C₄-alkyl steht,
R⁶ und R⁷ zusammen für einen gegebenenfalls durch Methyl oder Ethyl substituierten C₄-C₅-Alkylenrest stehen, in welchem gegebenenfalls eine Methylengruppe durch Sauerstoff oder Schwefel ersetzt ist.

4. Verbindungen der Formel (I), gemäß Anspruch 1, in welcher
W für Methoxy, Ethoxy, n-Propoxy, iso-Propoxy, n-Butoxy, iso-Butoxy, sec-Butoxy, Methoxy-ethyloxy, Ethoxy-ethyloxy, Cyclopropyl-methoxy, Cyclopentyl-methoxy oder Cyclohexyl-methoxy steht,
X für Methyl oder Ethyl steht,
Y für Chlor oder Brom steht,
CKE für eine der Gruppen 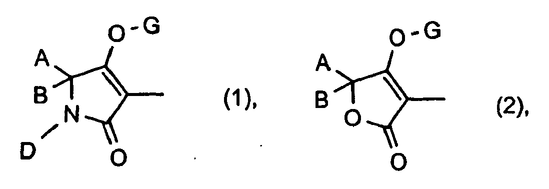 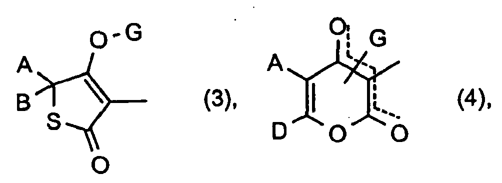 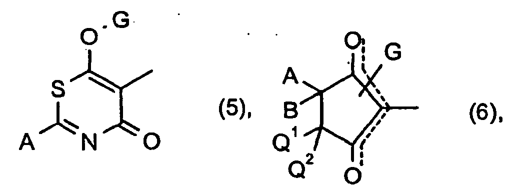 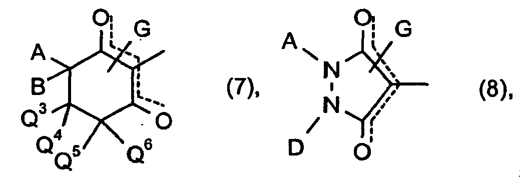 steht,
A für Wasserstoff, jeweils gegebenenfalls einfach bis dreifach durch Fluor substi- tuiertes C₁-C₄-Alkyl oder C₁-C₂-Alkoxy-C₁-C₂-alkyl, für Cyclopropyl, Cyclo- pentyl oder Cyclohexyl und nur im Fall der Verbindungen der Formel (1-5) für je- weils gegebenenfalls durch Fluor, Chlor, Brom, Methyl, Ethyl, n-Propyl, iso- Propyl, Methoxy, Ethoxy, Trifluormethyl, Trifluormethoxy, Cyano oder Nitro sub- stituiertes Phenyl steht,
B für Wasserstoff, Methyl oder Ethyl steht, oder
A, B und das Kohlenstoffatom an das sie gebunden sind, für gesättigtes C₅-C₆-Cyclo- alkyl stehen, in welchem gegebenenfalls ein Ringglied durch Sauerstoff oder Schwefel ersetzt ist und welches gegebenenfalls einfach durch Methyl, Ethyl, Propyl, Isopropyl, Trifluormethyl, Methoxy, Ethoxy, Propoxy oder Butoxy sub- stituiert ist, mit der Maßgabe, dass dann Q³ für Wasserstoff steht oder
A, B und das Kohlenstoffatom, an das sie gebunden sind, für C₆-Cyclbalkyl stehen, welches gegebenenfalls durch mit zwei nicht direkt benachbarten Sauerstoff- atomen enthaltende Alkylendioxyl-Gruppe substituiert ist, mit der Maßgabe, dass dann Q³ für Wasserstoff steht oder
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlen- stoffatomen, an die sie gebunden sind, für C₂-C₄-Alkandiyl oder C₂-C₄-Alkendiyl oder Butadiendiyl stehen, mit der Maßgabe, dass dann Q³ für Wasserstoff steht,
D für Wasserstoff, für jeweils gegebenenfalls einfach bis dreifach durch Fluor sub- stituiertes C₁-C₄-Alkyl, C₃-C₄-Alkenyl, C₁-C₄-Alkoxy-C₂-C₃-alkyl, für Cyclo- propyl, Cyclopentyl oder Cyclohexyl oder (jedoch nicht im Fall der Verbindungen der Formeln (I-1)) für gegebenenfalls einfach durch Fluor, Chlor, Methyl, Ethyl, n- Propyl, iso-Propyl, Methoxy, Ethoxy oder Trifluormethyl substituiertes Phenyl oder Pyridyl steht,
oder
A und D gemeinsam für gegebenenfalls einfach durch Methyl oder Methoxy substituiertes C₃-C₅-Alkandiyl stehen, worin gegebenenfalls ein Kohlenstoffatom durch Sauer- stoff oder Schwefel ersetzt ist oder für die Gruppe AD-1
A und Q¹ gemeinsam für gegebenenfalls einfach oder zweifach durch Methyl oder Methoxy substituiertes C₃-C₄-Alkandiyl stehen, oder
Q ¹ für Wasserstoff steht,
Q² für Wasserstoff steht,
Q⁴, Q⁵ und Q⁶ unabhängig voneinander für Wasserstoff oder Methyl stehen,
Q³ für Wasserstoff, Methyl, Ethyl oder Propyl steht, oder
Q³ und Q⁴ gemeinsam mit dem Kohlenstoff, an den sie gebunden sind, für einen gege- benenfalls einfach durch Methyl oder Methoxy substituierten gesättigten C₅-C₆- Ring stehen, mit der Maßgabe, dass dann A für Wasserstoff steht,
G für Wasserstoff (a) oder für eine der Gruppen 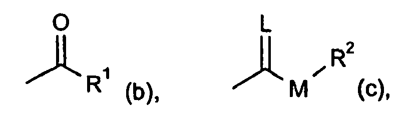 -SO₂-R³ (d) oder E (f) steht.
in welchen
L für Sauerstoff oder Schwefel steht,
M für Sauerstoff oder Schwefel steht und
E für ein Ammoniumion steht,
R¹ für C₁-C₆-Alkyl, C₂-C₁₇-Alkenyl, C₁-C₂-Alkoxy-C₁-alkyl, C₁-C₂-Alkylthio-C₁- alkyl oder jeweils gegebenenfalls einfach durch Fluor, Chlor, Methyl oder Methoxy substituiertes Cyclopropyl oder Cyclohexyl, für gegebenenfalls einfach durch Fluor, Chlor, Brom, Cyano, Nitro, Methyl, Methoxy, Trifluormethyl oder Trifluormethoxy substituiertes Phenyl steht,
R² für jeweils gegebenenfalls einfach durch Fluor substituiertes C₁-C₈-Alkyl, C₂-C₆- Alkenyl oder C₁-C₄-Alkoxy-C₂-C₃-alkyl, Phenyl oder Benzyl steht,
R³ für C₁-C₈-Alkyl steht.

5. Verbindungen der Formel (I) gemäß Anspruch 1, in welcher
W für Methoxy, Ethoxy, n-Propoxy, iso-Propoxy, n-Butoxy, iso-Butoxy, sec-Butoxy, Methoxy-ethyloxy, Ethoxy-ethyloxy oder Cyclopropyhnethoxy steht,
X für Methyl oder Ethyl steht,
Y für Chlor steht,
CKE für eine der Gruppen 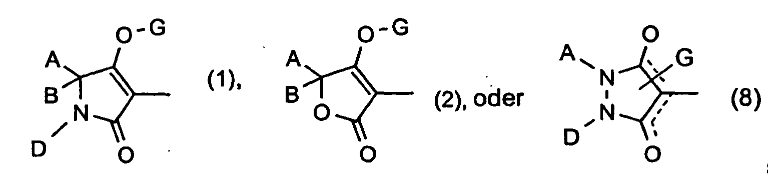 steht,
A für Wasserstoff, Methyl, Ethyl, Cyclopropyl, Isopropyl, n-Propyl, Isobutyl, n- Butyl, t-Butyl oder s-Butyl steht,
B für Wasserstoff, Methyl oder Ethyl steht,
A, B und das Kohlenstoffatom an das sie gebunden sind, für gesättigtes C₅-C₆- Cycloalkyl stehen, in welchem gegebenenfalls ein Ringglied durch Sauerstoff ersetzt ist und welches gegebenenfalls einfach durch Methyl, Methoxy, Ethoxy, n- Propoxy, n-Butoxy oder Trifluormethyl substituiert ist,
D für Wasserstoff, Methyl, Ethyl, Isopropyl, Cyclopropyl oder Cyclohexyl steht,
oder
A und D gemeinsam für C₃-C₅-Alkandiyl oder für die Gruppe AD-1 stehen,
G für Wasserstoff (a) oder für eine der Gruppen 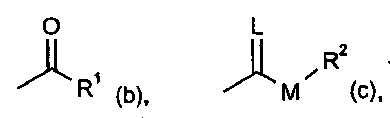 -SO₂-R³ (d) oder E (f)
steht,
in welchen
L für Sauerstoff steht,
M für Sauerstoff steht und
E für ein Ammoniumion (N⁺ (C₆H₁₃)₄) steht,
R¹ für C₁-C₈-Alkyl, C₁-C₂-Alkoxy-C₁-alkyl, oder C₂-C₁₇-Alkenyl steht,
R² für C₁-C₈-Alkyl oder C₂-C₆-Alkenyl steht,
R³ für C₁-C₄-Alkyl steht.

6. Verfahren zur Herstellung von Verbindungen deer Formel (I) gemäß Anspruch 1, **dadurch gekennzeichnet, dass** man zum Erhalt von
(A) Verbindungen der Formel (I-1-a) 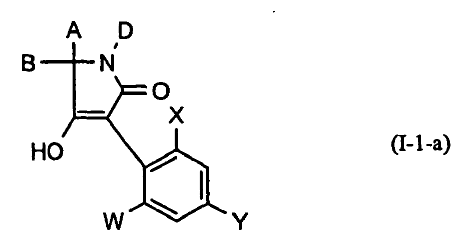 in welcher
A, B, D, W, X und Y die oben angegebenen Bedeutungen haben,
Verbindungen der Formel (II) 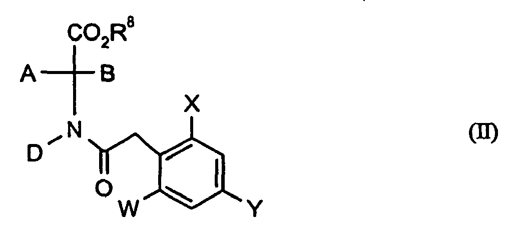 in welcher
A, B, D, W, X und Y die oben angegebenen Bedeutungen haben, und
R⁸ für Alkyl steht,
in Gegenwart eines Verdünnungsmittels und in Gegenwart einer Base intramolekular kondensiert.,
(B) Verbindungen der Formel (I-2-a) 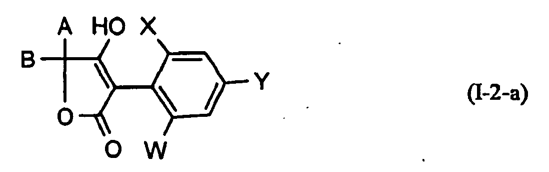 in welcher
A, B, W, X und Y die oben angegebenen Bedeutungen haben,
Verbindungen der Formel (III) 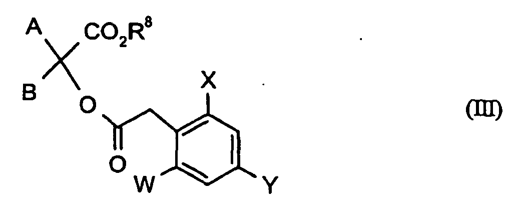 in welcher
A, B, W, X , Y und R⁸ die oben angegebenen Bedeutungen haben,
in Gegenwart eines Verdünnungsmittels und in Gegenwart einer Base intramolekular kondensiert,
(C) Verbindungen der Formel (I-3-a) 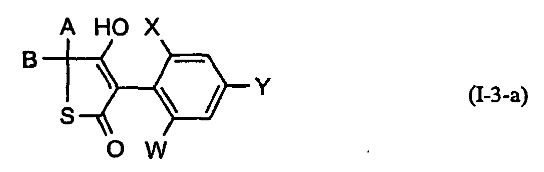 in welcher
A, B, W, X und Y die oben angegebenen Bedeutungen haben,
Verbindungen der Formel (IV) 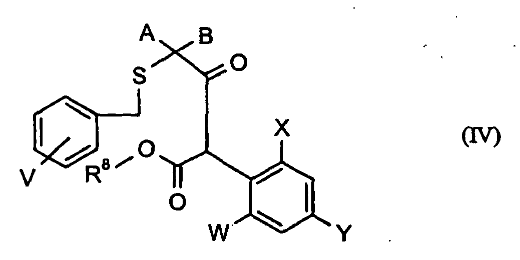 in welcher
A, B, W, X, Y und R⁸ die oben angegebenen Bedeutungen haben und
V für Wasserstoff, Halogen, Alkyl oder Alkoxy steht,
gegebenenfalls in Gegenwart eines Verdünnungsmittels und in Gegenwart einer Säure intramolekular cyclisiert,
(D) Verbindungen der Formel (I-4-a) 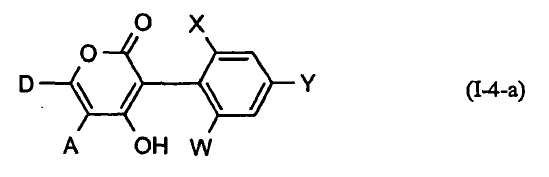 in welcher
A, D, W, X und Y die oben angegebenen Bedeutungen haben,
Verbindungen der Formel (V) 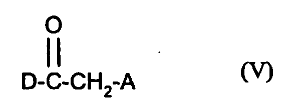 in welcher
A und D die oben angegebenen Bedeutungen haben,
oder deren Silylenolether der Formel (Va) 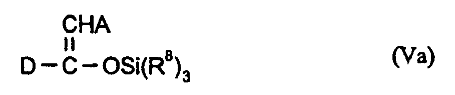 in welcher
A, D und R⁸ die oben angegebene Bedeutung haben,
mit Verbindungen der Formel (VI) 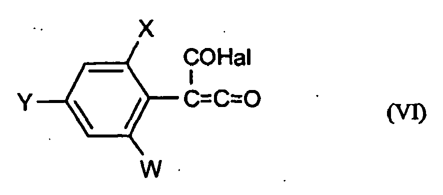 in welcher
W, X und Y die oben angegebenen Bedeutungen haben und
Hal für Halogen steht,
gegebenenfalls in Gegenwart eines Verdünnungsmittels und gegebenenfalls in Gegenwart eines Säureakzeptors umsetzt,
(E) Verbindungen der Formel (I-5-a) 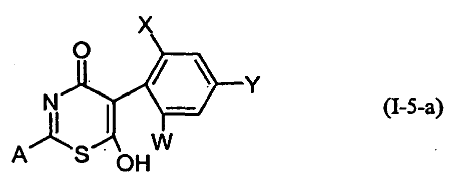 in welcher
A, W, X und Y die oben angegebene Bedeutung haben,
Verbindungen der Formel (VII) 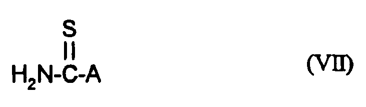 in welcher
A die oben angegebene Bedeutung hat,
mit Verbindungen der Formel (VI) 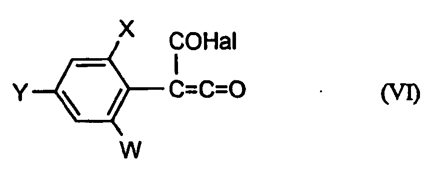 in welcher
Hal, W, X und Y die oben angegebenen Bedeutungen haben,
gegebenenfalls in Gegenwart eines Verdünnungsmittels und gegebenenfalls in Gegenwart eines Säureakzeptors umsetzt,
(F) Verbindungen der Formel (I-6-a) 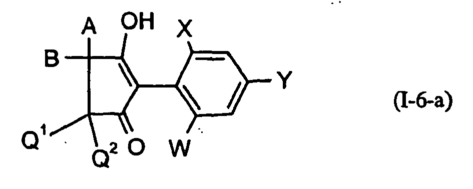 in welcher
A, B,Q¹, Q², W, X und Y die oben angegebene Bedeutung haben,
Verbindungen der Formel (VIII) 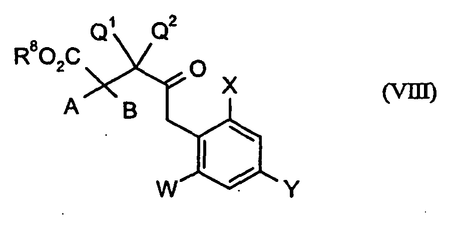 in welcher
A, B, Q¹, Q², W, X und Y die oben angegebene Bedeutung haben, und
R⁸ für Alkyl steht,
gegebenenfalls in Gegenwart eines Verdünnungsmittels und in Gegenwart einer Base intramolekular cyclisiert,
(G) Verbindungen der Formel (I-7-a) 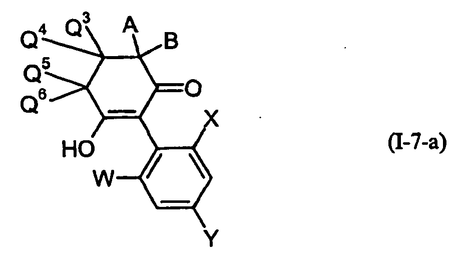 in welcher
A, B, Q³, Q⁴, Q⁵, Q⁶, W, X und Y die oben angegebene Bedeutung haben,
Verbindungen der Formel (IX) 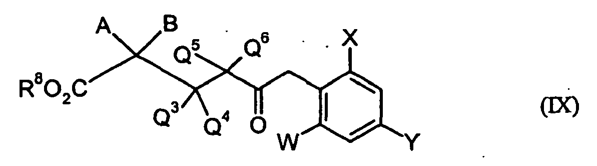 in welcher
A, B, Q³, Q⁴, Q⁵, Q⁶, W, X und Y die oben angegebene Bedeutung haben
und
R⁸ für Alkyl steht,
in Gegenwart eines Verdünnungsmittels und in Gegenwart einer Base intramolekular kondensiert,
(H) Verbindungen der Formel (I-8-a) 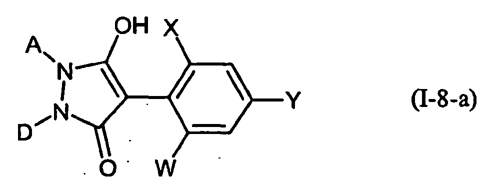 in welcher
A, D, W, X und Y die oben angegebenen Bedeutungen haben,
Verbindungen der Formel (X) 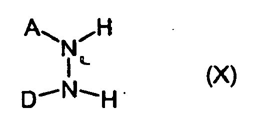 in welcher
A und D die oben angegebene Bedeutung haben,
α) mit Verbindungen der Formel (VI) 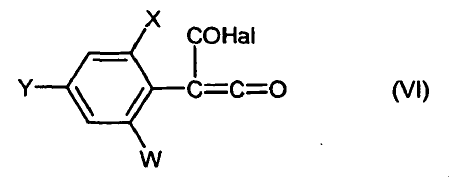 in welcher
Hal, X, Y und W die oben angegebenen Bedeutungen haben,
gegebenenfalls in Gegenwart eines Verdünnungsmittels und gegebenenfalls in Gegenwart eines Säureakzeptors umsetzt, oder
ß) mit Verbindungen der Formel (XI) 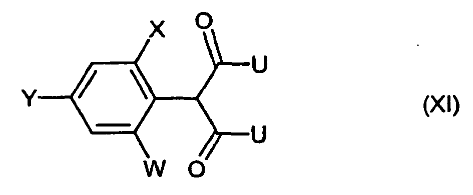 in welcher
W, X und Y die oben angegebene Bedeutung haben,
und U für NH₂ oder O-R⁸ stent, wobei R⁸ die oben genannte Bedeutung haf, gegebenenfalls in Gegenwart eines Verdünnungsmittels und gegebenenfalls in
Gegenwart einer Base umsetzt, oder
γ) Verbindungen der Formel (XII) 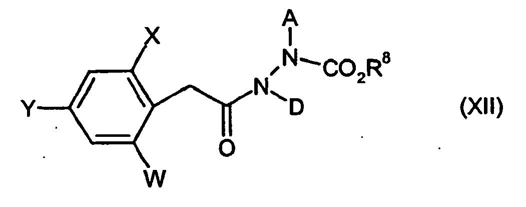 in welcher
A, D, W, X, Y und R⁸ die oben angegebene Bedeutung haben,
gegebenenfalls in Gegenwart eines Verdünnungsmittels und gegebenenfalls in Gegenwart einer Base umsetzt,
(I) Verbindungen der oben gezeigten Formeln (I-1-b) bis (I-8-b), in welchen A, B, D, Q¹, Q², Q³, Q⁴, Q⁵, Q⁶, R¹, W, X, und Y die oben angegebenen Bedeutungen haben, Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
(α) mit Säurehalogeniden der Formel (XIII) 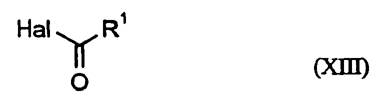 in welcher
R¹ die oben angegebene Bedeutung hat und
Hal für Halogen steht
oder
(ß) mit Carbonsäureanhydriden der Formel (XIV)
R¹-CO-O-CO-R¹ (XIV)
in welcher
R¹ die oben angegebene Bedeutung hat,
gegebenenfalls in Gegenwart eines Verdünnungsmittels und gegebenenfalls in Gegenwart eines Säurebindemittels umsetzt;
(J) Verbindungen der oben gezeigten Formeln (I-1-c) bis (I-8-c), in welchen A, B, D, Q¹, Q², Q³, Q⁴, Q⁵, Q⁶, R², M, W, X und Y die oben angegebenen Bedeutungen haben und L für Sauerstoff steht, Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
mit Chlorameisensäureestern oder Chlorameisensäurethioestern der Formel (XV)
R²-M-CO-Cl (XV)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(K) Verbindungen der oben gezeigten Formeln (I-1-c) bis (I-8-c), in welchen A, B, D, Q¹, Q², Q³, Q⁴, Q⁵, Q⁶, R², M, W, X und Y die oben angegebenen Bedeutungen haben und L für Schwefel steht, Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
mit Chlormonothioameisensäureestern oder Chlordithioameisensäureestern der Formel (XVI) 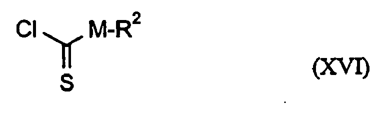 in welcher
M und R² die oben angegebenen Bedeutungen haben,
gegebenenfalls in Gegenwart eines Verdünnungsmittels und gegebenenfalls in Gegenwart eines Säurebindemittels umsetzt
und
(L) Verbindungen der oben gezeigten Formeln (I-1-d) bis (I-8-d), in welchen A, B, D, Q¹, Q², Q³, Q⁴, Q⁵, Q⁶, R³, W, X und Y die oben angegebenen Bedeutungen haben, Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
mit Sulfonsäurechloriden der Formel (XVII)
R³-SO₂-Cl (XVII)
in welcher
R³ die oben angegebene Bedeutung hat,
gegebenenfalls in Gegenwart eines Verdünnungsmittels und gegebenenfalls in Gegenwart eines Säurebindemittels umsetzt,
(M) Verbindungen der oben gezeigten Formeln (I-1-e) bis (I-8-e), in welchen A, B, D, L, Q¹, Q², Q³, Q⁴, Q⁵, Q⁶, R⁴, R⁵, W, X und Y die oben angegebenen Bedeutungen haben, Verbindungen der oben gezeigten Formeln (I-1-a) bis (I-8-a), in welchen A, B, D, Q¹, Q², Q³, Q⁴, Q⁵, Q⁶, W, X und Y die oben angegebenen Bedeutungen haben, jeweils
mit Phosphorverbindungen der Formel (XVIII) 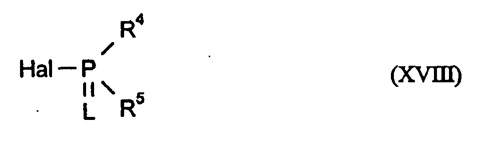 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(N) Verbindungen der oben gezeigten Formeln (I-1-f) bis (I-8-f), in welchen A, B, D, E, Q¹, Q², Q³, Q⁴, Q⁵, Q⁶, W, X und Y die oben angegebenen Bedeutungen haben, Verbindungen der Formeln (I-1-a) bis (I-8-a), in welchen A, B, D, Q¹, Q², Q³, Q⁴, Q⁵, Q⁶, W, X und Y die oben angegebenen Bedeutungen haben, jeweils
mit Metallverbindungen oder Aminen der Formeln (XIX) oder (XX)
Me(OR¹⁰)ₜ (XIX) 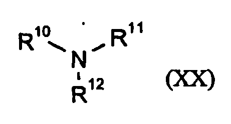 in welchen
Me für ein ein- oder zweiwertiges Metall,
t für die Zahl 1 oder 2 und
R¹⁰, R¹¹, R¹² unabhängig voneinander für Wasserstoff oder Alkyl stehen, gegebenenfalls in Gegenwart eines Verdünnungsmittels umsetzt,
(O) Verbindungen der oben gezeigten Formeln (I-1-g) bis (I-8-g), in welchen A, B, D, L, Q¹, Q², Q³, Q⁴, Q⁵, Q⁶, R⁶, R⁷, W, X und Y die oben angegebenen Bedeutungen haben, Verbindungen der oben gezeigten Formeln (I-1-a) bis (I-8-a), in welchen A, B, D, Q¹, Q², Q³, Q⁴, Q⁵, Q⁶, W , X u nd Y die oben an gegebenen Bedeutungen haben, jeweils
(α) mit Isocyanaten oder Isothiocyanaten der Formel (XXI)
R⁶-N=C=L (XXI)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(ß) mit Carbamidsäurechloriden oder Thiocarbamidsäurechloriden der Formel (XXII) 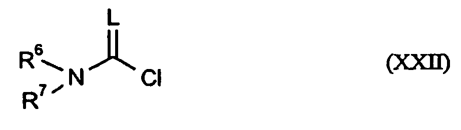
in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

7. Verbindungen der Formel (II) 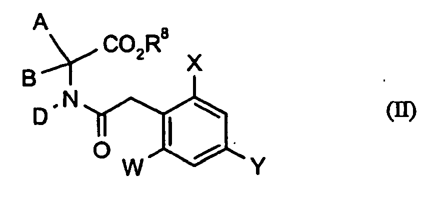 in welcher
A, B, D, W, X, Y und R⁸ die oben angegebenen Bedeutungen haben.

8. Verbindungen der Formel (III) 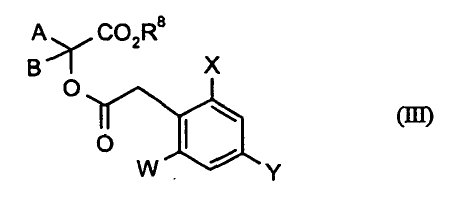 in welcher
A, B, W, X, Y und R⁸ die oben angegebenen Bedeutungen haben.

9. Verbindungen der Formel (IV) 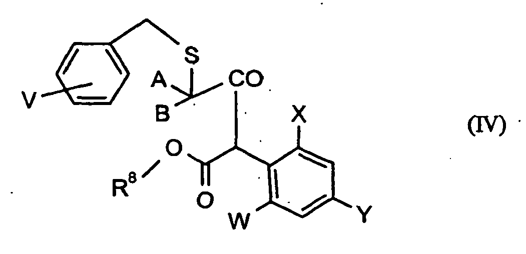 in welcher
A, B, V, W, X, Y und R⁸ die oben angegebenen Bedeutungen haben.

10. Verbindungen der Formel (VI) 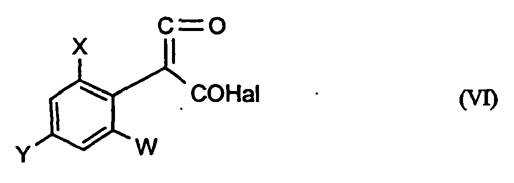 in welcher
Hal, W, X und Y die oben angegebenen Bedeutungen haben.

11. Verbindungen der Formel (VIII) 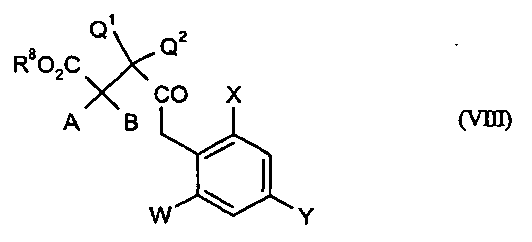 in welcher
A, B, Q¹, Q², W, X, Y und R⁸ die oben angegebene Bedeutung haben.

12. Verbindungen der Formel (IX) 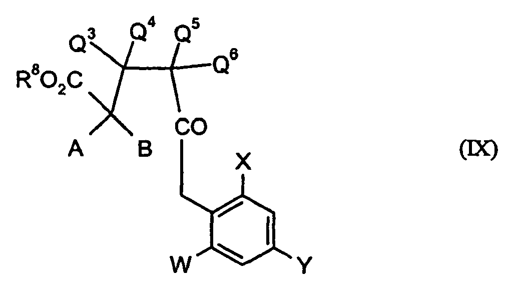 in welcher
A, B, Q³, Q⁴, Q⁵, Q⁶, W, X, Y und R⁸ die oben angegebene Bedeutung haben.

13. Verbindungen der Formel (XI) 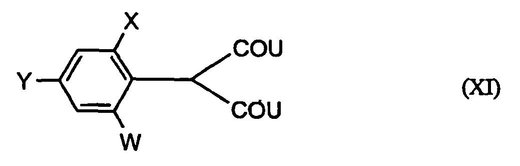 in welcher
U, W, X und Y die oben angegebene Bedeutung haben.

14. Verbindungen der Formel (XII) 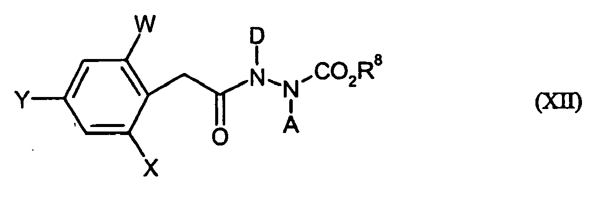 in welcher
A, D, W, X, Y und R⁸ die oben angegebene Bedeutung haben.

15. Verbindungen der Formel (XXIV) 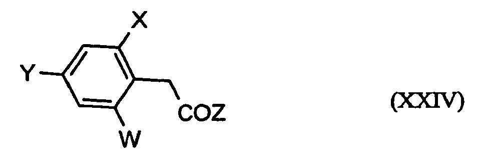 in welcher
W, X, und Y die oben angegebenen Bedeutungen haben und
Z für eine durch Carbonsäureaktivierungsreagenzien wie Carbonyldiimidazol, Carbonyl- diimide (wie z.B. Dicyclohexylcarbondiimid), Phosphorylierungsreagenzen (wie z.B. POCl₃, BOP-Cl), Halogenierungsmittel z.B. Thionylchlorid, Oxalylchlorid, Phosgen oder Chlorameisensäsureester eingeführte Abgangsgruppe steht.

16. Verbindungen der Formel (XXV) 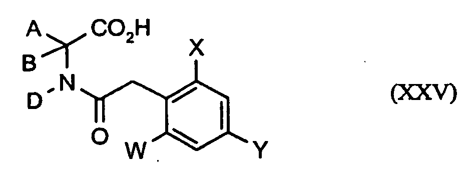 in welcher
A, B, D, W, X und Y die oben angegebenen Bedeutungen haben.

17. Verbindungen der Formel (XXVII) 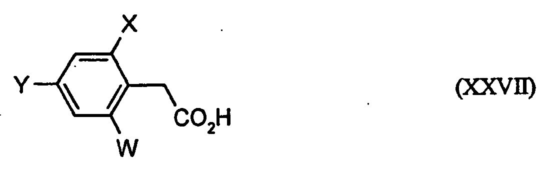 in welcher
W, X und Y die oben angegebene Bedeutung haben.

18. Verbindungen der Formel (XXIX) 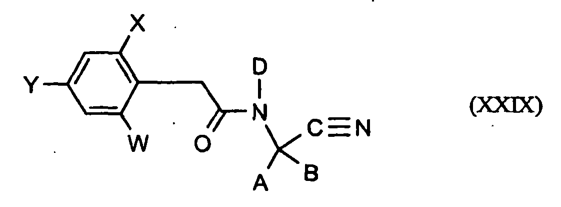 in welcher
A, B, D, W, X und Y die oben angegebenen Bedeutungen haben.

19. Verbindungen der Formel (XXXI) 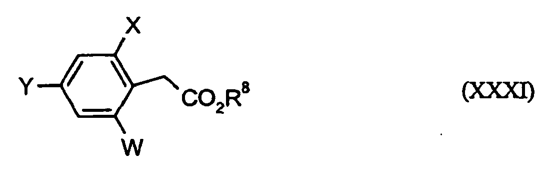 in welcher
W, X, Y und R⁸ die oben angegebene Bedeutung haben.

20. Verbindungen der Formel (XXXIII) 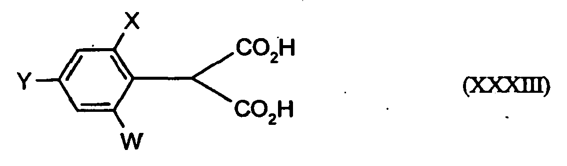 in welcher
W, X und Y die oben angegebenen Bedeutungen haben.

21. Verbindungen der Formel (XXXIV) 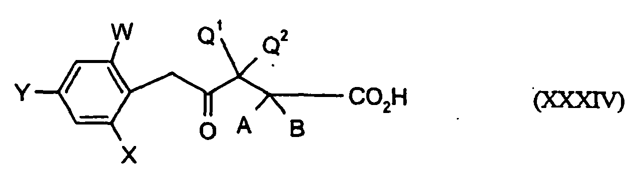 in welcher
A, B, Q¹, Q², W, X und Y die oben angegebene Bedeutung haben.

22. Verbindungen der Formel (XXXV) 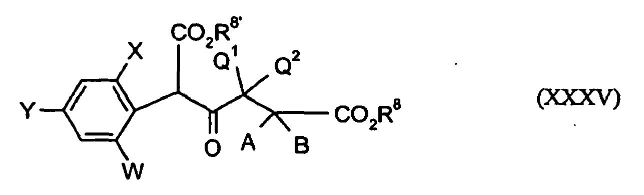 in welcher
A, B, Q¹, Q², R⁸, R⁸, W, X und Y die oben angegebene Bedeutung haben.

23. Verbindungen der Formel (XXXVIII) 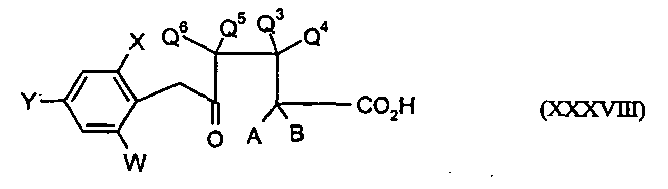 in welcher
A, B, Q³, Q⁴, Q⁵, Q⁶, W, X und Y die oben angegebene Bedeutung haben.

24. Verbindungen der Formel (XXXIX) 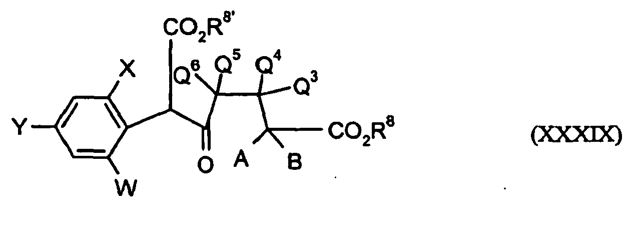 in welcher
A, B, Q³, Q⁴, Q⁵, Q⁶, R⁸, R⁸, W, X und Y die oben angegebene Bedeutung haben.

25. Mittel enthaltend einen wirksamen Gehalt an einer Wirkstoffkombination umfassend als Komponenten
(a') mindestens ein substituiertes, cyclisches Ketoenol der Formel (I) gemäß Anspruch 1, in welcher CKE, W, X und Y die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlorbenzyl)-lgl-methyl-1-phenyl-ethylpharnstoff (Cumyluron), α-(Cyanomethoximino)-phenylacetonitril (Cyometrinil), 2,4-Dichlor-phenoxyessigsäure (2,4-D), 4-(2,4-Dichlorphenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-rnethyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-arbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluorphenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5 -Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäwe-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxyessigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxybenzoyl)-4-[(methylamino-carbonyl)-amino]-benzolsulfonamid), 1-[4-(N-2-Methoxy-benzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethylharnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 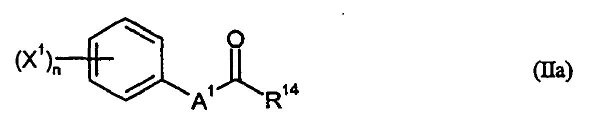 oder der allgemeinen Formel (IIb) 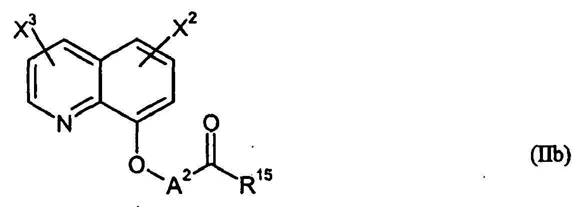 oder der Formel (IIc) 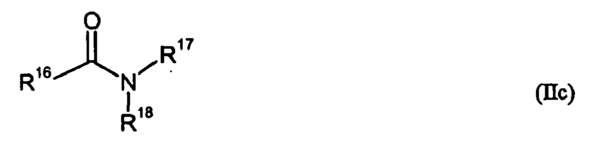 wobei
n für eine Zahl zwischen 0 und 5 steht,
A¹ für eine der nachstehend skizzierten divalenten heterocyclischen Gruppierungen steht, 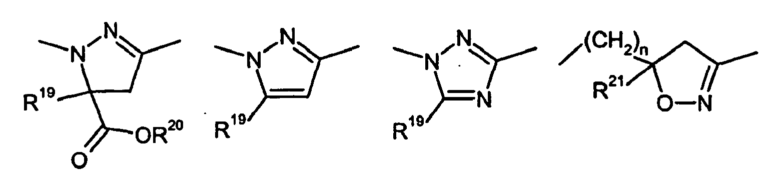
n für eine Zahl zwischen 0 und 5 steht,
A² für gegebenenfalls durch C₁-C₄-Alkyl und/oder C₁-C₄-Alkoxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₁-Alkylthio, C₁-C₆-Alkylamino oder Di-(C₁-C₄-alkyl)-amino steht,
R¹⁵ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁶ für jeweils gegebenenfalls durch Fluor, Chlor und/oder Brom substituiertes C₁-C₄- Alkyl steht,
R¹⁷ für Wasserstoff, jeweils gegebenenfalls durch Fluor, Chlor und/oder Brom substitu- iertes C₁-C₆-Alkyl, C₂-C₆-Alkenyl oder C₂-C₆-Alkinyl, C₁-C₄-Alkoxy-C₁-C₄-alkyl, Di- oxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substitu- iertes C₁-C₆-Alkyl, C₂-C₆-Alkenyl oder C₂-C₆-Alkinyl, C₁-C₄-Alkoxy-C₁-C₄-alkyl, Di- oxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, oder zusammen mit R¹⁷ für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁- C₄-Halogenalkoxy steht,
X² für Wasserstoff, Cyano, Nitro, Halogen, C₁-C₄-Alkyl, C₁-C₄-Halogenalkyl, C₁-C₄- Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄- alkoxy oder C₁-C₄-Halogenalkoxy steht,
und/oder die folgenden durch allgemeine Formeln definierten Verbindungen
der allgemeinen Formel (IId) 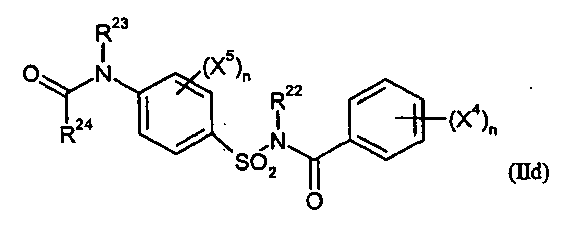 oder der allgemeinen Formel (IIe) 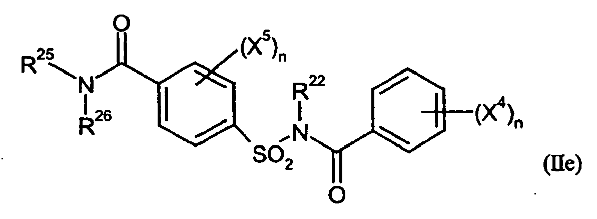 wobei
n für eine Zahl zwischen 0 und 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁- C₄-Alkyl substituiertes C₃-C₆-Cycloalkyl, C₃-C₆-Cycloalkyloxy, C₃-C₆-Cycloalkyl- thio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen sub- stituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen sub- stituiertes C₃-C₆-Alkenyl oder C₃-C₆-Akinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄- Halogenalkoxy substituiertes Phenyl steht, oder zusammen mit R²⁵ für jeweils gege- benenfalls durch C₁-C₄-Alkyl substituiertes C₂-C₆-Alkandiyl oder C₂-C₅-Oxa- alkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogen- alkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogen- alkoxy steht.

26. Mittel nach Anspruch 25, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen 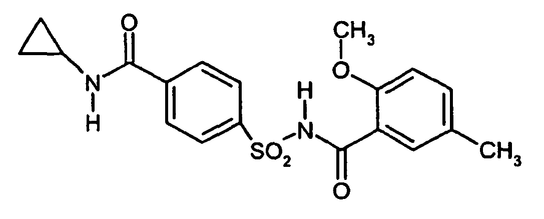 und

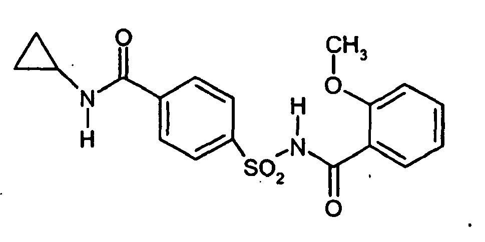

27. Mittel gemäß einem der Ansprüche 25 oder 26, bei denen die Kulturpflanzen-Verträglichkeit verbessernde Verbindung Cloquintocet-mexyl oder Mefenpyr-diethyl ist

28. Schädlingsbekämpfungsmittel und/oder Herbizide, **gekennzeichnet durch** einen Gehalt an mindestens einer Verbindung der Formel (I) gemäß Anspruch 1.

29. Verfahren zur Bekämpfung von tierischen Schädlingen und/oder unerwünschten Pflanzenbewuchs, **dadurch gekennzeichnet, dass** man Verbindungen der Formel (I) gemäß Anspruch 1 auf Schädlinge und/oder ihren Lebensraum einwirken lässt.

30. Verwendung von Verbindungen der Formel (I) gemäß Anspruch 1 zur Bekämpfung von tierischen Schädlingen und/oder unerwünschtem Pflanzenbewuchs.

31. Verfahren zur Herstellung von Schädlingsbekämpfungsmitteln und/oder Herbiziden, **dadurch gekennzeichnet, dass** man Verbindungen der Formel (I) gemäß Anspruch 1 mit Streckmitteln und/oder oberflächenaktiven Stoffen vermischt.

32. Verwendung von Verbindungen der Formel (I) gemäß Anspruch 1 zur Herstellung von Schädlingsbekämpfungsmitteln und/oder Herbiziden.

33. Verfahren zum Bekämpfen von unerwünschtem Pflanzenwuchs, **dadurch gekennzeichnet, dass** man ein Mittel gemäß Anspruch 25 auf die Pflanzen oder ihre Umgebung einwirken lässt.

34. Verwendung eines Mittels gemäß Anspruch 25 zum Bekämpfen von unerwünschten Pflanzenwuchs.

35. Verfahren zum Bekämpfen von unerwünschtem Pflanzenwuchs, **dadurch gekennzeichnet, dass** man eine Verbindung der Formel (I) gemäß Anspruch 25 und die die Kulturpflanzenverträglichkeit verbessernde Verbindung gemäß Anspruch 1 in zeitlich naher Abfolge getrennt auf die Pflanzen oder ihre Umgebung einwirken lässt.

## Claims

1. Compounds of the formula (I) 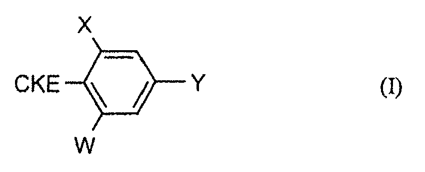 in which
W represents alkoxy, halogenoalkoxy, alkoxyalkyloxy, alkoxy-bis-alkyloxy or optionally substituted cycloalkylalkanediyloxy, which can optionally be interrupted by hetero atoms,
X represents alkyl,
Y represents chlorine, bromine or iodine,
CKE represents one of the groups 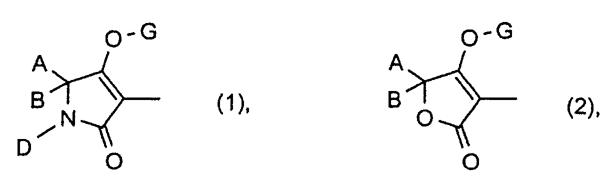 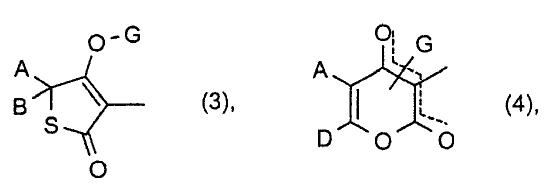 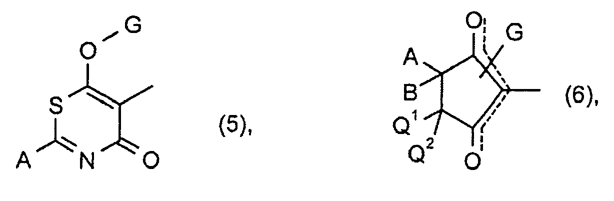 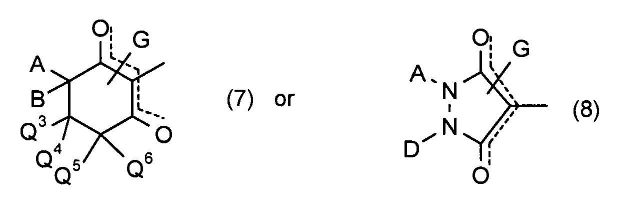
in which
A represents hydrogen, or represents alkyl, alkenyl, alkoxyalkyl or alkylthioalkyl, each of which is optionally substituted by halogen, or represents saturated or unsaturated, optionally substituted cycloalkyl in which at least one ring atom is optionally replaced by a hetero atom, or represents aryl, arylalkyl or hetaryl, each of which is optionally substituted by halogen, alkyl, halogenoalkyl, alkoxy, halogenoalkoxy, cyano or nitro,
B represents hydrogen, alkyl or alkoxyalkyl, or
A and B together with the carbon atom to which they are bonded represent a saturated or unsaturated, unsubstituted or substituted cycle which optionally contains at least one hetero atom,
D represents hydrogen or an optionally substituted radical from the series consisting of alkyl, alkenyl, alkinyl, alkoxyalkyl, saturated or unsaturated cycloalkyl in which one or more ring members optionally are replaced by hetero atoms; arylalkyl, aryl, hetarylalkyl or hetaryl, or
A and D together with the atoms to which they are bonded represent a saturated or unsaturated cycle which is unsubstituted or substituted in the A, D moiety and which optionally contains at least one (in the case of CKE = 8, a further) hetero atom, or
A and Q¹ together represent alkanediyl or alkenediyl which are optionally substituted by hydroxyl or by in each case optionally substituted alkyl, alkoxy, alkylthio, cycloalkyl, benzyloxy or aryl, or
Q¹ represents hydrogen or alkyl,
Q², Q⁴, Q⁵ and Q⁶ independently of one another represent hydrogen or alkyl,
Q³ represents hydrogen, optionally substituted alkyl, alkoxyalkyl, alkylthioalkyl, optionally substituted cycloalkyl (in which a methylene group is optionally replaced by oxygen or sulphur) or optionally substituted phenyl, or
Q³ and Q⁴ together with the carbon atom to which they are bonded represent a saturated or unsaturated, unsubstituted or substituted cycle which optionally contains a hetero atom,
G represents hydrogen (a) or one of the groups 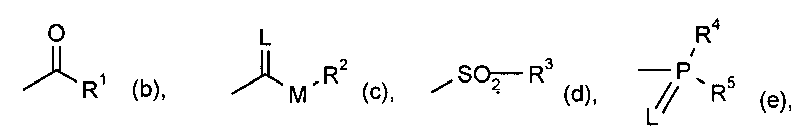 E(f) or 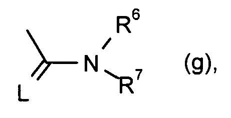 where
E represents a metal ion equivalent or an ammonium ion,
L represents oxygen or sulphur,
M represents oxygen or sulphur,
R¹ represents alkyl, alkenyl, alkoxyalkyl, alkylthioalkyl or polyalkoxyalkyl, each of which is optionally substituted by halogen, or represents cycloalkyl which can be interrupted by at least one hetero atom and which is optionally substituted by halogen, alkyl or alkoxy, or represents in each case optionally substituted phenyl, phenylalkyl, hetaryl, phenoxyalkyl or hetaryloxyalkyl,
R² represents alkyl, alkenyl, alkoxyalkyl or polyalkoxyalkyl, each of which is optionally substituted by halogen, or represents in each case optionally substituted cycloalkyl, phenyl or benzyl,
R³, R⁴ and R⁵ independently of one another represent alkyl, alkoxy, alkylamino, dialkylamino, alkylthio, alkenylthio or cycloalkylthio, each of which is optionally substituted by halogen, or represent in each case optionally substituted phenyl, benzyl, phenoxy or phenylthio,
R⁶ and R⁷ independently of one another represent hydrogen, or represent alkyl, cycloalkyl, alkenyl, alkoxy or alkoxyalkyl, each of which is optionally substituted by halogen, or represent optionally substituted phenyl, or represent optionally substituted benzyl, or together with the N atom to which they are bonded represent a cycle which is optionally interrupted by oxygen or sulphur.

2. Compounds of the formula (I) according to Claim 1 in which
W represents C₁-C₆-alkoxy, C₁-C₆-halogenoalkoxy, C₁-C₄-alkoxy-C₂-C₄- alkyloxy, C₁-C₄-alkoxy-bis-C₂-C₄-alkyloxy, or represents C₃-C₆- cycloalkyl-C₁-C₃-alkanediyloxy which is optionally monosubstituted to trisubstituted by fluorine, chlorine, C₁-C₃-alkyl or C₁-C₃-alkoxy and in which one methylene group of the ring can optionally be interrupted by oxygen or sulphur,
X represents C₁-C₆-alkyl,
Y represents chlorine, bromine or iodine,
CKE represents one of the groups 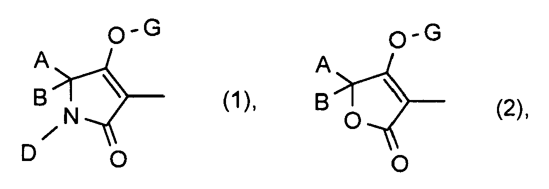 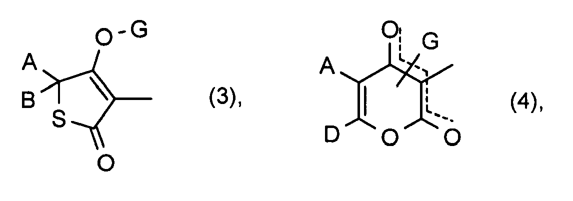 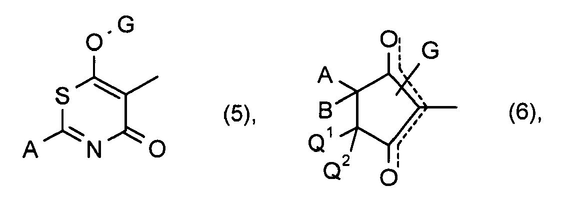 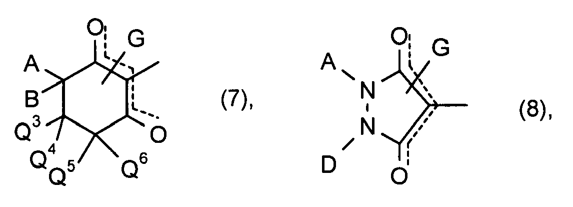
A represents hydrogen, or represents C₁-C₁₂-alkyl, C₃-C₈-alkenyl, C₁-C₁₀- alkoxy-C₁-C₈-alkyl, or C₁-C₁₀-alkylthio-C₁-C₆-alkyl, each of which is optionally substituted by halogen, or represents C₃-C₈-cycloalkyl in which one or two ring members which are not directly adjacent are optionally replaced by oxygen and/or sulphur and which is optionally substituted by halogen, C₁-C₆-alkyl or C₁-C₆-alkoxy, or represents phenyl, naphthyl, hetaryl having 5 to 6 ring atoms, phenyl-C₁-C₆-alkyl or naphthyl-C₁-C₆-alkyl, each of which is optionally substituted by halogen, C₁-C₆-alkyl, C₁-C₆-halogenoalkyl, C₁-C₆-alkoxy, C₁-C₆- halogenoalkoxy, cyano or nitro,
B represents hydrogen, C₁-C₁₂-alkyl or C₁-C₈-alkoxy-C₁-C₆-alkyl, or
A, B and the carbon atom to which they are bonded represent saturated C₃- C₁₀-cycloalkyl or unsaturated C₅-C₁₀-cycloalkyl in which one ring member is optionally replaced by oxygen or sulphur and which is optionally monosubstituted or disubstituted by C₁-C₈-alkyl, C₃-C₁₀- cycloalkyl, C₁-C₈-halogenoalkyl, C₁-C₈-alkoxy, C₁-C₈-alkylthio, halogen or phenyl, or
A, B and the carbon atom to which they are bonded represent C₃-C₆- cycloalkyl which is substituted by an alkylenediyl group which is optionally substituted by C₁-C₄-alkyl and which optionally contains one or two oxygen and/or sulphur atoms which are not directly adjacent, or by an alkylenedioxy or by an alkylenedithio group, this group together with the carbon atom to which it is bonded forming a further five- to eight-membered ring, or
A, B and the carbon atom to which they are bonded represent C₃-C₈- cycloalkyl or C₅-C₈-cycloalkenyl in which two substituents together with the carbon atoms to which they are bonded represent C₂-C₆- alkanediyl, C₂-C₆-alkenediyl or C₄-C₆-alkanedienediyl, in which one methylene group is optionally replaced by oxygen or sulphur and each of which is optionally substituted by C₁-C₆-alkyl, C₁-C₆-alkoxy or halogen,
D represents hydrogen, or represents C₁-C₁₂-alkyl, C₃-C₈-alkenyl, C₃-C₈- alkinyl, C₁-C₁₀-alkoxy-C₂-C₈-alkyl, each of which is optionally substituted by halogen, or represents C₃-C₈-cycloalkyl in which a ring member is optionally replaced by oxygen or sulphur and which is optionally substituted by halogen, C₁-C₄-alkyl, C₁-C₄-alkoxy or C₁-C₄- halogenoalkyl, or represents phenyl, hetaryl having 5 or 6 ring atoms, phenyl-C₁-C₆-alkyl or hetaryl-C₁-C₆-alkyl having 5 or 6 ring atoms, each of which is optionally substituted by halogen, C₁-C₆-alkyl, C₁-C₆- halogenoalkyl, C₁-C₆-alkoxy, C₁-C₆-halogenoalkoxy, cyano or nitro, or
A and D together represent in each case optionally substituted C₃-C₆-alkanediyl or C₃-C₆-alkenediyl, in which optionally one methylene group is replaced by a carbonyl group, oxygen or sulphur, and
where suitable substituents in each case are:
halogen, hydroxyl, mercapto, or C₁-C₁₀-alkyl, C₁-C₆-alkoxy, C₁-C₆- alkylthio, C₃-C₇-cycloalkyl, phenyl or benzyloxy, each of which is optionally substituted by halogen; or a further C₃-C₆-alkanediyl group, C₃-C₆-alkenediyl group or a butadienyl group which is optionally substituted by C₁-C₆-alkyl or in which two adjacent substituents together with the carbon atoms to which they are bonded optionally form a further saturated or unsaturated cycle having 5 or 6 ring atoms (in the case of the compound of the formula (I-1), A and D will now together with the atoms to which they are bonded represent for example the groups AD-1 to AD-10, which are detailed further below) which can contain oxygen or sulphur, or which optionally contains one of the following groups 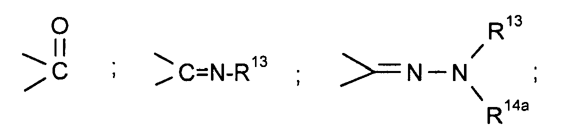 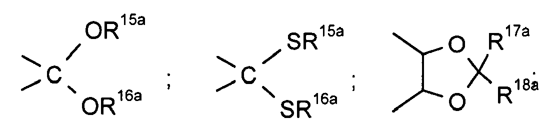 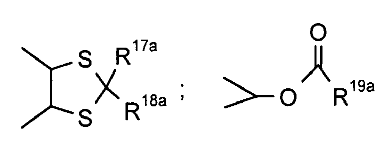 or 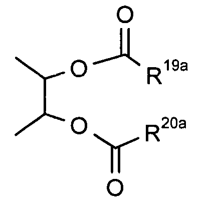 or
A and Q¹ jointly represent C₃-C₆-alkanediyl or C₄-C₆-alkenediyl, each of which is optionally monosubstituted or disubstituted by identical or different substituents from the series consisting of halogen, hydroxyl, or C₁-C₁₀- alkyl, C₁-C₆-alkoxy, C₁-C₆-alkylthio, C₃-C₇-cycloalkyl, each of which is optionally monosubstituted to trisubstituted by identical or different halogen substituents, or benzyloxy or phenyl, each of which is optionally monosubstituted to trisubstituted by identical or different substituents from the series consisting of halogen, C₁-C₆-alkyl or C₁-C₆-alkoxy, this C₃-C₆-alkanediyl or C₄-C₆-alkenediyl additionally optionally containing one of the following groups 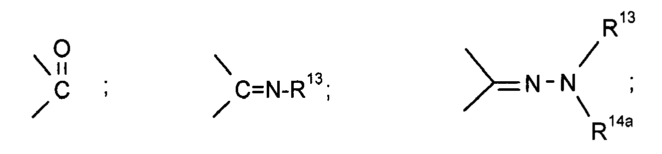 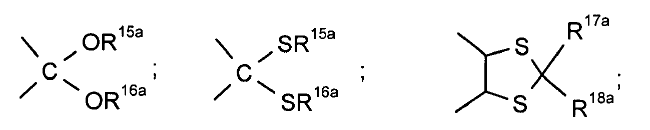 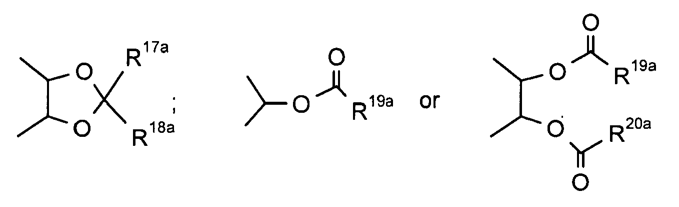 or being bridged via a C₁-C₂-alkanediyl group or by an oxygen atom, or
Q¹ represents hydrogen or C₁-C₄-alkyl,
Q², Q⁴, Q⁵ and Q⁶ independently of one another represent hydrogen or C₁-C₄- alkyl,
Q³ represents hydrogen, C₁-C₆-alkyl, C₁-C₆-alkoxy-C₁-C₂-alkyl, C₁-C₆- alkylthio-C₁-C₂-alkyl, C₃-C₈-cycloalkyl which is optionally substituted by C₁-C₄-alkyl or C₁-C₄-alkoxy and in which one methylene group is optionally replaced by oxygen or sulphur, or phenyl which is optionally substituted by halogen, C₁-C₄-alkyl, C₁-C₄-alkoxy, C₁-C₂-halogenoalkyl, C₁-C₂-halogenoalkoxy, cyano or nitro, or
Q³ and Q⁴ together with the carbon atom to which they are bonded represent a C₃-C₇ ring which is optionally substituted by C₁-C₄-alkyl, C₁-C₄-alkoxy or C₁-C₂-halogenoalkyl and in which one ring member is optionally replaced by oxygen or sulphur,
G represents hydrogen (a) or one of the groups 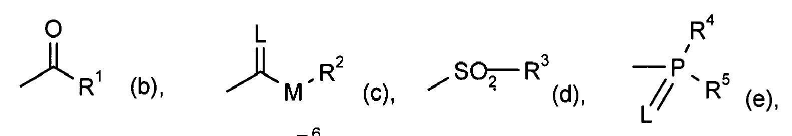 E (f) or 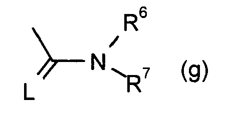 in which
E represents a metal ion equivalent or an ammonium ion,
L represents oxygen or sulphur and
M represents oxygen or sulphur,
R¹ represents C₁-C₂₀-alkyl, C₂-C₂₀-alkenyl, C₁-C₈-alkoxy-C₁-C₈-alkyl, C₁-C₈- alkylthio-C₁-C₈-alkyl or poly-C₁-C₈-alkoxy-C₁-C₈-alkyl, each of which is optionally substituted by halogen, or represents C₃-C₈-cycloalkyl in which one or more ring members which are not directly adjacent are optionally replaced by oxygen and/or sulphur and which is optionally substituted by halogen, C₁-C₆- alkyl or C₁-C₆-alkoxy,
or phenyl which is optionally substituted by halogen, cyano, nitro, C₁-C₆-alkyl, C₁-C₆-alkoxy, C₁-C₆-halogenoalkyl, C₁-C₆-halogenoalkoxy, C₁-C₆-alkylthio or C₁-C₆-alkylsulphonyl,
or phenyl-C₁-C₆-alkyl which is optionally substituted by halogen, nitro, cyano, C₁-C₆-alkyl, C₁-C₆-alkoxy, C₁-C₆-halogenoalkyl or C₁-C₆-halogenoalkoxy,
or 5- or 6-membered hetaryl which is optionally substituted by halogen or C₁-C₆-alkyl,
or phenoxy-C₁-C₆-alkyl which is optionally substituted by halogen or C₁-C₆- alkyl, or
5- or 6-membered hetaryloxy-C₁-C₆-alkyl which is optionally substituted by halogen, amino or C₁-C₆-alkyl,
R² represents C₁-C₂₀-alkyl, C₂-C₂₀-alkenyl, C₁-C₈-alkoxy-C₂-C₈-alkyl or poly- C₁-C₈-alkoxy-C₂-C₈-alkyl, each of which is optionally substituted by halogen,
or C₃-C₈-cycloalkyl which is optionally substituted by halogen, C₁-C₆-alkyl or C₁-C₆-alkoxy, or
phenyl or benzyl, each of which is optionally substituted by halogen, cyano, nitro, C₁-C₆-alkyl, C₁-C₆-alkoxy, C₁-C₆-halogenoalkyl or C₁-C₆- halogenoalkoxy,
R³ represents C₁-C₈-alkyl which is optionally substituted by halogen, or phenyl or benzyl, each of which is optionally substituted by halogen, C₁-C₆-alkyl, C₁-C₆- alkoxy, C₁-C₄-halogenoalkyl, C₁-C₄-halogenoalkoxy, cyano or nitro,
R⁴ and R⁵ independently of one another represent C₁-C₈-alkyl, C₁-C₈-alkoxy, C₁-C₈- alkylamino, di-(C₁-C₈-alkyl)amino, C₁-C₈-alkylthio, C₂-C₈-alkenylthio or C₃-C₇-cycloalkylthio, each of which is optionally substituted by halogen, or phenyl, phenoxy or phenylthio, each of which is optionally substituted by halogen, nitro, cyano, C₁-C₄-alkoxy, C₁-C₄-halogenoalkyl, C₁-C₄-alkylthio, C₁- C₄-halogenoalkylthio, C₁-C₄-alkyl or C₁-C₄-halogenoalkyl,
R⁶ and R⁷ independently of one another represent hydrogen, or C₁-C₈-alkyl, C₃-C₈- cycloalkyl, C₁-C₈-alkoxy, C₃-C₈-alkenyl or C₁-C₈-alkoxy-C₁-C₈-alkyl, each of which is optionally substituted by halogen, or phenyl which is optionally substituted by halogen, C₁-C₈-halogenoalkyl, C₁-C₈-alkyl or C₁-C₈-alkoxy, or benzyl which is optionally substituted by halogen, C₁-C₈-alkyl, C₁-C₈- halogenoalkyl or C₁-C₈-alkoxy, or together represent a C₃-C₆-alkylene radical which is optionally substituted by C₁-C₄-alkyl and in which one carbon atom is optionally replaced by oxygen or sulphur,
R¹³ represents hydrogen, C₁-C₈-alkyl or C₁-C₈-alkoxy, each of which is optionally substituted by halogen, C₃-C₈-cycloalkyl in which one methylene group is optionally replaced by oxygen or sulphur and which is optionally substituted by halogen, C₁-C₄-alkyl or C₁-C₄-alkoxy, or phenyl, phenyl-C₁-C₄-alkyl or phenyl- C₁-C₄-alkoxy, each of which is optionally substituted by halogen, C₁-C₆-alkyl, C₁-C₆-alkoxy, C₁-C₄-halogenoalkyl, C₁-C₄-halogenoalkoxy, nitro or cyano,
R^{14a} represents hydrogen or C₁-C₈-alkyl, or
R¹³ and R^{14a} together represent C₄-C₆-alkanediyl,
R^{15a} and R^{16a} are identical or different and represent C₁-C₆-alkyl, or
R^{15a} and R^{16a} together represent a C₂-C₄-alkanediyl radical which is optionally substituted by C₁-C₆-alkyl, C₁-C₆-halogenoalkyl or by phenyl which is optionally substituted by halogen, C₁-C₆-alkyl, C₁-C₄-halogenoalkyl, C₁-C₆- alkoxy, C₁-C₄-halogenoalkoxy, nitro or cyano,
R^{17a} and R^{18a} independently of one another represent hydrogen, C₁-C₈-alkyl which is optionally substituted by halogen, or phenyl which is optionally substituted by halogen, C₁-C₆-alkyl, C₁-C₆-alkoxy, C₁-C₄-halogenoalkyl, C₁-C₄- halogenoalkoxy, nitro or cyano, or
R^{17a} and R^{18a} together with the carbon atom to which they are bonded represent a carbonyl group or C₅-C₇-cycloalkyl in which one methylene group is optionally replaced by oxygen or sulphur and which is optionally substituted by halogen, C₁-C₄-alkyl or C₁-C₄-alkoxy,
R^{19a} and R^{20a} independently of one another represent C₁-C₁₀-alkyl, C₂-C₁₀-alkenyl, C₁-C₁₀-alkoxy, C₁-C₁₀-alkylamino, C₃-C₁₀-alkenylamino, di-(C₁-C₁₀- alkyl)amino or di-(C₃-C₁₀-alkenyl)amino.

3. Compounds of the formula (I) according to Claim 1 in which
W represents C₁-C₄-alkoxy, C₁-C₄-halogenoalkoxy, C₁-C₃-alkoxy-C₂-C₃- alkyloxy, C₁-C₂-alkoxy-bis-C₂-C₃-alkyloxy or C₃-C₆-cycloalkyl-C₁-C₂- alkanediyloxy in which one methylene group of the ring can optionally be interrupted by oxygen,
X represents C₁-C₃-alkyl,
Y represents chlorine or bromine,
CKE represents one of the groups 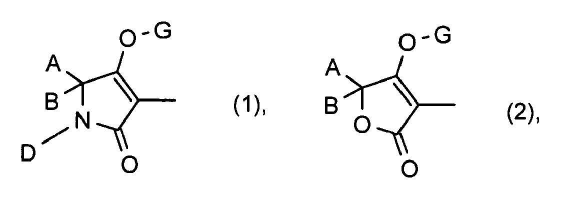 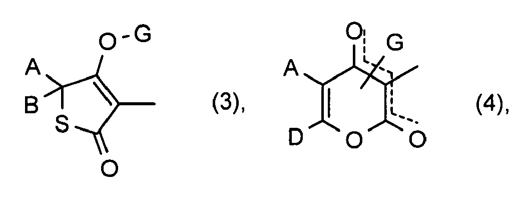 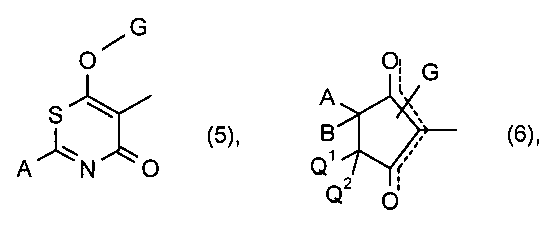 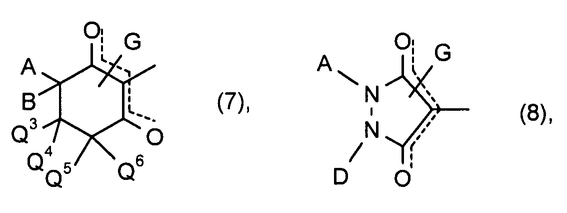
A represents hydrogen, or represents C₁-C₆-alkyl, C₁-C₄-alkoxy-C₁-C₂- alkyl, each of which is optionally monosubstituted to trisubstituted by fluorine or chlorine, or represents C₃-C₆-cycloalkyl which is optionally monosubstituted or disubstituted by C₁-C₂-alkyl or C₁-C₂-alkoxy, or (but not in the case of the compounds of formulae (I-3), (I-4), (I-6) and (I-7)) represents phenyl or benzyl, each of which is optionally monosubstituted or disubstituted by fluorine, chlorine, bromine, C₁-C₄-alkyl, C₁-C₂- halogenoalkyl, C₁-C₄-alkoxy, C₁-C₂-halogenoalkoxy, cyano or nitro,
B represents hydrogen, C₁-C₄-alkyl or C₁-C₂-alkoxy-C₁-C₂-alkyl, or
A, B and the carbon atom to which they are bonded represent saturated or unsaturated C₅-C₇-cycloalkyl in which one ring member is optionally replaced by oxygen or sulphur and which is optionally monosubstituted or disubstituted by C₁-C₆-alkyl, trifluoromethyl or C₁-C₆-alkoxy, with the proviso that, in this case, Q³ represents hydrogen or methyl, or
A, B and the carbon atom to which they are bonded represent C₅-C₆- cycloalkyl which is optionally substituted by an alkylenediyl group which optionally contains one or two oxygen or sulphur atoms which are not directly adjacent to each other and is optionally substituted by methyl or ethyl, or by an alkylenedioxyl or by an alkylenedithiol group, which group, together with the carbon atom to which it is bonded, forms a further five- or six-membered ring, with the proviso that, in this case, Q³ represents hydrogen or methyl,
A, B and the carbon atom to which they are bonded represent C₃-C₆- cycloalkyl or C₅-C₆-cycloalkenyl in which two substituents together with the carbon atoms to which they are bonded represent C₂-C₄-alkanediyl, C₂-C₄-alkenediyl or butadienediyl, each of which is optionally substituted by C₁-C₂-alkyl or C₁-C₂-alkoxy, with the proviso that, in this case, Q³ particularly preferably represents hydrogen or methyl,
D represents hydrogen, or represents C₁-C₆-alkyl, C₃-C₆-alkenyl, C₁-C₄- alkoxy-C₂-C₃-alkyl, each of which is optionally monosubstituted to trisubstituted by fluorine, or represents C₃-C₆-cycloalkyl which is optionally monosubstituted or disubstituted by C₁-C₄-alkyl, C₁-C₄- alkoxy or C₁-C₂-halogenoalkyl and in which one methylene group is optionally replaced by oxygen, or (but not in the case of the compounds of the formulae (I-1)) represents phenyl or pyridyl, each of which is optionally monosubstituted or disubstituted by fluorine, chlorine, bromine, C₁-C₄-alkyl, C₁-C₄-halogenoalkyl, C₁-C₄-alkoxy or C₁-C₄- halogenoalkoxy, or
A and D jointly represent C₃-C₅-alkanediyl which is optionally monosubstituted or disubstituted and in which one methylene group can be replaced by a carbonyl group (but not in the case of the compounds of the formula (I-1)), oxygen or sulphur, with C₁-C₂-alkyl or C₁-C₂-alkoxy being suitable substituents, or
A and D (in the case of the compounds of the formula (I-1)) together with the atoms to which they are bonded represent one of the groups AD-1 to AD-10: 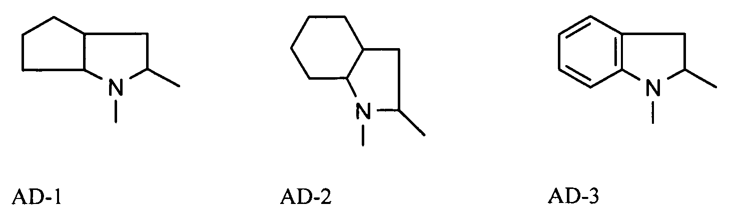 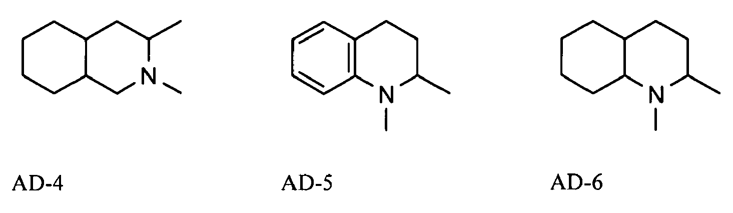 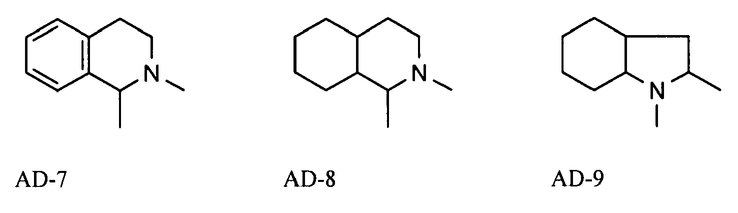 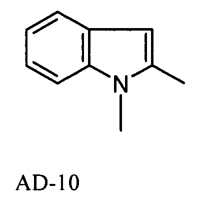 or
A and Q¹ together represent C₃-C₄-alkanediyl, in each case optionally monosubstituted or disubstituted by identical or different substituents from the series consisting of C₁-C₂-alkyl or C₁-C₂-alkoxy, or
Q¹ represents hydrogen,
Q² represents hydrogen,
Q⁴, Q⁵ and Q⁶ independently of one another represent hydrogen or C₁-C₃-alkyl,
Q³ represents hydrogen, C₁-C₄-alkyl or C₃-C₆-cycloalkyl which is optionally monosubstituted or disubstituted by methyl or methoxy, or
Q³ and Q⁴ together with the carbon atom to which they are bonded represent a saturated C₅-C₆ ring which is optionally substituted by C₁-C₂-alkyl or C₁-C₂-alkoxy and in which one ring member is optionally replaced by oxygen or sulphur, with the proviso that, in this case, A represents hydrogen or methyl, or
G represents hydrogen (a) or one of the groups 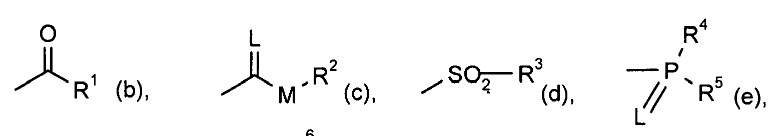 E (f) or 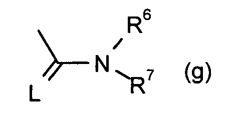 in which
E represents a metal ion equivalent or an ammonium ion,
L represents oxygen or sulphur and
M represents oxygen or sulphur,
R¹ represents C₁-C₈-alkyl, C₂-C₁₈-alkenyl, C₁-C₄-alkoxy-C₁-C₂-alkyl, C₁-C₄-alkylthio-C₁-C₂-alkyl, each of which is optionally monosubstituted to trisubstituted by fluorine or chlorine, or represents C₃-C₆-cycloalkyl which is optionally monosubstituted or disubstituted by fluorine, chlorine, C₁-C₂-alkyl or C₁-C₂-alkoxy and in which one or two ring members which are not directly adjacent are optionally replaced by oxygen,
or represents phenyl which is optionally monosubstituted or disubstituted by fluorine, chlorine, bromine, cyano, nitro, C₁-C₄-alkyl, C₁-C₄-alkoxy, C₁-C₂-halogenoalkyl or C₁-C₂-halogenoalkoxy,
R² represents C₁-C₈-alkyl, C₂-C₈-alkenyl or C₁-C₄-alkoxy- C₂-C₄-alkyl, each of which is optionally monosubstituted to trisubstituted by fluorine, or
represents C₃-C₆-cycloalkyl which is optionally monosubstituted by C₁-C₂-alkyl or C₁-C₂-alkoxy, or
represents phenyl or benzyl, each of which is optionally monosubstituted or disubstituted by fluorine, chlorine, bromine, cyano, nitro, C₁-C₄-alkyl, C₁-C₃-alkoxy, trifluoromethyl or trifluoromethoxy,
R³ represents C₁-C₆-alkyl which is optionally monosubstituted to trisubstituted by fluorine, or represents phenyl which is optionally in each case monosubstituted by fluorine, chlorine, bromine, C₁-C₄-alkyl, C₁-C₄-alkoxy, trifluoromethyl, trifluoromethoxy, cyano or nitro,
R⁴ represents C₁-C₆-alkyl, C₁-C₆-alkoxy, C₁-C₆-alkylamino, di-(C₁-C₆- alkyl)amino, C₁-C₆-alkylthio, C₃-C₄-alkenylthio, C₃-C₆-cycloalkylthio, or represents phenyl, phenoxy or phenylthio, each of which is optionally monosubstituted by fluorine, chlorine, bromine, nitro, cyano, C₁-C₃- alkoxy, C₁-C₃-halogenoalkoxy, C₁-C₃-alkylthio, C₁-C₃-halogeno- alkylthio, C₁-C₃-alkyl or trifluoromethyl,
R⁵ represents C₁-C₆-alkoxy or C₁-C₆-alkylthio,
R⁶ represents hydrogen, C₁-C₆-alkyl, C₃-C₆-cycloalkyl, C₁-C₆-alkoxy, C₃-C₆-alkenyl, C₁-C₆-alkoxy-C₁-C₄-alkyl, or represents phenyl which is optionally monosubstituted by fluorine, chlorine, bromine, trifluoromethyl, C₁-C₄-alkyl or C₁-C₄-alkoxy, or represents benzyl which is optionally monosubstituted by fluorine, chlorine, bromine, C₁-C₄- alkyl, trifluoromethyl or C₁-C₄-alkoxy,
R⁷ represents C₁-C₆-alkyl, C₃-C₆-alkenyl or C₁-C₆-alkoxy-C₁-C₄-alkyl,
R⁶ and R⁷ together represent a C₄-C₅-alkylene radical which is optionally substituted by methyl or ethyl and in which one methylene group is optionally replaced by oxygen or sulphur.

4. Compounds of the formula (I) according to Claim 1 in which
W represents methoxy, ethoxy, n-propoxy, iso-propoxy, n-butoxy, iso- butoxy, sec-butoxy, methoxy-ethyloxy, ethoxy-ethyloxy, cyclopropyl- methoxy, cyclopentyl-methoxy or cyclohexyl-methoxy,
X represents methyl or ethyl,
Y represents chlorine or bromine,
CKE represents one of the groups 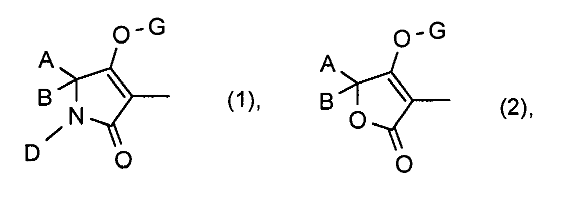 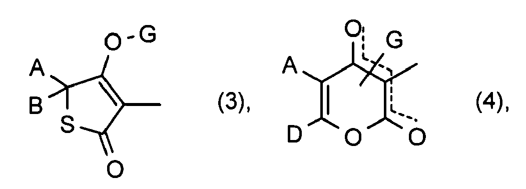 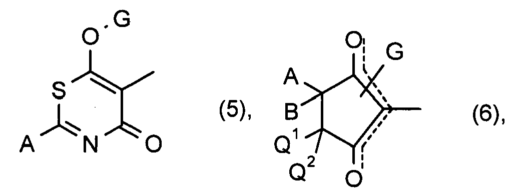 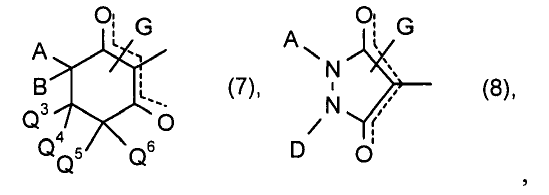
A represents hydrogen, or represents C₁-C₄-alkyl or C₁-C₂-alkoxy-C₁-C₂- alkyl, each of which is monosubstituted to trisubstituted by fluorine, or represents cyclopropyl, cyclopentyl or cyclohexyl, and only in the case of the compounds of the formula (I-5) represents phenyl which is optionally in each case substituted by fluorine, chlorine, bromine, methyl, ethyl, n-propyl, iso-propyl, methoxy, ethoxy, trifluoromethyl, trifluoromethoxy, cyano or nitro,
B represents hydrogen, methyl or ethyl, or
A, B and the carbon atom to which they are bonded represent saturated C₅-C₆- cycloalkyl in which one ring member is optionally replaced by oxygen or sulphur and which is optionally monosubstituted by methyl, ethyl, propyl, isopropyl, trifluoromethyl, methoxy, ethoxy, propoxy or butoxy, with the proviso that, in this case, Q³ represents hydrogen, or
A, B and the carbon atom to which they are bonded represent C₆-cycloalkyl which is optionally substituted by an alkylenedioxy group containing two oxygen atoms which are not directly adjacent, with the proviso that, in this case, Q³ represents hydrogen, or
A, B and the carbon atom to which they are bonded represent C₅-C₆- cycloalkyl or C₅-C₆-cycloalkenyl in which two substituents together with the carbon atoms to which they are bonded represent C₂-C₄-alkanediyl or C₂-C₄-alkenediyl or butadienediyl, with the proviso that, in this case, Q³ represents hydrogen,
D represents hydrogen, or represents C₁-C₄-alkyl, C₃-C₄-alkenyl, C₁-C₄-alkoxy-C₂-C₃-alkyl, each of which is optionally monosubstituted to trisubstituted by fluorine, or represents cyclopropyl, cyclopentyl or cyclohexyl, or (but not in the case of compounds of the formulae (I-1)) represents phenyl or pyridyl, each of which is optionally monosubstituted by fluorine, chlorine, methyl, ethyl, n-propyl, iso- propyl, methoxy, ethoxy or trifluoromethyl,
or
A and D together represent C₃-C₅-alkanediyl which is optionally mono- substituted by methyl or methoxy and in which one carbon atom is optionally replaced by oxygen or sulphur, or represent the group AD-1
A and Q¹ together represent C₃-C₄-alkanediyl which is optionally mono- substituted or disubstituted by methyl or methoxy or
Q¹ represents hydrogen,
Q² represents hydrogen,
Q⁴, Q⁵ and Q⁶ independently of one another represent hydrogen or methyl,
Q³ represents hydrogen, methyl, ethyl or propyl, or
Q³ and Q⁴ together with the carbon atom to which they are bonded represent a saturated C₅-C₆ ring which is optionally monosubstituted by methyl or methoxy, with the proviso that, in this case, A represents hydrogen,
G represents hydrogen (a) or one of the groups 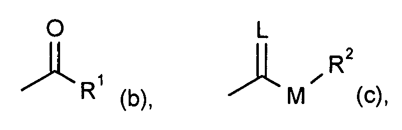 -SO₂-R³ (d) or E (f),
in which
L represents oxygen or sulphur,
M represents oxygen or sulphur and
E represents an ammonium ion,
R¹ represents C₁-C₆-alkyl, C₂-C₁₇-alkenyl, C₁-C₂-alkoxy-C₁-alkyl, C₁-C₂-alkylthio-C₁-alkyl or represents cyclopropyl or cyclohexyl, each of which is optionally monosubstituted by fluorine, chlorine, methyl or methoxy, or represents phenyl which is optionally monosubstituted by fluorine, chlorine, bromine, cyano, nitro, methyl, methoxy, trifluoromethyl or trifluoromethoxy,
R² represents C₁-C₈-alkyl, C₂-C₆-alkenyl or C₁-C₄-alkoxy-C₂-C₃-alkyl, phenyl or benzyl, each of which is optionally monosubstituted by fluorine,
R³ represents C₁-C₈-alkyl.

5. Compounds of the formula (I) according to Claim 1 in which
W represents methoxy, ethoxy, n-propoxy, iso-propoxy, n-butoxy, iso-butoxy, sec-butoxy, methoxy-ethyloxy, ethoxy-ethyloxy or cyclopropylmethoxy,
X represents methyl or ethyl,
Y represents chlorine,
CKE represents one of the groups 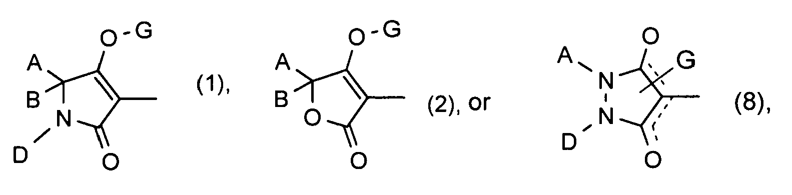
A represents hydrogen, methyl, ethyl, cyclopropyl, isopropyl, n-propyl, isobutyl, n-butyl, t-butyl or s-butyl,
B represents hydrogen, methyl or ethyl,
A, B and the carbon atom to which they are bonded represent saturated C₅-C₆-cycloalkyl in which one ring member is optionally replaced by oxygen and which is optionally monosubstituted by methyl, methoxy, ethoxy, n-propoxy, n-butoxy or trifluoromethyl (in particular methyl or methoxy),
D represents hydrogen, methyl, ethyl, isopropyl, cyclopropyl or cyclohexyl,
or
A and D together represent C₃-C₅-alkanediyl or the group AD-1,
G represents hydrogen (a) or one of the groups 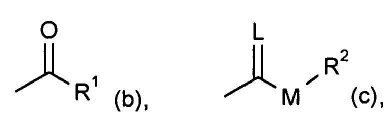 -SO₂-R³ (d) or E(f)
in which
L represents oxygen,
M represents oxygen and
E represents an ammonium ion (N⁺(C₆H₁₃)₄),
R¹ represents C₁-C₈-alkyl, C₁-C₂-alkoxy-C₁-alkyl, or C₂-C₁₇-alkenyl,
R² represents C₁-C₈-alkyl or C₂-C₆-alkenyl,
R³ represents C₁-C₄-alkyl.

6. Process for the preparation of compounds of the formula (I) according to Claim 1, **characterized in that**, to obtain
(A) compounds of the formula (I-1-a) 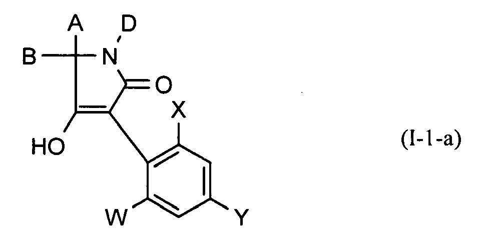 in which
A, B, D, W, X and Y have the abovementioned meanings,
compounds of the formula (II) 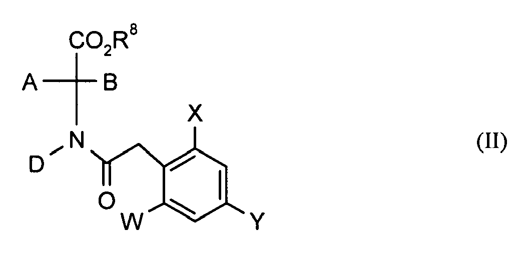 in which
A, B, D, W, X and Y have the abovementioned meanings,
and
R⁸ represents alkyl
are subjected to an intramolecular condensation reaction in the presence of a diluent and in the presence of a base,
(B) compounds of the formula (I-2-a) 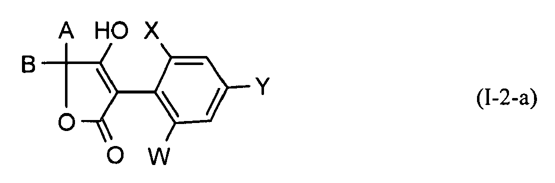 in which
A, B, W, X and Y have the abovementioned meanings,
compounds of the formula (III) 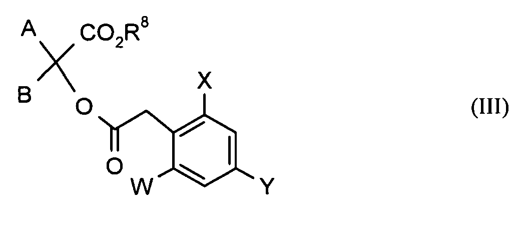 in which
A, B, W, X, Y and R⁸ have the abovementioned meanings are subjected to an intramolecular condensation reaction in the presence of a diluent and in the presence of a base,
(C) compounds of the formula (I-3-a) 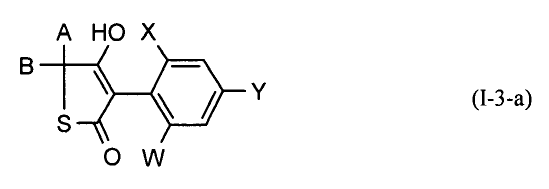 in which
A, B, W, X and Y have the abovementioned meanings,
compounds of the formula (IV) 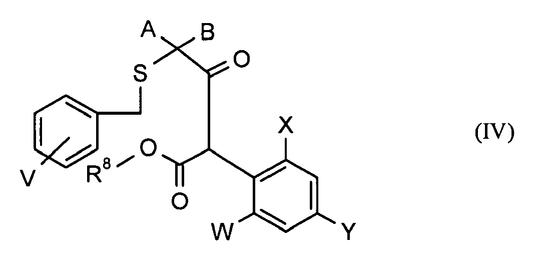 in which
A, B, W, X, Y and R⁸ have the abovementioned meanings and
V represents hydrogen, halogen, alkyl or alkoxy
are subjected to intramolecular cyclization, if appropriate in the presence of a diluent and in the presence of an acid,
(D) compounds of the formula (I-4-a) 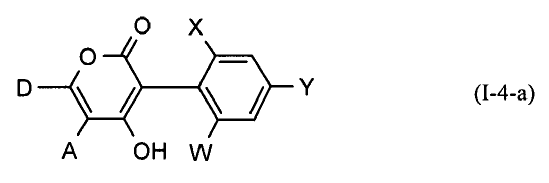 in which
A, D, W, X and Y have the abovementioned meanings,
compounds of the formula (V) 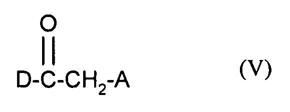 in which
A and D have the abovementioned meanings
or their silyl enol ethers of the formula (Va) 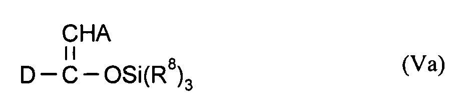 in which
A, D and R⁸ have the abovementioned meanings
are reacted with compounds of the formula (VI) 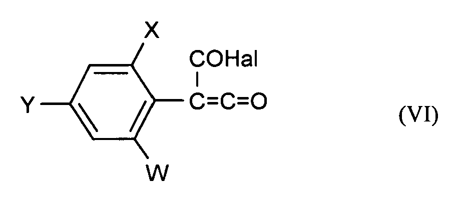 in which
W, X and Y have the abovementioned meanings and
Hal represents halogen,
if appropriate in the presence of a diluent and if appropriate in the presence of an acid acceptor,
(E) compounds of the formula (I-5-a) 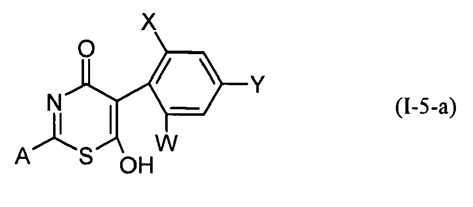 in which
A, W, X and Y have the abovementioned meanings,
compounds of the formula (VII) 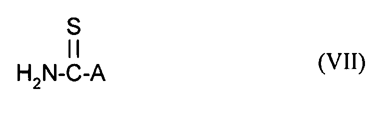 in which
A has the abovementioned meaning
are reacted with compounds of the formula (VI) 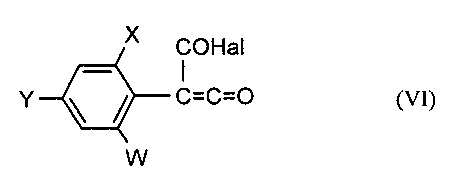 in which
Hal, W, X and Y have the abovementioned meanings,
if appropriate in the presence of a diluent and if appropriate in the presence of an acid acceptor,
(F) compounds of the formula (I-6-a) 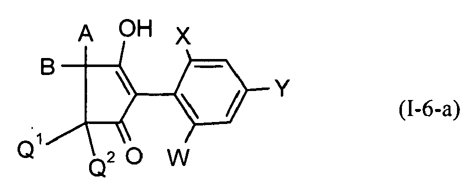 in which
A, B, Q¹, Q², W, X and Y have the abovementioned meanings,
compounds of the formula (VIII) 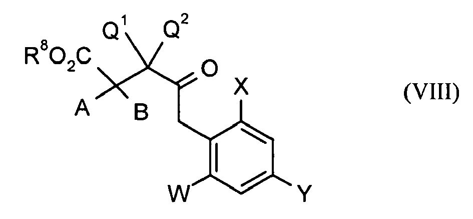 in which
A, B, Q¹, Q², W, X and Y have the abovementioned meanings and
R⁸ represents alkyl
are subjected to an intramolecular cyclization reaction, if appropriate in the presence of a diluent and in the presence of a base,
(G) compounds of the formula (I-7-a) 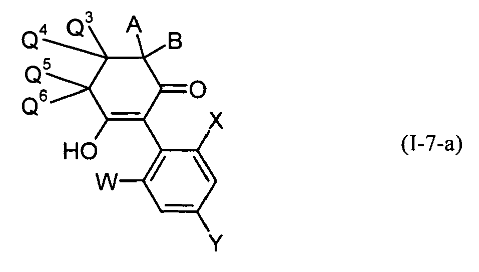 in which
A, B, Q³, Q⁴, Q⁵, Q⁶, W, X and Y have the abovementioned meanings,
compounds of the formula (IX) 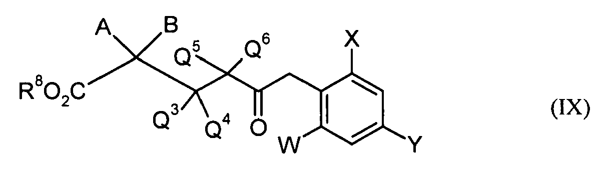 in which
A, B, Q³, Q⁴, Q⁵, Q⁶, W, X and Y have the abovementioned meanings
and
R⁸ represents alkyl
are subjected to an intramolecular condensation reaction in the presence of a diluent and in the presence of a base,
(H) compounds of the formula (I-8-a) 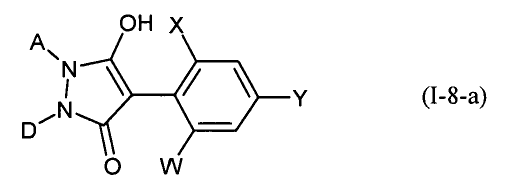 in which
A, D, W, X and Y have the abovementioned meanings,
compounds of the formula (X) 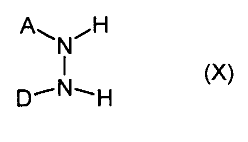 in which
A and D have the abovementioned meanings,
α) are reacted with compounds of the formula (VI) 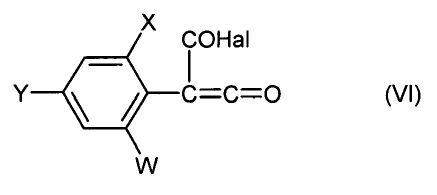 in which
Hal, X, Y and W have the abovementioned meanings,
if appropriate in the presence of a diluent and if appropriate in the presence of an acid acceptor, or
β) are reacted with compounds of the formula (XI) 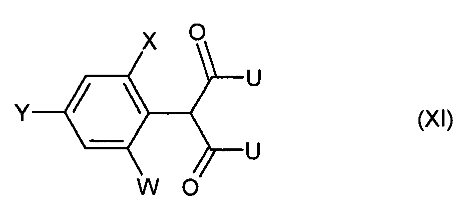 in which
W, X and Y have the abovementioned meanings,
and U represents NH₂ or O-R⁸,
where R⁸ has the abovementioned meaning,
if appropriate in the presence of a diluent and if appropriate in the presence of a base, or
γ) are reacted with compounds of the formula (XII) 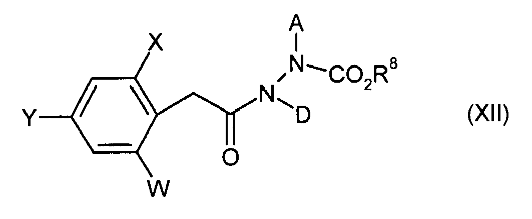 in which
A, D, W, X, Y and R⁸ have the abovementioned meanings,
if appropriate in the presence of a diluent and if appropriate in the presence of a base,
(I) compounds of the formulae (I-1-b) to (I-8-b) shown above in which A, B, D, Q¹, Q², Q³, Q⁴, Q⁵, Q⁶, R¹, W, X, and Y have the abovementioned meanings, compounds of the formulae (I-1-a) to (I-8-a) shown above in which A, B, D, Q¹, Q², Q³, Q⁴, Q⁵, Q⁶, W, X and Y have the abovementioned meanings
(α) are reacted with acid chlorides of the formula (XIII) 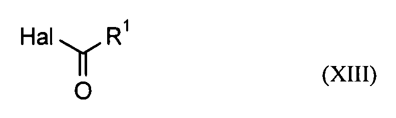 in which
R¹ has the abovementioned meaning and
Hal represents halogen
or
β) are reacted with carboxylic anhydrides of the formula (XIV)
R¹-CO-O-CO-R¹ (XIV)
in which
R¹ has the abovementioned meaning,
if appropriate in the presence of a diluent and if appropriate in the presence of an acid-binding agent;
(J) compounds of the formulae (I-1-c) to (I-8-c) shown above in which A, B, D, Q¹, Q², Q³, Q⁴, Q⁵, Q⁶, R², M, W, X and Y have the abovementioned meanings and L represents oxygen, compounds of the formulae (I-1-a) to (I-8-a) shown above in which A, B, D, Q¹, Q², Q³, Q⁴, Q⁵, Q⁶, W, X and Y have the abovementioned meanings
are reacted with chloroformic esters or chloroformic thioesters of the formula (XV)
R²-M-CO-Cl (XV)
in which
R² and M have the abovementioned meanings,
if appropriate in the presence of a diluent and if appropriate in the presence of an acid-binding agent;
(K) compounds of the formulae (I-1-c) to (I-8-c) shown above in which A, B, D, Q¹, Q², Q³, Q⁴, Q⁵, Q⁶, R², M, W, X and Y have the abovementioned meanings and L represents sulphur, compounds of the formulae (I-1-a) to (I-8-a) shown above in which A, B, D, Q¹, Q², Q³, Q⁴, Q⁵, Q⁶, W, X and Y have the abovementioned meanings
are reacted with chloromonothioformic esters or chlorodithioformic esters of the formula (XVI) 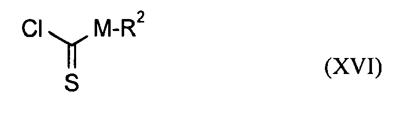 in which
M and R² have the abovementioned meanings,
if appropriate in the presence of a diluent and if appropriate in the presence of an acid-binding agent,
and
(L) compounds of the formulae (I-1-d) to (I-8-d) shown above in which A, B, D, Q¹, Q², Q³, Q⁴, Q⁵, Q⁶, R³, W, X and Y have the abovementioned meanings, compounds of the formulae (I-1-a) to (I-8-a) shown above in which A, B, D, Q¹, Q², Q³, Q⁴, Q⁵, Q⁶, W, X and Y have the abovementioned meanings
are reacted with sulphonyl chlorides of the formula (XVII)
R³-SO₂-Cl (XVII)
in which
R³ has the abovementioned meaning,
if appropriate in the presence of a diluent and if appropriate in the presence of an acid-binding agent,
(M) compounds of the formulae (I-1-e) to (I-8-e) shown above in which A, B, D, L, Q¹, Q², Q³, Q⁴, Q⁵, Q⁶, R⁴, R⁵, W, X and Y have the abovementioned meanings, compounds of the formulae (I-1-a) to (I-8-a) shown above in which A, B, D, Q¹, Q², Q³, Q⁴, Q⁵, Q⁶, W, X and Y have the abovementioned meanings
are reacted with phosphorus compounds of the formula (XVIII) 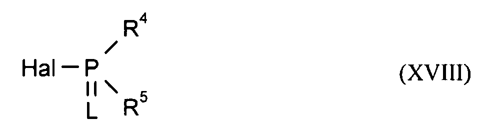 in which
L, R⁴ and R⁵ have the abovementioned meanings and
Hal represents halogen,
if appropriate in the presence of a diluent and if appropriate in the presence of an acid-binding agent,
(N) compounds of the formulae (I-1-f) to (I-8-f)shown above in which A, B, D, E, Q¹, Q², Q³, Q⁴, Q⁵, Q⁶, W, X and Y have the abovementioned meanings, compounds of the formulae (I-1-a) to (I-8-a) shown above in which A, B, D, Q¹, Q², Q³, Q⁴, Q⁵, Q⁶, W, X and Y have the abovementioned meanings
are reacted with metal compounds or amines of the formulae (XIX) or (XX)
Me(OR¹⁰)ₜ (XIX) 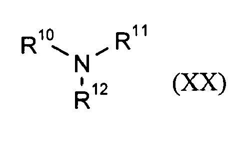 in which
Me represents a mono- or divalent metal,
t represents the number 1 or 2 and
R¹⁰, R¹¹ and R¹² independently of one another represent hydrogen or alkyl,
if appropriate in the presence of a diluent,
(O) compounds of the formulae (I-1-g) to (I-8-g) shown above in which A, B, D, L, Q¹, Q², Q³, Q⁴, Q⁵, Q⁶, R⁶, R⁷, W, X and Y have the abovementioned meanings, compounds of the formulae (I-1-a) to (I-8-a) shown above in which A, B, D, Q¹, Q², Q³, Q⁴, Q⁵, Q⁶, W, X and Y have the abovementioned meanings
are reacted
(α) with isocyanates or isothiocyanates of the formula (XXI)
R⁶-N=C=L (XXI)
in which
R⁶ and L have the abovementioned meanings,
if appropriate in the presence of a diluent and if appropriate in the presence of a catalyst, or
β) are reacted with carbamoyl chlorides or thiocarbamoyl chlorides of the formula (XXII) 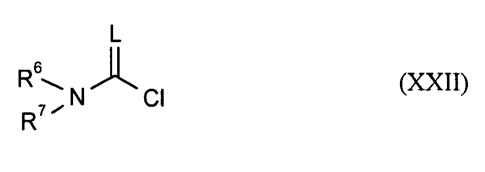 in which
L, R⁶ and R⁷ have the abovementioned meanings,
if appropriate in the presence of a diluent and if appropriate in the presence of an acid-binding agent.

7. Compounds of the formula (II) 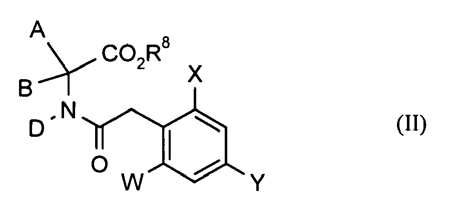 in which
A, B, D, W, X, Y and R⁸ have the abovementioned meanings.

8. Compounds of the formula (III) 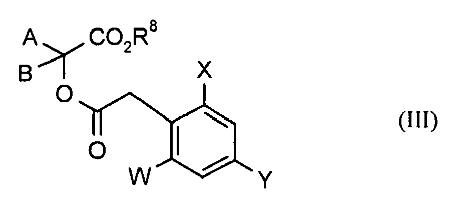 in which
A, B, W, X, Y and R⁸ have the abovementioned meanings.

9. Compounds of the formula (IV) 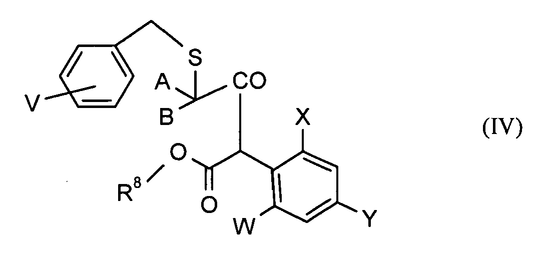 in which
A, B, V, W, X, Y and R⁸ have the abovementioned meanings.

10. Compounds of the formula (VI) 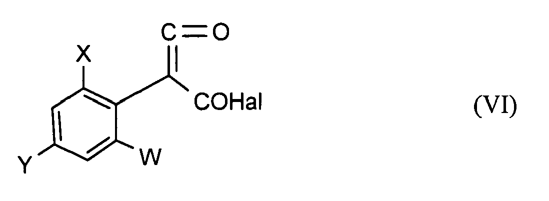 in which
Hal, W, X and Y have the abovementioned meanings.

11. Compounds of the formula (VIII) 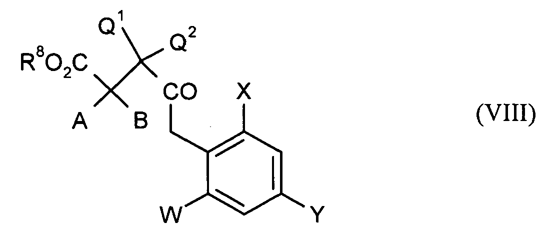 in which
A, B, Q¹, Q², W, X, Y and R⁸ have the abovementioned meanings.

12. Compounds of the formula (IX) 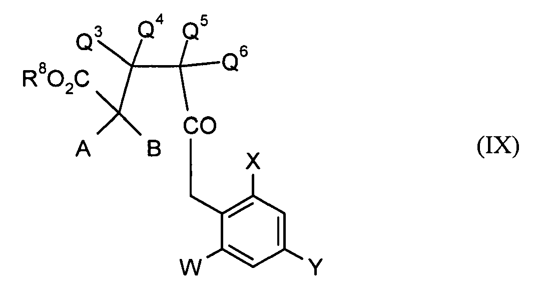 in which
A, B, Q³, Q⁴, Q⁵, Q⁶, W, X, Y and R⁸ have the abovementioned meanings.

13. Compounds of the formula (XI) 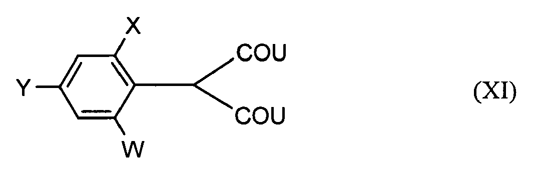 in which
U, W, X and Y have the abovementioned meanings.

14. Compounds of the formula (XII) 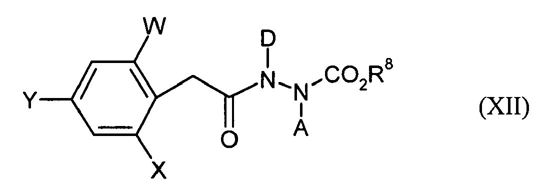 in which
A, D, W, X, Y and R⁸ have the abovementioned meanings.

15. Compounds of the formula (XXIV) 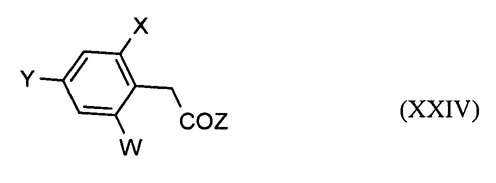 in which
W, X and Y have the abovementioned meanings and
Z represents a leaving group introduced by reagents for the activation of carboxylic acids, such as carbonyldiimidazole, carbonyldiimides (such as, for example, dicyclohexylcarbodiimide), phosphorylating reagents (such as, for example, POCl₃, BOP-Cl), halogenating agents, for example thionyl chloride, oxalyl chloride, phosgene or chloroformic esters

16. Compounds of the formula (XXV) 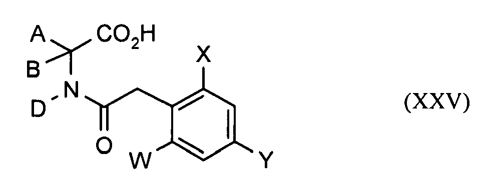 in which
A, B, D, W, X und Y have the abovementioned meanings.

17. Compounds of the formula (XXVII) 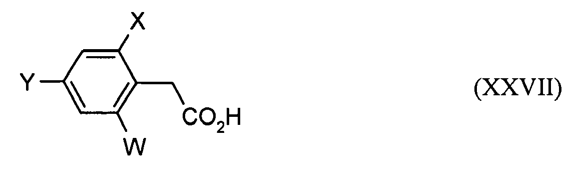 in which
W, X and Y have the abovementioned meanings.

18. Compounds of the formula (XXIX) 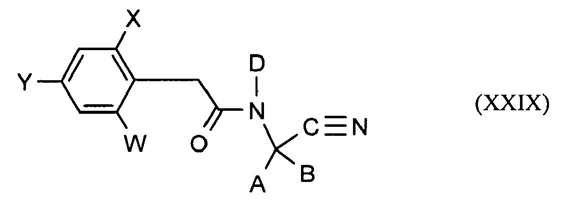 in which
A, B, D, W, X and Y have the abovementioned meanings.

19. Compounds of the formula (XXXI) 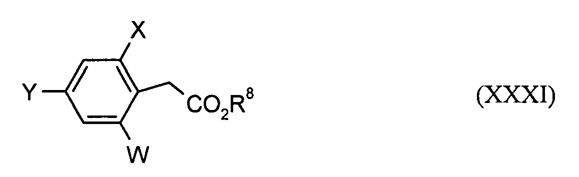 in which
W, X, Y and R⁸ have the abovementioned meanings.

20. Compounds of the formula (XXXIII) 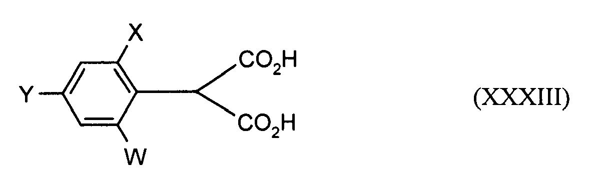 in which
W, X and Y have the abovementioned meanings.

21. Compounds of the formula (XXXIV) 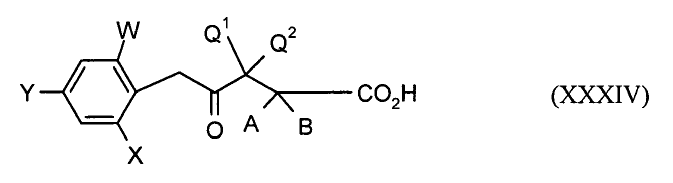 in which
A, B, Q¹, Q², W, X and Y have the abovementioned meanings.

22. Compounds of the formula (XXXV) 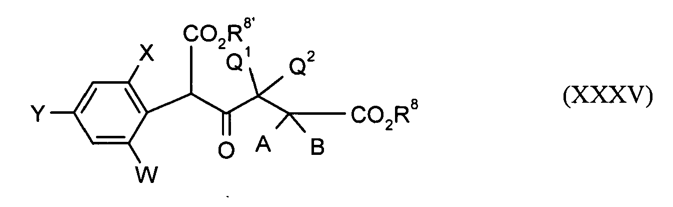 in which
A, B, Q¹, Q², R⁸, R^{8'}, W, X and Y have the abovementioned meanings.

23. Compounds of the formula (XXXVIII) 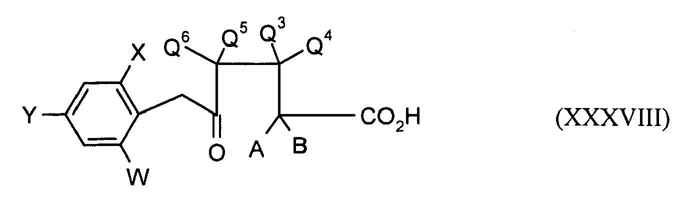 in which
A, B, Q³, Q⁴, Q⁵, Q⁶, W, X and Y have the abovementioned meanings.

24. Compounds of the formula (XXXIX) 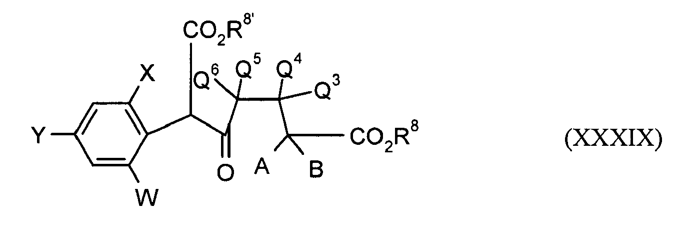 in which
A, B, Q³, Q⁴, Q⁵, Q⁶, R⁸, R^{8'}, W, X and Y have the abovementioned meanings.

25. Composition with an effective content of an active compound combination comprising, as components,
(a') at least one substituted cyclic ketoenol of the formula (I) according to Claim 1 in which CKE, W, X and Y have the abovementioned meanings
and
(b') at least one compound which improves crop plant tolerance and which is selected from the following group of compounds:
4-dichloroacetyl-1-oxa-4-aza-spiro[4.5]-decane (AD-67, MON-4660), 1-dichloroacetyl-hexahydro-3,3,8a-trimethylpyrrolo[1,2-a]-pyrimidin-6(2H)-one (dicyclonon, BAS-145138), 4-dichloroacetyl-3,4-dihydro-3-methyl-2H-1,4-benzoxazine (benoxacor), 1-methyl-hexyl 5-chloro-quinolin-8-oxy-acetate (cloquintocet-mexyl - cf. also related compounds in EP-A-86750, EP-A-94349, EP-A-191736, EP-A-492366), 3-(2-chloro-benzyl)-1-(1-methyl-1-phenylethyl)-urea (cumyluron), α-(cyanomethoximino)-phenylacetonitrile (cyometrinil), 2,4-dichloro-phenoxyacetic acid (2,4-D), 4-(2,4-dichloro-phenoxy)-butyric acid (2,4-DB), 1-(1-methyl-1-phenyl-ethyl)-3-(4-methyl-phenyl)-urea (daimuron, dymron), 3,6-dichloro-2-methoxy-benzoic acid (dicamba), S-1-methyl-1-phenyl-ethyl piperidine-1-thiocarboxylate (dimepiperate), 2,2-dichloro-N-(2-oxo-2-(2-propenylamino)-ethyl)-N-(2-propenyl)-acetamide (DKA-24), 2,2-dichloro-N,N-di-2-propenyl-acetamide (dichlormid), 4,6-dichloro-2-phenyl-pyrimidine (fenclorim), ethyl 1-(2,4-dichloro-phenyl)-5-trichloro-methyl-1H-1,2,4-triazole-3-carboxylate (fenchlorazole-ethyl - cf. also related compounds in EP-A-174562 and EP-A-346620), phenylmethyl 2-chloro-4-trifluoromethyl-thiazole-5-carboxylate (flurazole), 4-chloro-N-(1,3-dioxolan-2-yl-methoxy)-α-trifluoro-acetophenone oxime (fluxofenim), 3-dichloroacetyl-5-(2-furanyl)-2,2-dimethyl-oxazolidine (furilazole, MON-13900), ethyl 4,5-dihydro-5,5-diphenyl-3-isoxazolecarboxylate (isoxadifen-ethyl - cf. also related compounds in WO-A-95/07897), 1-(ethoxycarbonyl)-ethyl-3,6-dichloro-2-methoxybenzoate (lactidichlor), (4-chloro-o-tolyloxy)-acetic acid (MCPA), 2-(4-chloro-o-tolyloxy)-propionic acid (mecoprop), diethyl 1-(2,4-dichloro-phenyl)-4,5-dihydro-5-methyl-1H-pyrazole-3,5-dicarboxylate (mefenpyr-diethyl - cf. also related compounds in WO-A-91/07874), 2-dichloromethyl-2-methyl-1,3-dioxolane (MG-191), 2-propenyl-1-oxa-4-azaspiro[4.5]decane 4-carbodithioate (MG-838), 1,8-naphthalic anhydride, α-(1,3-dioxolan-2-yl-methoximino)-phenylacetonitrile (oxabetrinil), 2,2-dichloro-N-(1,3-dioxolan-2-yl-methyl)-N-(2-propenyl)-acetamide (PPG-1292), 3-dichloroacetyl-2,2-dimethyl-oxazolidine (R-28725), 3-dichloroacetyl-2,2,5-trimethyl-oxazolidine (R-29148), 4-(4-chloro-o-tolyl)-butyric acid, 4-(4-chloro-phenoxy)-butyric acid, diphenylmethoxyacetic acid, methyl diphenylmethoxyacetate, ethyl diphenylmethoxyacetate, methyl 1-(2-chloro-phenyl)-5-phenyl-1H-pyrazole-3-carboxylate, ethyl 1-(2,4-dichlorophenyl)-5-methyl-1H-pyrazole-3-carboxylate, ethyl 1-(2,4-dichloro-phenyl)-5-isopropyl-1H-pyrazole-3-carboxylate, ethyl 1-(2,4-dichloro-phenyl)-5-(1, 1-dimethyl-ethyl)-1H-pyrazole-3-carboxylate, ethyl 1-(2,4-dichloro-phenyl)-5-phenyl-1H-pyrazole-3-carboxylate (cf. also related compounds in EP-A-269806 and EP-A-333131), ethyl 5-(2,4-dichloro-benzyl)-2-isoxazoline-3-carboxylate,
ethyl 5-phenyl-2-isoxazoline-3-carboxylate, ethyl 5-(4-fluoro-phenyl)-5-phenyl-2-isoxazoline-3-carboxylate (cf. also related compounds in WO-A-91/08202), 1,3-dimethyl-but-1-yl 5-chloro-quinolin-8-oxy-acetate, 4-allyloxy-butyl 5-chloro-quinolin-8-oxy-acetate, 1-allyloxy-prop-2-yl 5-chloro-quinolin-8-oxyacetate, methyl 5-chloro-quinolin-8-oxy-acetate, ethyl 5-chloro-quinolin-8-oxyacetate, allyl 5-chloro-quinoxalin-8-oxy-acetate, 2-oxo-prop-l-yl 5-chloro-quinolin-8-oxy-acetate, diethyl 5-chloro-quinolin-8-oxy-malonate, diallyl 5-chloro-quinoxalin-8-oxy-malonate, diethyl 5-chloro-quinolin-8-oxy-malonate (cf. also related compounds in EP-A-582198), 4-carboxy-chroman-4-yl-acetic acid (AC-304415, cf. EP-A-613618), 4-chloro-phenoxy-acetic acid, 3,3'-dimethyl-4-methoxy-benzophenone, 1-bromo-4-chloromethylsulphonyl-benzene, 1-[4-(N-2-methoxybenzoylsulphamoyl)-phenyl]-3-methyl-urea (alias N-(2-methoxy-benzoyl)-4-[(methylamino-carbonyl)-amino]-benzenesulphonamide), 1-[4-(N-2-methoxybenzoylsulphamoyl)-phenyl]-3,3-dimethyl-urea, 1-[4-(N-4,5-dimethylbenzoylsulphamoyl)-phenyl]-3-methyl-urea, 1-[4-(N-naphthylsulphamoyl)-phenyl]-3,3-dimethyl-urea, N-(2-methoxy-5-methyl-benzoyl)-4-(cyclopropylaminocarbonyl)-benzenesulphonamide,
and/or one of the following compounds (defined by general formulae)
of the general formula (IIa) 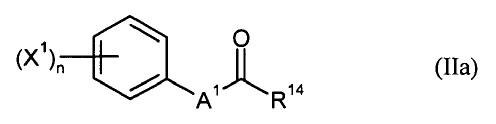 or of the general formula (IIb) 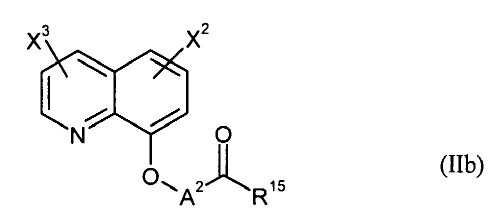 or of the formula (IIc) 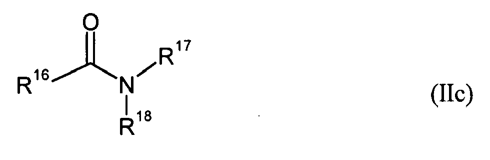 where
n represents a number of between 0 and 5,
A¹ represents one of the divalent heterocyclic groups outlined hereinbelow, 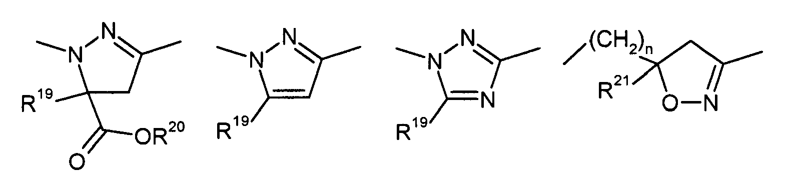
n represents a number of between 0 and 5,
A² represents alkanediyl having 1 or 2 carbon atoms which is optionally substituted by C₁-C₄-alkyl and/or C₁-C₄-alkoxycarbonyl,
R¹⁴ represents hydroxyl, mercapto, amino, C₁-C₆-alkoxy, C₁-C₆-alkylthio, C₁-C₆-alkylamino or di(C₁-C₄-alkyl)amino,
R¹⁵ represents hydroxyl, mercapto, amino, C₁-C₆-alkoxy, C₁-C₆-alkylthio, C₁-C₆-alkylamino or di(C₁-C₄-alkyl)amino,
R¹⁶ represents C₁-C₄-alkyl which is optionally substituted in each case by fluorine, chlorine and/or bromine,
R¹⁷ represents hydrogen, or represents C₁-C₆-alkyl, C₂-C₆-alkenyl or C₂-C₆- alkinyl, C₁-C₄-alkoxy-C₁-C₄-alkyl, dioxolanyl-C₁-C₄-alkyl, furyl, furyl- C₁-C₄-alkyl, thienyl, thiazolyl, piperidinyl, each of which is optionally substituted by fluorine, chlorine and/or bromine, or represents phenyl which is optionally substituted by fluorine, chlorine and/or bromine or C₁-C₄-alkyl,
R¹⁸ represents hydrogen, or represents C₁-C₆-alkyl, C₂-C₆-alkenyl or C₂-C₆- alkinyl, C₁-C₄-alkoxy-C₁-C₄-alkyl, dioxolanyl-C₁-C₄-alkyl, furyl, furyl- C₁-C₄-alkyl, thienyl, thiazolyl, piperidinyl, each of which is optionally substituted by fluorine, chlorine and/or bromine, or represents phenyl which is optionally substituted by fluorine, chlorine and/or bromine or C₁-C₄-alkyl, or together with R¹⁷ represents C₃-C₆-alkanediyl or C₂-C₅- oxaalkanediyl, each of which is optionally substituted by C₁-C₄-alkyl, phenyl, furyl, a fused benzene ring or by two substituents which, together with the C atom to which they are bonded, form a 5- or 6- membered carbocycle,
R¹⁹ represents hydrogen, cyano, halogen, or represents C₁-C₄-alkyl, C₃-C₆- cycloalkyl or phenyl, each of which is optionally substituted by fluorine, chlorine and/or bromine,
R²⁰ represents hydrogen, or represents C₁-C₆-alkyl, C₃-C₆-cycloalkyl or tri(C₁-C₄-alkyl)silyl, in each case optionally substituted by hydroxyl, cyano, halogen or C₁-C₄-alkoxy,
R²¹ represents hydrogen, cyano, halogen, or represents C₁-C₄-alkyl, C₃-C₆- cycloalkyl or phenyl, each of which is optionally substituted by fluorine, chlorine and/or bromine,
X¹ represents nitro, cyano, halogen, C₁-C₄-alkyl, C₁-C₄-halogenoalkyl, C₁-C₄-alkoxy or C₁-C₄-halogenoalkoxy,
X² represents hydrogen, cyano, nitro, C₁-C₄-alkyl, C₁-C₄-halogenoalkyl, C₁-C₄-alkoxy or C₁-C₄-halogenoalkoxy,
X³ represents hydrogen, cyano, nitro, C₁-C₄-alkyl, C₁-C₄-halogenoalkyl, C₁-C₄-alkoxy or C₁-C₄-halogenoalkoxy,
and/or the following compounds (defined by general formulae)
of the general formula (IId) 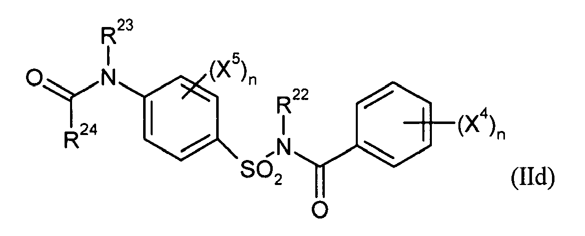 or of the general formula (IIe) 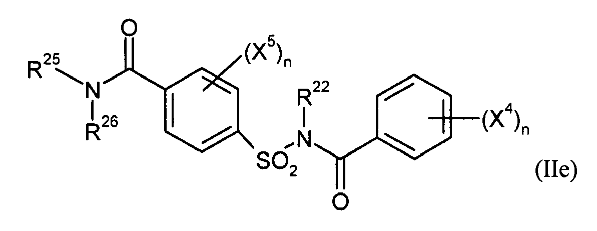 where
n represents a number of between 0 and 5,
R²² represents hydrogen or C₁-C₄-alkyl,
R²³ represents hydrogen or C₁-C₄-alkyl,
R²⁴ represents hydrogen, or represents C₁-C₆-alkyl, C₁-C₆-alkoxy, C₁-C₆- alkylthio, C₁-C₆-alkylamino or di(C₁-C₄-alkyl)amino, each of which is optionally substituted by cyano, halogen or C₁-C₄-alkoxy, or represents C₃-C₆-cycloalkyl, C₃-C₆-cycloalkyloxy, C₃-C₆-cycloalkylthio or C₃-C₆- cycloalkylamino, each of which is optionally substituted by cyano, halogen or C₁-C₄-alkyl,
R²⁵ represents hydrogen, or represents C₁-C₆-alkyl which is optionally substituted by cyano, hydroxyl, halogen or C₁-C₄-alkoxy, or represents C₃-C₆-alkenyl or C₃-C₆-alkinyl, each of which is optionally substituted by cyano or halogen, or represents C₃-C₆-cycloalkyl which is optionally substituted by cyano, halogen or C₁-C₄-alkyl,
R²⁶ represents hydrogen, or represents C₁-C₆-alkyl which is optionally substituted by cyano, hydroxyl, halogen or C₁-C₄-alkoxy, or represents C₃-C₆-alkenyl or C₃-C₆-alkinyl, each of which is optionally substituted by cyano or halogen, or represents C₃-C₆-cycloalkyl which is optionally substituted by cyano, halogen or C₁-C₄-alkyl, or represents phenyl which is optionally substituted by nitro, cyano, halogen, C₁-C₄-alkyl, C₁-C₄- halogenoalkyl, C₁-C₄-alkoxy or C₁-C₄-halogenoalkoxy, or together with R²⁵ represents C₂-C₆-alkanediyl or C₂-C₅-oxaalkanediyl, each of which is optionally substituted by C₁-C₄-alkyl,
X⁴ represents nitro, cyano, carboxyl, carbamoyl, formyl, sulphamoyl, hydroxyl, amino, halogen, C₁-C₄-alkyl, C₁-C₄-halogenoalkyl, C₁-C₄- alkoxy or C₁-C₄-halogenoalkoxy, and
X⁵ represents nitro, cyano, carboxyl, carbamoyl, formyl, sulphamoyl, hydroxyl, amino, halogen, C₁-C₄-alkyl, C₁-C₄-halogenoalkyl, C₁-C₄- alkoxy or C₁-C₄-halogenoalkoxy.

26. Composition according to Claim 25, in which the compound which improves crop plant tolerance is selected from the following group of compounds:
cloquintocet-mexyl, fenchlorazole-ethyl, isoxadifen-ethyl, mefenpyr-diethyl, furilazole, fenclorim, cumyluron, dymron or the compounds 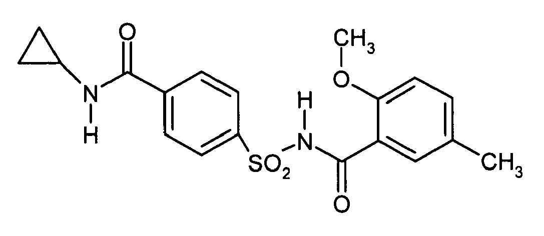 and

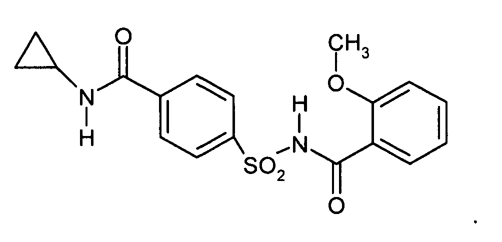

27. Composition according to Claim 25 or 26 in which the compound which improves crop plant tolerance is cloquintocet-mexyl or mefenpyr-diethyl.

28. Pesticides and/or herbicides, **characterized by** a content of at least one compound of the formula (I) according to Claim 1.

29. Method for controlling animal pests and/or undesired vegetation, **characterized in that** compounds of the formula (I) according to Claim 1 are allowed to act on pests and/or their environment.

30. Use of compounds of the formula (I) according to Claim 1 for controlling animal pests and/or undesired vegetation.

31. Process for the preparation of pesticides and/or herbicides, **characterized in that** compounds of the formula (I) according to Claim 1 are mixed with extenders and/or surface-active substances.

32. Use of compounds of the formula (I) according to Claim 1 for the preparation of pesticides and/or herbicides.

33. Method for controlling undesired vegetation, **characterized in that** a composition according to Claim 25 is allowed to act on the plants or their environment.

34. Use of a composition according to Claim 25 for controlling undesired vegetation.

35. Method for controlling undesired vegetation, **characterized in that** a compound of the formula (I) according to Claim 25 and the compound which improves crop plant tolerance according to Claim 1 are allowed to act separately at short intervals on the plants or their environment.

## Revendications

1. Composés de formule (I) 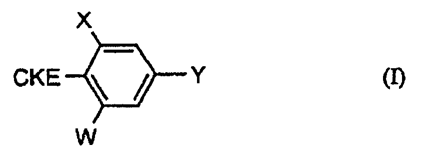 dans laquelle
W est un groupe alcoxy, halogènalcoxy, alcoxyalkyloxy, alcoxy-bisalcoxy ou cycloalkyl-alcanediyl-oxy éventuellement substitué, qui éventuellement peut être interrompu par des hétéroatomes,
X représente un groupe alkyle,
Y représente le chlore, le brome ou l'iode,
CKE représente l'un des groupes 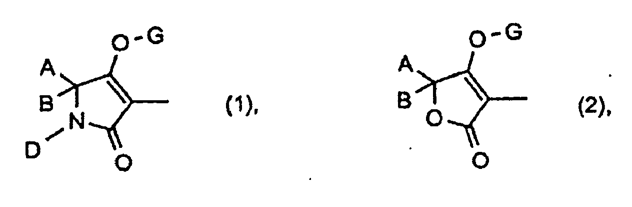 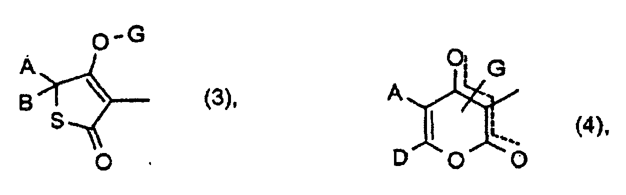 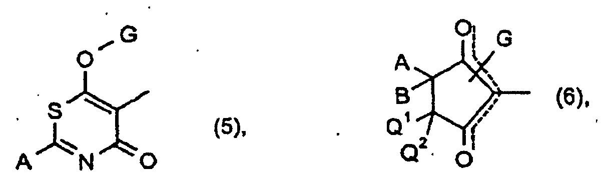 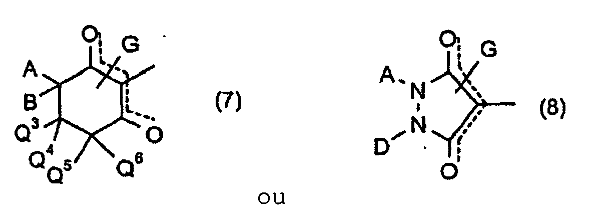 dans lesquelles
A représente un atome d'hydrogène, un groupe alkyle, alcényle, alcoxyalkyle, alkylthioalkyle, chacun étant éventuellement halogéné, cycloalkyle éventuellement substitué, saturé ou insaturé, dans lequel éventuellement au moins un atome nucléaire est remplacé par un hétéroatome, ou un groupe aryle, arylalkyle ou hétéroaryle, dont chacun est éventuellement substitué par un ou des substituants halogéno, alkyle, halogènalkyle, alcoxy, halogènalcoxy, cyano ou nitro,
B est un atome d'hydrogène ou un groupe alkyle ou alcoxyalkyle, ou
A et B, ensemble avec l'atome de carbone auquel ils sont liés, représentent un radical cyclique saturé ou insaturé, substitué ou non substitué, contenant au moins un hétéroatome,
D représente un atome d'hydrogène ou un radical éventuellement substitué, de la série comprenant les groupes alkyle, alcényle, alcynyle, alcoxyalkyle, cycloalkyle saturé ou insaturé, dans lequel éventuellement un ou plusieurs chaînons du cycle sont remplacés par des hétéroatomes, arylalkyle, aryle, hétéroarylalkyle ou hétéroaryle, ou
A et D, ensemble avec les atomes auxquels ils sont liés, forment un radical cyclique saturé ou non saturé, contenant éventuellement au moins un (dans le cas de CKE-8, au moins un autre) hétéroatome, non substitué ou substitué dans la partie A-D, ou encore
A et Q¹ forment ensemble un radical alcanediyle ou alcènediyle éventuellement substitué par un ou des groupes hydroxy, par un ou des groupes alkyle, alcoxy, alkylthio, cycloalkyle, benzyloxy ou aryle, dont chacun est éventuellement substitué, ou
Q¹ est un atome d'hydrogène ou un groupe alkyle,
Q², Q⁴, Q⁵ et Q⁶ représentent chacun indépendamment des autres un atome d'hydrogène ou un groupe alkyle,
Q³ représente un atome d'hydrogène, un groupe alkyle, cycloalkyle, alkylthioalkyle éventuellement substitué, cycloalkyle éventuellement substitué (dans lequel éventuellement un groupe méthylène est remplacé par un atome d'oxygène ou de soufre), ou phényle éventuellement substitué, ou
Q³ et Q⁴, ensemble avec l'atome de carbone auquel ils sont liés, représentent un radical cyclique saturé ou insaturé, contenant éventuellement un hétéroatome, non substitué ou substitué,
G représente un atome d'hydrogène (a) ou l'un des groupes 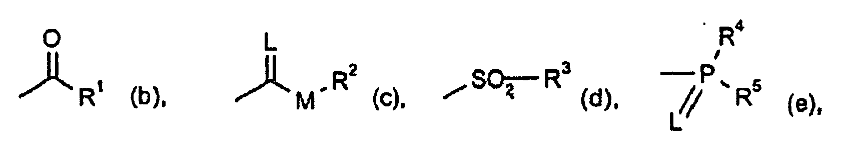 E(f) ou 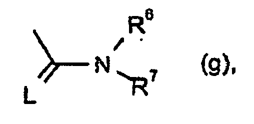 où
E est un équivalent d'un ion métallique ou un ion ammonium,
L est un atome d'oxygène ou de soufre,
M est un atome d'oxygène ou de soufre,
R¹ représente un groupe alkyle, alcényle, alcoxyalkyle, alkylthioalkyle, polyalcoxyalkyle, dont chacun est éventuellement halogéné, ou cycloalkyle éventuellement substitué par un ou plusieurs groupes halogéno, alkyle ou alcoxy, qui peut être interrompu par au moins un hétéroatome, phényle, phénylalkyle, hétéroaryle, phénoxyalkyle ou hétéroaryloxyalkyle dont chacun est éventuellement substitué,
R² représente un groupe alkyle, alcényle, alcoxyalkyle, polyalcoxyalkyle dont chacun est éventuellement halogéné, ou cycloalkyle, phényle ou benzyle dont chacun est éventuellement substitué,
R³, R⁴ et R⁵ représentent chacun indépendamment de l'autre un groupe alkyle, alcoxy, alkylamino, dialkylamino, alkylthio, alcénylthio, cycloalkylthio, dont chacun est éventuellement halogéné, ou phényle, benzyle, phénoxy ou phénylthio dont chacun est éventuellement substitué,
R⁶ et R⁷ représentent chacun indépendamment de l'autre un atome d'hydrogène, un groupe alkyle, cycloalkyle, alcényle, alcoxy, alcoxyalkyle dont chacun est éventuellement halogéné, phényle éventuellement substitué, benzyle éventuellement substitué, ou, ensemble avec l'atome d'azote auquel ils sont liés, représentent un groupe cyclique éventuellement interrompu par un ou des atomes d'oxygène ou de soufre.

2. Composés de formule (I) selon la revendication 1, dans laquelle
W est un groupe alcoxy en C₁-C₆, halogènalcoxy en C₁-C₆, (alcoxy en C₁-C₄) - (alkyloxy en C₂-C₄), (alcoxy en C₁-C₄) - bis (alkyloxy en C₂-C₄), ou (cycloalkyle en C₃-C₆) - (alcanediyle en C₁-C₃)-oxy éventuellement une à trois fois substitué par des substituants fluoro, chloro, alkyle en C₁-C₃ ou alcoxy en C₁-C₃, où éventuellement un groupe méthylène du noyau peut être interrompu par un atome d'oxygène ou de soufre,
X est un groupe alkyle en C₁-C₆,
Y représente le chlore, le brome ou l'iode,
CKE représente l'un des groupes 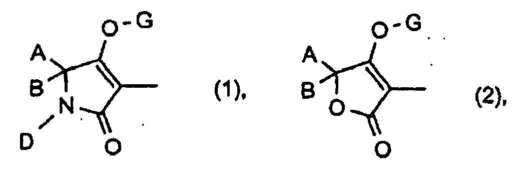 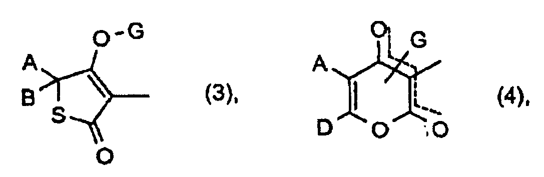 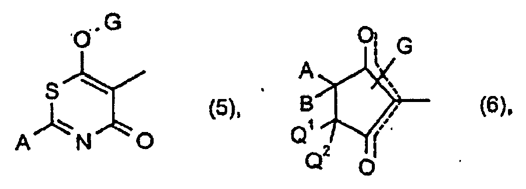 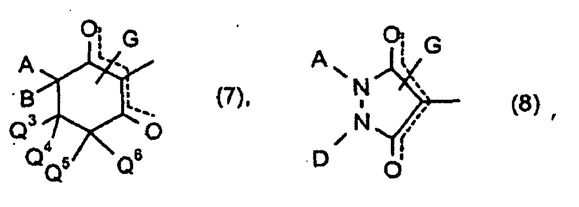 A représente un atome d'hydrogène ou un groupe alkyle en C₁-C₁₂, alcényle en C₃-C₈, (alcoxy en C₁-C₁₀)-(alkyle en C₁-C₈), (alkylthio en C₁-C₁₀)-(alkyle en C₁-C₆) dont chacun est éventuellement halogéné, cycloalkyle en C₃-C₈ éventuellement substitué par un ou des substituants halogéno, alkyle en C₁-C₆ ou alcoxy en C₁-C₆, dans lequel éventuellement un ou deux chaînons du cycle non immédiatement voisins sont remplacés par des atomes d'oxygène et/ou de soufre, ou phényle, naphtyle, hétéroaryle ayant 5 à 6 atomes nucléaires, phényl-(alkyle en C₁-C₆) ou naphtyl-(alkyle en C₁-C₆) dont chacun est éventuellement substitué par un ou des substituants halogéno, alkyle en C₁-C₆, halogènalkyle en C₁-C₆, alcoxy en C₁-C₆, halogènalcoxy en C₁-C₆, cyano ou nitro,
B est un atome d'hydrogène, un groupe alkyle en C₁-C₁₂ ou (alcoxy en C₁-C₈)-(alkyle en C₁-C₆), ou
A, B, et l'atome de carbone auquel ils sont liés, représentent un groupe cycloalkyle en C₃-C₁₀ saturé ou cycloalkyle en C₅-C₁₀ insaturé, dans lequel éventuellement un chaînon du cycle est remplacé par un atome d'oxygène ou de soufre, et qui sont éventuellement une ou deux fois substitués par des substituants alkyle en C₁-C₈, cycloalkyle en C₃-C₁₀, halogènalkyle en C₁-C₈, alcoxy en C₁-C₈, alkylthio en C₁-C₈, halogéno ou phényle, ou
A, B, et l'atome de carbone auquel ils sont liés, représentent un groupe cycloalkyle en C₃-C₆, qui est substitué par un groupe alkylènediyle, éventuellement substitué par un ou des substituants alkyle en C₁-C₄, contenant éventuellement un ou deux atomes d'oxygène ou de soufre non immédiatement voisins, ou par un groupe alkylènedioxyle ou par un groupe alkylènedithioyle, qui avec l'atome de carbone auquel il est lié forme un autre cycle à cinq à huit chaînons, ou
A, B et l'atome de carbone auquel ils sont liés représentent un groupe cycloalkyle en C₃-C₈ ou cycloalcényle en C₅-C₈, dans lesquels deux substituants, ensemble avec les atomes de carbone auxquels ils sont liés, représentent chacun un radical alcanediyle en C₂-C₆, alcènediyle en C₂-C₆ ou (alcane en C₄-C₆)diènediyle, éventuellement substitué par un ou deux substituants alkyle en C₁-C₆, alcoxy en C₁-C₆ ou halogéno, où éventuellement un groupe méthylène est remplacé par un atome d'oxygène ou de soufre,
D représente un atome d'hydrogène, un groupe alkyle en C₁-C₁₂, alcényle en C₃-C₈, alcynyle en C₃-C₈, (alcoxy en C₁-C₁₀)-(alkyle en C₂-C₈) dont chacun est éventuellement halogéné, cycloalkyle en C₃-C₈ éventuellement substitué par un ou des substituants halogéno, alkyle en C₁-C₄, alcoxy en C₁-C₄ ou halogènalkyle en C₁-C₄, dans lequel éventuellement un chaînon du cycle est remplacé par un atome d'oxygène ou de soufre, ou phényle, hétéroaryle ayant 5 ou 6 atomes nucléaires, phényl-(alkyle en C₁-C₆) ou hétéroaryle-(alkyle en C₁-C₆) ayant 5 ou 6 atomes nucléaires, dont chacun est éventuellement substitué par un ou des substituants halogéno, alkyle en C₁-C₆, halogènalkyle en C₁-C₆, alcoxy en C₁-C₆, halogènalcoxy en C₁-C₆, cyano ou nitro, ou
A et D représentent ensemble un radical alcanediyle en C₃-C₆ ou alcènediyle en C₃-C₆ dont chacun est éventuellement substitué, où éventuellement un groupe méthylène est remplacé par un groupe carbonyle, un atome d'oxygène ou de soufre, etoù entrent en ligne de compte en tant que substituants les groupes suivants : halogéno, hydroxy, mercapto, ou alkyle en C₁-C₁₀, alcoxy en C₁-C₆, alkylthio en C₁-C₆, cycloalkyle en C₃-C₇, phényle ou benzyloxy, dont chacun est éventuellement halogéné, ou un groupement alcanediyle en C₃-C₆, un groupement alcènediyle en C₃-C₆ ou un groupement butadiényle supplémentaire, qui éventuellement est substitué par un ou des substituants alkyle en C₁-C₆, ou dans lequel éventuellement deux substituants voisins, avec les atomes de carbone auxquels ils sont liés, forment un groupe cyclique saturé ou insaturé supplémentaire ayant 5 ou 6 atomes nucléaires (dans le cas du composé de formule (I-1), A et D représentent alors, ensemble avec les atomes auxquels ils sont liés, par exemple les groupes AD-1 à AD-10 qui sont mentionnés plus en détail ci-dessous), lequel peut contenir un atome d'oxygène ou de soufre, ou où éventuellement l'un des groupes suivants 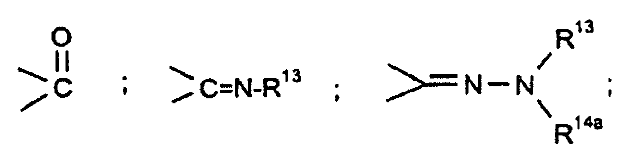 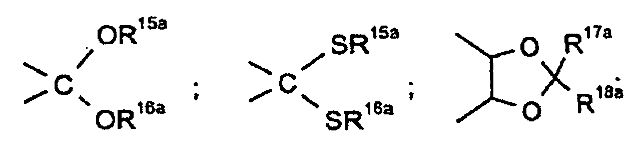 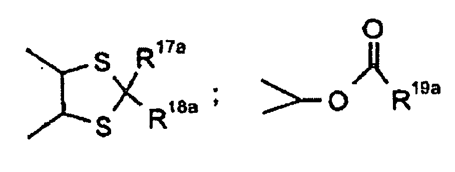 ou 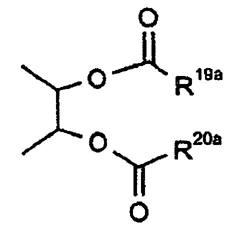 est présent,
où
A et Q¹ représentent ensemble un radical alcanediyle en C₃-C₆ ou alcènediyle en C₄-C₆, dont chacun est éventuellement substitué, une ou deux fois, d'une manière identique ou différente, par des substituants halogéno, hydroxy ; alkyle en C₁-C₁₀, alcoxy en C₁-C₆, alkylthio en C₁-C₆, cycloalkyle en C₃-C₇, dont chacun est éventuellement une à trois fois substitué d'une manière identique ou différente par des substituants halogéno, ou par un ou des substituants benzyloxy ou phényle, dont chacun est éventuellement une à trois fois substitué d'une manière identique ou différente par des substituants halogéno, alkyle en C₁-C₆ ou alcoxy en C₁-C₆, et qui en outre contient éventuellement l'un des groupes ci-après 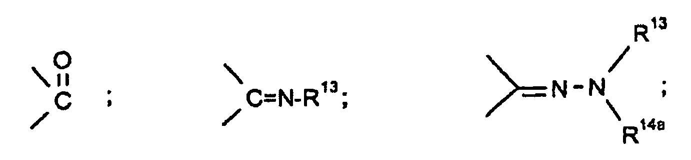 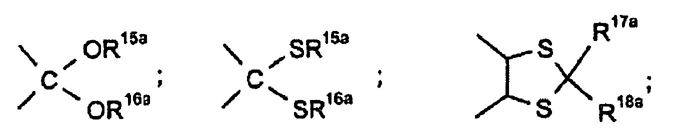 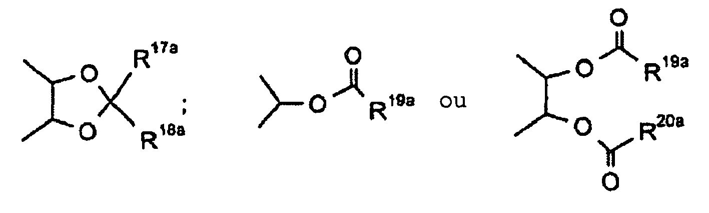 ou est ponté par un radical alcanediyle en C₁-C₂ ou par un atome d'oxygène, ou
Q¹ est un atome d'hydrogène ou un groupe alkyle en C₁-C₄,
Q², Q⁴, Q⁵ et Q⁶ représentent chacun indépendamment des autres un atome d'hydrogène ou un groupe alkyle en C₁-C₄,
Q³ est un atome d'hydrogène, un groupe alkyle en C₁-C₆, (alcoxy en C₁-C₆)-(alkyle en C₁-C₂), (alkylthio en C₁-C₆)-(alkyle en C₁-C₂), cycloalkyle en C₃-C₈ éventuellement substitué par un ou des substituants alkyle en C₁-C₄ ou alcoxy en C₁-C₄, où éventuellement un groupe méthylène est remplacé par un atome d'oxygène ou de soufre, et phényle éventuellement substitué par un ou des substituants halogéno, alkyle en C₁-C₄, alcoxy en C₁-C₄, halogènalkyle en C₁-C₂, halogènalcoxy en C₁-C₂, cyano ou nitro, ou
Q³ et Q⁴, ensemble avec l'atome de carbone auquel ils sont liés, représentent un radical cyclique en C₃-C₇ éventuellement substitué par un ou des substituants alkyle en C₁-C₄, alcoxy en C₁-C₄ ou halogènalkyle en C₁-C₂, et dans lequel éventuellement un chaînon du cycle est remplacé par un atome d'oxygène ou de soufre,
G est un atome d'hydrogène (a) ou l'un des groupes 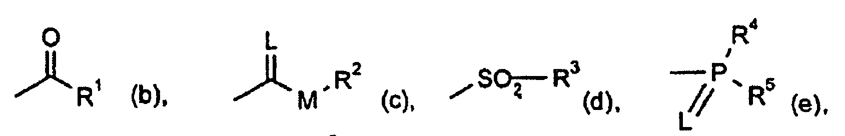 E (f) ou 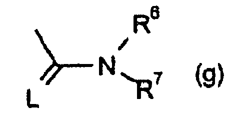 dans lesquels
E est un équivalent d'un ion métallique ou un ion ammonium,
L est un atome d'oxygène ou de soufre,
M est un atome d'oxygène ou de soufre,
R¹ représente un groupe alkyle en C₁-C₂₀, alcényle en C₂-C₂₀, (alcoxy en C₁-C₈)-(alkyle en C₁-C₈), (alkylthio en C₁-C₈)-(alkyle en C₁-C₈), poly((alcoxy en C₁-C₈)-(alkyle en C₁-C₈)) dont chacun est éventuellement halogéné, ou un groupe cycloalkyle en C₃-C₈ éventuellement substitué par un ou des substituants halogéno, alkyle en C₁-C₆ ou alcoxy en C₁-C₆, dans lequel éventuellement un ou plusieurs chaînons du cycle qui ne sont pas immédiatement voisins sont remplacés par des atomes d'oxygène et/ou de soufre,
phényle, éventuellement substitué par un ou des substituants halogéno, cyano, nitro, alkyle en C₁-C₆, alcoxy en C₁-C₆, halogènalkyle en C₁-C₆, halogènalcoxy en C₁-C₆, alkylthio en C₁-C₆ ou alkylsulfonyle en C₁-C₆,
phényl-(alkyle en C₁-C₆), éventuellement substitué par un ou des substituants halogéno, nitro, cyano, alkyle en C₁-C₆, alcoxy en C₁-C₆, halogènalkyle en C₁-C₆ ou halogènalcoxy en C₁-C₆,
hétéroaryle à 5 ou 6 chaînons, éventuellement substitué par un ou des substituants halogéno ou alkyle en C₁-C₆,
phénoxy-(alkyle en C₁-C₆) éventuellement substitué par un ou des substituants halogéno ou alkyle en C₁-C₆,
hétéroaryloxy-(alkyle en C₁-C₆) à 5 ou 6 chaînons, éventuellement substitué par un ou des substituants halogéno, amino ou alkyle en C₁-C₆,
R² représente un groupe alkyle en C₁-C₂₀, alcényle en C₂-C₂₀, (alcoxy en C₁-C₈)-(alkyle en C₂-C₈), poly((alcoxy en C₁-C₈)-(alkyle en C₂-C₈)) dont chacun est éventuellement halogéné,
cycloalkyle en C₃-C₈ éventuellement substitué par un ou des substituants halogéno, alkyle en C₁-C₆ ou alcoxy en C₁-C₆,
phényle ou benzyle, dont chacun est éventuellement substitué par un ou des substituants halogéno, cyano, nitro, alkyle en C₁-C₆, alcoxy en C₁-C₆, halogènalkyle en C₁-C₆ ou halogènalcoxy en C₁-C₆,
R³ représente un groupe alkyle en C₁-C₈ éventuellement halogéné, ou un groupe phényle ou benzyle, dont chacun est éventuellement substitué par un ou des substituants halogéno, alkyle en C₁-C₆, alcoxy en C₁-C₆, halogènalkyle en C₁-C₄, halogènalcoxy en C₁-C₄, cyano ou nitro,
R⁴ et R⁵ représentent chacun indépendamment de l'autre un groupe alkyle en C₁-C₈, alcoxy en C₁-C₈, alkylamino en C₁-C₈, di (alkyle en C₁-C₈) amino, alkylthio en C₁-C₈, alcénylthio en C₂-C₈, cycloalkylthio en C₃-C₇ dont chacun est éventuellement halogéné, ou un groupe phényle, phénoxy ou phénylthio, dont chacun est éventuellement substitué par un ou des substituants halogéno, nitro, cyano, alcoxy en C₁-C₄, halogènalcoxy en C₁-C₄, alkylthio en C₁-C₄, halogènalkylthio en C₁-C₄, alkyle en C₁-C₄ ou halogènalkyle en C₁-C₄,
R⁶ et R⁷ représentent chacun indépendamment de l'autre un atome d'hydrogène, un groupe alkyle en C₁-C₈, cycloalkyle en C₃-C₈, alcoxy en C₁-C₈, alcényle en C₃-C₈, (alcoxy en C₁-C₈)-(alkyle en C₁-C₈) dont chacun est éventuellement halogéné, phényle éventuellement substitué par un ou des substituants halogéno, halogènalkyle en C₁-C₈, alkyle en C₁-C₈ ou alcoxy en C₁-C₈, benzyle éventuellement substitué par un ou des substituants halogéno, alkyle en C₁-C₈, halogènalkyle en C₁-C₈ ou alcoxy en C₁-C₈, ou représentent ensemble un radical alkylène en C₃-C₆ éventuellement substitué par un ou des substituants alkyle en C₁-C₄, radical dans lequel éventuellement un atome de carbone est remplacé par un atome d'oxygène ou de soufre,
R¹³ est un atome d'hydrogène, un groupe alkyle en C₁-C₈ ou alcoxy en C₁-C₈ dont chacun est éventuellement halogéné, cycloalkyle en C₃-C₈ éventuellement substitué par un ou des substituants halogéno, alkyle en C₁-C₄ ou alcoxy en C₁-C₄, dans lequel éventuellement un groupe méthylène est remplacé par un atome d'oxygène ou de soufre, ou phényle, phényl-(alkyle en C₁-C₄) ou phényl-(alcoxy en C₁-C₄) dont chacun est éventuellement substitué par un ou des substituants halogéno, alkyle en C₁-C₆, alcoxy en C₁-C₆, halogènalkyle en C₁-C₄, halogènalcoxy en C₁-C₄, nitro ou cyano,
R^{14a} est un atome d'hydrogène ou un groupe alkyle en C₁-C₈, ou
R¹³ et R^{14a} forment ensemble un radical alcanediyle en C₄-C₆,
R^{15a} et R^{16a} sont identiques ou différents et représentent chacun un groupe alkyle en C₁-C₆, ou
R^{15a} et R^{16a} forment ensemble un radical alcanediyle en C₂-C₄, qui est éventuellement substitué par un ou des substituants alkyle en C₁-C₆, halogènalkyle en C₁-C₆, ou par un ou des groupes phényle éventuellement substitués par un ou des substituants halogéno, alkyle en C₁-C₆, halogènalkyle en C₁-C₄, alcoxy en C₁-C₆, halogènalcoxy en C₁-C₄, nitro ou cyano,
R^{17a} et R^{18a} représentent chacun indépendamment de l'autre un atome d'hydrogène, un groupe alkyle en C₁-C₈ éventuellement halogéné, ou un groupe phényle éventuellement substitué par un ou des substituants halogéno, alkyle en C₁-C₆, alcoxy en C₁-C₆, halogènalkyle en C₁-C₄, halogènalcoxy en C₁-C₄, nitro ou cyano, ou
R^{17a} et R^{18a}, ensemble avec l'atome de carbone auquel ils sont liés, forment un groupe carbonyle, ou un groupe cycloalkyle en C₅-C₇ éventuellement substitué par un ou des substituants halogéno, alkyle en C₁-C₄ ou alcoxy en C₁-C₄, et dans lequel éventuellement un groupe méthylène est remplacé par un atome d'oxygène ou de soufre,
R^{19a} et R^{20a} représentent chacun indépendamment de l'autre un groupe alkyle en C₁-C₁₀, alcényle en C₂-C₁₀, alcoxy en C₁-C₁₀, alkylamino en C₁-C₁₀, alcénylamino en C₃-C₁₀, di (alkyle en C₁-C₁₀) amino ou di (alcényle en C₃-C₁₀) amino.

3. Composés de formule (I) selon la revendication 1, dans laquelle
W représente un groupe alcoxy en C₁-C₄, halogènalcoxy en C₁-C₄, (alcoxy en C₁-C₃) - (alkyloxy en C₂-C₃) , (alcoxy en C₁-C₂) -bis (alkyloxy en C₂-C₃) ou (cycloalkyle en C₃-C₆) - (alcanediyloxy en C₁-C₂), dans lequel éventuellement un groupe méthylène du cycle peut être interrompu par un atome d'oxygène,
X est un groupe alkyle en C₁-C₃,
Y est le chlore ou le brome,
CKE représente l'un des groupes 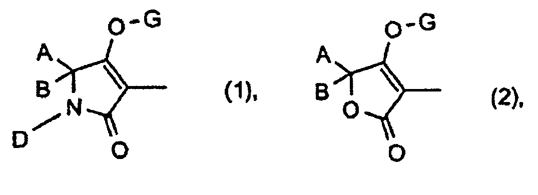 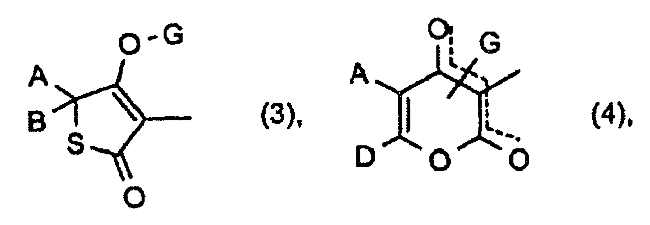 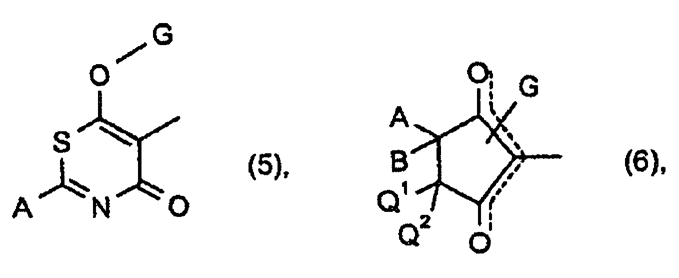 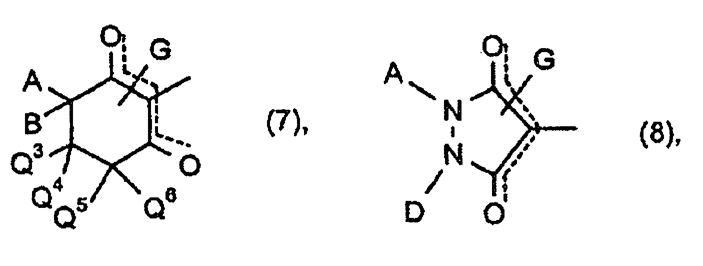 A est un atome d'hydrogène, un groupe alkyle en C₁-C₆, (alcoxy en C₁-C₄)-(alkyle en C₁-C₂) dont chacun est éventuellement substitué une à trois fois par des groupes fluoro ou chloro, cycloalkyle en C₃-C₆ éventuellement une à deux fois substitué par des substituants alkyle en C₁-C₂ ou alcoxy en C₁-C₂, ou (mais pas dans le cas des composés des formules (1-3), (I-4), (I-6) et (I-7)), phényle ou benzyle, dont chacun est une à deux fois éventuellement substitué par des substituants fluoro, chloro, bromo, alkyle en C₁-C₄, halogènalkyle en C₁-C₂, alcoxy en C₁-C₄, halogènalcoxy en C₁-C₂, cyano ou nitro,
B est un atome d'hydrogène, un groupe alkyle en C₁-C₄ ou (alcoxyle en C₁-C₂) - (alkyle en C₁-C₂) ou
A, B et l'atome de carbone auquel ils sont liés représentent un groupe cycloalkyle en C₅-C₇ saturé ou insaturé, dans lequel éventuellement un chaînon du cycle est remplacé par un atome d'oxygène ou de soufre, et qui est éventuellement substitué une à deux fois par des substituants alkyle en C₁-C₆, trifluorométhyle ou alcoxy en C₁-C₆, à la condition qu'alors Q³ soit un atome d'hydrogène ou le groupe méthyle, ou
A, B et l'atome de carbone auquel ils sont liés forment un groupe cycloalkyle en C₅-C₆, qui est substitué par un groupe alkylènediyle, ou par un groupe alkylènedioxyle, ou par un groupe alkylènedithiol, éventuellement substitué par un ou des substituants méthyle ou éthyle, contenant éventuellement un ou deux atomes d'oxygène ou de soufre qui ne sont pas immédiatement voisins, groupe qui, avec l'atome de carbone auquel il est lié, forme un cycle supplémentaire à 5 ou 6 chaînons, à la condition qu'alors Q³ soit un atome d'hydrogène ou le groupe méthyle,
A, B et l'atome de carbone auquel ils sont liés représentent un groupe cycloalkyle en C₃-C₆ ou cycloalcényle en C₅-C₆, dans lesquels deux substituants, ensemble avec l'atome de carbone auquel ils sont liés, représentent des radicaux alcanediyle en C₂-C₄, alcènediyle en C₂-C₄ ou butadiènediyle dont chacun est éventuellement substitué par un ou des substituants alkyle en C₁-C₂ ou alcoxy en C₁-C₂, à la condition qu'alors Q³ soit d'une manière particulièrement préférée un atome d'hydrogène ou le groupe méthyle,
D est un atome d'hydrogène, un groupe alkyle en C₁-C₆, alcényle en C₃-C₆, (alcoxy en C₁-C₄) - (alkyle en C₂-C₃) dont chacun est éventuellement une à trois fois substitué par des substituants fluoro, cycloalkyle en C₃-C₆ éventuellement substitué une à deux fois par des substituants alkyle en C₁-C₄, alcoxy en C₁-C₄ ou halogènalkyle en C₁-C₂, dans lequel éventuellement un groupe méthylène est remplacé par un atome d'oxygène, ou (mais pas dans le cas des composés de formules (I-1)), un groupe phényle ou pyridyle, dont chacun est éventuellement substitué une à deux fois par des substituants fluoro, chloro, bromo, alkyle en C₁-C₄, halogènalkyle en C₁-C₄, alcoxy en C₁-C₄ ou halogènalcoxy en C₁-C₄, ou
A et D forment ensemble un radical alcanediyle en C₃-C₅ éventuellement une à deux fois substitué, dans lequel un groupe méthylène peut être remplacé par un groupe carbonyle (mais pas dans le cas des composés de formule (I-1)), par un atome d'oxygène ou de soufre, où en tant que substituants entrent en ligne de compte les groupes alkyle en C₁-C₂ ou alcoxy en C₁-C₂, ou
A et D (dans le cas des composés de formule (I-1)), ensemble avec les atomes auxquels ils sont liés, représentent l'un des groupes AD-1 à AD-10 : 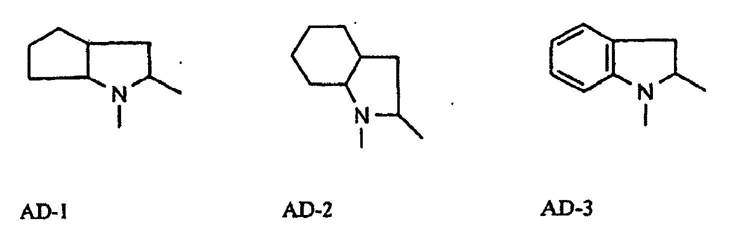 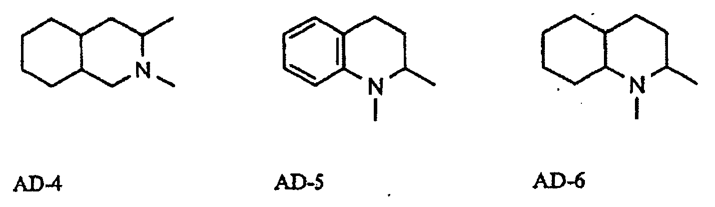 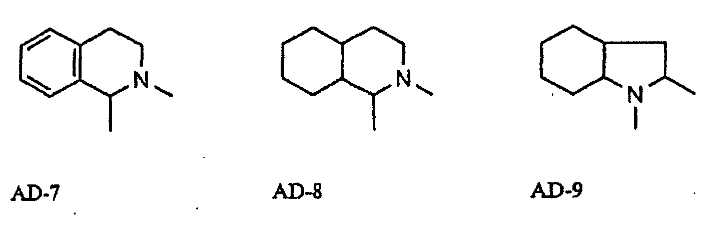 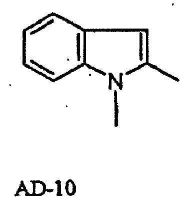 ou
A et Q¹ forment ensemble un radical alcanediyle en C₃-C₄, dont chacun est éventuellement une ou deux fois substitué, d'une manière identique ou différente, par des substituants alkyle en C₁-C₂ ou alcoxy en C₁-C₂, ou
Q¹ est un atome d'hydrogène,
Q² est un atome d'hydrogène,
Q⁴, Q⁵ et Q⁶ représentent chacun indépendamment des autres un atome d'hydrogène ou un groupe alkyle en C₁-C₃,
Q³ représente un atome d'hydrogène, un groupe alkyle en C₁-C₄, ou cycloalkyle en C₃-C₆ éventuellement une à deux fois substitué par des substituants méthyle ou méthoxy, ou
Q³ et Q⁴, ensemble avec l'atome de carbone auquel ils sont liés, forment un radical cyclique en C₅-C₆ saturé, éventuellement substitué par un ou des substituants alkyle en C₁-C₂ ou alcoxy en C₁-C₂, et dans lequel éventuellement un chaînon du cycle est remplacé par un atome d'oxygène ou de soufre, à la condition qu'alors A soit un atome d'hydrogène ou le groupe méthyle, ou
G est un atome d'hydrogène (a) ou l'un des groupes 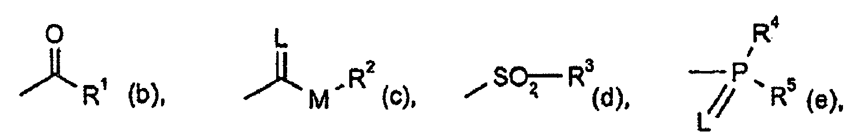 E (f) ou 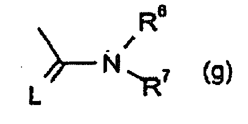 dans lesquels
E est un équivalent d'un ion métallique ou un ion ammonium,
L est un atome d'oxygène ou de soufre, et
M est un atome d'oxygène ou de soufre,
R¹ représente un groupe alkyle en C₁-C₈, alcényle en C₂-C₁₈, (alcoxy en C₁-C₄)-(alkyle en C₁-C₂), (alkylthio en C₁-C₄) - (alkyle en C₁-C₂) dont chacun est éventuellement une à trois fois substitué par des substituants fluoro ou chloro, ou cycloalkyle en C₃-C₆, éventuellement une à deux fois substitué par des substituants fluoro, chloro, alkyle en C₁-C₂ ou alcoxy en C₁-C₂, dans lequel éventuellement un ou deux chaînons du cycle qui ne sont pas immédiatement voisins sont remplacés par des atomes d'oxygène,
un groupe phényle éventuellement une à deux fois substitué par des substituants fluoro, chloro, bromo, cyano, nitro, alkyle en C₁-C₄, alcoxy en C₁-C₄, halogènalkyle en C₁-C₂ ou halogènalcoxy en C₁-C₂,
R² représente un groupe alkyle en C₁-C₈, alcényle en C₂-C₈ ou (alcoxy en C₁-C₄) - (alkyle en C₂-C₄) dont chacun est éventuellement une à trois fois substitué par des substituants fluoro,
cycloalkyle en C₃-C₆ éventuellement une fois substitué par un substituant alkyle en C₁-C₂ ou alcoxy en C₁-C₂, ou phényle ou benzyle, dont chacun est éventuellement une à deux fois substitué par des substituants fluoro, chloro, bromo, cyano, nitro, alkyle en C₁-C₄, alcoxy en C₁-C₃, trifluorométhyle ou trifluorométhoxy,
R³ représente un groupe alkyle en C₁-C₆ éventuellement une à trois fois substitué par des substituants fluoro, ou un groupe phényle, chacun étant éventuellement une fois substitué par un substituant fluoro, chloro, bromo, alkyle en C₁-C₄, alcoxy en C₁-C₄, trifluorométhyle, trifluorométhoxy, cyano ou nitro,
R⁴ est un groupe alkyle en C₁-C₆, alcoxy en C₁-C₆, alkylamino en C₁-C₆, di (alkyle en C₁-C₆) amino, alkylthio en C₁-C₆, alcénylthio en C₃-C₄, cycloalkylthio en C₃-C₆, ou phényle, phénoxy ou phénylthio dont chacun est éventuellement substitué une fois par un substituant fluoro, chloro, bromo, nitro, cyano, alcoxy en C₁-C₃, halogènalcoxy en C₁-C₃, alkylthio en C₁-C₃, halogènalkylthio en C₁-C₃, alkyle en C₁-C₃ ou trifluorométhyle,
R⁵ est un groupe alcoxy en C₁-C₆ ou alkylthio en C₁-C₆,
R⁶ est un atome d'hydrogène, un groupe alkyle en C₁-C₆, cycloalkyle en C₃-C₆, alcoxy en C₁-C₆, alcényle en C₃-C₆, (alcoxy en C₁-C₆) - (alkyle en C₁-C₄) , phényle éventuellement une fois substitué par un substituant fluoro, chloro, bromo, trifluorométhyle, alkyle en C₁-C₄ ou alcoxy en C₁-C₄, benzyle éventuellement une fois substitué par un substituant fluoro, chloro, bromo, alkyle en C₁-C₄, trifluorométhyle ou alcoxy en C₁-C₄,
R⁷ est un groupe alkyle en C₁-C₆, alcényle en C₃-C₆ ou (alcoxy en C₁-C₆) - (alkyle en C₁-C₄) ,
R⁶ et R⁷ forment ensemble un radical alkylène en C₄-C₅ éventuellement substitué par un ou des substituants méthyle ou éthyle, dans lequel éventuellement un groupe méthylène est remplacé par un atome d'oxygène ou de soufre.

4. Composés de formule (I) selon la revendication 1, dans laquelle
W est le groupe méthoxy, éthoxy, n-propoxy, iso-propoxy, n-butoxy, iso-butoxy, sec-butoxy, méthoxy-éthyloxy, éthoxy-éthyloxy, cyclopropyl-méthoxy, cyclopentyl-méthoxy ou cyclohexyl-méthoxy,
X est le groupe méthyle ou éthyle,
Y est le chlore ou le brome,
CKE est l'un des groupes 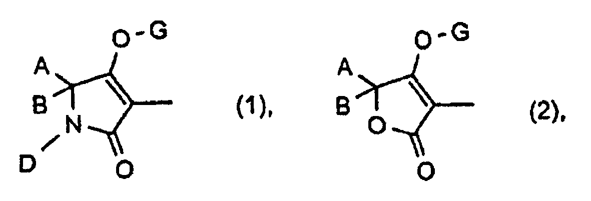 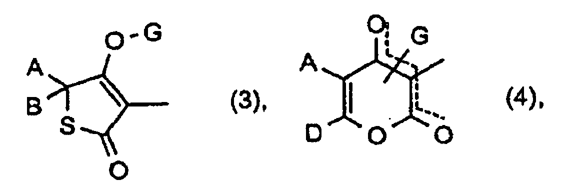 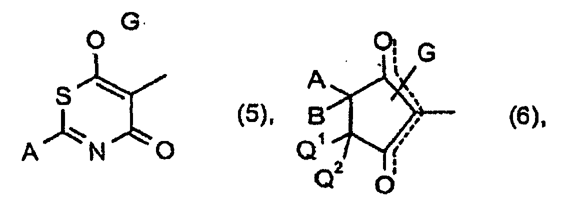 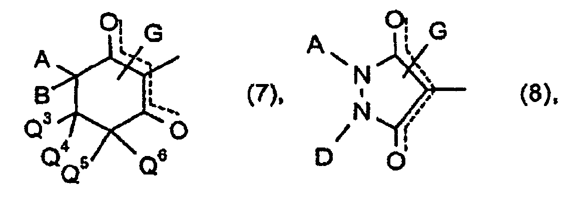 A est un atome d'hydrogène, un groupe alkyle en C₁-C₄ ou (alcoxy en C₁-C₂) - (alkyle en C₁-C₂) dont chacun est éventuellement une à trois fois substitué par des substituants fluoro ; cyclopropyle, cyclopentyle ou cyclohexyle ; et, seulement dans le cas des composés de formule (1-5), phényle, dont chacun est éventuellement substitué par un ou des substituants fluoro, chloro, bromo, méthyle, éthyle, n-propyle, isopropyle, méthoxy, éthoxy, trifluorométhyle, trifluorométhoxy, cyano ou nitro,
B est un atome d'hydrogène, le groupe méthyle ou éthyle, ou
A, B et l'atome de carbone auquel ils sont liés représentent un groupe cycloalkyle en C₅-C₆ saturé, dans lequel éventuellement un chaînon du cycle est remplacé par un atome d'oxygène ou de soufre, et qui éventuellement est une fois substitué par un substituant méthyle, éthyle, propyle, isopropyle, trifluorométhyle, méthoxy, éthoxy, propoxy ou butoxy, à la condition qu'alors Q³ soit un atome d'hydrogène, ou
A, B et l'atome de carbone auquel ils sont liés forment un groupe cycloalkyle en C₆, qui éventuellement est substitué par deux groupes alkylènedioxyle contenant des atomes d'oxygène qui ne sont pas immédiatement voisins, à la condition qu'alors Q³ soit un atome d'hydrogène, ou
A, B et l'atome de carbone auquel ils sont liés forment un groupe cycloalkyle en C₅-C₆ ou cycloalcényle en C₅-C₆, où deux substituants, avec les atomes de carbone auxquels ils sont liés, représentent des radicaux alcanediyle en C₂-C₄ ou alcènediyle en C₂-C₄ ou butadiènediyle, à la condition qu'alors Q³ soit un atome d'hydrogène,
D est un atome d'hydrogène, un groupe alkyle en C₁-C₄, alcényle en C₃-C₄, (alcoxy en C₁-C₄) - (alkyle en C₂-C₃) dont chacun est éventuellement une à trois fois substitué par des substituants fluoro ; cyclopropyle, cyclopentyle ou cyclohexyle, ou (mais pas dans le cas des composés de formules (I-1)) un groupe phényle ou pyridyle, éventuellement une fois substitué par un substituant fluoro, chloro, méthyle, éthyle, n-propyle, isopropyle, méthoxy, éthoxy ou trifluorométhyle, ou
A et D forment ensemble un radical alcanediyle en C₃-C₅ éventuellement une fois substitué par un substituant méthyle ou méthoxy, où éventuellement un atome de carbone est remplacé par un atome d'oxygène ou de soufre ; ou le groupe AD-1,
A et Q¹ forment ensemble un radical alcanediyle en C₃-C₄ éventuellement une ou deux fois substitué par des substituants méthyle ou méthoxy, ou
Q¹ est un atome d'hydrogène,
Q² est un atome d'hydrogène,
Q⁴, Q⁵ et Q⁶ représentent chacun indépendamment des autres un atome d'hydrogène ou le groupe méthyle,
Q³ est un atome d'hydrogène, le groupe méthyle, éthyle ou propyle, ou
Q³ et Q⁴, ensemble avec l'atome de carbone auquel ils sont liés, forment un radical cyclique en C₅-C₆ saturé, éventuellement une fois substitué par un substituant méthyle ou méthoxy, à la condition qu'alors A soit un atome d'hydrogène,
G est un atome d'hydrogène (a) ou l'un des groupes 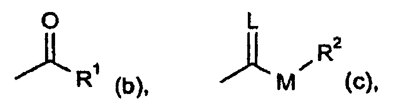 -SO₂-R³ (d) ou E (f),
dans lesquels
L est un atome d'oxygène ou de soufre,
M est un atome d'oxygène ou de soufre, et
E est un ion ammonium,
R¹ est un groupe alkyle en C₁-C₆, alcényle en C₂-C₁₇, (alcoxy en C₁-C₂) - (alkyle en C₁), (alkylthio en C₁-C₂)-(alkyle en C₁), ou cyclopropyle ou cyclohexyle dont chacun est éventuellement une fois substitué par un substituant fluoro, chloro, méthyle ou méthoxy,
phényle éventuellement une fois substitué par un substituant fluoro, chloro, bromo, cyano, nitro, méthyle, méthoxy, trifluorométhyle ou trifluorométhoxy,
R² est un groupe phényle ou benzyle dont chacun est éventuellement une fois substitué par un substituant alkyle en C₁-C₈, alcényle en C₂-C₆ ou (alcoxy en C₁-C₄)-(alkyle en C₂-C₃) fluoré,
R³ est un groupe alkyle en C₁-C₈.

5. Composés de formule (I) selon la revendication 1, dans laquelle
W est le groupe méthoxy, éthoxy, n-propoxy, iso-propoxy, n-butoxy, iso-butoxy, sec-butoxy, méthoxy-éthyloxy, éthoxy-éthyloxy ou cyclopropylméthoxy,
X est le groupe méthyle ou éthyle,
Y est le chlore,
CKE représente l'un des groupes 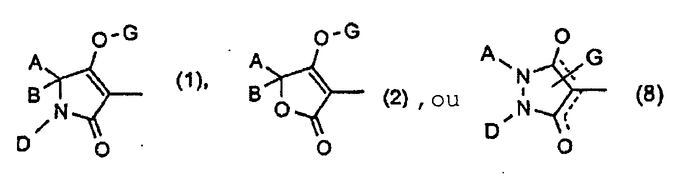 A représente un atome d'hydrogène, le groupe méthyle, éthyle, cyclopropyle, isopropyle, n-propyle, isobutyle, n-butyle, tert-butyle ou s-butyle,
B est un atome d'hydrogène, le groupe méthyle ou éthyle,
A, B et l'atome de carbone auquel ils sont liés représentent un groupe cycloalkyle en C₅-C₆ saturé, dans lequel éventuellement un chaînon du cycle est remplacé par un atome d'oxygène et qui est éventuellement une fois substitué par un substituant méthyle, méthoxy, éthoxy, n-propoxy, n-butoxy ou trifluorométhyle,
D est un atome d'hydrogène, le groupe méthyle, éthyle, isopropyle, cyclopropyle ou cyclohexyle,
ou
A et D forment ensemble un radical alcanediyle C₃-C₅ ou le groupe AD-1,
G est un atome d'hydrogène (a) ou l'un des groupes 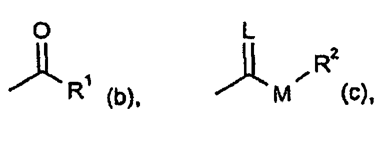 -SO₂-R³ (d) ou E (f)
dans lesquels
L est un atome d'oxygène,
M est un atome d'oxygène, et
E est un ion ammonium (N⁺ (C₆H₁₃) ₄),
R¹ est un groupe alkyle en C₁-C₈, (alcoxy en C₁-C₂) - (alkyle en C₁) ou alcényle en C₂-C₁₇,
R² est un groupe alkyle en C₁-C₈ ou alcényle en C₂-C₆,
R³ est un groupe alkyle en C₁-C₄.

6. Procédé de préparation de composés de formule (I) selon la revendication 1, **caractérisé en ce que**
(A) pour obtenir des composés de formule (I-1-a) 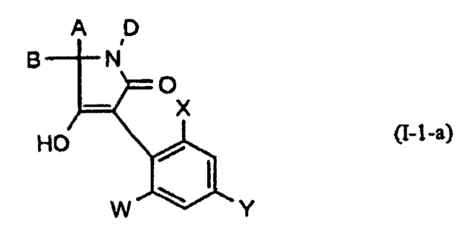 dans laquelle A, B, D, W, X et Y ont les significations données ci-dessus,
on soumet à une condensation intramoléculaire en présence d'un diluant et en présence d'une base des composés de formule (II) 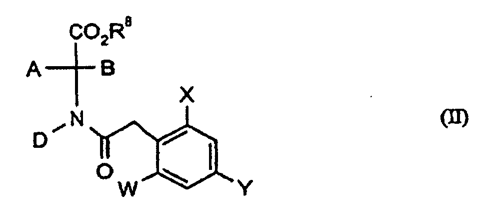 dans laquelle A, B, D, W, X et Y ont les significations données ci-dessus, et
R⁸ est un groupe alkyle,
(B) pour obtenir des composés de formule (I-2-a) 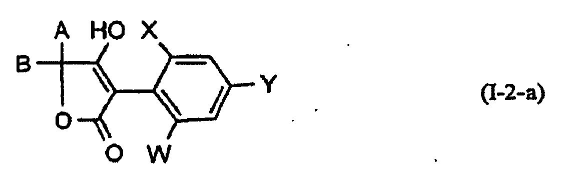 dans laquelle A, B, W, X et Y ont les significations données ci-dessus,
on soumet à une condensation intramoléculaire en présence d'un diluant et en présence d'une base des composés de formule (III) 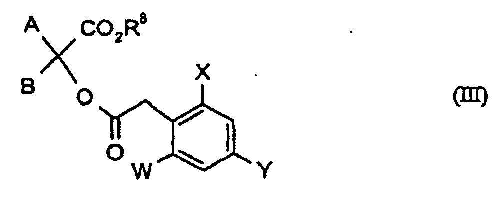 dans laquelle
A, B, W, X, Y et R⁸ ont les significations données ci-dessus,
(C) pour obtenir des composés de formule (I-3-a) 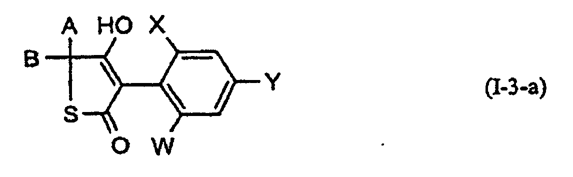 dans laquelle A, B, W, X et Y ont les significations données ci-dessus,
on soumet à une cyclisation intramoléculaire, éventuellement en présence d'un diluant et en présence d'un acide, des composés de formule (IV) 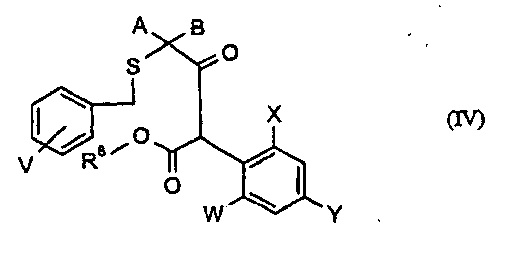 dans laquelle
A, B, W, X, Y et R⁸ ont les significations données ci-dessus, et
V représente un atome d'hydrogène, un groupe halogéno, alkyle ou alcoxy,
(D) pour obtenir des composés de formule (I-4-a) 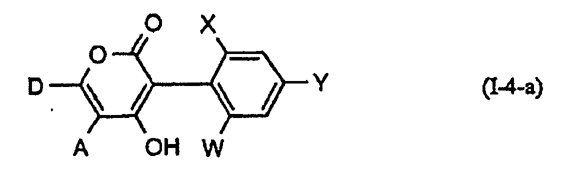 dans laquelle A, B, W, X et Y ont les significations données ci-dessus,
on fait réagir, éventuellement en présence d'un diluant et éventuellement en présence d'un accepteur d'acide, des composés de formule (V) 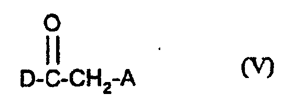 dans laquelle A et D ont les significations données ci-dessus,
ou leur silylénoléther de formule (Va) 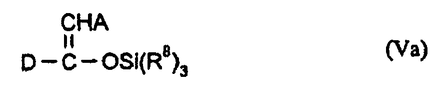 dans laquelle A, D et R⁸ ont les significations données ci-dessus,
avec des composés de formule (VI) 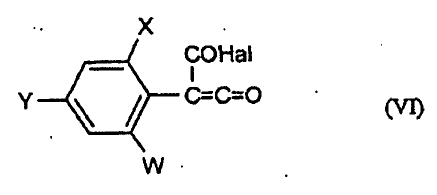 dans laquelle
W, X et Y ont les significations données ci-dessus, et Hal est un halogène,
(E) pour obtenir des composés de formule (I-5-a) 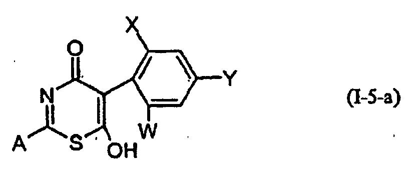 dans laquelle
A, W, X et Y ont les significations données ci-dessus, on fait réagir éventuellement en présence d'un diluant et éventuellement en présence d'un accepteur d'acide, des composés de formule (VII) 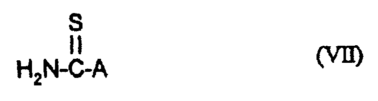 dans laquelle
A a les significations données ci-dessus,
avec des composés de formule (VI) 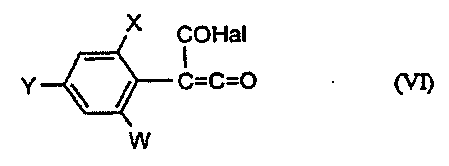 dans laquelle
Hal, W, X et Y ont les significations données ci-dessus,
(F) pour obtenir des composés de formule (I-6-a) 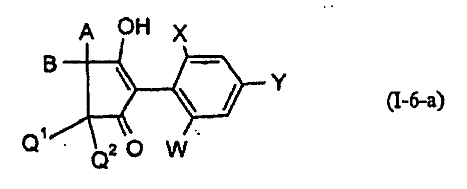 dans laquelle
A, B, Q¹, Q², W, X et Y ont les significations données ci-dessus,
on soumet à une cyclisation intramoléculaire, éventuellement en présence d'un diluant et en présence d'une base, des composés de formule (VIII) 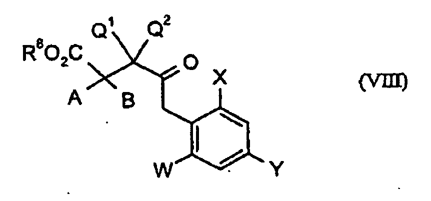 dans laquelle A, B, Q¹, Q², W, X et Y ont les significations données ci-dessus, et
R⁸ est un groupe alkyle,
(G) pour obtenir des composés de formule (I-7-a) 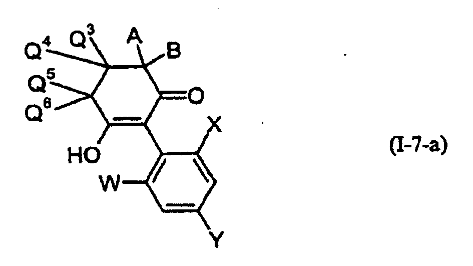 dans laquelle A, B, Q³, Q⁴, Q⁵, Q⁶, W, X et Y ont les significations données ci-dessus,
on soumet à une condensation intramoléculaire en présence d'un diluant et en présence d'une base des composés de formule (IX) 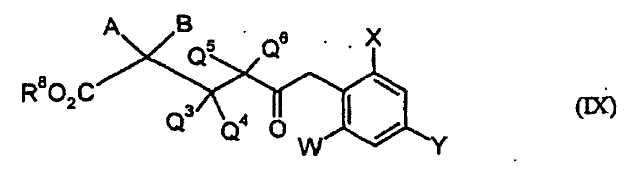 dans laquelle A, B, Q³, Q⁴, Q⁵, Q⁶, W, X et Y ont les significations données ci-dessus, et
R⁸ est un groupe alkyle,
(H) pour obtenir des composés de formule (I-8-a) 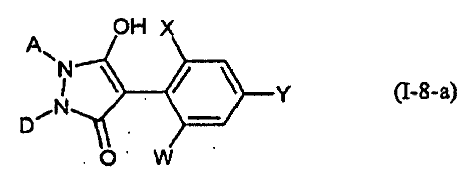 dans laquelle
A, D, W, X et Y ont les significations données ci-dessus,
on fait réagir des composés de formule (X) 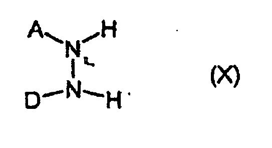 dans laquelle A et D ont les significations données ci-dessus,
α) éventuellement en présence d'un diluant et éventuellement en présence d'un accepteur d'acide, avec des composés de formule (VI) 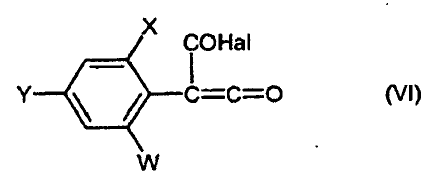 dans laquelle Hal, X, Y et W ont les significations données ci-dessus,
P) éventuellement en présence d'un diluant et éventuellement en présence d'une base, avec des composés de formule (XI) 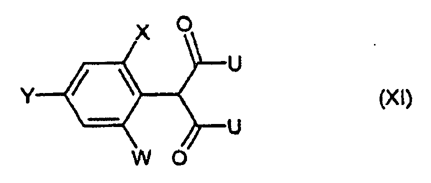 dans laquelle W, X et Y ont les significations données ci-dessus, et U est NH₂ ou O-R⁸, R⁸ ayant les significations données ci-dessus, ou
γ) éventuellement en présence d'un diluant et éventuellement en présence d'une base, avec des composés de formule (XII) 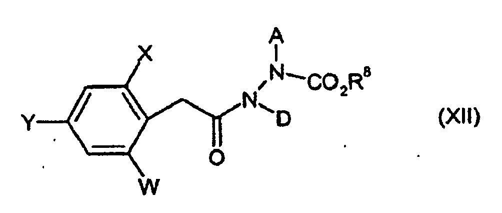 dans laquelle A, D, W, X, Y et R⁸ ont les significations données ci-dessus,
(I) pour obtenir des composés ayant les formules (I-1-b) à (I-8-b) présentées ci-dessus, dans lesquelles A, B, D, Q¹, Q², Q³, Q⁴, Q⁵, Q⁶, R¹, W, X et Y ont les significations données ci-dessus, on fait réagir éventuellement en présence d'un diluant et éventuellement en présence d'un agent liant les acides, chacun des composés ayant les formules (I-1-a) à (1-8-a) présentées ci-dessus, dans lesquelles A, B, D, Q¹, Q², Q³, Q⁹, Q⁵, Q⁶, W, X et Y ont les significations données ci-dessus,
(α) avec des halogénures d'acide de formule (XIII) 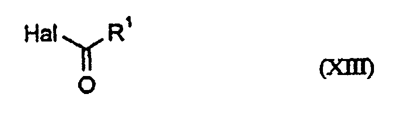 dans laquelle
R¹ a les significations données ci-dessus et
Hal est un halogène,
ou
(β) avec des anhydrides carboxyliques de formule (XIV)
R¹-CO-O-CO-R¹ (XIV)
dans laquelle
R¹ a les significations données ci-dessus ;
(J) pour obtenir des composés ayant les formules (I-1-c) à (I-8-c) présentées ci-dessus, dans lesquelles A, B, D, Q¹, Q², Q³, Q⁴, Q⁵, Q⁶, R², M, W, X et Y ont les significations données ci-dessus et L est un atome d'oxygène, on fait réagir éventuellement en présence d'un diluant et éventuellement en présence d'un agent liant les acides chacun des composés ayant les formules (I-1-a) à (I-8-a) présentées ci-dessus, dans lesquelles A, B, D, Q¹, Q², Q³, Q⁴, Q⁵, Q⁶, W, X et Y ont les significations données ci-dessus,
avec des esters de l'acide chloroformique ou des thioesters de l'acide chloroformique de formule (XV)
R²-M-CO-Cl (XV)
dans laquelle
R² et M ont les significations données ci-dessus,
(K) pour obtenir des composés ayant les formules (I-1-c) à (I-8-c) présentées ci-dessus, dans lesquelles A, B, D, Q¹, Q², Q³, Q⁹, Q⁵, Q⁶, R², M, W, X et Y ont les significations données ci-dessus et L est un atome de soufre, on fait réagir éventuellement en présence d'un diluant et éventuellement en présence d'un agent liant les acides chacun des composés ayant les formules (I-1-a) à (I-8-a) présentées ci-dessus, dans lesquelles A, B, D, Q¹, Q², Q³, Q⁴, Q⁵, Q⁶, W, X et Y ont les significations données ci-dessus,
avec des esters de l'acide chloromonothioformique ou des esters de l'acide chlorodithioformique de formule (XVI) 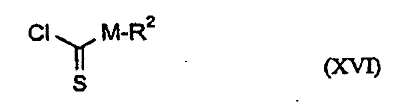 dans laquelle M et R² ont les significations données ci-dessus,
et
(L) pour obtenir des composés ayant les formules (I-1-d) à (I-8-d) présentées ci-dessus dans lesquelles A, B, D, Q¹, Q², Q³, Q⁴, Q⁵, Q⁶, R³, W, X et Y ont les significations données ci-dessus, on fait réagir éventuellement en présence d'un diluant et éventuellement en présence d'un agent liant les acides chacun des composés ayant les formules (I-1-a) à (1-8-a) présentées ci-dessus, dans lesquelles A, B, D, Q¹, Q², Q³, Q⁴, Q⁵, Q⁶, W, X et Y ont les significations données ci-dessus,
avec des chlorures de sulfonyle de formule (XVII)
R³-SO₂-Cl (XVII)
dans laquelle
R³ a les significations données ci-dessus,
(M) pour obtenir des composés ayant les formules (I-1-e) à (I-8-e) présentées ci-dessus, dans lesquelles A, B, D, L, Q¹, Q², Q³, Q⁴, Q⁵, Q⁶, R⁴, R⁵, W, X et Y ont les significations données ci-dessus, on fait réagir, éventuellement en présence d'un diluant et éventuellement en présence d'un agent liant les acides, chacun des composés ayant les formules (I-1-a) à (1-8-a) présentées ci-dessus, dans lesquelles A, B, D, Q¹, Q², Q³, Q⁴, Q⁵, Q⁶, W, X et Y ont les significations données ci-dessus,
avec des composés phosphorés de formule (XVIII) 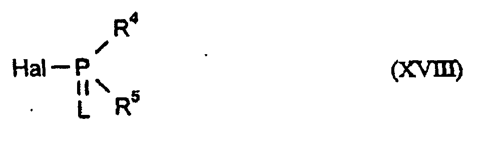 dans laquelle
L, R⁴ et R⁵ ont les significations données ci-dessus, et
Hal est un atome d'halogène,
(N) pour obtenir des composés ayant les formules (I-1-f) à (I-8-f) présentées ci-dessus, dans lesquelles A, B, D, E, Q¹, Q², Q³, Q⁴, Q⁵, Q⁶, W, X et Y ont les significations données ci-dessus, on fait réagir, éventuellement en présence d'un diluant, chacun des composés de formule (I-1-a) à (I-8-a), dans lesquelles A, B, D, Q¹, Q², Q³, Q⁴, Q⁵, Q⁶, W, X et Y ont les significations données ci-dessus,
avec des composés métalliques ou des amines de formules (XIX) ou (XX)
Me (OR¹⁰)ₜ (XIX) 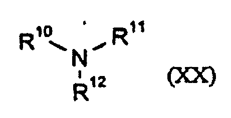 dans lesquelles
Me est un métal monovalent ou divalent,
t représente le nombre 1 ou 2, et
R¹⁰, R¹¹ et R¹² représentent chacun indépendamment des autres un atome d'hydrogène ou un groupe alkyle,
(O) pour obtenir des composés ayant les formules (I-1-g) à (I-8-g) présentées ci-dessus, dans lesquelles A, B, D, L, Q¹, Q², Q³, Q⁴, Q⁵, Q⁶, R⁶, R⁷, W, X et Y ont les significations données ci-dessus, on fait réagir chacun des composés ayant les formules (I-1-a) à (I-8-a) présentées ci-dessus, dans lesquelles A, B, D, Q¹, Q², Q³, Q⁴, Q⁵, Q⁶, W, X et Y ont les significations données ci-dessus,
(α) éventuellement en présence d'un diluant et éventuellement en présence d'un catalyseur, avec des isocyanates ou des isothiocyanates de formule (XXI)
R⁶-N=C=L (XXI)
dans laquelle
R⁶ et L ont les significations données ci-dessus, ou
(β) éventuellement en présence d'un diluant et éventuellement en présence d'un agent liant les acides, avec des chlorures de carbamoyle ou des chlorures de thiocarbamoyle de formule (XXII) 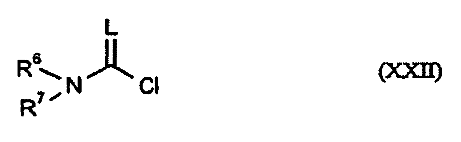 dans laquelle L, R⁶ et R⁷ ont les significations données ci-dessus.

7. Composés de formule (II) 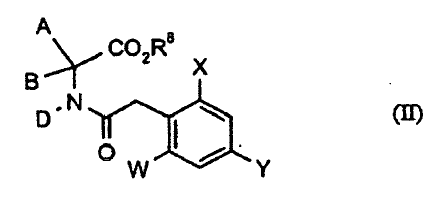 dans laquelle
A, B, D, W, X, Y et R⁸ ont les significations données ci-dessus.

8. Composés de formule (III) 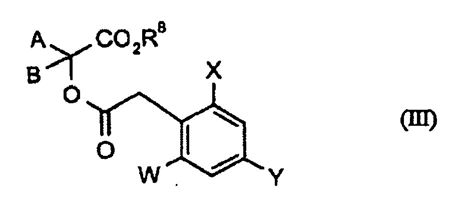 dans laquelle
A, B, W, X, Y et R⁸ ont les significations données ci-dessus.

9. Composés de formule (IV) 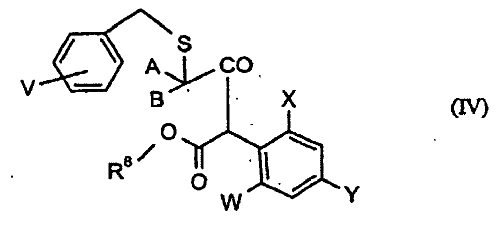 dans laquelle
A, B, V, W, X, Y et R⁸ ont les significations données ci-dessus.

10. Composés de formule (VI) 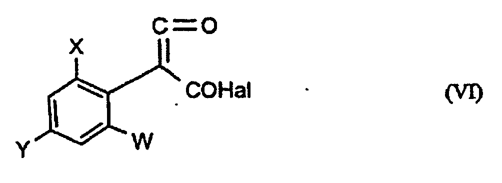 dans laquelle
Hal, W, X et Y ont les significations données ci-dessus.

11. Composés de formule (VIII) 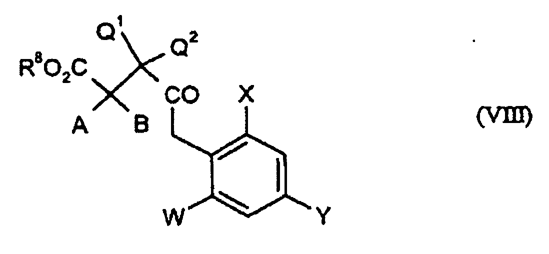 dans laquelle
A, B, Q¹, Q², W, X, Y et R⁸ ont les significations données ci-dessus.

12. Composés de formule (IX) 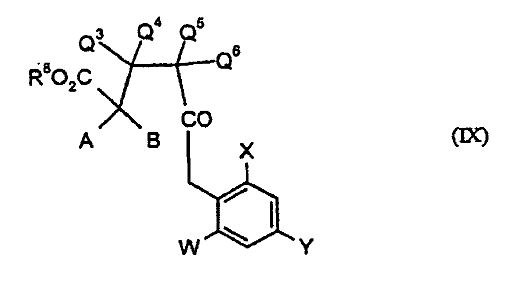 dans laquelle
A, B, Q³, Q⁴, Q⁵, Q⁶, W, X, Y et R⁸ ont les significations données ci-dessus.

13. Composés de formule (XI) 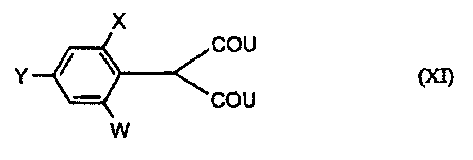 dans laquelle
U, W, X et Y ont les significations données ci-dessus.

14. Composés de formule (XII) 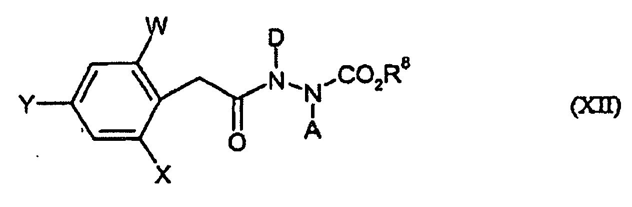 dans laquelle
A, D, W, X, Y et R⁸ ont les significations données ci-dessus.

15. Composés de formule (XXIV) 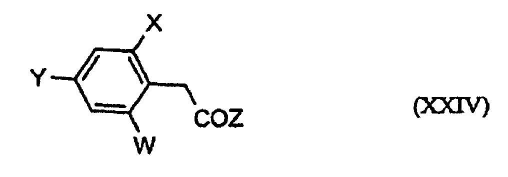 dans laquelle
W, X et Y ont les significations données ci-dessus, et Z est un groupe éliminable, introduit par des réactifs d'activation d'acides carboxyliques tels que le carbonyldiimidazole, les carbonyldiimides (tels que par exemple le dicyclohexylcarbodiimide), des réactifs de phosphorylation (tels que par exemple POCl₃, BOP-Cl), des agents d'halogénation, par exemple le chlorure de thionyle, le chlorure d'oxalyle, le phosgène ou les esters de l'acide chloroformique.

16. Composés de formule (XXV) 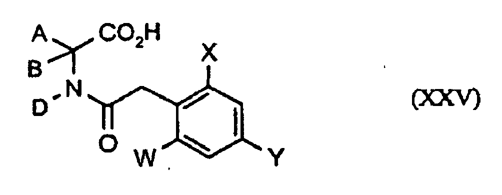 dans laquelle
A, B, D, W, X et Y ont les significations données ci-dessus.

17. Composés de formule (XXVII) 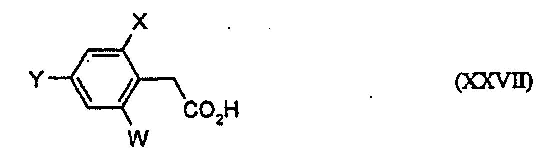 dans laquelle
W, X et Y ont les significations données ci-dessus.

18. Composés de formule (XXIX) 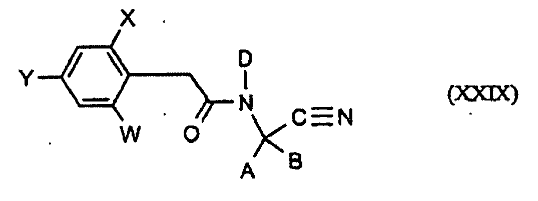 dans laquelle
A, B, D, W, X et Y ont les significations données ci-dessus.

19. Composés de formule (XXXI) 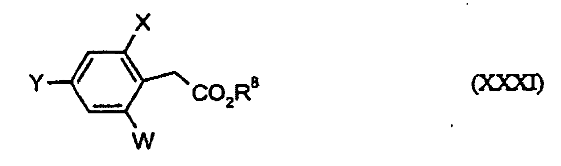 dans laquelle
W, X, Y et R⁸ ont les significations données ci-dessus.

20. Composés de formule (XXXIII) 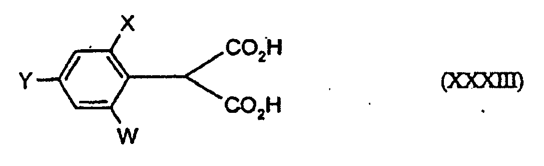 dans laquelle
W, X et Y ont les significations données ci-dessus.

21. Composés de formule (XXXIV) 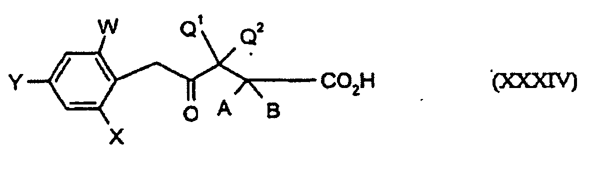 dans laquelle
A, B, Q¹, Q², W, X et Y ont les significations données ci-dessus.

22. Composés de formule (XXXV) 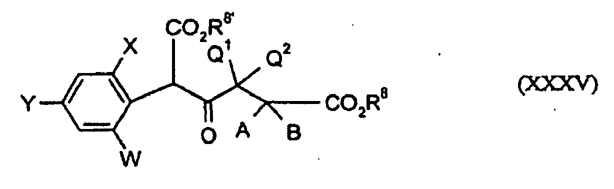 dans laquelle
A, B, Q¹, Q², R⁸, R^{8'}, W, X et Y ont les significations données ci-dessus.

23. Composés de formule (XXXVIII) 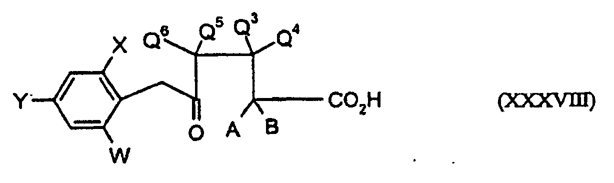 dans laquelle
A, B, Q³, Q⁴, Q⁵, Q⁶, W, X et Y ont les significations données ci-dessus.

24. Composés de formule (XXXIX) 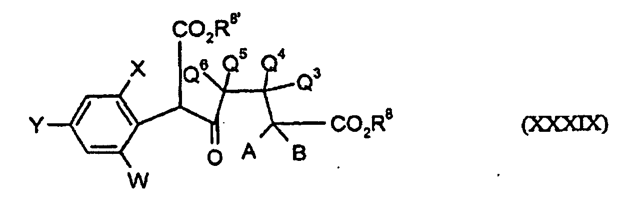 dans laquelle
A, B, Q³, Q⁹, Q⁵, Q⁶, R⁸, R^{8'}, W, X et Y ont les significations données ci-dessus.

25. Produit contenant une quantité efficace d'une combinaison de matières actives comprenant en tant que composants
(a') au moins un cétoénol cyclique substitué de formule (I) selon la revendication 1, dans laquelle CKE, W, X et Y ont les significations données ci-dessus, et
(b') au moins un composé améliorant la compatibilité de plantes de culture, appartenant au groupe suivant de composés:
4-dichloracétyl-1-oxa-4-aza-spiro[4.5]-décane (AD-67, MON-4660), 1-dichloracétyl-hexahydro-3,3,8a-triméthylpyrrolo[1,2-a]-pyrimidin-6(2H)-one (dicyclonon, BAS-145138), 4-dichloracétyl-3,4-dihydro-3-méthyl-2H-1,4-benzoxazine (benoxacor), ester 1-méthyl-hexylique de l'acide 5-chloro-quinoléin-8-oxyacétique (cloquintocet-mexyl- Cf. aussi des composés apparentés dans EP-A-86 750, EP-A-94 349, EP-A-19 17 36, EP-A-49 23 66), 3-(2-chlorobenzyl)-1-(1-méthyl-1-phényl-éthyl)-urée (cumyluron), α-(cyanométhoximino)-phénylacétronitrile (cyométrinil), acide 2,4-dichloro-phénoxyacétique (2,4-D), acide 4-(2,4-dichlorophénoxy)-butyrique (2,4-DB), 1-(1-méthyl-1-phényl-éthyl)-3-(4-méthyl-phényl)-urée (daimuron, dymron), acide 3,6-dichloro-2-méthoxy-benzoïque (dicamba), ester S-1-méthyl-1-phényl-éthylique de l'acide pipéridine-1-thiocarboxylique (dimépipérate), 2,2-dichloro-N-(2-oxo-2-(2-propénylamino)-éthyl)-N-(2-propényl)-acétamide (DKA-24), 2,2-dichloro-N,N-di-2-propényl-acétamide (dichlormide), 4,6-dichloro-2-phényl-pyrimidine (fenclorim), 1-(2,4-dichlorophényl)-5-trichlorométhyl-1H-1,2,4-triazole-3-carboxylate d'éthyle (fenchlorazole-éthyl - Cf. aussi des composés apparentés dans EP-A-17 45 62 et EP-A-34 66 20), 2-chloro-4-trifluorométhyl-thiazole-5-carboxylate de phénylméthyle (flurazole), 4-chloro-N-(1,3-dioxolanne-2-yl-méthoxy)-α-trifluoro-acétophénonoxime (fluxofénim), 3-dichloracétyl-5-(2-furannyl)-2,2-diméthyl-oxazolidine (flurilazole, MON-13900), 4,5-dihydro-5,5-diphényl-3-isoxazolecarboxylate d'éthyle (isoxadifène-éthyl - Cf. aussi des composés apparentés dans WO-A-95/07 897), 3,6-dichloro-2-méthoxybenzoate de 1-(éthoxycarbonyl)-éthyle (lactidichlor), acide (4-chloro-o-tolyloxy)-acétique (MCPA), acide 2-(4-chloro-o-tolyloxy)-propionique (mécoprop), 1-(2,4-dichloro-phényl)-4,5-dihydro-5-méthyl-1H-pyrazole-3,5-dicarboxylate de diéthyle (méfenpyr-diéthyl - Cf. aussi des composés apparentés dans WO-A-91/07874), 2-dichlorométhyl-2-méthyl-1,3-dioxolanne (MG-191), 1-oxa-4-azaspiro[4.5]décane-4-carbodithioate de 2-propényle (MG-838), anhydride 1,8-naphtalique, α-(1,3-dioxolann-2-yl-méthoximino)-phénylacétonitrile (oxabétrinil), 2,2-dichloro-N-(1,3-dioxolann-2-yl-méthyl)-N-(2-propényl)-acétamide (PPG-1292), 3-dichloracétyl-2,2-diméthyl-oxazolidine (R-28725), 3-dichloracétyl-2,2,5-triméthyl-oxazolidine (R-29148), acide 4-(4-chloro-o-tolyl)-butyrique, acide 4-(4-chloro-phénoxy)-butyrique, acide diphénylméthoxyacétique, diphénylméthoxyacétate de méthyle, diphénylméthoxyacétate d'éthyle, 1-(2-chloro-phényl)-5-phényl-1H-pyrazole-3-carboxylate de méthyle, 1-(2,4-dichloro-phényl)-5-méthyl-1H-pyrazole-3-carboxylate d'éthyle, 1-(2,4-dichloro-phényl)-5-isopropyl-1H-pyrazole-3-carboxylate d'éthyle, 1-(2,4-dichloro-phényl)-5-(1,1-diméthyl-éthyl)-1H-pyrazole-3-carboxylate d'éthyle, 1-(2,4-dichloro-phényl)-5-phényl-1H-pyrazole-3-carboxylate d'éthyle (Cf. aussi des composés apparentés dans EP-A-26 98 06 et EP-A-333 131), 5-(2,4-dichloro-benzyl)-2-isoxazoline-3-carboxylate d'éthyle, 5-phényl-2-isoxazoline-3-carboxylate d'éthyle, 5-(4-fluoro-phényl)-5-phényl-2-isoxazoline-3-carboxylate d'éthyle (Cf. aussi des composés apparentés dans WO-A-91/08 202), ester 1,3-diméthyl-but-1-ylique de l'acide 5-chloro-quinoléin-8-oxy-acétique, ester 4-allyloxy-butylique de l'acide 5-chloro-quinoléin-8-oxy-acétique, ester 1-allyloxy-prop-2-ylique de l'acide 5-chloro-quinoléin-8-oxy-acétique, 5-chloro-quinoxalin-8-oxy-acétate de méthyle, 5-chloro-quinoléin-8-oxy-acétate d'éthyle, 5-chloro-quinoxalin-8-oxy-acétate d'allyle, ester 2-oxo-prop-1-ylique de l'acide 5-chloro-quinoléin-8-oxy-acétique, 5-chloro-quinoléin-8-oxy-malonate de diéthyle, 5-chloro-quinoxalin-8-oxy-malonate de diallyle, 5-chloro-quinoléin-8-oxy-malonate de diéthyle (Cf. aussi des composés apparentés dans EP-A-58 21 98), acide 4-carboxy-chromann-4-yl-acétique (AC-30 44 15, Cf. EP-A-61 36 18), acide 4-chloro-phénoxy-acétique, 3,3'-diméthyl-4-méthoxy-benzophénone, 1-bromo-4-chlorométhylsulfonyl-benzène, 1-[4-(N-2-méthoxybenzoylsulfamoyl)-phényl]-3-méthyl-urée (connu aussi sous le nom de N-(2-méthoxy-benzoyl)-4-[(méthylamino-carbonyl)-amino]-benzènesulfonamide), 1-[4-(N-2-méthoxy-benzoylsulfamoyl)-phényl]-3,3-diméthyl-urée, 1-[4-(N-4,5-diméthylbenzoylsulfamoyl)-phényl]-3-méthyl-urée, 1-[4-(N-naphtylsulfamoyl)-phényl]-3,3-diméthyl-urée, N-(2-méthoxy-5-méthyl-benzoyl)-4-(cyclopropylaminocarbonyl)-benzènesulfonamide,
et/ou l'un des composés suivants, définis par des formules générales, de formule générale (IIa) 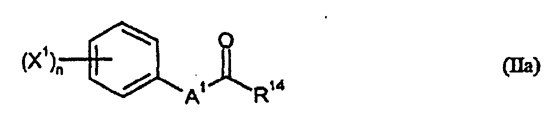 ou de formule générale (IIb) 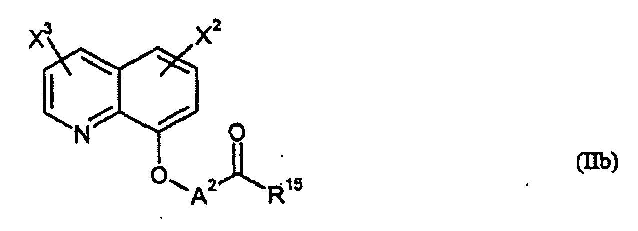 ou de formule (IIc) 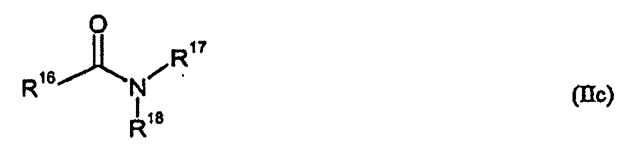 dans lesquelles
n est un nombre compris entre 0 et 5,
A¹ représente l'un des groupements hétérocycliques divalents représentés ci-après: 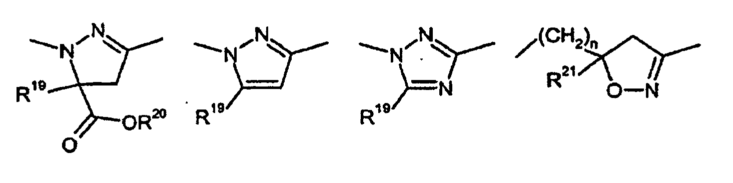 n est un nombre compris entre 0 et 5,
A² représente un radical alcanediyle ayant 1 ou 2 atomes de carbone, éventuellement substitué par un ou des substituants alkyle en C₁-C₄ et/ou (alcoxy en C₁-C₄)-carbonyle,
R¹⁴ représente un groupe hydroxy, mercapto, amino, alcoxy en C₁-C₆, alkylthio en C₁-C₆, alkylamino en C₁-C₆ ou di (alkyle en C₁-C₄)-amino,
R¹⁵ représente un groupe hydroxy, mercapto, amino, alcoxy en C₁-C₆, alkylthio en C₁-C₆, alkylamino en C₁-C₆ ou di (alkyle en C₁-C₄)-amino,
R¹⁶ représente un groupe alkyle en C₁-C₄, dont chacun est éventuellement substitué par un ou des substituants fluoro, chloro et/ou bromo,
R¹⁷ représente un atome d'hydrogène, un groupe alkyle en C₁-C₆, alcényle en C₂-C₆ ou alcynyle en C₂-C₆, (alcoxy en C₁-C₄)-(alkyle en C₁-C₄), dioxolannyl-(alkyle en C₁-C₄), furyle, furyl-(alkyle en C₁-C₄), thiényle, thiazolyle, pipéridyle, dont chacun est éventuellement substitué par un ou des substituants fluoro, chloro et/ou bromo, ou phényle, éventuellement substitué par un ou des substituants fluoro, chloro et/ou bromo, ou alkyle en C₁-C₄,
R¹⁸ représente un atome d'hydrogène, un groupe alkyle en C₁-C₆, alcényle en C₂-C₆ ou alcynyle en C₂-C₆, (alcoxy en C₁-C₄)-(alkyle en C₁-C₄), dioxolannyl-(alkyle en C₁-C₄), furyle, furyl-(alkyle en C₁-C₄), thiényle, thiazolyle, pipéridinyle, dont chacun est éventuellement substitué par un ou des substituants fluoro, chloro et/ou bromo, ou phényle éventuellement substitué par un ou des substituants fluoro, chloro et/ou bromo ou alkyle en C₁-C₄, ou, ensemble avec R¹⁷, représentent un radical alcanediyle en C₃-C₆ ou oxaalcanediyle en C₂-C₅ dont chacun est éventuellement substitué par un ou des substituants alkyle en C₁-C₄, phényle, furyle, par un noyau benzénique condensé, ou par deux substituants qui, ensemble avec l'atome de carbone auquel ils sont liés, forment un radical carbocyclique à 5 ou 6 chaînons,
R¹⁹ représente un atome d'hydrogène, un groupe cyano, halogéno, ou alkyle en C₁-C₄, cycloalkyle en C₃-C₆ ou phényle, dont chacun est éventuellement substitué par un ou des substituants fluoro, chloro et/ou bromo,
R²⁰ représente un atome d'hydrogène, un groupe alkyle en C₁-C₆, cycloalkyle en C₃-C₆ ou tri(alkyle en C₁-C₄)-silyle, éventuellement substitué par un ou des substituants hydroxy, cyano, halogéno ou alcoxy en C₁-C₄,
R²¹ représente un atome d'hydrogène, un groupe cyano, halogéno, ou alkyle en C₁-C₄, cycloalkyle en C₃-C₆ ou phényle, dont chacun est éventuellement substitué par un ou des substituants fluoro, chloro et/ou bromo,
X¹ représente un groupe nitro, cyano, halogéno, alkyle en C₁-C₄, halogènalkyle en C₁-C₄, alcoxy en C₁-C₄ ou halogènalcoxy en C₁-C₄,
X² représente un atome d'hydrogène, un groupe cyano, nitro, halogéno, alkyle en C₁-C₄, halogènalkyle en C₁-C₄, alcoxy en C₁-C₄ ou halogènalcoxy en C₁-C₄,
X³ représente un atome d'hydrogène, un groupe cyano, nitro, halogéno, alkyle en C₁-C₄, halogènalkyle en C₁-C₄, alcoxy en C₁-C₄ ou halogènalcoxy en C₁-C₄,
et/ou les composés suivants, définis par des formules générales
de formule générale (IId) 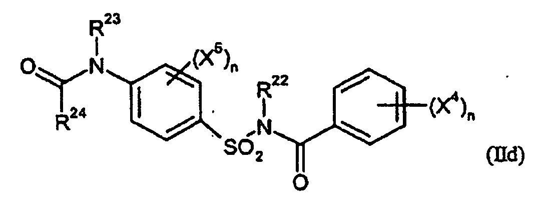 ou de formule générale (IIe) 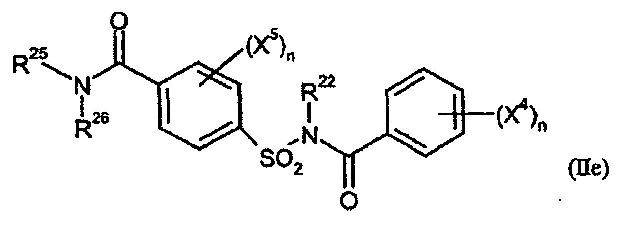 où
n est un nombre compris entre 0 et 5
R²² représente un atome d'hydrogène ou un groupe alkyle en C₁-C₄,
R²³ représente un atome d'hydrogène ou un groupe alkyle en C₁-C₄,
R²⁴ représente un atome d'hydrogène, un groupe alkyle en C₁-C₆, alcoxy en C₁-C₆, alkylthio en C₁-C₆, alkylamino en C₁-C₆ ou di(alkyle en C₁-C₄), dont chacun est éventuellement substitué par un ou des substituants cyano, halogéno ou alcoxy en C₁-C₄; ou cycloalkyle en C₃-C₆, cycloalkyloxy en C₃-C₆, cycloalkylthio en C₃-C₆ ou cycloalkylamino en C₃-C₆, dont chacun est éventuellement substitué par un ou des substituants cyano, halogéno ou alkyle en C₁-C₄,
R²⁵ représente un atome d'hydrogène, un groupe alkyle en C₁-C₆ éventuellement substitué par un ou des substituants cyano, hydroxy, halogéno ou alcoxy en C₁-C₄, alcényle en C₃-C₆ ou alcynyle en C₃-C₆ dont chacun est éventuellement substitué par un ou des substituants cyano ou halogéno, ou cycloalkyle en C₃-C₆ éventuellement substitué par un ou des substituants cyano, halogéno ou alkyle en C₁-C₄,
R²⁶ représente un atome d'hydrogène, un groupe alkyle en C₁-C₆ éventuellement substitué par un ou des substituants cyano, hydroxy, halogéno ou alcoxy en C₁-C₄, alcényle en C₃-C₆ ou alcynyle en C₃-C₆ dont chacun est éventuellement substitué par un ou des substituants cyano ou halogéno, cycloalkyle en C₃-C₆ éventuellement substitué par un ou des substituants cyano, halogéno ou alkyle en C₁-C₄, ou phényle éventuellement substitué par un ou des substituants nitro, cyano, halogéno, alkyle en C₁-C₄, halogènalkyle en C₁-C₄, alcoxy en C₁-C₄ ou halogènalcoxy en C₁-C₄, ou, ensemble avec R²⁵, forme un radical alcanediyle en C₂-C₆ ou oxaalcanediyle en C₂-C₅, dont chacun est éventuellement substitué par un ou des substituants alkyle en C₁-C₄,
X⁹ représente un groupe nitro, cyano, carboxy, carbamoyle, formyle, sulfamoyle, hydroxy, amino, halogéno, alkyle en C₁-C₄, halogènalkyle en C₁-C₄, alcoxy en C₁-C₄ ou halogènalcoxy en C₁-C₄, et
X⁵ représente un groupe nitro, cyano, carboxy, carbamoyle, formyle, sulfamoyle, hydroxy, amino, halogéno, alkyle en C₁-C₄, halogènalkyle en C₁-C₄, alcoxy en C₁-C₄ ou halogènalcoxy en C₁-C₄.

26. Produit selon la revendication 25, dans lequel le composé améliorant la compatibilité avec les plantes de culture est choisi dans le groupe suivant de composés : cloquintocet-mexyl, fenchlorazole-éthyl, isoxadifène-éthyl, méfenpyr-diéthyl, furilazole, fenclorim, cumyluron, dymron, ou les composés 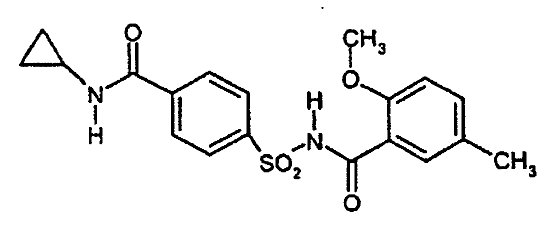 et

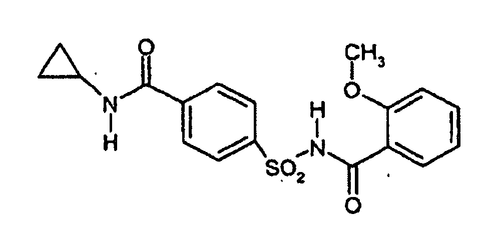

27. Produits selon l'une des revendications 25 ou 26, dans lesquelles le composé améliorant la compatibilité avec les plantes de culture est le cloquintocet-mexyl ou le méfenpyr-diéthyl.

28. Produit pesticide et/ou herbicide, **caractérisé en ce qu'**il contient au moins un composé de formule (I) selon la revendication 1.

29. Procédé pour la maîtrise de ravageurs animaux et/ou d'une végétation indésirable, **caractérisé en ce qu'**on fait agir des composés de formule (I) selon la revendication 1 sur les ravageurs et/ou leur habitat.

30. Utilisation de composés de formule (I) selon la revendication 1 pour la maîtrise de ravageurs animaux et/ou d'une végétation indésirable.

31. Procédé de fabrication de pesticides et/ou d'herbicides, **caractérisé en ce qu'**on mélange des composés de formule (I) selon la revendication 1 avec des diluants et/ou des substances tensioactives.

32. Utilisation de composés de formule (I) selon la revendication 1 pour fabriquer des produits pesticides et/ou des herbicides.

33. Procédé de maîtrise d'une végétation indésirable, **caractérisé en ce qu'**on laisse agir un produit selon la revendication 25 sur les plantes ou leur environnement.

34. Utilisation d'un produit selon la revendication 25 pour maîtriser une végétation indésirable.

35. Procédé pour la maîtrise d'une végétation indésirable, **caractérisé en ce qu'**on laisse agir un composé de formule (I) selon la revendication 25, et le composé améliorant la compatibilité avec les plantes de culture selon la revendication 1, séparément selon une séquence temporelle serrée, sur les plantes ou leur environnement.
